# EUROPEAN PATENT SPECIFICATION

(11) **EP 1 349 870 B1**
(45) Date of publication and mention of the grant of the patent: **08.10.2008**
(21) Application number: 01954748.8
(22) Date of filing: 17.07.2001
(51) Int. Cl.: C07K 14/47, C12N 15/12, C12N 15/85, C12N 5/10, C07K 16/30, G01N 33/574, C12Q 1/68, C12N 15/62, C12N 5/06, A61K 39/00, A61K 39/395

(54) **COMPOSITIONS FOR THE THERAPY AND DIAGNOSIS OF OVARIAN CANCER**
ZUSAMMENSETZUNGEN ZUR BEHANDLUNG UND DIAGNOSE VON EIERSTOCKKREBS
COMPOSITIONS UTILISÉS DANS LA THÉRAPIE ET LE DIAGNOSTIC DU CANCER DES OVAIRES

(30) Priority: 17.07.2000 US 617747; 10.08.2000 US 636801; 20.09.2000 US 667857; 04.04.2001 US 827271; 18.06.2001 US 884441
(43) Date of publication of application: 08.10.2003
(73) Proprietor: CORIXA CORPORATION, Wilmington, DE 19808 (US)
(72) Inventor: MITCHAM, Jennifer, L., Redmond, WA 98052 (US); KING, Gordon, E., Shoreline, WA 98177 (US); ALGATE, Paul, A., Issaquah, WA 98027 (US); FLING, Steven, P., Bainbridge Island, WA 98110 (US); RETTER, Marc, W., Carnation, WA 98014 (US); FANGER, Gary, Richard, Mill Creek, WA 98012 (US); REED, Steven, G., Bellevue, WA 98005 (US); VEDVICK, Thomas, S., Federal Way, WA 98003 (US); CARTER, Darrick, Seattle, WA 98102 (US); HILL, Paul, Everett, WA 98201 (US); ALBONE, Earl, Plymouth Meeting, PA 19462 (US)
(74) Representative: Walker, Ross Thomson
(86) International application number: PCT/US2001/022635
(87) International publication number: WO 2002/006317

(56) References cited:
- WO-A-00/12758
- WO-A-00/36107
- WO-A-01/94641
- WO-A-02/02624
- WO-A-99/63088
- FRANKEL A E ET AL: "Targeted toxins" CLINICAL CANCER RESEARCH, THE AMERICAN ASSOCIATION FOR CANCER RESEARCH, US, vol. 6, no. 2, February 2000 (2000-02), pages 326-334, XP002223271 ISSN: 1078-0432

### SEQUENCE LISTING

<110> Corixa Corporation
   Mitcham, Jennifer L.
   King, Gordon E.
   Algate, Paul A.
   Fling, Steven P.
   Retter, Marc W.
   Fanger, Gary Richard
   Reed, Steven G.
   Vedvick, Thomas S.
   Carter, Darrick
   Hill, Paul
   Albone, Earl
<120> COMPOSITIONS AND METHODS FOR THE THERAPY AND DIAGNOSIS OF OVARIAN CANCER
<130> 210121.46201PC
<140> PCT
   <141> 2001-07-17
<160> 596
<170> FastSEQ for Windows Version 4.0
<210> 1
   <211> 461
   <212> DNA
   <213> Homo sapiens
<400> 1
   ttagagaggc acagaaggaa gaagagttaa aagcagcaaa gccgggtttt tttgttttgt 60
   tttgttttgt tttgttttga gatggagtct cactctgttg cccaagctgg agtacaacgg 120
   catgatctca gctcgctgca acctccgcct cccacgttca agtgattctc ctgcctcagc 180
   ctcccaagta gctgggatta caggcgcccg ccaccacgct cagctaattt tttttgtatt 240
   tttagtagag acagggtttc accaggttgg ccaggctgct cttgaactcc tgacctcagg 300
   tgatccaccc gcctcggcct cccaaagtgc tgggattaca ggcgtgagcc accacgcccg 360
   gcccccaaag ctgtttcttt tgtctttagc gtaaagctct cctgccatgc agtatctaca 420
   taactgacgt gactgccagc aagctcagtc actccgtggt c                     461
<210> 2
   <211> 540
   <212> DNA
   <213> Homo sapiens
<400> 2
   taggatgtgt tggaccctct gtgtcaaaaa aaacctcaca aagaatcccc tgctcattac 60
   agaagaagat gcatttaaaa tatgggttat tttcaacttt ttatctgagg acaagtatcc 120
   attaattatt gtgtcagaag agattgaata cctgcttaag aagcttacag aagctatggg 180
   aggaggttgg cagcaagaac aatttgaaca ttataaaatc aactttgatg acagtaaaaa 240
   tggcctttct gcatgggaac ttattgagct tattggaaat ggacagttta gcaaaggcat 300
   ggaccggcag actgtgtcta tggcaattaa tgaagtcttt aatgaactta tattagatgt 360
   gttaaagcag ggttacatga tgaaaaaggg ccacagacgg aaaaactgga ctgaaagatg 420
   gtttgtacta aaacccaaca taatttctta ctatgtgagt gaggatctga aggataagaa 480
   aggagacatt ctcttggatg aaaattgctg tgtagagtcc ttgcctgaca aagatggaaa 540
<210> 3
   <211> 461
   <212> DNA
   <213> Homo sapiens
<400> 3
   ttagagaggc acagaaggaa gaagagttaa aagcagcaaa gccgggtttt tttgttttgt 60
   tttgttttgt tttgttttga gatggagtct cactctgttg cccaagctgg agtacaacgg 120
   catgatctca gctcgctgca acctccgcct cccacgttca agtgattctc ctgcctcagc 180
   ctcccaagta gctgggatta caggcgcccg ccaccacgct cagctaattt tttttgtatt 240
   tttagtagag acagggtttc accaggttgg ccaggctgct cttgaactcc tgacctcagg 300
   tgatccaccc gcctcggcct cccaaagtgc tgggattaca ggcgtgagcc accacgcccg 360
   gcccccaaag ctgtttcttt tgtctttagc gtaaagctct cctgccatgc agtatctaca 420
   taactgacgt gactgccagc aagctcagtc actccgtggt c 461
<210> 4
   <211> 531
   <212> DNA
   <213> Homo sapiens
<220>
   <221> misc_feature
   <222> 454, 492, 526
   <223> n = A,T,C or G
<400> 4
   tctttttctt tcgatttcct tcaatttgtc acgtttgatt ttatgaagtt gttcaagggc 60
   taactgctgt gtattatagc tttctctgag ttccttcagc tgattgttaa atgaatccat 120
   ttctgagagc ttagatgcag tttctttttc aagagcatct aattgttctt taagtctttg 180
   gcataattct tccttttctg atgacttttt atgaagtaaa ctgatccctg aatcaggtgt 240
   gttactgagc tgcatgtttt taattctttc gtttaatagc tgcttctcag ggaccagata 300
   gataagctta ttttgatatt ccttaagctc ttgttgaagt tgtttgattt ccataatttc 360
   caggtcacac tgtttatcca aaacttctag ctcagtcttt tgtgtttgct ttctgatttg 420
   gacatcttgt agtctgcctg agatctgctg atgntttcca ttcactgctt ccagttccag 480
   gtggagactt tnctttctgg agctcagcct gacaatgcct tcttgntccc t 531
<210> 5
   <211> 531
   <212> DNA
   <213> Homo sapiens
<400> 5
   agccagatgg ctgagagctg caagaagaag tcaggatcat gatggctcag tttcccacag 60
   cgatgaatgg agggccaaat atgtgggcta ttacatctga agaacgtact aagcatgata 120
   aacagtttga taacctcaaa ccttcaggag gttacataac aggtgatcaa gcccgtactt 180
   ttttcctaca gtcaggtctg ccggccccgg ttttagctga aatatgggcc ttatcagatc 240
   tgaacaagga tgggaagatg gaccagcaag agttctctat agctatgaaa ctcatcaagt 300
   taaagttgca gggccaacag ctgcctgtag tcctccctcc tatcatgaaa caacccccta 360
   tgttctctcc actaatctct gctcgttttg ggatgggaag catgcccaat ctgtccattc 420
   atcagccatt gcctccagtt gcacctatag caacaccctt gtcttctgct acttcaggga 480
   ccagtattcc tcccctaatg atgcctgctc ccctagtgcc ttctgttagt a 531

<210> 6
   <211> 531
   <212> DNA
   <213> Homo sapiens
<400> 6
   aatagattta atgcagagtg tcaacttcaa ttgattgata gtggctgcct agagtgctgt 60
   gttgagtagg tttctgagga tgcaccctgg cttgaagaga aagactggca ggattaacaa 120
   tatctaaaat ctcacttgta ggagaaacca caggcaccag agctgccact ggtgctggca 180
   ccagctccac caaggccagc gaagagccca aatgtgagag tggcggtcag gctggcacca 240
   gcactgaagc caccactggt gctggcactg gcactggcac tgttattggt actggtactg 300
   gcaccagtgc tggcactgcc actctcttgg gctttggctt tagcttctgc tcccgcctgg 360
   atccgggctt tggcccaggg tccgatatca gcttcgtccc agttgcaggg cccggcagca 420
   ttctccgagc cgagcccaat gcccattcga gctctaatct cggccctagc cttggcttca 480
   gctgcagcct cagctgcagc cttcaaatcc gcttccatcg cctctcggta c 531
<210> 7
   <211> 531
   <212> DNA
   <213> Homo sapiens
<400> 7
   gccaagaaag cccgaaaggt gaagcatctg gatggggaag aggatggcag cagtgatcag 60
   agtcaggctt ctggaaccac aggtggccga agggtctcaa aggccctaat ggcctcaatg 120
   gcccgcaggg cttcaagggg tcccatagcc ttttgggccc gcagggcatc aaggactcgg 180
   ttggctgctt gggcccggag agccttgctc tccctgagat cacctaaagc ccgtaggggc 240
   aaggctcgcc gtagagctgc caagctccag tcatcccaag agcctgaagc accaccacct 300
   cgggatgtgg cccttttgca agggagggca aatgatttgg tgaagtacct tttggctaaa 360
   gaccagacga agattcccat caagcgctcg gacatgctga aggacatcat caaagaatac 420
   actgatgtgt accccgaaat cattgaacga gcaggctatt ccttggagaa ggtatttggg 480
   attcaattga aggaaattga taagaatgac cacttgtaca ttcttctcag c 531
<210> 8
   <211> 531
   <212> DNA
   <213> Homo sapiens
<220>
   <221> misc_feature
   <222> 481
   <223> n = A,T,C or G
<400> 8
   gaggtctcac tatgttgccc aggctgttct tgaactcctg ggatcaagca atccacccat 60
   gttggtctcc aaaagtgctg ggatcatagg cgtgagccac ctcacccagc caccaatttt 120
   caatcaggaa gactttttcc ttcttcaaga agtgaagggt ttccagagta tagctacact 180
   attgcttgcc tgagggtgac tacaaaattg cttgctaaaa ggttaggatg ggtaaagaat 240
   tagattttct gaatgcaaaa ataaaatgtg aactaatgaa ctttaggtaa tacatattca 300
   taaaataatt attcacatat ttcctgattt atcacagaaa taatgtatga aatgctttga 360
   gtttcttgga gtaaactcca ttactcatcc caagaaacca tattataagt atcactgata 420
   ataagaacaa caggaccttg tcataaattc tggataagag aaatagtctc tgggtgtttg 480
   ntcttaattg ataaaattta cttgtccatc ttttagttca gaatcacaaa a 531
<210> 9
   <211> 531
   <212> DNA
   <213> Homo sapiens
<220>
   <221> misc_feature
   <222> 528
   <223> n = A,T,C or G
<400> 9
   aagcggaaat gagaaaggag ggaaaatcat gtggtattga gcggaaaact gctggatgac 60
   agggctcagt cctgttggag aactctgggt ggtgctgtag aacagggcca ctcacagtgg 120
   ggtgcacaga ccagcacggc tctgtgacct gtttgttaca ggtccatgat gaggtaaaca 180
   atacactgag tataagggtt ggtttagaaa ctcttacagc aatttgacaa agtaatcttc 240
   tgtgcagtga atctaagaaa aaaattgggg ctgtatttgt atgttccttt ttttcatttc 300
   atgttctgag ttacctattt ttattgcatt ttacaaaagc atccttccat gaaggaccgg 360
   aagttaaaaa caaagcaggt cctttatcac agcactgtcg tagaacacag ttcagagtta 420
   tccacccaag gagccaggga gctgggctaa accaaagaat tttgcttttg gttaatcatc 480
   aggtacttga gttggaattg ttttaatccc atcattacca ggctggangt g          531
<210> 10
   <211> 861
   <212> DNA
   <213> Homo sapiens
<400> 10
   ccgcggctcc tgtccagacc ctgaccctcc ctcccaaggc tcaaccgtcc cccaacaacc 60
   gccagccttg tactgatgtc ggctgcgaga gcctgtgctt aagtaagaat caggccttat 120
   tggagacatt caagcaaagg ttggacaact acttttccag aacagaaagg aaactcatgc 180
   atcagaaaag gtgactaata aaggtaccag aagaatatgg ctgcacaaat accagaatct 240
   gatcagataa aacagtttaa ggaatttctg gggacctaca ataaacttac agagacctgc 300
   tttttggact gtgttagaga cttcacaaca agagaagtaa aacctgaaga gaccacctgt 360
   tcagaacatt gcttacagaa atatttaaaa atgacacaaa gaatatccat gagatttcag 420
   gaatatcata ttcagcagaa tgaagccctg gcagccaaag caggactcct tggccaacca 480
   cgatagagaa gtcctgatgg atgaactttt gatgaaagat tgccaacagc tgctttattg 540
   gaaatgagga ctcatctgat agaatcccct gaaagcagta gccaccatgt tcaaccatct 600
   gtcatgactg tttggcaaat ggaaaccgct ggagaaacaa aattgctatt taccaggaat 660
   aatcacaata gaaggtctta ttgttcagtg aaataataag atgcaacatt tgttgaggcc 720
   ttatgattca gcagcttggt cacttgatta gaaaaataaa ccattgtttc ttcaattgtg 780
   actgttaatt ttaaagcaac ttatgtgttc gatcatgtat gagatagaaa aatttttatt 840
   actcaaagta aaataaatgg a                                           861
<210> 11
   <211> 541
   <212> DNA
   <213> Homo sapiens
<400> 11
   gaaaaaaaat ataaaacaca cttttgcgaa aacggtggcc ctaaaagagg aaaagaattt 60
   caccaatata aatccaattt tatgaaaact gacaatttaa tccaagaatc acttttgtaa 120
   atgaagctag caagtgatga tatgataaaa taaacgtgga ggaaataaaa acacaagact 180
   tggcataaga tatatccact tttgatatta aacttgtgaa gcatattctt cgacaaattg 240
   tgaaagcgtt cctgatcttg cttgttctcc atttcaaata aggaggcata tcacatccca 300
   agagtaacag aaaaagaaaa aagacatttt tgcattttga gatgaaccaa agacacaaaa 360
   caaaacgaac aaagtgtcat gtctaattct agcctctgaa ataaaccttg aacatctcct 420
   acaaggcacc gtgatttttg taattctaac ctgaagaaat gtgatgactt ttgtggacat 480
   gaaaatcaga tgagaaaact gtggtctttc caaagcctga actcccctga aaacctttgc 540
   a                                                                 541
<210> 12
   <211> 541
   <212> DNA
   <213> Homo sapiens
<400> 12
   ctgggatcat ttctcttgat gtcataaaag actcttcttc ttcctcttca tcctcttctt 60
   catcctcttc tgtacagtgc tgccgggtac aacggctatc tttgtcttta tcctgagatg 120
   aagatgatgc ttctgtttct cctaccataa ctgaagaaat ttcgctggaa gtcgtttgac 180
   tggctgtttc tctgacttca ccttctttgt caaacctgag tctttttacc tcatgcccct 240
   cagcttccac agcatcttca tctggatgtt tatttttcaa agggctcact gaggaaactt 300
   ctgattcaga ggtcgaagag tcactgtgat ttttctcctc attttgctgc aaatttgcct 360
   ctttgctgtc tgtgctctca ggcaacccat ttgttgtcat gggggctgac aaagaaacct 420
   ttggtcgatt aagtggcctg ggtgtcccag gcccatttat attagacctc tcagtatagc 480
   ttggtgaatt tccaggaaac ataacaccat tcattcgatt taaactattg gaattggttt 540
   t                                                                 541
<210> 13
   <211> 441
   <212> DNA
   <213> Homo sapiens
<400> 13
   gagggttggt ggtagcggct tggggaggtg ctcgctctgt cggtcttgct ctctcgcacg 60
   cttcccccgg ctcccttcgt ttcccccccc cggtcgcctg cgtgccggag tgtgtgcgag 120
   ggagggggag ggcgtcgggg gggtgggggg aggcgttccg gtccccaaga gacccgcgga 180
   gggaggcgga ggctgtgagg gactccggga agccatggac gtcgagaggc tccaggaggc 240
   gctgaaagat tttgagaaga gggggaaaaa ggaagtttgt cctgtcctgg atcagtttct 300
   ttgtcatgta gccaagactg gagaaacaat gattcagtgg tcccaattta aaggctattt 360
   tattttcaaa ctggagaaag tgatggatga tttcagaact tcagctcctg agccaagagg 420
   tcctcccaac cctaatgtcg a                                           441
<210> 14
   <211> 131
   <212> DNA
   <213> Homo sapiens
<220>
   <221> misc_feature
   <222> 126
   <223> n = A,T,C or G
<400> 14
   aagcaggcgg ctcccgcgct cgcagggccg tgccacctgc ccgcccgccc gctcgctcgc 60
   tcgcccgccg cgccgcgctg ccgaccgcca gcatgctgcc gagagtgggc tgccccgcgc 120
   tgccgntgcc g                                                      131
<210> 15
   <211> 692
   <212> DNA
   <213> Homo sapiens
<400> 15
   atctcttgta tgccaaatat ttaatataaa tctttgaaac aagttcagat gaaataaaaa 60
   tcaaagtttg caaaaacgtg aagattaact taattgtcaa atattcctca ttgccccaaa 120
   tcagtatttt ttttatttct atgcaaaagt atgccttcaa actgcttaaa tgatatatga 180
   tatgatacac aaaccagttt tcaaatagta aagccagtca tcttgcaatt gtaagaaata 240
   ggtaaaagat tataagacac cttacacaca cacacacaca cacacacgtg tgcacgccaa 300
   tgacaaaaaa caatttggcc tctcctaaaa taagaacatg aagaccctta attgctgcca 360
   ggagggaaca ctgtgtcacc cctccctaca atccaggtag tttcctttaa tccaatagca 420
   aatctgggca tatttgagag gagtgattct gacagccacg ttgaaatcct gtggggaacc 480
   attcatgtcc acccactggt gccctgaaaa aatgccaata atttttcgct cccacttctg 540
   ctgctgtctc ttccacatcc tcacatagac cccagacccg ctggcccctg gctgggcatc 600
   gcattgctgg tagagcaagt cataggtctc gtctttgacg tcacagaagc gatacaccaa 660
   attgcctggt cggtcattgt cataaccaga ga                               692

<210> 16
   <211> 728
   <212> DNA
   <213> Homo sapiens
<400> 16
   cagacggggt ttcactatgt tggctaggct ggtcttgaac tcctgacttc aggtgatctg 60
   cctgccttgg cctcccaaag tgctgggatt acaggcataa gccactgcgc ccggctgatc 120
   tgatggtttc ataaggcttt tccccctttt gctcagcact tctccttcct gccgccatgt 180
   gaagaaggac atgtttgctt ccccttccac cacgattgta agttgtttcc tgaggcctcc 240
   ccggccatgc tgaactgtga gtcaattaaa cctctttcct ttataaatta tccagttttg 300
   ggtatgtctt tattagtaga atgagaacag actaatacaa cccttaaagg agactgacgg 360
   agaggattct tcctggatcc cagcacttcc tctgaatgct actgacattc ttcttgagga 420
   ctttaaactg ggagatagaa aacagattcc atggctcagc agcctgagag cagggaggga 480
   gccaagctat agatgacatg ggcagcctcc cctgaggcca ggtgtggccg aacctgggca 540
   gtgctgccac ccaccccacc agggccaagt cctgtccttg gagagccaag cctcaatcac 600
   tgctagcctc aagtgtcccc aagccacagt ggctaggggg actcagggaa cagttcccag 660
   tctgccctac ttctcttacc tttacccctc atacctccaa agtagaccat gttcatgagg 720
   tccaaagg                                                          728
<210> 17
   <211> 531
   <212> DNA
   <213> Homo sapiens
<220>
   <221> misc_feature
   <222> 518, 528
   <223> n = A,T,C or G
<400> 17
   aagcgaggaa gccactgcgg ctcctggctg aaaagcggcg ccaggctcgg gaacagaggg 60
   aacgcgaaga acaggagcgg aagctgcagg ctgaaaggga caagcgaatg cgagaggagc 120
   agctggcccg ggaggctgaa gcccgggctg aacgtgaggc cgaggcgcgg agacgggagg 180
   agcaggaggc tcgagagaag gcgcaggctg agcaggagga gcaggagcga ctgcagaagc 240
   agaaagagga agccgaagcc cggtcccggg aagaagctga gcgccagcgc caggagcggg 300
   aaaagcactt tcagaaggag gaacaggaga gacaagagcg aagaaagcgg ctggaggaga 360
   taatgaagag gactcggaaa tcagaagccg ccgaaaccaa gaagcaggat gcaaaggaga 420
   ccgcagctaa caattccggc ccagaccctt gtgaaagctg tagagactcg gccctctggg 480
   cttccagaaa ggattctatt gcagaaagga aggagctngg ccccccangg a          531
<210> 18
   <211> 1041
   <212> DNA
   <213> Homo sapiens
<220>
   <221> misc_feature
   <222> 544
   <223> n = A,T,C or G
<400> 18
   ctctgtggaa aactgatgag gaatgaattt accattaccc atgttctcat ccccaagcaa 60
   agtgctgggt ctgattactg caacacagag aacgaagaag aacttttcct catacaggat 120
   cagcagggcc tcatcacact gggctggatt catactcacc ccacacagac cgcgtttctc 180
   tccagtgtcg acctacacac tcactgctct taccagatga tgttgccaga gtcagtagcc 240
   attgtttgct cccccaagtt ccaggaaact ggattcttta aactaactga ccatggacta 300
   gaggagattt cttcctgtcg ccagaaagga tttcatccac acagcaagga tccacctctg 360
   ttctgtagct gcagccacgt gactgttgtg gacagagcag tgaccatcac agaccttcga 420
   tgagcgtttg agtccaacac cttccaagaa caacaaaacc atatcagtgt actgtagccc 480
   cttaatttaa gctttctaga aagctttgga agtttttgta gatagtagaa aggggggcat 540
   cacntgagaa agagctgatt ttgtatttca ggtttgaaaa gaaataactg aacatatttt 600
   ttaggcaagt cagaaagaga acatggtcac ccaaaagcaa ctgtaactca gaaattaagt 660
   tactcagaaa ttaagtagct cagaaattaa gaaagaatgg tataatgaac ccccatatac 720
   ccttccttct ggattcacca attgttaaca tttttttcct ctcagctatc cttctaattt 780
   ctctctaatt tcaatttgtt tatatttacc tctgggctca ataagggcat ctgtgcagaa 840
   atttggaagc catttagaaa atcttttgga ttttcctgtg gtttatggca atatgaatgg 900
   agcttattac tggggtgagg gacagcttac tccatttgac cagattgttt ggctaacaca 960
   tcccgaagaa tgattttgtc aggaattatt gttatttaat aaatatttca ggatattttt 1020
   cctctacaat aaagtaacaa t                                           1041
<210> 19
   <211> 1043
   <212> DNA
   <213> Homo sapiens
<400> 19
   ctctgtggaa aactgatgag gaatgaattt accattaccc atgttctcat ccccaagcaa 60
   agtgctgggt ctgattactg caacacagag aacgaagaag aacttttcct catacaggat 120
   cagcagggcc tcatcacact gggctggatt catactcacc ccacacagac cgcgtttctc 180
   tccagtgtcg acctacacac tcactgctct taccagatga tgttgccaga gtcagtagcc 240
   attgtttgct cccccaagtt ccaggaaact ggattcttta aactaactga ccatggacta 300
   gaggagattt cttcctgtcg ccagaaagga tttcatccac acagcaagga tccacctctg 360
   ttctgtagct gcagccacgt gactgttgtg gacagagcag tgaccatcac agaccttcga 420
   tgagcgtttg agtccaacac cttccaagaa caacaaaacc atatcagtgt actgtagccc 480
   cttaatttaa gctttctaga aagctttgga agtttttgta gatagtagaa aggggggcat 540
   cacctgagaa agagctgatt ttgtatttca ggtttgaaaa gaaataactg aacatatttt 600
   ttaggcaagt cagaaagaga acatggtcac ccaaaagcaa ctgtaactca gaaattaagt 660
   tactcagaaa ttaagtagct cagaaattaa gaaagaatgg tataatgaac ccccatatac 720
   ccttccttct ggattcacca attgttaaca tttttttcct ctcagctatc cttctaattt 780
   ctctctaatt tcaatttgtt tatatttacc tctgggctca ataagggcat ctgtgcagaa 840
   atttggaagc catttagaaa atcttttgga ttttcctgtg gtttatggca atatgaatgg 900
   agcttattac tggggtgagg gacagcttac tccatttgac cagattgttt ggctaacaca 960
   tcccgaagaa tgattttgtc aggaattatt gttatttaat aaatatttca ggatattttt 1020
   cctctacaat aaagtaacaa tta                                           1043
<210> 20
   <211> 448
   <212> DNA
   <213> Homo sapiens
<400> 20
   ggacgacaag gccatggcga tatcggatcc gaattcaagc ctttggaatt aaataaacct 60
   ggaacaggga aggtgaaagt tggagtgaga tgtcttccat atctatacct ttgtgcacag 120
   ttgaatggga actgtttggg tttagggcat cttagagttg attgatggaa aaagcagaca 180
   ggaactggtg ggaggtcaag tggggaagtt ggtgaatgtg gaataactta cctttgtgct 240
   ccacttaaac cagatgtgtt gcagctttcc tgacatgcaa ggatctactt taattccaca 300
   ctctcattaa taaattgaat aaaagggaat gttttggcac ctgatataat ctgccaggct 360
   atgtgacagt aggaaggaat ggtttcccct aacaagccca atgcactggt ctgactttat 420
   aaattattta ataaaatgaa ctattatc                                      448
<210> 21
   <211> 411
   <212> DNA
   <213> Homo sapiens
<400> 21
   ggcagtgaca ttcaccatca tgggaaccac cttccctttt cttcaggatt ctctgtagtg 60
   gaagagagca cccagtgttg ggctgaaaac atctgaaagt agggagaaga acctaaaata 120
   atcagtatct cagagggctc taaggtgcca agaagtctca ctggacattt aagtgccaac 180
   aaaggcatac tttcggaatc gccaagtcaa aactttctaa cttctgtctc tctcagagac 240
   aagtgagact caagagtcta ctgctttagt ggcaactaca gaaaactggt gttacccaga 300
   aaaacaggag caattagaaa tggttccaat atttcaaagc tccgcaaaca ggatgtgctt 360
   tcctttgccc atttagggtt tcttctcttt cctttctctt tattaaccac t          411
<210> 22
   <211> 896
   <212> DNA
   <213> Homo sapiens
<220>
   <221> misc_feature
   <222> 230, 320
   <223> n = A,T,C or G
<400> 22
   tgcgctgaaa acaacggcct cctttactgt taaaatgcag ccacaggtgc ttagccgtgg 60
   gcatctcaac caccagcctc tgtggggggc aggtgggcgt ccctgtgggc ctctgggccc 120
   acgtccagcc tctgtcctct gccttccgtt cttcgacagt gttcccggca tccctggtca 180
   cttggtactt ggcgtgggcc tcctgtgctg ctccagcagc tcctccaggn ggtcggcccg 240
   cttcaccgca gcctcatgtt gtgtccggag gctgctcacg gcctcctcct tcctcgcgag 300
   ggctgtcttc accctccggn gcacctcctc cagctccagc tgctggcggg cctgcagcgt 360
   ggccagctcg gccttggcct gccgcgtctc ctcctcarag gctgccagcc ggtcctcgaa 420
   ctcctggcgg atcacctggg ccaggttgct gcgctcgcta gaaagctgct cgttcaccgc 480
   ctgcgcatcc tccagcgccc gctccttctg ccgcacaagg ccctgcagac gcagattctc 540
   gccctcggcc tccccaagct ggcccttcag ctccgagcac cgctcctgaa gcttccgctc 600
   cgactgctcc agctcggaga gctcggcctc gtacttgtcc cgtaagcgct tgatgcggct 660
   ctcggcagcc ttctcactct cctccttggc cagcgccatg tcggcctcca gccggtgaat 720
   gaccagctca atctccttgt cccggccttt ccggatttct tccctcagct cctgttcccg 780
   gttcagcagc cacgcctcct ccttcctggt gcggccggcc tcccacgcct gcctctccag 840
   ctccagctgc tgcttcaggg tattcagctc catctggcgg gcctgcagcg tggcca     896
<210> 23
   <211> 111
   <212> DNA
   <213> Homo sapiens
<400> 23
   caacttatta cttgaaatta taatatagcc tgtccgtttg ctgtttccag gctgtgatat 60
   attttcctag tggtttgact ttaaaaataa ataaggttta attttctccc c          111
<210> 24
   <211> 531
   <212> DNA
   <213> Homo sapiens
<220>
   <221> misc_feature
   <222> 472, 494
   <223> n = A,T,C or G
<400> 24
   tgcaagtcac gggagtttat ttatttaatt tttttcccca gatggagact ctgtcgccca 60
   ggctggagtg caatggtgtg atcttggctc actgcaacct ccacctcctg ggttcaagcg 120
   attctcctgc cacagcctcc cgagtagctg ggattacagg tgcccgccac cacacccagc 180
   taatttttat atttttagta aagacagggt ttccccatgt tggccaggct ggtcttgaac 240
   ttctgacctc aggtgatcca cctgcctcgg cctcccaaag tgttgggatt acaggcgtga 300
   gctacccgtg cctggccagc cactggagtt taaaggacag tcatgttggc tccagcctaa 360
   ggcggcattt tcccccatca gaaagcccgc ggctcctgta cctcaaaata gggcacctgt 420
   aaagtcagtc agtgaagtct ctgctctaac tggccacccg gggccattgg cntctgacac 480
   agccttgcca ggangcctgc atctgcaaaa gaaaagttca cttcctttcc g          531

<210> 25
   <211> 471
   <212> DNA
   <213> Homo sapiens
<220>
   <221> misc_feature
   <222> 377
   <223> n = A,T,C or G
<400> 25
   cagagaatct kagaaagatg tcgcgttttc ttttaatgaa tgagagaagc ccatttgtat 60
   ccctgaatca ttgagaaaag gcggcggtgg cgacagcggc gacctaggga tcgatctgga 120
   gggacttggg gagcgtgcag agacctctag ctcgagcgcg agggacctcc cgccgggatg 180
   cctggggagc agatggaccc tactggaagt cagttggatt cagatttctc tcagcaagat 240
   actccttgcc tgataattga agattctcag cctgaaagcc aggttctaga ggatgattct 300
   ggttctcact tcagtatgct atctcgacac cttcctaatc tccagacgca caaagaaaat 360
   cctgtgttgg atgttgngtc caatccttga acaaacagct ggagaagaac gaggagaccg 420
   gtaatagtgg gttcaatgaa catttgaaag aaaaccaggt tgcagaccct g         471
<210> 26
   <211> 541
   <212> DNA
   <213> Homo sapiens
<400> 26
   gactgtcctg aacaagggac ctctgaccag agagctgcag gagatgcaga gtggtggcag 60
   gagtggaagc caaagaacac ccaccttcct cccttgaagg agtagagcaa ccatcagaag 120
   atactgtttt attgctctgg tcaaacaagt cttcctgagt tgacaaaacc tcaggctctg 180
   gtgacttctg aatctgcagt ccactttcca taagttcttg tgcagacaac tgttcttttg 240
   cttccatagc agcaacagat gctttggggc taaaaggcat gtcctctgac cttgcaggtg 300
   gtggattttg ctcttttaca acatgtacat ccttactggg ctgtgctgtc acagggatgt 360
   ccttgctgga ctgttctgct atggggatat cttcgttgga ctgttcttca tgcttaattg 420
   cagtattagc atccacatca gacagcctgg tataaccaga gttggtggtt actgattgta 480
   gctgctcttt gtccacttca tatggcacaa gtattttcct caacatcctg gctctgggaa 540
   g                                                                 541
<210> 27
   <211> 461
   <212> DNA
   <213> Homo sapiens
<220>
   <221> misc_feature
   <222> 367
   <223> n = A,T,C or G
<400> 27
   gaaatgtata tttaatcatt ctcttgaacg atcagaactc traaatcagt tttctataac 60
   arcatgtaat acagtcaccg tggctccaag gtccaggaag gcagtggtta acacatgaag 120
   agtgtgggaa gggggctgga aacaaagtat tcttttcctt caaagcttca ttcctcaagg 180
   cctcaattca agcagtcatt gtccttgctt tcaaaagtct gtgtgtgctt catggaaggt 240
   atatgtttgt tgccttaatt tgaattgtgg ccaggaaggg tctggagatc taaattcaga 300
   gtaagaaaac ctgagctaga actcaggcat ttctcttaca gaacttggct tgcagggtag 360
   aatgaangga aagaaactta gaagctcaac aagctgaaga taatcccatc aggcatttcc 420
   cataggcctt gcaactctgt tcactgagag atgttatcct g                     461
<210> 28
   <211> 541
   <212> DNA
   <213> Homo sapiens
<400> 28
   agtctggagt gagcaaacaa gagcaagaaa caarragaag ccaaaagcag aaggctccaa 60
   tatgaacaag ataaatctat cttcaaagac atattagaag ttgggaaaat aattcatgtg 120
   aactagacaa gtgtgttaag agtgataagt aaaatgcacg tggagacaag tgcatcccca 180
   gatctcaggg acctccccct gcctgtcacc tggggagtga gaggacagga tagtgcatgt 240
   tctttgtctc tgaattttta gttatatgtg ctgtaatgtt gctctgagga agcccctgga 300
   aagtctatcc caacatatcc acatcttata ttccacaaat taagctgtag tatgtaccct 360
   aagacgctgc taattgactg ccacttcgca actcaggggc ggctgcattt tagtaatggg 420
   tcaaatgatt cactttttat gatgcttccc aaggtgcctt ggcttctctt cccaactgac 480
   aaatgcccaa gttgagaaaa atgatcataa ttttagcata aaccgagcaa tcggcgaccc 540
   c                                                                 541
<210> 29
   <211> 411
   <212> DNA
   <213> Homo sapiens
<400> 29
   tagctgtctt cctcactctt atggcaatga ccccatatct taatggatta agataatgaa 60
   agtgtatttc ttacactctg tatctatcac cagaagctga ggtgatagcc cgcttgtcat 120
   tgtcatccat attctgggac tcaggcggga actttctgga atattgccag ggagcatggc 180
   agaggggcac agtgcattct gggggaatgc acattggctc agcctgggta atgagtgata 240
   tacattacct ctgttcacaa ctcattgccc agcaccagtc acaaggcccc accaaatacc 300
   agagcccaag aaatgtagtc ctgttgatat ggttttgctg tgtcccaacc caaatctcat 360
   cttgaattgt aagctcccat aattcccatg tgttgtggga gggacctggt g          411
<210> 30
   <211> 511
   <212> DNA
   <213> Homo sapiens
<400> 30
   atcatgagga tgttaccaaa gggatggtac taaaccattt gtattcgtct gttttcacac 60
   tgctttgaag atactacctg agactgggta atttataaac aaaagagatt taattgactc 120
   acagttctgc atggctgaag aggcctcagg aaacttacag tcatggtgga aggcaaagga 180
   ggagcaaggc atgtcttaca tgtcagtagg agagagagcg agagcaggag aacctgccac 240
   ttataaacca ttcagatctc ataactccct atcatgagaa aaacatggag gaaaccaccc 300
   tcatgatcca atcacctccc gccaggtccc tccctcgaca cgtggggatt ataattcagg 360
   attagaggga cacagagaca aaccatatca tcattcatga gaaatccacc ctcatagtcc 420
   aatcagctcc taccaggccc cacctccaac actggggatt gcaattcaac atgagatttg 480
   gatggggaca cagattcaaa ccatatcata c                                511
<210> 31
   <211> 827
   <212> DNA
   <213> Homo sapiens
<400> 31
   catggccttt ctccttagag gccagaggtg ctgccctggc tgggagtgaa gctccaggca 60
   ctaccagctt tcctgatttt cccgtttggt ccatgtgaag agctaccacg agccccagcc 120
   tcacagtgtc cactcaaggg cagcttggtc ctcttgtcct gcagaggcag gctggtgtga 180
   ccctgggaac ttgacccggg aacaacaggt ggcccagagt gagtgtggcc tggcccctca 240
   acctagtgtc cgtcctcctc tctcctggag ccagtcttga gtttaaaggc attaagtgtt 300
   agatacaagc tccttgtggc tggaaaaaca cccctctgct gataaagctc agggggcact 360
   gaggaagcag aggccccttg ggggtgccct cctgaagaga gcgtcaggcc atcagctctg 420
   tccctctggt gctcccacgt ctgttcctca ccctccatct ctgggagcag ctgcacctga 480
   ctggccacgc gggggcagtg gaggcacagg ctcagggtgg ccgggctacc tggcacccta 540
   tggcttacaa agtagagttg gcccagtttc cttccacctg aggggagcac tctgactcct 600
   aacagtcttc cttgccctgc catcatctgg ggtggctggc tgtcaagaaa ggccgggcat 660
   gctttctaaa cacagccaca ggaggcttgt agggcatctt ccaggtgggg aaacagtctt 720
   agataagtaa ggtgacttgc ctaaggcctc ccagcaccct tgatcttgga gtctcacagc 780
   agactgcatg tsaacaactg gaaccgaaaa catgcctcag tataaaa               827
<210> 32
   <211> 291
   <212> DNA
   <213> Homo sapiens
<400> 32
   ccagaacctc cttctctttg gagaatgggg aggcctcttg gagacacaga gggtttcacc 60
   ttggatgacc tctagagaaa ttgcccaaga agcccacctt ctggtcccaa cctgcagacc 120
   ccacagcagt cagttggtca ggccctgctg tagaaggtca cttggctcca ttgcctgctt 180
   ccaaccaatg ggcaggagag aaggccttta tttctcgccc acccattctc ctgtaccagc 240
   acctccgttt tcagtcagyg ttgtccagca acggtaccgt ttacacagtc a          291
<210> 33
   <211> 491
   <212> DNA
   <213> Homo sapiens
<400> 33
   tgcatgtagt tttatttatg tgttttsgtc tggaaaacca agtgtcccag cagcatgact 60
   gaacatcact cacttcccct acttgatcta caaggccaac gccgagagcc cagaccagga 120
   ttccaaacac actgcacgag aatattgtgg atccgctgtc aggtaagtgt ccgtcactga 180
   cccaracgct gttacgtggc acatgactgt acagtgccac gtaacagcac tgtacttttc 240
   tcccatgaac agttacctgc catgtatcta catgattcag aacattttga acagttaatt 300
   ctgacacttg aataatccca tcaaaaaccg taaaatcact ttgatgtttg taacgacaac 360
   atagcatcac tttacgacag aatcatctgg aaaaacagaa caacgaatac atacatctta 420
   aaaaatgctg gggtgggcca ggcacagctt cacgcctgta atcccagcac tttgggaggc 480
   ttaagcgggt g 491
<210> 34
   <211> 521
   <212> DNA
   <213> Homo sapiens
<220>
   <221> misc_feature
   <222> 453, 476, 487
   <223> n = A,T,C or G
<400> 34
   tggggcggaa agaagccaag gccaaggagc tggtgcggca gctgcagctg gaggccgagg 60
   agcagaggaa gcagaagaag cggcagagtg tgtcgggcct gcacagatac cttcacttgc 120
   tggatggaaa tgaaaattac ccgtgtcttg tggatgcaga cggtgatgtg atttccttcc 180
   caccaataac caacagtgag aagacaaagg ttaagaaaac gacttctgat ttgtttttgg 240
   aagtaacaag tgccaccagt ctgcagattt gcaaggatgt catggatgcc ctcattctga 300
   aaatggcaag aaatgaaaaa gtacacttta gaaaataaag aggaaggatc actctcagat 360
   actgaagccg atgcagtctc tggacaactt ccagatccca caacgaatcc cagtgctgga 420
   aaggacgggc ccttccttct ggtggtggaa cangtcccgg tggtggatct tggaanggaa 480
   cctgaangtg gtgtaccccg tccaaggccg accttggcca c 521

<210> 35
   <211> 161
   <212> DNA
   <213> Homo sapiens
<220>
   <221> misc_feature
   <222> 18
   <223> n = A,T,C or G
<400> 35
   tcccgcgctc gcagggcncg tgccacctgc cygtccgccc gctcgctcgc tcgcccgccg 60
   cgccgcgctg ccgaccgyca gcatgctgcc gagagtgggc tgccccgcgc tgccgctgcc 120
   gccgccgccg ctgctgccgc tgctgccgct gctgctgctg c 161
<210> 36
   <211> 341
   <212> DNA
   <213> Homo sapiens
<400> 36
   ggcgggtagg catggaactg agaagaacga agaagctttc agactacgtg gggaagaatg 60
   aaaaaaccaa aattatcgcc aagattcagc aaaggggaca gggagctcca gcccgagagc 120
   ctattattag cagtgaggag cagaagcagc tgatgctgta ctatcacaga agacaagagg 180
   agctcaagag attggaagaa aatgatgatg atgcctattt aaactcacca tgggcggata 240
   acactgcttt gaaaagacat tttcatggag tgaaagacat aaagtggaga ccaagatgaa 300
   gttcaccagc tgatgacact tccaaagaga ttagctcacc t                     341
<210> 37
   <211> 521
   <212> DNA
   <213> Homo sapiens
<220>
   <221> misc_feature
   <222> 516
   <223> n = A,T,C or G
<400> 37
   tctgaaggtt aaatgtttca tctaaatagg gataatgrta aacacctata gcatagagtt 60
   gtttgagatt aaatgagata atacatgtaa aattatgtgc ctggcataca gcaagattgt 120
   tgttgttgtt gatgatgatg atgatgatga taatattttt ctatccccag tgcacaactg 180
   cttgaaccta ttagataatc aatacatgtt tcttgaactg agatcaattt ccccatgttg 240
   tctgactgat gaagccctac attttcttct agaggagatg acatttgagc aagatcttaa 300
   agaaaatcag atgccttcac ctgaccactg cttggtgatc ccatggcact ttgtacatct 360
   ctccattagc tctcatctca ccagcccatc attattgtat gtgctgcctt ctgaagcttg 420
   cagctggcta ccatcmggta gaataaaaat catcctttca taaaatagtg accctccttt 480
   tttatttgca tttcccaaag ccaagcaccg tggganggta g                     521
<210> 38
   <211> 461
   <212> DNA
   <213> Homo sapiens
<400> 38
   tatgaagaag ggaaaagaag ataatttgtg aaagaaatgg gtccagttac tagtctttga 60
   aaagggtcag tctgtagctc ttcttaatga gaataggcag ctttcagttg ctcagggtca 120
   gatttcctta gtggtgtatc taatcacagg aaacatctgt ggttccctcc agtctctttc 180
   tgggggactt gggcccactt ctcatttcat ttaattagag gaaatagaac tcaaagtaca 240
   atttactgtt gtttaacaat gccacaaaga catggttggg agctatttct tgatttgtgt 300
   aaaatgctgt ttttgtgtgc tcataatggt tccaaaaatt gggtgctggc caaagagaga 360
   tactgttaca gaagccagca agaagacctc tgttcattca cacccccggg gatatcagga 420
   attgactcca gtgtgtgcaa atccagtttg gcctatcttc t                     461
<210> 39
   <211> 769
   <212> DNA
   <213> Homo sapiens
<400> 39
   tgagggactg attggtttgc tctctgctat tcaattcccc aagcccactt gttcctgcag 60
   cgtcctcctt ctcattccct ttagttgtac cctctctttc atctgagacc tttccttctt 120
   gatgtcgcct tttcttcttc ttgctttttc tgatgttctg ctcagcatgt tctgggtgct 180
   tctcatctgc atcattcctt tcagatgctg tagcttcttc ctcctctttc tgcctccttt 240
   tctttttctt ttttttgggg ggcttgctct ctgactgcag ttgaggggcc ccagggtcct 300
   ggcctttgag acgagccagg aaggcctgct cctgggcctc taggcgagca agcttggcct 360
   tcattgtgat cccaagacgg gcagccttgt gtgctgttcg cccctcacag gcttggagca 420
   gcatctcatc agtcagaatc tttggggact tggacccctg gttgtcgtca tcactgcagc 480
   tctccaagtc tttgtttggc ttctctccac ctgaagtcaa tgtagccatc ttcacaaact 540
   tctgatacag caagttgggc ttgggatgat tataacgggt ggtctcctta gaaaggctcc 600
   ttatctgtac tccatcctgc ccagtttcca ctaccaagtt ggccgcagtc ttgttgaaga 660
   gctcattcca ccagtggttt gtgaactcct tggcagggtc atgtcctacc ccatgagtgt 720
   cttgcttcag ygtcaccctg agagcctgag tgataccatt ctccttccg 769
<210> 40
   <211> 292
   <212> DNA
   <213> Homo sapiens
<400> 40
   gacaacatga aataaatcct agaggacaaa attaaactca atagagtgta gtctagttaa 60
   aaactcgaaa aatgagcaag tctggtggga gtggaggaag ggctatacta taaatccaag 120
   tgggcctcct gatcttaaca agccatgctc attatacaca tctctgaact ggacatacca 180
   cctttacgca ggaaacaggg cttggaactt ctaagggaaa ttaacatgca ccacccacat 240
   ctaacctacc tgccgggtag gtaccatccc tgcttcgctg aaatcagtgc tc 292
<210> 41
   <211> 406
   <212> DNA
   <213> Homo sapiens
<400> 41
   ttggaattaa ataaacctgg aacagggaag gtgaaagttg gagtgagatg tcttccatat 60
   ctataccttt gtgcacagtt gaatgggaac tgtttgggtt tagggcatct tagagttgat 120
   tgatggaaaa agcagacagg aactggtggg aggtcaagtg gggaagttgg tgaatgtgga 180
   ataacttacc tttgtgctcc acttaaacca gatgtgttgc agctttcctg acatgcaagg 240
   atctacttta attccacact ctcattaata aattgaataa aagggaatgt tttggcacct 300
   gatataatct gccaggctat gtgacagtag gaaggaatgg tttcccctaa caagcccaat 360
   gcactggtct gactttataa attatttaat aaaatgaact attatc 406
<210> 42
   <211> 381
   <212> DNA
   <213> Homo sapiens
<400> 42
   aaactggacc tgcaacaggg acatgaattt actgcarggt ctgagcaagc tcagcccctc 60
   tacctcaggg ccccacagcc atgactacct cccccaggag cgggagggtg aagggggcct 120
   gtctctgcaa gtggagccag agtggaggaa tgagctctga agacacagca cccagccttc 180
   tcgcaccagc caagccttaa ctgcctgcct gaccctgaac cagaacccag ctgaactgcc 240
   cctccaaggg acaggaaggc tgggggaggg agtttacaac ccaagccatt ccaccccctc 300
   ccctgctggg gagaatgaca catcaagctg ctaacaattg ggggaagggg aaggaagaaa 360
   actctgaaaa caaaatcttg t                                            381
<210> 43
   <211> 451
   <212> DNA
   <213> Homo sapiens
<400> 43
   catgcgtttc accactgttg gccaggctgg tctcgaactc ctggcctcaa gcaatccacc 60
   cgcctcagcc tccaaaagtg ctgggattac agatgtgagc catggcacca tgccaaaagg 120
   ctatattcct ggctctgtgt ttccgagact gcttttaatc ccaacttctc tacatttaga 180
   ttaaaaaata ttttattcat ggtcaatctg gaacataatt actgcatctt aagtttccac 240
   tgatgtatat agaaggctaa aggcacaatt tttatcaaat ctagtagagt aaccaaacat 300
   aaaatcatta attactttca acttaataac taattgacat tcctcaaaag agctgttttc 360
   aatcctgata ggttctttat tttttcaaaa tatatttgcc atgggatgct aatttgcaat 420
   aaggcgcata atgagaatac cccaaactgg a                                451
<210> 44
   <211> 521
   <212> DNA
   <213> Homo sapiens
<400> 44
   gttggacccc cagggactgg aaagacactt cttgcccgag ctgtggcggg agaagctgat 60
   gttccttttt attatgcttc tggatccgaa tttgatgaga tgtttgtggg tgtgggagcc 120
   agccgtatca gaaatctttt tagggaagca aaggcgaatg ctccttgtgt tatatttatt 180
   gatgaattag attctgttgg tgggaagaga attgaatctc caatgcatcc atattcaagg 240
   cagaccataa atcaacttct tgctgaaatg gatggtttta aacccaatga aggagttatc 300
   ataataggag ccacaaactt cccagaggca ttagataatg ccttaatacc gtcctggtcg 360
   ttttgacatg caagttacag ttccaaggcc agatgtaaaa ggtcgaacag aaattttgaa 420
   atggtatctc aataaaataa agtttgatca atcccgttga tccagaaatt atagcctcga 480
   ggtactggtg gcttttccgg aagcagagtt gggagaatct t                      521
<210> 45
   <211> 585
   <212> DNA
   <213> Homo sapiens
<400> 45
   gcctacaaca tccagaaaga gtctaccctg cacctggtgc tscgtctcag aggtgggatg 60
   cagatcttcg tgaagaccct gactggtaag accatcactc tcgaagtgga gccgagtgac 120
   accatygaga acgtcaaagc aaagatccar gacaaggaag gcrtycctcc tgaccagcag 180
   aggttgatct ttgccggaaa gcagctggaa gatggdcgca ccctgtctga ctacaacatc 240
   cagaaagagt cyaccctgca cctggtgctc cgtctcagag gtgggatgca ratcttcgtg 300
   aagaccctga ctggtaagac catcaccctc gaggtggagc ccagtgacac catcgagaat 360
   gtcaaggcaa agatccaaga taaggaaggc atccctcctg atcagcagag gttgatcttt 420
   gctgggaaac agctggaaga tggacgcacc ctgtctgact acaacatcca gaaagagtcc 480
   actctgcact tggtcctgcg cttgaggggg ggtgtctaag tttccccttt taaggtttcm 540
   acaaatttca ttgcactttc ctttcaataa agttgttgca ttccc                 585

<210> 46
   <211> 481
   <212> DNA
   <213> Homo sapiens
<400> 46
   gaactgggcc ctgagcccaa gtcatgcctt gtgtccgcat ctgccgtgtc acctctgtkc 60
   ctgcccctca cccctccctc ctggtcttct gagccagcac catctccaaa tagcctattc 120
   cttcctgcaa atcacacaca catgcgggcc acacatacct gctgccctgg agatggggaa 180
   gtaggagaga tgaatagagg cccatacatt gtacagaagg aggggcaggt gcagataaaa 240
   gcagcagacc cagcggcagc tgaggtgcat ggagcacggt tggggccggc attgggctga 300
   gcacctgatg ggcctcatct cgtgaatcct cgaggcagcg ccacagcaga ggagttaagt 360
   ggcacctggg ccgagcagag caggagactg agggtcagag tggaggctaa gctgccctgg 420
   aactcctcaa tcttgcctgc cccctagtat gaagccccct tcctgcccct acaattcctg 480
   a                                                                 481
<210> 47
   <211> 461
   <212> DNA
   <213> Homo sapiens
<220>
   <221> misc_feature
   <222> 128
   <223> n = A,T,C or G
<400> 47
   atggatctta ctttgccacc caggttggag tgcagtgctg caatcttggc tcactgcagc 60
   cttaacctcc caggctcaag ctatcctcct gccaaagcct tccacatagc tgggactaca 120
   ggtacacngc caccacaccc agctaaaatt tttgtatttt ttgtagagac gggatctcgc 180
   cacgttgccc aggctggtcc catcctgacc tcaagcagat ctgcccacct cagcccccca 240
   acgtgctagg attacaggcg tgagccaccg cacccagcct ttgttttgct tttaatggaa 300
   tcaccagttc ccctccgtgt ctcagcagca gctgtgagaa atgctttgca tctgtgacct 360
   ttatgaaggg gaacttccat gctgaatgag ggtaggatta catgctcctg tttcccgggg 420
   gtcaagaaag cctcagactc cagcatgata agcagggtga g                     461
<210> 48
   <211> 571
   <212> DNA
   <213> Homo sapiens
<400> 48
   ataggggctt taaggaggga attcaggttc aatgaggtcg taaggccagg gctcttatcc 60
   agtaagactg gggtccttag atgagaaaga gacacccgag gtccttctct ctgccgtgtg 120
   aggatgcatc aagaaggcgg ccgtctgcaa gcgaaggaga ggccgcacca gaaaccgaca 180
   ccttcatctt ggacttgcag cctctagaac tgagaaaata actgtctgtt ggttaagcca 240
   cccagtttgt agtattctct tatggcttcc taagcagact aacaaacaaa cacccaaaat 300
   taactgatgg cttcgctgtc ttctgtaaaa attgctatga gagaactttt cactcactgt 360
   tttgcagttt ctccctcagt ccctggttct ttcttctcac ataatcccaa tttcaattta 420
   tagttcatgg cccaggcaga gtcattcatc acggcatctc ctgagctaaa ccagcacctg 480
   ctctgctcac ttcttgactg gctgctcatc atcagccctc ttgcagagat ttcatttcct 540
   cccgtgccag gtacttcacg caccaagctc a 571
<210> 49
   <211> 511
   <212> DNA
   <213> Homo sapiens
<400> 49
   ggataatgaa gttgttttat ttagcttgga caaaaaggca tattcctcta ttttcttata 60
   caacaaatat ccccaaaata aagcaagcat atatatcttg aatgtgtaat aatccagtga 120
   taaacaagag cagtacttta aaagaaaaaa aaatatgtat ttctgtcagg ttaaaatgag 180
   aatcaaaacc atttactctg ctaactcatt attttttgct ttctttttgg ttaagagagg 240
   caatgcaata cactgaaaaa ggtttttatc ttatctggca ttggaattag acatattcaa 300
   accccagccc ccatttccaa actttaagac cacaaacaag taatttactt ttctgaacat 360
   tggttttttc tggaaaatgg gaattataaa atagactttg cagactctta tgagattaaa 420
   taagataatg tatgaaattc tttcttcttt tttacttctt tttccttttt gagatggagt 480
   ctcaccccgt cacccaggct ggagtacagt g 511
<210> 50
   <211> 561
   <212> DNA
   <213> Homo sapiens
<400> 50
   ccactgcact ccagcctggg tgacggagtg agactctgtc tcaaaaaaac aaacaaacaa 60
   acaaacaaaa aactgaaaag gaaatagagt tcctctttcc tcatatatga atatattatt 120
   tcaacagatt gttgatcacc taccatatgc ttggtattgt tctaattgct ggggatacag 180
   caagaggttc tgcagaactt catggagcat gaaagtaaat aaacaaagtt aatttcaagg 240
   ccaggcatgg ttgctcacac ctttagtccc agcactttgg gaggctgagg caggtggatc 300
   acttgggccc aggagttcaa ggctgcagtg agccaagatt gtgccactac tctccaggct 360
   gggcaacaga gcaagaccct gtctcagggg gaacaaaaag ttaatttcag attttgttaa 420
   gtgctgtaaa ggaagtaaat aggttgatat tcaagagagc acctgaaggc caggcgtggt 480
   ggctcacgcc tgtggtctaa cgctttggga agcccgagcg ggcggatcac aaggtcagga 540
   gaattttggc caggcatggt g 561
<210> 51
   <211> 451
   <212> DNA
   <213> Homo sapiens
<400> 51
   agaatccatt tattgggttt taaactagtt acacaactga aatcagtttg gcactacttt 60
   atacagggat tacgcctgtg tatgccgaca cttaaatact gtaccaggac cactgctgtg 120
   cttaggtctg tattcagtca ttcagcatgt agatactaaa aatatactgt agtgttcctt 180
   taaggaagac tgtacagggt gtgttgcaag atgacattca ccaatttgtg aattatttca 240
   acccagaaga tacctttcac tctataaact tgtcataggc aaacatgtgg tgttagcatt 300
   gagagatgca cacaaaaatg ttacataaaa gttcagacat tctaatgata agtgaactga 360
   aaaaaaaaaa aaccccacat ctcaattttt gtaacaagat aaagaaaata atttaaaaac 420
   acaaaaaatg gcattcagtg ggtacaaagc c                                451
<210> 52
   <211> 682
   <212> DNA
   <213> Homo sapiens

<400> 52
   caaatattta atataaatct ttgaaacaag ttcagakgaa ataaaaatca aagtttgcaa 60
   aaacgtgaag attaacttaa ttgtcaaata ttcctcattg ccccaaatca gtattttttt 120
   tatttctatg caaaagtatg ccttcaaact gcttaaatga tatatgatat gatacacaaa 180
   ccagttttca aatagtaaag ccagtcatct tgcaattgta agaaataggt aaaagattat 240
   aagacacctt acacacacac acacacacac acacacacgt gtgcaccgcc aatgacaaaa 300
   aacaatttgg cctctcctaa aataagaaca tgaagaccct taattgctgc caggagggaa 360
   cactgtgtca cccctcccta caatccaggt agtttccttt aatccaatag caaatctggg 420
   catatttgag aggagtgatt ctgacagcca csgttgaaat cctgtgggga accattcatg 480
   tccacccact ggtgccctga aaaaatgcca ataatttttc gctcccactt ctgctgctgt 540
   ctcttccaca tcctcacata gaccccagac ccgctggccc ctggctgggc atcgcattgc 600
   tggtagagca agtcataggt ctcgtctttg acgtcacaga agcgatacac caaattgcct 660
   ggtcggtcat tgtcataacc ag                                          682
<210> 53
   <211> 311
   <212> DNA
   <213> Homo sapiens
<220>
   <221> misc_feature
   <222> 208
   <223> n = A,T,C or G
<400> 53
   tttgacttta gtaggggtct gaactattta ttttactttg ccmgtaatat ttaraccyta 60
   tatatctttc attatgccat cttatcttct aatgbcaagg gaacagwtgc taamctggct 120
   tctgcattwa tcacattaaa aatggctttc ttggaaaatc ttcttgatat gaataaagga 180
   tcttttavag ccatcattta aagcmggntt ctctccaaca cgagtctgct sasggggggk 240
   gagctgtgaa ctctggctga aggctttccc atacacactg caatgacmtg gtttctgacc 300
   agbgtgagtt a                                                      311
<210> 54
   <211> 561
   <212> DNA
   <213> Homo sapiens
<400> 54
   agagaagccc cataaatgca atcagtgtgg gaaggccttc agtcagagct caagcctttt 60
   cctccatcat cgggttcata ctggagagaa accctatgta tgtaatgaat gcggcagagc 120
   ctttggtttt aactctcatc ttactgaaca cgtaaggatt cacacaggag aaaaacccta 180
   tgtttgtaat gagtgcggca aagcctttcg tcggagttcc actcttgttc agcatcgaag 240
   agttcacact ggggagaagc cctaccagtg cgttgaatgt gggaaagctt tcagccagag 300
   ctcccagctc accctacatc agccgagttc acactggaga gaagccctat gactgtggtg 360
   actgtgggaa ggccttcagc cggaggtcaa ccctcattca gcatcagaaa gttcacagcg 420
   gagagactcg taagtgcaga aaacatggtc cagcctttgt tcatggctcc agcctcacag 480
   cagatggaca gattcccact ggagagaagc acggcagaac ctttaaccat ggtgcaaatc 540
   tcattctgcg ctggacagtt c                                           561
<210> 55
   <211> 811
   <212> DNA
   <213> Homo sapiens
<400> 55
   gagacagggt ctcactttgt cacccaggct ggaatgcagt ggtgcgatct tacgtagctc 60
   actgcagccc tgacctcctg gactcaaaca attctcctgc ctcagccctg caagtagctg 120
   ggactgtggg tgcatgccac catgcctggc taacttttgt agtttttgta aagatggggt 180
   tttgccatgt tgcacatgct ggtcttgaac tcctgagctc aaacgatctg cccacctcgg 240
   cctcccagaa tgttgggatt acaggggtaa accaccacgc ctggccccat tagggtattc 300
   ttagcatcca cttgctcact gagattaatc ataagagatg ataagcactg gaagaaaaaa 360
   atttttacta ggctttggat atttttttcc tttttcagct ttatacagag gattggatct 420
   ttagttttcc tttaactgat aataaaacat tgaaaggaaa taagtttacc tgagattcac 480
   agagataacc ggcatcactc ccttgctcaa ttccagtctt taccacatca attattttca 540
   gaggtgcagg ataaaggcct ttagtctgct ttcgcacttt ttcttccact tttttgtaaa 600
   cctgttgcct gacaaatgga attgacagcg tatgccatga ctattccatt tgtcaggcat 660
   acgctgtcaa tttttccacc aatcccttgt ctctctttgg agagatcttc ttatcagcta 720
   gtcctttggc aaaagtaatt gcaacttctt ctaggtattc tattgtccgt tccactggtg 780
   gaacccctgg gaccaggact aaaacctcca g                                811
<210> 56
   <211> 591
   <212> DNA
   <213> Homo sapiens
<220>
   <221> misc_feature
   <222> 45, 477, 490, 561
   <223> n = A,T,C or G
<400> 56
   atctcatata tatatttctt cctgacttta tttgcttgct tctgncacgc atttaaaata 60
   tcacagagac caaaatagag cggctttctg gtggaacgca tggcagtcac aggacaaaat 120
   acaaaactag ggggctctgt cttctcatac atcatacaat tttcaagtat tttttttatg 180
   tacaaagagc tactctatct gaaaaaaaat taaaaaataa atgagacaag atagtttatg 240
   catcctagga agaaagaatg ggaagaaaga acggggcagt tgggtacaga ttcctgtccc 300
   ctgttcccag ggaccactac cttcctgcca ctgagttccc ccacagcctc acccatcatg 360
   tcacagggca agtgccaggg taggtgggga ccagtggaga caggaaccag caacatactt 420
   tggcctggaa gataaggaga aagtctcaga aacacactgg tgggaagcaa tcccacnggc 480
   cgtgccccan gagcttccca cctgctgctg gctccctggg tggctttggg aacagcttgg 540
   gcaggccctt ttgggtgggg nccaactggg cctttgggcc cgtgtggaaa g          591
<210> 57
   <211> 481
   <212> DNA
   <213> Homo sapiens
<400> 57
   aaacattgag atggaatgat agggtttccc agaatcaggt ccatatttta actaaatgaa 60
   aattatgatt tatagccttc tcaaatacct gccatacttg atatctcaac cagagctaat 120
   tttacctctt tacaaattaa ataagcaagt aactggatcc acaatttata atacctgtca 180
   attttttctg tattaaacct ctatcatagt ttaagcctat tagggtactt aatccttaca 240
   aataaacagg tttaaaatca cctcaatagg caactgccct tctggttttc ttctttgact 300
   aaacaatctg aatgcttaag attttccact ttgggtgcta gcagtacaca gtgttacact 360
   ctgtattcca gacttcttaa attatagaaa aaggaatgta cactttttgt attctttctg 420
   agcagggccg ggaggcaaca tcatctacca tggtagggac ttgtatgcat ggactacttt 480
   a                                                                 481
<210> 58
   <211> 141
   <212> DNA
   <213> Homo sapiens
<400> 58
   actctgtcgc ccaggctgga gcccabtggm gcgatctcga ctccctgcaa gctmcgcctc 60
   acaggwtcat gccattctcc tgcctcagca tctggagtag ctgggactac aggcgccagc 120
   caccatgccc agctaatttt t                                           141
<210> 59
   <211> 191
   <212> DNA
   <213> Homo sapiens
<400> 59
   accttaaaga cataggagaa tttatactgg gagagaaagc ttacaaatgt aaggtttctg 60
   acaagacttg ggagtgattc acacctggaa caacatactg gacttcacac tggabagaaa 120
   ccttacaagt gtaatgagtg tggcaaagcc tttggcaagc agtcaacact tattcaccat 180
   caggcaattc a                                                      191
<210> 60
   <211> 480
   <212> DNA
   <213> Homo sapiens
<400> 60
   agtcaggatc atgatggctc agtttcccac agcgatgaat ggagggccaa atatgtgggc 60
   tattacatct gaagaacgta ctaagcatga taaacagttt gataacctca aaccttcagg 120
   aggttacata acaggtgatc aagcccgtac ttttttccta cagtcaggtc tgccggcccc 180
   ggttttagct gaaatatggg ccttatcaga tctgaacaag gatgggaaga tggaccagca 240
   agagttctct atagctatga aactcatcaa gttaaagttg cagggccaac agctgcctgt 300
   agtcctccct cctatcatga aacaaccccc tatgttctct ccactaatct ctgctcgttt 360
   tgggatggga agcatgccca atctgtccat tcatcagcca ttgcctccag ttgcacctat 420
   agcaacaccc ttgtcttctg ctacttcagg gaccagtatt cctccctaat gatgcctgct 480
<210> 61
   <211> 381
   <212> DNA
   <213> Homo sapiens
<400> 61
   ctttcgattt ccttcaattt gtcacgtttg attttatgaa gttgttcaag ggctaactgc 60
   tgtgtattat agctttctct gagttccttc agctgattgt taaatgaatc catttctgag 120
   agcttagatg cagtttcttt ttcaagagca tctaattgtt ctttaagtct ttggcataat 180
   tcttcctttt ctgatgactt tctatgaagt aaactgatcc ctgaatcagg tgtgttactg 240
   agctgcatgt ttttaattct ttcgtttaat agctgcttct cagggaccag atagataagc 300
   ttattttgat attccttaag ctcttggtga agttgttcga tttccataat ttccaggtca 360
   cactggttat cccaaacttc t                                          381
<210> 62
   <211> 906
   <212> DNA
   <213> Homo sapiens
<400> 62
   gtggaggtga aacggaggca agaaaggggg ctacctcagg agcgagggac aaagggggcg 60
   tgaggcacct aggccgcggc accccggcga caggaagccg tcctgaaccg ggctaccggg 120
   taggggaagg gcccgcgtag tcctcgcagg gccccagagc tggagtcggc tccacagccc 180
   cgggccgtcg gcttctcact tcctggacct ccccggcgcc cgggcctgag gactggctcg 240
   gcggagggag aagaggaaac agacttgagc agctccccgt tgtctcgcaa ctccactgcc 300
   gaggaactct catttcttcc ctcgctcctt caccccccac ctcatgtaga aaggtgctga 360
   agcgtccgga gggaagaaga acctgggcta ccgtcctggc cttcccmccc ccttcccggg 420
   gcgctttggt gggcgtggag ttggggttgg gggggtgggt gggggttctt ttttggagtg 480
   ctggggaact tttttccctt cttcaggtca ggggaaaggg aatgcccaat tcagagagac 540
   atgggggcaa gaaggacggg agtggaggag cttctggaac tttgcagccg tcatcgggag 600
   gcggcagctc taacagcaga gagcgtcacc gcttggtatc gaagcacaag cggcataagt 660
   ccaaacactc caaagacatg gggttggtga cccccgaagc agcatccctg ggcacagtta 720
   tcaaaccttt ggtggagtat gatgatatca gctctgattc cgacaccttc tccgatgaca 780
   tggccttcaa actagaccga agggagaacg acgaacgtcg tggatcagat cggagcgacc 840
   gcctgcacaa acatcgtcac caccagcaca ggcgttcccg ggacttacta aaagctaaac 900
   agaccg                                                           906

<210> 63
   <211> 491
   <212> DNA
   <213> Homo sapiens
<400> 63
   gacatgtttg cctgcagggg accagagaca atgggattag ccagtgctca ctgttcttta 60
   tgcttccaga gaggatgggg acagctctca ggtcagaatc caggctgaga aggccatgct 120
   ggttgggggc ccccggaagc acggtccgga tcctccctgg catcagcgta gacccgctgc 180
   tcaggcttgg ggtaccaaac tcatgctctg tactgttttg gccccatgcg gtgagaggaa 240
   aacctagaaa aagattggtc gtgctaagga atcagctgcc ccctcatcct ccgcatccaa 300
   tgctggtgac aacatattcc ctctcccagg acacagactc ggtgactcca cactgggctg 360
   agtggcctct ggaggctcgt ggcctaaggc agggctccgt aaggctgatc ggctgaactg 420
   ggtggggtga gggtttctga cccttcgctt cccatcccat aaccgctgtc aatgagctca 480
   cactgtggtc a 491
<210> 64
   <211> 511
   <212> DNA
   <213> Homo sapiens
<400> 64
   gatggcatgg tcgttgctaa tgtgcctgct gggatggagc acttcctcct gtgagcccag 60
   gggacccgcc tgtccctgga gcttggggca aggagggaag agtgatacca ggaaggtggg 120
   gctgcagcca ggggccagag tcagttcagg gagtggtcct cggccctcaa agctcctccg 180
   gggactgctc aggagtgatg gtgccctgga gtttgcccca acttccctgg ccaccctgga 240
   aggtgcctgg ctgctccagg cctctaggct gggctgatgg gtttctccag gacacaagta 300
   tcattaaagc caccctctcc tcagcttgtc aggccgcaca tgtgggacag gctgtgctca 360
   caaccccctc gcctgccctg ccctccatca ggaggagcca gtggaacctt cggaaagctc 420
   ccagcatctc agcagccctc aaaagtcgtc ctggggcaag ctctggttct cctgactgga 480
   ggtcatctgg gcttggcctg ctctctctcg c 511
<210> 65
   <211> 394
   <212> DNA
   <213> Homo sapiens
<400> 65
   taaaaaagtg taacaaaggt ttatttagac tttcttcatg cccccagatc caggatgtct 60
   atgtaaaccg ttatcttaca aagaaagcac aatatttggt ataaactaag tcagtgactt 120
   gcttaactga aatagcgtcc atccaaaagt gggtttaagg taaaactacc tgacgatatt 180
   ggcggggatc ctgcagtttg gactgcttgc cgggtttgtc cagggttccg ggtctgttct 240
   tggcactcat ggggacaggc atcctgctcg tctgtggggc cccgctggag cccttacgtg 300
   aagctgaagg tatcgaccst agggggctct agggcagtgg gaccttcatc cggaactaac 360
   aagggtcggg gagaggcctc ttgggctatg tggg 394
<210> 66
   <211> 359
   <212> DNA
   <213> Homo sapiens
<400> 66
   caagcgttcc tttatggatg taaattcaaa cagtcatgct gagccatccc gggctgacag 60
   tcacgttwaa gacactaggt cgggcgccac agtgccaccc aaggagaaga agaatttgga 120
   atttttccat gaagatgtac ggaaatctga tgttgaatat gaaaatggcc cccaaatgga 180
   attccaaaag gttaccacag gggctgtaag acctagtgac cctcctaagt gggaaagagg 240
   aatggagaat agtatttctg atgcatcaag aacatcagaa tataaaactg agatcataat 300
   gaaggaaaat tccatatcca atatgagttt actcagagac agtagaaact attcccagg  359
<210> 67
   <211> 450
   <212> DNA
   <213> Homo sapiens
<220>
   <221> misc_feature
   <222> 425
   <223> n = A, T, C or G
<400> 67
   taggaataac aaatgtttat tcagaaatgg ataagtaata cataatcacc cttcatctct 60
   taatgcccct tcctctcctt ctgcacagga gacacagatg ggtaacatag aggcatggga 120
   agtggaggag gacacaggac tagcccacca ccttctcttc ccggtctccc aagatgactg 180
   cttatagagt ggaggaggca aacaggtccc ctcaatgtac cagatggtca cctatagcac 240
   cagctccaga tggccacgtg gttgcagctg gactcaatga aactctgtga caaccagaag 300
   atacctgctt tgggatgaga gggaggataa agccatgcag ggaggatatt taccatccct 360
   accctaagca cagtgcaagc agtgagcccc cggctcccag tacctgaaaa accaaggcct 420
   actgnctttt ggatgctctc ttgggccacg                                  450
<210> 68
   <211> 511
   <212> DNA
   <213> Homo sapiens
<400> 68
   aagcctcctg ccctggaaat ctggagcccc ttggagctga gctggacggg gcagggaggg 60
   gctgagaggc aagaccgtct ccctcctgct gcagctgctt ccccagcagc cactgctggg 120
   cacagcagaa acgccagcag agaaaatggg agccgagagt ccttagccct ggagctgagg 180
   ctgcctctgg gctgacccgc tggctgtacg tggccagaac tggggttggc atctggcatc 240
   catttgaggc cagggtggag gaaagggagg ccaacagagg aaaacctatt cctgctgtga 300
   caacacagcc cttgtcccac gcagcctaag tgcagggagc gtgatgaagt caggcagcca 360
   gtcggggagg acgaggtaac tcagcagcaa tgtcaccttg tagcctatgc gctcaatggc 420
   ccggaggggc agcaaccccc cgcacacgtc agccaacagc agtgcctctg caggcaccaa 480
   gagagcgatg atggacttga gcgccgtgtt c                                511
<210> 69
   <211> 511
   <212> DNA
   <213> Homo sapiens
<400> 69
   gtttggcaga agacatgttt aataacattt tcatatttaa aaaatacagc aacaattctc 60
   tatctgtcca ccatcttgcc ttgcccttcc tggggctgag gcagacaaag gaaaggtaat 120
   gaggttaggg cccccaggcg ggctaagtgc tattggcctg ctcctgctca aagagagcca 180
   tagccagctg ggcacggccc cctagcccct ccaggttgct gaggcggcag cggtggtaga 240
   gttcttcact gagccgtggg ctgcagtctc gcagggagaa cttctgcacc agccctggct 300
   ctacggcccg aaagaggtgg agccctgaga accggaggaa aacatccatc acctccagcc 360
   cctccagggc ttcctcctct tcctggcctg ccagttcacc tgccagccgg gctcgggccg 420
   ccaggtagtc agcgttgtag aagcagccct ccgcagaagc ctgccggtca aatctccccg 480
   ctataggagc cccccgggag gggtcagcac c                                511
<210> 70
   <211> 511
   <212> DNA
   <213> Homo sapiens
<400> 70
   caagttgaac gtcaggcttg gcagaggtgg agtgtagatg aaaacaaagg tgtgattatg 60
   aagaggatgt gagtcctttg ggtgtaggag agaaaggctg ttgagcttct atttcaagat 120
   acttttacct gtgcaaaaag cacattttcc acctccttct catggcattt gtgtaaggtg 180
   agtatgattc ctattccatc tgcattttag aggtgaagaa taacgtacaa gggattcagt 240
   gattagcaag ggacccctca ctaagtgttg atggagttag gacagagctc agctgtttga 300
   atctcagagc ccaggcagct ggagctgggt aggatcctgg agctggcact aatgtgaggt 360
   gcattccctc caacccaggc tcagatccgg aacctgaccg tgctgacccc cgaaggggag 420
   gcagggctga gctggcccgt tgggctccct gctcctttca caccacactc tcgctttgag 480
   gtgctgggct gggactactt cacagagcag c                                511
<210> 71
   <211> 511
   <212> DNA
   <213> Homo sapiens
<400> 71
   tggcctgggc aggattggga gagaggtagc tacccggatg cagtcctttg ggatgaagac 60
   tatagggtat gaccccatca tttccccaga ggtctcggcc tcctttggtg ttcagcagct 120
   gcccctggag gagatctggc ctctctgtga tttcatcact gtgcacactc ctctcctgcc 180
   ctccacgaca ggcttgctga atgacaacac ctttgcccag tgcaagaagg gggtgcgtgt 240
   ggtgaactgt gcccgtggag ggatcgtgga cgaaggcgcc ctgctccggg ccctgcagtc 300
   tggccagtgt gccggggctg cactggacgt gtttacggaa gagccgccac gggaccgggc 360
   cttggtggac catgagaatg tcatcagctg tccccacctg ggtgccagca ccaaggaggc 420
   tcagagccgc tgtggggagg aaattgctgt tcagttcgtg gacatggtga aggggaaatc 480
   tctcacgggg gttgtgaatg cccaggccct t                                511
<210> 72
   <211> 2017
   <212> DNA
   <213> Homo sapiens
<400> 72
   agccagatgg ctgagagctg caagaagaag tcaggatcat gatggctcag tttcccacag 60
   cgatgaatgg agggccaaat atgtgggcta ttacatctga agaacgtact aagcatgata 120
   aacagtttga taacctcaaa ccttcaggag gttacataac aggtgatcaa gcccgtactt 180
   ttttcctaca gtcaggtctg ccggccccgg ttttagctga aatatgggcc ttatcagatc 240
   tgaacaagga tgggaagatg gaccagcaag agttctctat agctatgaaa ctcatcaagt 300
   taaagttgca gggccaacag ctgcctgtag tcctccctcc tatcatgaaa caacccccta 360
   tgttctctcc actaatctct gctcgttttg ggatgggaag catgcccaat ctgtccattc 420
   atcagccatt gcctccagtt gcacctatag caacaccctt gtcttctgct acttcaggga 480
   ccagtattcc tcccctaatg atgcctgctc ccctagtgcc ttctgttagt acatcctcat 540
   taccaaatgg aactgccagt ctcattcagc ctttatccat tccttattct tcttcaacat 600
   tgcctcatgc atcatcttac agcctgatga tgggaggatt tggtggtgct agtatccaga 660
   aggcccagtc tctgattgat ttaggatcta gtagctcaac ttcctcaact gcttccctct 720
   cagggaactc acctaagaca gggacctcag agtgggcagt tcctcagcct tcaagattaa 780
   agtatcggca aaaatttaat agtctagaca aaggcatgag cggatacctc tcaggttttc 840
   aagctagaaa tgcccttctt cagtcaaatc tctctcaaac tcagctagct actatttgga 900
   ctctggctga catcgatggt gacggacagt tgaaagctga agaatttatt ctggcgatgc 960
   acctcactga catggccaaa gctggacagc cactaccact gacgttgcct cccgagcttg 1020
   tccctccatc tttcagaggg ggaaagcaag ttgattctgt taatggaact ctgccttcat 1080
   atcagaaaac acaagaagaa gagcctcaga agaaactgcc agttactttt gaggacaaac 1140
   ggaaagccaa ctatgaacga ggaaacatgg agctggagaa gcgacgccaa gtgttgatgg 1200
   agcagcagca gagggaggct gaacgcaaag cccagaaaga gaaggaagag tgggagcgga 1260
   aacagagaga actgcaagag caagaatgga agaagcagct ggagttggag aaacgcttgg 1320
   agaaacagag agagctggag agacagcggg aggaagagag gagaaaggag atagaaagac 1380
   gagaggcagc aaaacaggag cttgagagac aacgccgttt agaatgggaa agactccgtc 1440
   ggcaggagct gctcagtcag aagaccaggg aacaagaaga cattgtcagg ctgagctcca 1500
   gaaagaaaag tctccacctg gaactggaag cagtgaatgg aaaacatcag cagatctcag 1560
   gcagactaca agatgtccaa atcagaaagc aaacacaaaa gactgagcta gaagttttgg 1620
   ataaacagtg tgacctggaa attatggaaa tcaaacaact tcaacaagag cttaaggaat 1680
   atcaaaataa gcttatctat ctggtccctg agaagcagct attaaacgaa agaattaaaa 1740
   acatgcagct cagtaacaca cctgattcag ggatcagttt acttcataaa aagtcatcag 1800
   aaaaggaaga attatgccaa agacttaaag aacaattaga tgctcttgaa aaagaaactg 1860
   catctaagct ctcagaaatg gattcattta acaatcagct gaaggaactc agagaaagct 1920
   ataatacaca gcagttagcc cttgaacaac ttcataaaat caaacgtgac aaattgaagg 1980
   aaatcgaaag aaaaagatta gagcaaaaaa aaaaaaa                          2017

<210> 73
   <211> 414
   <212> DNA
   <213> Homo sapiens
<400> 73
   atggcagtga cattcaccat catgggaacc accttccctt ttcttcagga ttctctgtag 60
   tggaagagag cacccagtgt tgggctgaaa acatctgaaa gtagggagaa gaacctaaaa 120
   taatcagtat ctcagagggc tctaaggtgc caagaagtct cactggacat ttaagtgcca 180
   acaaaggcat actttcggaa tcgccaagtc aaaactttct aacttctgtc tctctcagag 240
   acaagtgaga ctcaagagtc tactgcttta gtggcaacta cagaaaactg gtgttaccca 300
   gaaaaacagg agcaattaga aatggttcca atatttcaaa gctccgcaaa caggatgtgc 360
   tttcctttgc ccatttaggg tttcttctct ttcctttctc tttattaacc acta       414
<210> 74
   <211> 1567
   <212> DNA
   <213> Homo sapiens
<400> 74
   atatctagaa gtctggagtg agcaaacaag agcaagaaac aaaaagaagc caaaagcaga 60
   aggctccaat atgaacaaga taaatctatc ttcaaagaca tattagaagt tgggaaaata 120
   attcatgtga actagacaag tgtgttaaga gtgataagta aaatgcacgt ggagacaagt 180
   gcatccccag atctcaggga cctccccctg cctgtcacct ggggagtgag aggacaggat 240
   agtgcatgtt ctttgtctct gaatttttag ttatatgtgc tgtaatgttg ctctgaggaa 300
   gcccctggaa agtctatccc aacatatcca catcttatat tccacaaatt aagctgtagt 360
   atgtacccta agacgctgct aattgactgc cacttcgcaa ctcaggggcg gctgcatttt 420
   agtaatgggt caaatgattc actttttatg atgcttccaa aggtgccttg gcttctcttc 480
   ccaactgaca aatgccaaag ttgagaaaaa tgatcataat tttagcataa acagagcagt 540
   cggcgacacc gattttataa ataaactgag caccttcttt ttaaacaaac aaatgcgggt 600
   ttatttctca gatgatgttc atccgtgaat ggtccaggga aggacctttc accttgacta 660
   tatggcatta tgtcatcaca agctctgagg cttctccttt ccatcctgcg tggacagcta 720
   agacctcagt tttcaatagc atctagagca gtgggactca gctggggtga tttcgccccc 780
   catctccggg ggaatgtctg aagacaattt tgttacctca atgagggagt ggaggaggat 840
   acagtgctac taccaactag tggataaagg ccagggatgc tgctcaacct cctaccatgt 900
   acaggacgtc tccccattac aactacccaa tccgaagtgt caactgtgtc aggactaaga 960
   aaccctggtt ttgagtagaa aagggcctgg aaagagggga gccaacaaat ctgtctgctt 1020
   cctcacatta gtcattggca aataagcatt ctgtctcttt ggctgctgcc tcagcacaga 1080
   gagccagaac tctatcgggc accaggataa catctctcag tgaacagagt tgacaaggcc 1140
   tatgggaaat gcctgatggg attatcttca gcttgttgag cttctaagtt tctttccctt 1200
   cattctaccc tgcaagccaa gttctgtaag agaaatgcct gagttctagc tcaggttttc 1260
   ttactctgaa tttagatctc cagacccttc ctggccacaa ttcaaattaa ggcaacaaac 1320
   atataccttc catgaagcac acacagactt ttgaaagcaa ggacaatgac tgcttgaatt 1380
   gaggccttga ggaatgaagc tttgaaggaa aagaatactt tgtttccagc cccttcccca 1440
   cactcttcat gtgttaacca ctgccttcct ggaccttgga gccacggtga ctgtattaca 1500
   tgttgttata gaaaactgat tttagagttc tgatcgttca agagaatgat taaatataca 1560
   tttccta                                                           1567
<210> 75
   <211> 240
   <212> DNA
   <213> Homo sapiens
<400> 75
   tcgagcggcc gcccgggcag gtccttcaga cttggactgt gtcacactgc caggcttcca 60
   gggctccaac ttgcagacgg cctgttgtgg gacagtctct gtaatcgcga aagcaaccat 120
   ggaagacctg ggggaaaaca ccatggtttt atccaccctg agatctttga acaacttcat 180
   ctctcagcgt gcggagggag gctctggact ggatatttct acctcggccg cgaccacgct 240
<210> 76
   <211> 330
   <212> DNA
   <213> Homo sapiens
<220>
   <221> misc_feature
   <222> 288
   <223> n = A,T,C or G
<400> 76
   tagcgyggtc gcggccgagg yctgcttytc tgtccagccc agggcctgtg gggtcagggc 60
   ggtgggtgca gatggcatcc actccggtgg cttccccatc tttctctggc ctgagcaagg 120
   tcagcctgca gccagagtac agagggccaa cactggtgtt cttgaacaag ggccttagca 180
   ggccctgaag grccctctct gtagtgttga acttcctgga gccaggccac atgttctcct 240
   cataccgcag gytagygatg gtgaagttga gggtgaaata gtattmangr agatggctgg 300
   caracctgcc cgggcggccg ctcsaaatcc                                  330
<210> 77
   <211> 361
   <212> DNA
   <213> Homo sapiens
<400> 77
   agcgtggtcg cggccgaggt gtccttcagg gtctgcttat gcccttgttc aagaacacca 60
   gtgtcagctc tctgtactct ggttgcagac tgaccttgct caggcctgag aaggatgggg 120
   cagccaccag agtggatgct gtctgcaccc atcgtcctga ccccaaaagc cctggactgg 180
   acagagagcg gctgtactgg aagctgagcc agctgaccca cggcatcact gagctgggcc 240
   cctacaccct ggacagggac agtctctatg tcaatggttt cacccatcgg agctctgtac 300
   ccaccaccag caccggggtg gtcagcgagg agccattcaa cctgcccggg cggccgctcg 360
   a                                                                 361
<210> 78
   <211> 356
   <212> DNA
   <213> Homo sapiens
<220>
   <221> mise feature
   <222> 7, 346, 350, 353
   <223> n = A,T,C or G
<400> 78
   ttggggnttt mgagcggccg cccgggcagg taccggggtg gtcagcgagg agccattcac 60
   actgaacttc accatcaaca acctgcggta tgaggagaac atgcagcacc ctggctccag 120
   gaagttcaac accacggaga gggtccttca gggcctgctc aggtccctgt tcaagagcac 180
   cagtgttggc cctctgtact ctggctgcag actgactttg ctcagacttg agaaacatgg 240
   ggcagccact ggagtggacg ccatctgcac cctccgcctt gatcccactg gtcctggact 300
   ggacagagag cggctatact gggagctgag ccagtcctct ggcggngacn ccnctt     356
<210> 79
   <211> 226
   <212> DNA
   <213> Homo sapiens
<400> 79
   agcgtggtcg cggccgaggt ccagtcgcag catgctcttt ctcctgccca ctggcacagt 60
   gaggaagatc tctgctgtca gtgagaaggc tgtcatccac tgagatggca gtcaaaagtg 120
   catttaatac acctaacgta tcgaacatca tagcttggcc caggttatct catatgtgct 180
   cagaacactt acaatagcct gcagacctgc ccgggcggcc gctcga                226
<210> 80
   <211> 444
   <212> DNA
   <213> Homo sapiens
<220>
   <221> misc_feature
   <222> 23
   <223> n = A,T,C or G
<400> 80
   tgtggtgttg aacttcctgg agncagggtg acccatgtcc tccccatact gcaggttggt 60
   gatggtgaag ttgagggtga atggtaccag gagagggcca gcagccataa ttgtsgrgck 120
   gsmgmssgag gmwggwgtyy cwgaggttcy rarrtccact gtggaggtcc caggagtgct 180
   ggtggtgggc acagagstcy gatgggtgaa accattgaca tagagactgt tcctgtccag 240
   ggtgtagggg cccagctctt yratgycatt ggycagttkg ctyagctccc agtacagccr 300
   ctctckgyyg mgwccagsgc ttttggggtc aagatgatgg atgcagatgg catccactcc 360
   agtggctgct ccatccttct cggacctgag agaggtcagt ctgcagccag agtacagagg 420
   gccaacactg gtgttctttg aata                                        444
<210> 81
   <211> 310
   <212> DNA
   <213> Homo sapiens
<400> 81
   tcgagcggcc gcccgggcag gtcaggaagc acattggtct tagagccact gcctcctgga 60
   ttccacctgt gctgcggaca tctccaggga gtgcagaagg gaagcaggtc aaactgctca 120
   gatcagtcag actggctgtt ctcagttctc acctgagcaa ggtcagtctg cagccagagt 180
   acagagggcc aacactggtg ttcttgaaca agggcttgag cagaccctgc agaaccctct 240
   tccgtggtgt tgaacttcct ggaaaccagg gtgttgcatg tttttcctca taatgcaagg 300
   ttggtgatgg                                                        310
<210> 82
   <211> 571
   <212> DNA
   <213> Homo sapiens
<220>
   <221> misc_feature
   <222> 202
   <223> n = A,T,C or G
<400> 82
   acggtttcaa tggacacttt tattgtttac ttaatggatc atcaattttg tctcactacc 60
   tacaaatgga atttcatctt gtttccatgc tgagtagtga aacagtgaca aagctaatca 120
   taataaccta catcaaaaga gaactaagct aacactgctc actttctttt taacaggcaa 180
   aatataaata tatgcactct anaatgcaca atggtttagt cactaaaaaa ttcaaatggg 240
   atcttgaaga atgtatgcaa atccagggtg cagtgaagat gagctgagat gctgtgcaac 300
   tgtttaaggg ttcctggcac tgcatctctt ggccactagc tgaatcttga catggaaggt 360
   tttagctaat gccaagtgga gatgcagaaa atgctaagtt gacttagggg ctgtgcacag 420
   gaactaaaag gcaggaaagt actaaatatt gctgagagca tccaccccag gaaggacttt 480
   accttccagg agctccaaac tggcaccacc cccagtgctc acatggctga ctttatcctc 540
   cgtgttccat ttggcacagc aagtggcagt g 571
<210> 83
   <211> 551
   <212> DNA
   <213> Homo sapiens
<400> 83
   aaggctggtg ggtttttgat cctgctggag aacctccgct ttcatgtgga ggaagaaggg 60
   aagggaaaag atgcttctgg gaacaaggtt aaagccgagc cagccaaaat agaagctttc 120
   cgagcttcac tttccaagct aggggatgtc tatgtcaatg atgcttttgg cactgctcac 180
   agagcccaca gctccatggt aggagtcaat ctgccacaga aggctggtgg gtttttgatg 240
   aagaaggagc tgaactactt tgcaaaggcc ttggagagcc cagagcgacc cttcctggcc 300
   atcctgggcg gagctaaagt tgcagacaag atccagctca tcaataatat gctggacaaa 360
   gtcaatgaga tgattattgg tggtggaatg gcttttacct tccttaaggt gctcaacaac 420
   atggagattg gcacttctct gtttgatgaa gagggagcca agattgtcaa agacctaatg 480
   tccaaagctg agaagaatgg tgtgaagatt accttgcctg ttgactttgt cactgctgac 540
   aagtttgatg a 551
<210> 84
   <211> 571
   <212> DNA
   <213> Homo sapiens
<400> 84
   tttgttcctt acatttttct aaagagttac ttaaatcagt caactggtct ttgagactct 60
   taagttctga ttccaactta gctaattcat tctgagaact gtggtatagg tggcgtgtct 120
   cttctagctg ggacaaaagt tctttgtttt ccccctgtag agtatcacag accttctgct 180
   gaagctggac ctctgtctgg gccttggact cccaaatctg cttgtcatgt tcaagcctgg 240
   aaatgttaat ctttaattct tccatatgga tggacatctg tctaagttga tcctttagaa 300
   cactgcaatt atcttctttg agtctaattt cttcttcttt gctttgaatc gcatcactaa 360
   acttcctctc ccatttctta gcttcatcta tcaccctgtc acgatcatcc tggagggaag 420
   acatgctctt agtaaaggct gcaagctggg tcacagtact gtccaagttt tcctgaagtt 480
   gctgaacttc cttgtctttc ttgttcaaag taacctgaat ctctccaatt gtctcttcca 540
   agtggacttt ttctctgcgc aaagcatcca g 571
<210> 85
   <211> 561
   <212> DNA
   <213> Homo sapiens
<400> 85
   tcattgcctg tgatggcatc tggaatgtga tgagcagcca ggaagttgta gatttcattc 60
   aatcaaagga ttcagcatgt ggtggaagct gtgaggcaag agaaacaaga actgtatggc 120
   aagttaagaa gcacagaggc aaacaagaag gagacagaaa agcagttgca ggaagctgag 180
   caagaaatgg aggaaatgaa agaaaagatg agaaagtttg ctaaatctaa acagcagaaa 240
   atcctagagc tggaagaaga gaatgaccgg cttagggcag aggtgcaccc tgcaggagat 300
   acagctaaag agtgtatgga aacacttctt tcttccaatg ccagcatgaa ggaagaactt 360
   gaaagggtca aaatggagta tgaaaccctt tctaagaagt ttcagtcttt aatgtctgag 420
   aaagactctc taagtgaaga ggttcaagat ttaaagcatc agatagaagg taatgtatct 480
   aaacaagcta acctagaggc caccgagaaa catgataacc aaacgaatgt cactgaagag 540
   ggaacacagt ctataccagg t                                           561

<210> 86
   <211> 795
   <212> DNA
   <213> Homo sapiens
<400> 86
   aagccaataa tcaccattta ttacttaata tatgccaacc actgtacttg gcagttcaca 60
   aattctcacc gttacaacaa ccccatgagg tatttattcc cattctatag atagggaaac 120
   cacagctcaa gtaagttagg aaactgagcc aagtatacac agaatacgaa gtggcaaaac 180
   tagaaggaaa gactgacact gctatctgct ggcctccagt gtcctggctc ttttcacacg 240
   ggttcaatgt ctccagcgct gctgctgctg ctgcattacc atgccctcat tgtttttctt 300
   cctctggtgt tcaactgcat ccttcaaaga atctaactca ttccagagac cacttatttc 360
   tttctctctt tctgaaatta cttttaataa ttcttcatga gggggaaaag aagatgcctg 420
   ttggtagttt tgttgtttaa gctgctcaat ttgggactta aacaatttgt tttcatcttg 480
   tacatcctgt aacagctgtg ttttgctaga aagatcactc tccctctctt ttagcatggc 540
   ttctaacctc ttcaattcat tttccttttc tttcaacaca atctcaagtt cttcaaactg 600
   tgatgcagaa gaggcctctt tcaagttatg ttgtgctact tcctgaacat gtgcttttaa 660
   agattcattt tcttcttgaa gatcctgtaa ccacttccct gtattggcta ggtctttctc 720
   tttctcttcc aaaacagcct tcatggtatt catctgttcc tcttttcctt ttaataagtt 780
   caggagcttc agaac                                                  795
<210> 87
   <211> 594
   <212> DNA
   <213> Homo sapiens
<400> 87
   caagcttttt tttttttttt aaaaagtgtt agcattaatg ttttattgtc acgcagatgg 60
   caactgggtt tatgtcttca tattttatat ttttgtaaat taaaaaaatt acaagtttta 120
   aatagccaat ggctggttat attttcagaa aacatgatta gactaattca ttaatggtgg 180
   cttcaagctt ttccttattg gctccagaaa attcacccac cttttgtccc ttcttaaaaa 240
   actggaatgt tggcatgcat ttgacttcac actctgaagc aacatcctga cagtcatcca 300
   catctacttc aaggaatatc acgttggaat acttttcaga gagggaatga aagaaaggct 360
   tgatcatttt gcaaggccca caccacgtgg ctgagaagtc aactactaca agtttatcac 420
   ctgcagcgtc caaggcttcc tgaaaagcag tcttgctctc gatctgcttc accatcttgg 480
   ctgctggagt ctgacgagcg gctgtaagga ccgatggaaa tggatccaaa gcaccaaaca 540
   gagcttcaag actcgctgct tggcttgaat tcggatccga tatcgccatg gcct       594
<210> 88
   <211> 557
   <212> DNA
   <213> Homo sapiens
<400> 88
   aagtgttagc attaatgttt tattgtcacg cagatggcaa ctgggtttat gtcttcatat 60
   tttatatttt tgtaaattaa aaaaattmca agttttaaat agccaatggc tggttatatt 120
   ttcagaaaac atgattagac taattcatta atggtggctt caagcttttc cttattggct 180
   ccagaaaatt cacccacctt ttgtcccttc ttaaaaaact ggaatgttgg catgcatttg 240
   acttcacact ctgaagcaac atcctgacag tcatccacat ctacttcaag gaatatcacg 300
   ttggaatact tttcagagag ggaatgaaag aaaggcttga tcattttgca aggcccacac 360
   cacgtggctg agaagtcaac tactacaagt ttatcacctg cagcgtccaa ggcttcctga 420
   aaagcagtct tgctctcgat ctgcttcacc atcttggctg ctggagtctg acgagcggct 480
   gtaaggaccg atggaaatgg atccaaagca ccaaacagag cttcaagact cgctgcttgg 540
   catgaattcg gatccga                                                557
<210> 89
   <211> 561
   <212> DNA
   <213> Homo sapiens
<220>
   <221> misc_feature
   <222> 544, 551
   <223> n = A,T,C or G
<400> 89
   tacaaacttt attgaaacgc acacgcgcac acacacaaac acccctgtgg atagggaaaa 60
   gcacctggcc acagggtcca ctgaaacggg gaggggatgg cagcttgtaa tgtggctttt 120
   gccacaaccc ccttctgaca gggaaggcct tagattgagg ccccacctcc catggtgatg 180
   gggagctcag aatggggtcc agggagaatt tggttagggg gaggtgctag ggaggcatga 240
   gcagagggca ccctccgagt ggggtcccga gggctgcaga gtcttcagta ctgtccctca 300
   cagcagctgt ctcaaggctg ggtccctcaa aggggcgtcc cagcgcgggg cctccctgcg 360
   caaacacttg gtacccctgg ctgcgcagcg gaagccagca ggacagcagt ggcgccgatc 420
   agcacaacag acgccctggc ggtagggaca gcaggcccag ccctgtcggt tgtctcggca 480
   gcaggtctgg ttatcatggc agaagtgtcc ttcccacact tcacgtcctt cacacccacg 540
   tganggctac nggccaggaa g                                           561
<210> 90
   <211> 561
   <212> DNA
   <213> Homo sapiens
<400> 90
   cccgtgggtg ccatccacgg agttgttacc tgatctttgg aagcaggatc gcccgtctgc 60
   actgcagtgg aagccccgtg ggcagcagtg atggccatcc ccgcatgcca cggcctctgg 120
   gaaggggcag caactggaag tccctgagac ggtaaagatg caggagtggc cggcagagca 180
   gtgggcatca acctggcagg ggccacccag atgcctgctc agtgttgtgg gccatttgtc 240
   cagaagggga cggcagcagc tgtagctggc tcctccgggg tccaggcagc aggccacagg 300
   gcagaactga ccatctgggc accgcgttcc agccaccagc cctgctgtta aggccaccca 360
   gctcaccagg gtccacatgg tctgcctgcg tccgactccg cggtccttgg gccctgatgg 420
   ttctacctgc tgtgagctgc ccagtgggaa gtatggctgc tgccaatgcc caacgccacc 480
   tgctgctccg atcacctgca ctgctgcccc aagacactgt gtgtgacctg atccagagta 540
   agtgcctctc caaggagaac g                                           561
<210> 91
   <211> 541
   <212> DNA
   <213> Homo sapiens
<220>
   <221> misc_feature
   <222> 480, 491
   <223> n = A,T,C or G
<400> 91
   gaatcacctt tctggtttag ctagtacttt gtacagaaca atgaggtttc ccacagcgga 60
   gtctccctgg gctctgtttg gctctcggta aggcaggcct acaccttttc ctctcctcta 120
   tggagagggg aatatgcatt aaggtgaaaa gtcaccttcc aaaagtgaga aagggattcg 180
   attgctgctt caggactgtg gaattatttg gaatgtttta caaatggttg ctacaaaaca 240
   acaaaaaagg taattacaaa atgtgtacat cacaacatgc tttttaaaga cattatgcat 300
   tgtgctcaca ttcccttaaa tgttgtttcc aaaggtgctc agcctctagc ccagctggat 360
   tctccgggaa gaggcagaga cagtttggcg aaaaagacac agggaaggag ggggtggtga 420
   aaggagaaag cagccttcca gttaaagatc agccctcagt taaaggtcag cttcccgcan 480
   gctggcctca ngcggagtct gggtcagagg gaggagcagc agcagggtgg gactggggcg 540
   t                                                                 541
<210> 92
   <211> 551
   <212> DNA
   <213> Homo sapiens
<400> 92
   aaccggagcg cgagcagtag ctgggtgggc accatggctg ggatcaccac catcgaggcg 60
   gtgaagcgca agatccaggt tctgcagcag caggcagatg atgcagagga gcgagctgag 120
   cgcctccagc gagaagttga gggagaaagg cgggcccggg aacaggctga ggctgaggtg 180
   gcctccttga accgtaggat ccagctggtt gaagaagagc tggaccgtgc tcaggagcgc 240
   ctggccactg ccctgcaaaa gctggaagaa gctgaaaaag ctgctgatga gagtgagaga 300
   ggtatgaagg ttattgaaaa ccgggcctta aaagatgaag aaaagatgga actccaggaa 360
   atccaactca aagaagctaa gcacattgca gaagaggcag ataggaagta tgaagaggtg 420
   gctcgtaagt tggtgatcat tgaaggagac ttggaacgca cagaggaacg agctgagctg 480
   gcagagtccc gttgccgaga gatggatgag cagattagac tgatggacca gaacctgaag 540
   tgtctgagtg c                                                      551
<210> 93
   <211> 531
   <212> DNA
   <213> Homo sapiens
<400> 93
   gagaacttgg cctttattgt gggcccagga gggcacaaag gtcaggaggc ccaagggagg 60
   gatctggttt tctggatagc caggtcatag catgggtatc agtaggaatc cgctgtagct 120
   gcacaggcct cacttgctgc agttccgggg agaacacctg cactgcatgg cgttgatgac 180
   ctcgtggtac acgacagagc cattggtgca gtgcaagggc acgcgcatgg gctccgtcct 240
   cgagggcagg cagcaggagc attgctcctg cacatcctcg atgtcaatgg agtacacagc 300
   tttgctggca cactttccct ggcagtaatg aatgtccact tcctcttggg acttacaatc 360
   tcccactttg atgtactgca ccttggctgt gatgtctttg caatcaggct cctcacatgt 420
   gtcacagcag gtgcctggaa ttttcacgat tttgcctcct tcagccagac acttgtgttc 480
   atcaaatggt gggcagcccg tgaccctctt ctcccagatg tactctcctc t          531
<210> 94
   <211> 531
   <212> DNA
   <213> Homo sapiens
<220>
   <221> misc_feature
   <222> 517
   <223> n = A,T,C or G
<400> 94
   gcctggacct tgccggatca gtgccacaca gtgacttgct tggcaaatgg ccagaccttg 60
   ctgcagagtc atcgtgtcaa ttgtgaccat ggaccccggc cttcatgtgc caacagccag 120
   tctcctgttc gggtggagga gacgtgtggc tgccgctgga cctgcccttg tgtgtgcacg 180
   ggcagttcca ctcggcacat cgtcaccttc gatgggcaga atttcaagct tactggtagc 240
   tgctcctatg tcatctttca aaacaaggag caggacctgg aagtgctcct ccacaatggg 300
   gcctgcagcc ccggggcaaa acaagcctgc atgaagtcca ttgagattaa gcatgctggc 360
   gtctctgctg agctgcacag taacatggag atggcagtgg atgggagact ggtccttgcc 420
   ccgtacgttg gtgaaaacat ggaagtcagc atctacggcg ctatcatgta tgaagtcagg 480
   tttacccatc ttggccacat cctcacatac accgccncaa aacaacgagt t          531
<210> 95
   <211> 605
   <212> DNA
   <213> Homo sapiens
<400> 95
   agatcaacct ctgctggtca ggaggaatgc cttccttgtc ttggatcttt gctttgacgt 60
   tctcgatagt rwcaactkkr ytsramskma agkgyratgr wmttksywgw rasyktmwwm 120
   rsgraraytt agacaycccm cctcwgagac gsagkaccar gtgcagaggt ggactctttc 180
   tggatgttgt agtcagacag ggtgcgtcca tcttccagct gtttcccagc aaagatcaac 240
   ctctgctgat caggagggat gccttcctta tcttggatct ttgccttgac attctcgatg 300
   gtgtcactgg gctccacctc gagggtgatg gtcttaccag tcagggtctt cacgaagaty 360
   tgcatcccac ctctgagacg gagcaccagg tgcagggtrg actctttctg gatgttgtag 420
   tcagacaggg tgcgyccatc ttccagctgc tttccsagca aagatcaacc tctgctggtc 480
   aggaggratg ccttccttgt cytggatctt tgcyttgacr ttctcratgg tgtcactcgg 540
   ctccacttcg agagtgatgg tcttaccagt cagggtcttc acgaagatct gcatcccacc 600
   tctaa                                                             605
<210> 96
   <211> 531
   <212> DNA
   <213> Homo sapiens
<400> 96
   aagtcacaaa cagacaaaga ttattaccag ctgcaagcta tattagaagc tgaacgaaga 60
   gacagaggtc atgattctga gatgattgga gaccttcaag ctcgaattac atctttacaa 120
   gaggaggtga agcatctcaa acataatctc gaaaaagtgg aaggagaaag aaaagaggct 180
   caagacatgc ttaatcactc agaaaaggaa aagaataatt tagagataga tttaaactac 240
   aaacttaaat cattacaaca acggttagaa caagaggtaa atgaacacaa agtaaccaaa 300
   gctcgtttaa ctgacaaaca tcaatctatt gaagaggcaa agtctgtggc aatgtgtgag 360
   atggaaaaaa agctgaaaga agaaagagaa gctcgagaga aggctgaaaa tcgggttgtt 420
   cagattgaga aacagtgttc catgctagac gttgatctga agcaatctca gcagaaacta 480
   gaacatttga ctggaaataa agaaaggatg gaggatgaag ttaagaatct a          531
<210> 97
   <211> 1017
   <212> DNA
   <213> Homo sapiens
<220>
   <221> misc_feature
   <222> 963, 995, 1001, 1008, 1010
   <223> n = A,T,C or G
<400> 97
   cgcctccacc atgtccatca gggtgaccca gaagtcctac aaggtgtcca cctctggccc 60
   ccgggccttc agcagccgct cctacacgag tgggcccggt tcccgcatca gctcctcgag 120
   cttctcccga gtgggcagca gcaactttcg cggtggcctg ggcggcggct atggtggggc 180
   cagcggcatg ggaggcatca ccgcagttac ggtcaaccag agcctgctga gcccccttgt 240
   cctggaggtg gaccccaaca tccaggccgt gcgcacccag gagaaggagc agatcaagac 300
   cctcaacaac aagtttgcct ccttcataga caaggtacgg ttcctggagc agcagaacaa 360
   gatgctggag accaagtgga gcctcctgca gcagcagaag acggctcgaa gcaacatgga 420
   caacatgttc gagagctaca tcaacarcct taggcggcag ctggagactc tgggccagga 480
   gaagctgaag ctggaggcgg agcttggcaa catgcagggg ctggtggagg acttcaagaa 540
   caagtatgag gatgagatca ataagcgtac agagatggag aacgaatttg tcctcatcaa 600
   gaaggatgtg gatgaagctt acatgaacaa ggtagagctg gagtctcgcc tggaagggct 660
   gaccgacgag atcaacttcc tcaggcagct gtatgaagag gagatccggg agctgcagtc 720
   ccagatctcg gacacatctg tggtgctgtc catggacaac agccgctccc tggacatgga 780
   cagcatcatt gctgaggtca aggcacagta cgaggatatt gccaaccgca gccgggctga 840
   ggctgagagc atgtaccagg tcaagtatga ggagctgcag agcctggctg ggaagcacgg 900
   ggatgacctg cggcgcacaa agactgagat ctctgagatg aacccggaac atcagcccgg 960
   ctncaggctg agattgaggg cctcaaaggc caganggctt ncctggangn ccgccat    1017

<210> 98
   <211> 561
   <212> DNA
   <213> Homo sapiens
<400> 98
   cccggagcca gccaacgagc ggaaaatggc agacaatttt tcgctccatg atgcgttatc 60
   tgggtctgga aacccaaacc ctcaaggatg gcctggcgca tgggggaacc agcctgctgg 120
   ggcagggggc tacccagggg cttcctatcc tggggcctac cccgggcagg cacccccagg 180
   ggcttatcct ggacaggcac ctccaggcgc ctaccctgga gcacctggag cttatcccgg 240
   agcacctgca cctggagtct acccagggcc acccagcggc cctggggcct acccatcttc 300
   tggacagcca agtgccaccg gagcctaccc tgccactggc ccctatggcg cccctgctgg 360
   gccactgatt gtgccttata acctgccttt gcctggggga gtggtgcctc gcatgctgat 420
   aacaattctg ggcacggtga agcccaatgc aaacagaatt gctttagatt tccaaagagg 480
   gaatgatgtt gccttccact ttaacccacg cttcaatgag aacaacagga gagtcattgg 540
   ttgcaataca aagctggata a                                           561
<210> 99
   <211> 636
   <212> DNA
   <213> Homo sapiens
<400> 99
   gggaatgcaa caactttatt gaaaggaaag tgcaatgaaa tttgttgaaa ccttaaaagg 60
   ggaaacttag acaccccccc tcragcgmag kaccargtgc araggtggac tctttctgga 120
   tgttgtagtc agacagggtr cgwccatctt ccagctgttt yccrgcaaag atcaacctct 180
   gctgatcagg aggratgcct tccttatctt ggatctttgc cttgacattc tcgatggtgt 240
   cactgggctc cacctcgagg gtgatggtct taccagtcag ggtcttcacg aagatytgca 300
   tcccacctct gagacggagc accaggtgca gggtrgactc tttctggatg ttgtagtcag 360
   acagggtgcg yccatcttcc agctgctttc csagcaaaga tcaacctctg ctggtcagga 420
   ggratgcctt ccttgtcytg gatctttgcy ttgacrttct caatggtgtc actcggctcc 480
   acttcgagag tgatggtctt accagtcagg gtcttcacga agatctgcat cccacctcta 540
   agacggagca ccaggtgcag ggtggactct ttctggatgg ttgtagtcag acagggtgcg 600
   tccatcttcc agctgtttcc cagcaaagat caacct 636
<210> 100
   <211> 697
   <212> DNA
   <213> Homo sapiens
<400> 100
   aggttgatct ttgctgggaa acagctggaa gatggacgca ccctgtctga ctacaaccat 60
   ccagaaagag tccaccctgc acctggtgct ccgtcttaga ggtgggatgc agatcttcgt 120
   gaagaccctg actggtaaga ccatcactct cgaagtggag ccgagtgaca ccattgagaa 180
   ygtcaargca aagatccarg acaaggaagg catycctcct gaccagcaga ggttgatctt 240
   tgctsggaaa gcagctggaa gatggrcgca ccctgtctga ctacaacatc cagaaagagt 300
   cyaccctgca cctggtgctc cgtctcagag gtgggatgca ratcttcgtg aagaccctga 360
   ctggtaagac catcaccctc gaggtggagc ccagtgacac catcgagaat gtcaaggcaa 420
   agatccaaga taaggaaggc atccctcctg atcagcagag gttgatcttt gctgggaaac 480
   agctggaaga tggacgcacc ctgtctgact acaacatcca gaaagagtcc acctytgcac 540
   ytggtmctbc gtctyagagg kgggrtgcaa atctwmgtkw agacactcac tkkyaagryy 600
   atcamcmwtg akktcgakys castkwcact wtcrakaamg tyrwwgcawa gatccmagac 660
   aaggaaggca ttcctcctga ccagcagagg ttgatct 697
<210> 101
   <211> 451
   <212> DNA
   <213> Homo sapiens
<400> 101
   atggagtctc actctgtcga ccaggctgga gcgctgtggt gcgatatcgg ctcactgcag 60
   tctccacttc ctgggttcaa gcgatcctcc tgcctcagcc tcccgagtag ctgggactac 120
   aggcaggcgt caccataatt tttgtatttt tagtagagac atggtttcgc catgttggct 180
   gggctggtct cgaactcctg acctcaagtg atctgtcctg gcctcccaaa gtgttgggat 240
   tacaggcgaa agccaacgct cccggccagg gaacaacttt agaatgaagg aaatatgcaa 300
   aagaacatca catcaaggat caattaatta ccatctatta attactatat gtgggtaatt 360
   atgactattt cccaagcatt ctacgttgac tgcttgagaa gatgtttgtc ctgcatggtg 420
   gagagtggag aagggccagg attcttaggt t 451
<210> 102
   <211> 571
   <212> DNA
   <213> Homo sapiens
<400> 102
   agcgcggtct tccggcgcga gaaagctgaa ggtgatgtgg ccgccctcaa ccgacgcatc 60
   cagctcgttg aggaggagtt ggacagggct caggaacgac tggccacggc cctgcagaag 120
   ctggaggagg cagaaaaagc tgcagatgag agtgagagag gaatgaaggt gatagaaaac 180
   cgggccatga aggatgagga gaagatggag attcaggaga tgcagctcaa agaggccaag 240
   cacattgcgg aagaggctga ccgcaaatac gaggaggtag ctcgtaagct ggtcatcctg 300
   gagggtgagc tggagagggc agaggagcgt gcggaggtgt ctgaactaaa atgtggtgac 360
   ctggaagaag aactcaagaa tgttactaac aatctgaaat ctctggaggc tgcatctgaa 420
   aagtattctg aaaaggagga caaatatgaa gaagaaatta aacttctgtc tgacaaactg 480
   aaagaggctg agacccgtgc tgaatttgca gagagaacgg ttgcaaaact ggaaaagaca 540
   attgatgacc tggaagagaa acttgcccag c 571
<210> 103
   <211> 451
   <212> DNA
   <213> Homo sapiens
<400> 103
   gtgcacaggt cccatttatt gtagaaaata ataataatta cagtgatgaa tagctcttct 60
   taaattacaa aacagaaacc acaaagaagg aagaggaaaa accccaggac ttccaagggt 120
   gaagctgtcc cctcctccct gccaccctcc caggctcatt agtgtccttg gaaggggcag 180
   aggactcaga ggggatcagt ctccaggggc cctgggctga agcgggtgag gcagagagtc 240
   ctgaggccac agagctgggc aacctgagcc gcctctctgg ccccctcccc caccactgcc 300
   caaacctgtt tacagcacct tcgcccctcc cctctaaacc cgtccatcca ctctgcactt 360
   cccaggcagg tgggtgggcc aggcctcagc catactcctg ggcgcgggtt tcggtgagca 420
   aggcacagtc ccagaggtga tatcaaggcc t 451
<210> 104
   <211> 441
   <212> DNA
   <213> Homo sapiens
<400> 104
   gcaaggaact ggtctgctca cacttgctgg cttgcgcatc aggactggct ttatctcctg 60
   actcacggtg caaaggtgca ctctgcgaac gttaagtccg tccccagcgc ttggaatcct 120
   acggccccca cagccggatc ccctcagcct tccaggtcct caactcccgt ggacgctgaa 180
   caatggcctc catggggcta caggtaatgg gcatcgcgct ggccgtcctg ggctggctgg 240
   ccgtcatgct gtgctgcgcg ctgcccatgt ggcgcgtgac ggccttcatc ggcagcaaca 300
   ttgtcacctc gcagaccatc tgggagggcc tatggatgaa ctgcgtggtg cagagcaccg 360
   gccagatgca gtgcaaggtg tacgactcgc tgctggcact gccgcaggac ctgcaggcgg 420
   cccgcgccct cgtcatcatc a 441
<210> 105
   <211> 509
   <212> DNA
   <213> Homo sapiens
<220>
   <221> misc_feature
   <222> 195
   <223> n = A, T, C or G
<400> 105
   tgcaaaaggg acacaggggt tcaaaaataa aaatttctct tccccctccc caaacctgta 60
   ccccagctcc ccgaccacaa cccccttcct cccccgggga aagcaagaag gagcaggtgt 120
   ggcatctgca gctgggaaga gagaggccgg ggaggtgccg agctcggtgc tggtctcttt 180
   ccaaatataa atacntgtgt cagaactgga aaatcctcca gcacccacca cccaagcact 240
   ctccgttttc tgccggtgtt tggagagggg cggggggcag gggcgccagg caccggctgg 300
   ctgcggtcta ctgcatccgc tgggtgtgca ccccgcgagc ctcctgctgc tcattgtaga 360
   agagatgaca ctcggggtcc ccccggatgg tgggggctcc ctggatcagc ttcccggtgt 420
   tggggttcac acaccagcac tccccacgct gcccgttcag agacatcttg cactgtttga 480
   ggttgtacag gccatgcttg tcacagttg                                  509
<210> 106
   <211> 571
   <212> DNA
   <213> Homo sapiens
<400> 106
   gggttggagg gactggttct ttatttcaaa aagacacttg tcaatattca gtatcaaaac 60
   agttgcacta ttgatttctc tttctcccaa tcggccccaa agagaccaca taaaaggaga 120
   gtacatttta agccaataag ctgcaggatg tacacctaac agacctccta gaaaccttac 180
   cagaaaatgg ggactgggta gggaaggaaa cttaaaagat caacaaactg ccagcccacg 240
   gactgcagag gctgtcacag ccagatgggg tggccagggt gccacaaacc caaagcaaag 300
   tttcaaaata atataaaatt taaaaagttt tgtacataag ctattcaaga tttctccagc 360
   actgactgat acaaagcaca attgagatgg cacttctaga gacagcagct tcaaacccag 420
   aaaagggtga tgagatgagt ttcacatggc taaatcagtg gcaaaaacac agtcttcttt 480
   ctttctttct ttcaaggagg caggaaagca attaagtggt cacctcaaca taagggggac 540
   atgatccatt ctgtaagcag ttgtgaaggg g                                571
<210> 107
   <211> 555
   <212> DNA
   <213> Homo sapiens
<400> 107
   caggaaccgg agcgcgagca gtagctgggt gggcaccatg gctgggatca ccaccatcga 60
   ggcggtgaag cgcaagatcc aggttctgca gcagcaggca gatgatgcag aggagcgagc 120
   tgagcgcctc cagcgagaag ttgagggaga aaggcgggcc cgggaacagg ctgaggctga 180
   ggtggcctcc ttgaaccgta ggatccagct ggttgaagaa gagctggacc gtgctcagga 240
   gcgcctggcc actgccctgc aaaagctgga agaagctgaa aaagctgctg atgagagtga 300
   gagaggtatg aaggttattg aaaaccgggc cttaaaagat gaagaaaaga tggaactcca 360
   ggaaatccaa ctcaaagaag ctaagcacat tgcagaagag gcagatagga agtatgaaga 420
   ggtggctcgt aagttggtga tcattgaagg agacttggaa cgcacagagg aacgagctga 480
   gctggcagag tcccgttgcc gagagatgga tgagcagatt agactgatgg accagaacct 540
   gaagtgtctg agtgc                                                  555
<210> 108
   <211> 541
   <212> DNA
   <213> Homo sapiens
<400> 108
   atctacgtca tcaatcaggc tggagacacc atgttcaatc gagctaagct gctcaatatt 60
   ggctttcaag aggccttgaa ggactatgat tacaactgct ttgtgttcag tgatgtggac 120
   ctcattccga tggacgaccg taatgcctac aggtgttttt cgcagccacg gcacatttct 180
   gttgcaatgg acaagttcgg gtttagcctg ccatatgttc agtattttgg aggtgtctct 240
   gctctcagta aacaacagtt tcttgccatc aatggattcc ctaataatta ttggggttgg 300
   ggaggagaag atgacgacat ttttaacaga ttagttcata aaggcatgtc tatatcacgt 360
   ccaaatgctg tagtagggag gtgtcgaatg atccggcatt caagagacaa gaaaaatgag 420
   cccaatcctc agaggtttga ccggatcgca catacaaagg aaacgatgcg cttcgatggt 480
   ttgaactcac ttacctacaa ggtgttggat gtcagagata cccgttatat acccaaatca 540
   c                                                                 541
<210> 109
   <211> 411
   <212> DNA
   <213> Homo sapiens
<400> 109
   ctagacctct aattaaaagg cacaatcatg ctggagaatg aacagtctga ccccgagggc 60
   cacagcgaat tttagggaag gaggcaaaga ggtgagaagg gaaaggaaag aaggaaggaa 120
   ggagaacaat aagaactgga gacgttgggt gggtcaggga gtgtggtgga ggctcggaga 180
   gatggtaaac aaacctgact gctatgagtt ttcaacccca tagtctaggg ccatgagggc 240
   gtcagttctt ggtggctgag ggtccttcca cccagcccac ctgggggagt ggagtgggga 300
   gttctgccag gtaagcagat gttgtctccc aagttcctga cccagatgtc tggcaggata 360
   acgctgacct gttccctcaa caagggacct gaaagtaatt ttgctcttta c          411
<210> 110
   <211> 451
   <212> DNA
   <213> Homo sapiens
<400> 110
   ccgaattcaa gcgtcaacga tccytccctt accatcaaat caattggcca ccaatggtac 60
   tgaacctacg agtacaccga ctacgggcgg actaatcttc aactcctaca tacttccccc 120
   attattccta gaaccaggcg acctgcgact ccttgacgtt gacaatcgag tagtactccc 180
   gattgaagcc cccattcgta taataattac atcacaagac gtcttgcact catgagctgt 240
   ccccacatta ggcttaaaaa cagatgcaat tcccggacgt ctaagccaaa ccactttcac 300
   cgctacacga ccgggggtat actacggtca atgctctgaa atctgtggag caaaccacag 360
   tttcatgccc atcgtcctag aattaattcc cctaaaaatc tttgaaatag ggcccgtatt 420
   taccctatag caccccctct accccctcta g                                451

<210> 111
   <211> 541
   <212> DNA
   <213> Homo sapiens
<400> 111
   gctcttcaca cttttattgt taattctctt cacatggcag atacagagct gtcgtcttga 60
   agaccaccac tgaccaggaa atgccacttt tacaaaatca tccccccttt tcatgattgg 120
   aacagttttc ctgaccgtct gggagcgttg aagggtgacc agcacatttg cacatgcaaa 180
   aaaggagtga ccccaaggcc tcaaccacac ttcccagagc tcaccatggg ctgcaggtga 240
   cttgccaggt ttggggttcg tgagctttcc ttgctgctgc ggtggggagg ccctcaagaa 300
   ctgagaggcc ggggtatgct tcatgagtgt taacatttac gggacaaaag cgcatcatta 360
   ggataaggaa cagccacagc acttcatgct tgtgagggtt agctgtagga gcgggtgaaa 420
   ggattccagt ttatgaaaat ttaaagcaaa caacggtttt tagctgggtg ggaaacagga 480
   aaactgtgat gtcggccaat gaccaccatt tttctgccca tgtgaaggtc cccatgaaac 540
   c                                                                  541
<210> 112
   <211> 521
   <212> DNA
   <213> Homo sapiens
<400> 112
   caagcgcttg gcgtttggac ccagttcagt gaggttcttg ggttttgtgc ctttggggat 60
   tttggtttga cccaggggtc agccttagga aggtcttcag gaggaggccg agttcccctt 120
   cagtaccacc cctctctccc cactttccct ctcccggcaa catctctggg aatcaacagc 180
   atattgacac gttggagccg agcctgaaca tgcccctcgg ccccagcaca tggaaaaccc 240
   ccttccttgc ctaaggtgtc tgagtttctg gctcttgagg catttccaga cttgaaattc 300
   tcatcagtcc attgctcttg agtctttgca gagaacctca gatcaggtgc acctgggaga 360
   aagactttgt ccccacttac agatctatct cctcccttgg gaagggcagg gaatggggac 420
   ggtgtatgga ggggaaggga tctcctgcgc ccttcattgc cacacttggt gggaccatga 480
   acatctttag tgtctgagct tctcaaatta ctgcaatagg a                      521
<210> 113
   <211> 568
   <212> DNA
   <213> Homo sapiens
<400> 113
   agcgtcaaat cagaatggaa aagactcaaa accatcatca acaccaagat caaaaggaca 60
   agratccttc aagaaacagg aaaaaactcc taaaacacca aaaggaccta gttctgtaga 120
   agacattaaa gcaaaaatgc aagcaagtat agaaaaaggt ggttctcttc ccaaagtgga 180
   agccaaattc atcaattatg tgaagaattg cttccggatg actgaccaag aggctattca 240
   agatctctgg cagtggagga agtctcttta agaaaatagt ttaaacaatt tgttaaaaaa 300
   ttttccgtct tatttcattt ctgtaacagt tgatatctgg ctgtcctttt tataatgcag 360
   agtgagaact ttccctaccg tgtttgataa atgttgtcca ggttctattg ccaagaatgt 420
   gttgtccaaa atgcctgttt agtttttaaa gatggaactc caccctttgc ttggttttaa 480
   gtatgtatgg aatgttatga taggacatag tagtagcggt ggtcagacat ggaaatggtg 540
   ggsmgacaaa aatatacatg tgaaataa                                     568
<210> 114
   <211> 483
   <212> DNA
   <213> Homo sapiens
<400> 114
   tccgaattcc aagcgaatta tggacaaacg attcctttta gaggattact tttttcaatt 60
   tcggttttag taatctaggc tttgcctgta aagaatacaa cgatggattt taaatactgt 120
   ttgtggaatg tgtttaaagg attgattcta gaacctttgt atatttgata gtatttctaa 180
   ctttcatttc tttactgttt gcagttaatg ttcatgttct gctatgcaat cgtttatatg 240
   cacgtttctt taattttttt agattttcct ggatgtatag tttaaacaac aaaaagtcta 300
   tttaaaactg tagcagtagt ttacagttct agcaaagagg aaagttgtgg ggttaaactt 360
   tgtattttct ttcttataga ggcttctaaa aaggtatttt tatatgttct ttttaacaaa 420
   tattgtgtac aacctttaaa acatcaatgt ttggatcaaa acaagaccca gcttattttc 480
   tgc                                                               483
<210> 115
   <211> 521
   <212> DNA
   <213> Homo sapiens
<400> 115
   tgtggtggcg cgggctgagg tggaggccca ggactctgac cctgcccctg ccttcagcaa 60
   ggcccccggc agcgccggcc actacgaact gccgtgggtt gaaaaatata ggccagtaaa 120
   gctgaatgaa attgtcggga atgaagacac cgtgagcagg ctagaggtct ttgcaaggga 180
   aggaaatgtg cccaacatca tcattgcggg ccctccagga accggcaaga ccacaagcat 240
   tctgtgcttg gcccgggccc tgctgggccc agcactcaaa gatgccatgt tggaactcaa 300
   tgcttcaaat gacaggggca ttgacgttgt gaggaataaa attaaaatgt ttgctcaaca 360
   aaaagtcact cttcccaaag gccgacataa gatcatcatt ctggatgaag cagacagcat 420
   gaccgacgga gcccagcaag ccttgaggag aaccatggaa atctactcta aaaccactcg 480
   ttcgcccttg cttgtaatgc ttcggataag atcatcgagc c                     521
<210> 116
   <211> 501
   <212> DNA
   <213> Homo sapiens
<400> 116
   ctttgcaaag cttttatttc atgtctgcgg catggaatcc acctgcacat ggcatcttag 60
   ctgtgaagga gaaagcagtg cacgagaagg aatgagtggg cggaaccaac ggcctccaca 120
   agctgccttc cagcagcctg ccaaggccat ggcagagaga gactgcaaac aaacacaagc 180
   aaacagagtc tcttcacagc tggagtctga aagctcatag tggcatgtgt gaatctgaca 240
   aaattaaaag tgtgcatagt ccattacatg cataaaacac taataataat cctgtttaca 300
   cgtgactgca gcaggcaggt ccagctccac cactgccctc ctgccacatc acatcaagtg 360
   ccatggttta gagggttttt catatgtaat tcttttattc tgtaaaaggt aacaaaatat 420
   acagaacaaa actttccctt tttaaaacta atgttacaaa tctgtattat cacttggata 480
   taaatagtat ataagctgat c                                           501
<210> 117
   <211> 451
   <212> DNA
   <213> Homo sapiens
<220>
   <221> misc_feature
   <222> 320
   <223> n = A,T,C or G
<400> 117
   caagggatat atgttgaggg tacrgrgtga cactgaacag atcacaaagc acgagaaaca 60
   ttagttctct ccctccccag cgtctccttc gtctccctgg ttttccgatg tccacagagt 120
   gagattgtcc ctaagtaact gcatgatcag agtgctgkct ttataagact cttcattcag 180
   cgtatccaat tcagcaattg cttcatcaaa tgccgttttt gccaggctac aggccttttc 240
   aggagagttt agaatctcat agtaaaagac tgagaaattt agtgccagac caagacgaat 300
   tgggtgtgta ggctgcattn ctttcttact aatttcaaat gcttcctggt aagcctgctg 360
   ggagttcgac acaagtggtt tgtttgttgc tccagatgcc acttcagaaa gatacctaaa 420
   ataatctcct ttcattttca aagtagaaca c                                451
<210> 118
   <211> 501
   <212> DNA
   <213> Homo sapiens
<400> 118
   tccggagccg gggtagtcgc cgccgccgcc gccggtgcag ccactgcagg caccgctgcc 60
   gccgcctgag tagtgggctt aggaaggaag aggtcatctc gctcggagct tcgctcggaa 120
   gggtctttgt tccctgcagc cctcccacgg gaatgacaat ggataaaagt gagctggtac 180
   agaaagccaa actcgctgag caggctgagc gatatgatga tatggctgca gccatgaagg 240
   cagtcacaga acaggggcat gaactctcca acgaagagag aaatctgctc tctgttgcct 300
   acaagaatgt ggtaaggccg cccgccgctc ttcctggcgt gtcatctcca gcattgagca 360
   gaaaacagag aggaatgaga agaagcagca gatgggcaaa gagtaccgtg agaagataga 420
   ggcagaactg caggacatct gcaatgatgt tctggagctt gttggacaaa tatcttattc 480
   caatgctaca caacccagaa a                                           501
<210> 119
   <211> 391
   <212> DNA
   <213> Homo sapiens
<400> 119
   aaaaagcagc argttcaaca caaaatagaa atctcaaatg taggatagaa caaaaccaag 60
   tgtgtgaggg gggaagcaac agcaaaagga agaaatgaga tgttgcaaaa aagatggagg 120
   agggttcccc tctcctctgg ggactgactc aaacactgat gtggcagtat acaccattcc 180
   agagtcaggg gtgttcattc ttttttggga gtaagaaaag gtggggatta agaagacgtt 240
   tctggaggct tagggaccaa ggctggtctc tttcccccct cccaaccccc ttgatccctt 300
   tctctgatca ggggaaagga gctcgaatga gggaggtaga gttggaaagg gaaaggattc 360
   cacttgacag aatgggacag actccttccc a                                391
<210> 120
   <211> 421
   <212> DNA
   <213> Homo sapiens
<220>
   <221> misc_feature
   <222> 409
   <223> n = A,T,C or G
<400> 120
   tggcaatagc acagccatcc aggagctctt cargcgcatc tcggagcagt tcactgccat 60
   gttccgccgg aaggccttcc tccactggta cacaggcgag ggcatggacg agatggagtt 120
   caccgaggct gagagcaaca tgaacgacct cgtctctgag tatcaagcag taccaggatg 180
   ccaccgcaga agaggaggag gatttcggtg aggaggccga agaggaggcc taaggcagag 240
   cccccatcac ctcaggcttc tcagttccct tagccgtctt actcaactgc ccctttcctc 300
   tccctcagaa tttgtgtttg ctgcctctat cttgtttttt gttttttctt ctgggggggt 360
   ctagaacagt gcctggcaca tagtaggcgc tcaataaata cttggttgnt gaatgtctcc 420
   t                                                                 421
<210> 121
   <211> 206
   <212> DNA
   <213> Homo sapiens
<400> 121
   agctggcgct agggctcggt tgtgaaatac agcgtrgtca gcccttgcgc tcagtgtaga 60
   aacccacgcc tgtaaggtcg gtcttcgtcc atctgctttt ttctgaaata cactaagagc 120
   agccacaaaa ctgtaacctc aaggaaacca taaagcttgg agtgccttaa tttttaacca 180
   gtttccaata aaacggttta ctacct                                      206

<210> 122
   <211> 131
   <212> DNA
   <213> Homo sapiens
<400> 122
   ggagatgaag atgaggaagc tgagtcagct acgggcargc gggcagctga agatgatgag 60
   gatgacgatg tcgataccaa gaagcagaag accgacgagg atgactagac agcaaaaaag 120
   gaaaagttaa a                                                      131
<210> 123
   <211> 231
   <212> DNA
   <213> Homo sapiens
<220>
   <221> misc_feature
   <222> 166, 202, 222, 225
   <223> n = A,T,C or G
<400> 123
   gatgaaaatt aaatacttaa attaatcaaa aggcactacg ataccaccta aaacctactg 60
   cctcagtggc agtakgctaa kgaagatcaa gctacagsac atyatctaat atgaatgtta 120
   gcaattacat akcargaagc atgtttgctt tccagaagac tatggnacaa tggtcattwg 180
   ggcccaagag gatatttggc cnggaaagga tcaagataga tnaangtaaa g          231
<210> 124
   <211> 521
   <212> DNA
   <213> Homo sapiens
<220>
   <221> misc_feature
   <222> 284, 412, 513
   <223> n = A,T,C or G
<400> 124
   gagtagcaac gcaaagcgct tggtattgag tctgtgggsg acttcggttc cggtctctgc 60
   agcagccgtg atcgcttagt ggagtgctta gggtagttgg ccaggatgcc gaatatcaaa 120
   atcttcagca ggcagctccc accaggactt atctcasaaa attgctgacc gcctgggcct 180
   ggagctaggc aaggtggtga ctaagaaatt cagcaaccag gagacctgtg tggaaattgg 240
   tgaaagtgta ccgtggagag gatgtctaca ttgttcagag tggntgtggc gaaatcaatg 300
   acaatttaat ggagcttttg atcatgatta atgcctgcaa gattgcttca gccagccggg 360
   ttactgcagt catcccatgc ttcccttatg ccccggcagg ataagaaaga tnagagccgg 420
   gccgccaatc tcagccaagc ttggtgcaaa tatgctatct gtagcagtgc agatcatatt 480
   atcaccatgg acctacatgc ttctcaaatt canggctttt t                     521
<210> 125
   <211> 341
   <212> DNA
   <213> Homo sapiens
<220>
   <221> misc_feature
   <222> 277
   <223> n = A,T,C or G
<400> 125
   atgcaaaagg ggacacaggg ggttcaaaaa taaaaatttc tcttccccct ccccaaacct 60
   gtaccccagc tccccgacca caaccccctt cctcccccgg ggaaagcaag aaggagcagg 120
   tgtggcatct gcagctggga agagagaggc cggggaggtg ccgagctcgg tgctggtctc 180
   tttccaaata taaatacgtg tgtcagaact ggaaaatcct ccagcaccca ccacccaagc 240
   actctccgtt ttctgccggt gtttggagag gggcggnggg caggggcgcc aggcaccggc 300
   tggctgcggt ctactgcatc cgctgggtgt gcaccccgcg a                     341
<210> 126
   <211> 521
   <212> DNA
   <213> Homo sapiens
<220>
   <221> misc_feature
   <222> 353, 399, 455
   <223> n = A,T,C or G
<400> 126
   aggttggaga aggtcatgca ggtgcagatt gtccaggskc agccacaggg tcaagcccaa 60
   caggcccaga gtggcactgg acagaccatg caggtgatgc agcagatcat cactaacaca 120
   ggagagatcc agcagatccc ggtgcagctg aatgccggcc agctgcagta tatccgctta 180
   gcccagcctg tatcaggcac tcaagttgtg cagggacaga tccagacact tgccaccaat 240
   gctcaacaga ttacacagac agaggtccag caaggacagc agcagttcaa gccagttcac 300
   aagatggaca gcagctctac cagatccagc aagtcaccat gcctgcgggc cangacctcg 360
   ccagcccatg ttcatccagt caagccaacc agcccttcna cgggcaggcc ccccaggtga 420
   ccggcgactg aagggcctga gctggcaagg ccaangacac ccaacacaat ttttgccata 480
   cagcccccag gcaatgggca cagcctttct tcccagagga c                      521
<210> 127
   <211> 351
   <212> DNA
   <213> Homo sapiens
<400> 127
   tgagatttat tgcatttcat gcagcttgaa gtccatgcaa aggrgactag cacagttttt 60
   aatgcattta aaaaataaaa gggaggtggg cagcaaacac acaaagtcct agtttcctgg 120
   gtccctggga gaaaagagtg tggcaatgaa tccacccact ctccacaggg aataaatctg 180
   tctcttaaat gcaaagaatg tttccatggc ctctggatgc aaatacacag agctctgggg 240
   tcagagcaag ggatggggag aggaccacga gtgaaaaagc agctacacac attcacctaa 300
   ttccatctga gggcaagaac aacgtggcaa gtcttggggg tagcagctgt t          351
<210> 128
   <211> 521
   <212> DNA
   <213> Homo sapiens
<400> 128
   tccagacatg ctcctgtcct aggcggggag caggaaccag acctgctatg ggaagcagaa 60
   agagttaagg gaaggtttcc tttcattcct gttccttctc ttttgctttt gaacagtttt 120
   taaatatact aatagctaag tcatttgcca gccaggtccc ggtgaacagt agagaacaag 180
   gagcttgcta agaattaatt ttgctgtttt tcaccccatt caaacagagc tgccctgttc 240
   cctgatggag ttccattcct gccagggcac ggctgagtaa cacgaagcca ttcaagaaag 300
   gcgggtgtga aatcactgcc accccatgga cagacccctc actcttcctt cttagccgca 360
   gcgctactta ataaatatat ttatactttg aaattatgat aaccgatttt tcccatgcgg 420
   catcctaagg gcacttgcca gctcttatcc ggacagtcaa gcactgttgt tggacaacag 480
   ataaaggaaa agaaaaagaa gaaaacaacc gcaacttctg t                      521

<210> 129
   <211> 521
   <212> DNA
   <213> Homo sapiens
<400> 129
   tgagacggac cactggcctg gtcccccctc atktgctgtc gtaggacctg acatgaaacg 60
   cagatctagt ggcagagagg aagatgatga ggaacttctg agacgtcggc agcttcaaga 120
   agagcaatta atgaagctta actcaggcct gggacagttg atcttgaaag aagagatgga 180
   gaaagagagc cgggaaaggt catctctgtt agccagtcgc tacgattctc ccatcaactc 240
   agcttcacat attccatcat ctaaaactgc atctctccct ggctatggaa gaaatgggct 300
   tcaccggcct gtttctaccg acttcgctca gtataacagc tatggggatg tcagcggggg 360
   agtgcgagat taccagacac ttccagatgg ccacatgcct gcaatgagaa tggaccgagg 420
   agtgtctatg cccaacatgt tggaaccaaa gatatttcca tatgaaatgc tcatggtgac 480
   caacagaggg ccgaaaccaa atctcagaga ggtggacaga a                     521
<210> 130
   <211> 270
   <212> DNA
   <213> Homo sapiens
<400> 130
   tcactttatt tttcttgtat aaaaacccta tgttgtagcc acagctggag cctgagtccg 60
   ctgcacggag actctggtgt gggtcttgac gaggtggtca gtgaactcct gatagggaga 120
   cttggtgaat acagtctcct tccagaggtc gggggtcagg tagctgtagg tcttagaaat 180
   ggcatcaaag gtggccttgg cgaagttgcc cagggtggca gtgcagcccc gggctgaggt 240
   gtagcagtca tcgataccag ccatcatgag                                  270
<210> 131
   <211> 341
   <212> DNA
   <213> Homo sapiens
<400> 131
   ctggaatata gacccgtgat cgacaaaact ttgaacgagg ctgactgtgc caccgtcccg 60
   ccagccattc gctcctactg atgagacaag atgtggtgat gacagaatca gcttttgtaa 120
   ttatgtataa tagctcatgc atgtgtccat gtcataactg tcttcatacg cttctgcact 180
   ctggggaaga aggagtacat tgaagggaga ttggcaccta gtggctggga gcttgccagg 240
   aacccagtgg ccagggagcg tggcacttac ctttgtccct tgcttcattc ttgtgagatg 300
   ataaaactgg gcacagctct taaataaaat ataaatgaac a                     341
<210> 132
   <211> 844
   <212> DNA
   <213> Homo sapiens
<220>
   <221> misc_feature
   <222> 37
   <223> n = A,T,C or G
<400> 132
   tgaatgggga ggagctgacc caggaaatgg agcttgngga gaccaggcct gcaggggatg 60
   gaaccttcca gaagtgggca tctgtggtgg tgcctcttgg gaaggagcag aagtacacat 120
   gccatgtgga acatgagggg ctgcctgagc ccctcaccct gagatggggc aaggaggagc 180
   ctccttcatc caccaagact aacacagtaa tcattgctgt tccggttgtc cttggagctg 240
   tggtcatcct tggagctgtg atggcttttg tgatgaagag gaggagaaac acaggtggaa 300
   aaggagggga ctatgctctg gctccaggct cccagagctc tgatatgtct ctcccagatt 360
   gtaaagtgtg aagacagctg cctggtgtgg acttggtgac agacaatgtc ttcacacatc 420
   tcctgtgaca tccagagacc tcagttctct ttagtcaagt gtctgatgtt ccctgtgagt 480
   ctgcgggctc aaagtgaaga actgtggagc ccagtccacc cctgcacacc aggaccctat 540
   ccctgcactg ccctgtgttc ccttccacag ccaaccttgc tgctccagcc aaacattggt 600
   ggacatctgc agcctgtcag ctccatgcta ccctgacctt caactcctca cttccacact 660
   gagaataata atttgaatgt gggtggctgg agagatggct cagcgctgac tgctcttcca 720
   aaggtcctga gttcaaatcc cagcaaccac atggtggctc acaaccatct gtaatgggat 780
   ctaataccct cttctgcagt gtctgaagac asctacagtg tacttacata taataataaa 840
   taag                                                              844
<210> 133
   <211> 601
   <212> DNA
   <213> Homo sapiens
<400> 133
   ggccgggcgc gcgcgccccc gccacacgca cgccgggcgt gccagtttat aaagggagag 60
   agcaagcagc gagtcttgaa gctctgtttg gtgctttgga tccatttcca tcggtcctta 120
   cagccgctcg tcagactcca gcagccaaga tggtgaagca gatcgagagc aagactgctt 180
   ttcaggaagc cttggacgct gcaggtgata aacttgtagt agttgacttc tcagccacgt 240
   ggtgtgggcc ttgcaaaatg atcaagcctt tctttcattc cctctctgaa aagtattcca 300
   acgtgatatt ccttgaagta gatgtggatg actgtcagga tgttgcttca gagtgtgaag 360
   tcaaatgcat gccaacattc cagtttttta agaagggaca aaaggtgggt gaattttctg 420
   gagccaataa ggaaaagctt gaagccacca ttaatgaatt agtctaatca tgttttctga 480
   aaatataacc agccattggc tatttaaaac ttgtaatttt tttaatttac aaaaatataa 540
   aatatgaaga cataaacccm gttgccatct gcgtgacaat aaaacattaa tgctaacact 600
   t                                                                 601
<210> 134
   <211> 421
   <212> DNA
   <213> Homo sapiens
<400> 134
   tcacataaga aatttaagca agttacrcta tcttaaaaaa cacaacgaat gcattttaat 60
   agagaaaccc ttccctccct ccacctccct cccccaccct cctcatgaat taagaatcta 120
   agagaagaag taaccataaa accaagtttt gtggaatcca tcatccagag tgcttacatg 180
   gtgattaggt taatattgcc ttcttacaaa atttctattt taaaaaaaat tataaccttg 240
   attgcttatt acaaaaaaat tcagtacaaa agttcaatat attgaaaaat gcttttcccc 300
   tccctcacag caccgtttta tatatagcag agaataatga agagattgct agtctagatg 360
   gggcaatctt caaattacac caagacgcac agtggtttat ttaccctccc cttctcataa 420
   g                                                                 421
<210> 135
   <211> 511
   <212> DNA
   <213> Homo sapiens
<400> 135
   ggaaaggatt caagaattag aggacttgct tgctrragaa aaagacaact ctcgtcgcat 60
   gctgacagac aaagagagag agatggcgga aataagggat caaatgcagc aacagctgaa 120
   tgactatgaa cagcttcttg atgtaaagtt agccctggac atggaaatca gtgcttacag 180
   gaaactctta gaaggcgaag aagagaggtt gaagctgtct ccaagccctt cttcccgtgt 240
   gacagtatcc cgagcatcct caagtcgtag tgtaccgtac aactagagga aagcggaaga 300
   gggttgatgt ggaagaatca gaggcgaagt agtagtgtta gcatctctca ttccgcctca 360
   accactggaa atgtttgcat cgaagaaatt gatgttgatg ggaaatttat cccgcttgaa 420
   gaacacttct gaacaggatc aaccaatggg aaggcttggg agatgatcag aaaaattgga 480
   gacacatcag tcagttataa atatacctca a                                 511
<210> 136
   <211> 341
   <212> DNA
   <213> Homo sapiens
<400> 136
   catgggtttc accaggttgg ccaggctgct cttgaactsc tgacctcagg tgatccaccc 60
   gcctcggcct cccaaagtgc tgggattaca ggcgtgagcc accacgcccg gcccccaaag 120
   ctgtttcttt tgtctttagc gtaaagctct cctgccatgc agtatctaca taactgacgt 180
   gactgccagc aagctcagtc actccgtggt ctttttctct ttccagttct tctctctctc 240
   ttcaagttct gcctcagtga aagctgcagg tccccagtta agtgatcagg tgagggttct 300
   ttgaacctgg ttctatcagt cgaattaatc cttcatgatg g                      341
<210> 137
   <211> 551
   <212> DNA
   <213> Homo sapiens
<400> 137
   gatgtgttgg accctctgtg tcaaaaaaaa cctcacaaag aatcccctgc tcattacaga 60
   agaagatgca tttaaaatat gggttatttt caacttttta tctgaggaca agtatccatt 120
   aattattgtg tcagaagaga ttgaatacct gcttaagaag cttacagaag ctatgggagg 180
   aggttggcag caagaacaat ttgaacatta taaaatcaac tttgatgaca gtaaaaatgg 240
   cctttctgca tgggaactta ttgagcttat tggaaatgga cagtttagca aaggcatgga 300
   ccggcagact gtgtctatgg caattaatga agtctttaat gaacttatat tagatgtgtt 360
   aaagcagggt tacatgatga aaaagggcca cagacggaaa aactggactg aaagatggtt 420
   tgtactaaaa cccaacataa tttcttacta tgtgagtgag gatctgaagg ataagaaagg 480
   agacattctc ttggatgaaa attgctgtgt agaagtcctt gcctgacaaa agatggaaag 540
   aaatgccttt t                                                       551
<210> 138
   <211> 531
   <212> DNA
   <213> Homo sapiens
<220>
   <221> misc_feature
   <222> 490
   <223> n = A, T, C or G
<400> 138
   gactggttct ttatttcaaa aagacacttg tcaatattca gtrtcaaaac agttgcacta 60
   ttgatttctc tttctcccaa tcggccccaa agagaccaca taaaaggaga gtacatttta 120
   agccaataag ctgcaggatg tacacctaac agacctccta gaaaccttac cagaaaatgg 180
   ggactgggta gggaaggaaa cttaaaagat caacaaactg ccagcccacg gactgcagag 240
   gctgtcacag ccagatgggg tggccagggt gccacaaacc caaagcaaag tttcaaaata 300
   atataaaatt taaaaagttt tgtacataag ctattcaaga tttctccagc actgactgat 360
   acaaagcaca attgagatgg cacttctaga gacagcagct tcaaacccag aaaagggtga 420
   tgagatgaag tttcacatgg ctaaatcagt ggcaaaaaca cagtcttctt tctttctttc 480
   tttcaaggan gcaggaaagc aattaagtgg tcaccttaac ataaggggga c         531
<210> 139
   <211> 521
   <212> DNA
   <213> Homo sapiens
<220>
   <221> misc_feature
   <222> 517
   <223> n = A,T,C or G
<400> 139
   tgggtgggca ccatggctgg gatcaccacc atcgaggcgg tgaagcgcaa gatccaggtt 60
   ctgcagcagc aggcagatga tgcagaggag cgagctgagc gcctccagcg agaagttgag 120
   ggagaaaggc gggcccggga acaggctgag gctgaggtgg cctccttgaa ccgtaggatc 180
   cagctggttg aagaagagct ggaccgtgct caggagcgcc tggccactgc cctgcaaaag 240
   ctggaagaag ctgaaaaagc tgctgatgag agtgagagag gtatgaaggt tattgaaaac 300
   cgggccttaa aagatgaaga aaagatggaa ctccaggaaa tccaactcaa agaagctaag 360
   cacattgcag aagaggcaga taggaagtat gaagaggtgg ctcgtaagtt ggtgatcatt 420
   gaaggagact tggaaccgca cagaaggaac gagcttgagc ttggcaaaag tcccgttgcc 480
   cagagatggg atgaaccaga ttagactgat ggaccanaac c                    521

<210> 140
   <211> 571
   <212> DNA
   <213> Homo sapiens
<220>
   <221> misc_feature
   <222> 7
   <223> n = A, T, C or G
<400> 140
   aggggcngcg ggtgcgtggg ccactgggtg accgacttag cctggccaga ctctcagcac 60
   ctggaagcgc cccgagagtg acagcgtgag gctgggaggg aggacttggc ttgagcttgt 120
   taaactctgc tctgagcctc cttgtcgcct gcatttagat ggctcccgca aagaagggtg 180
   gcgagaagaa aaagggccgt tctgccatca acgaagtggt aacccgagaa tacaccatca 240
   acattcacaa gcgcatccat ggagtgggct tcaagaagcg tgcacctcgg gcactcaaag 300
   agattcggaa atttgccatg aaggagatgg gaactccaga tgtgcgcatt gacaccaggc 360
   tcaacaaagc tgtctgggcc aaaggaataa ggaatgtgcc ataccgaatc cggtgtgcgg 420
   ctgtccagaa aacgtaatga ggatgaagat tcaccaaata agctatatac tttggttacc 480
   tatgtacctg ttaccacttt caaaaatcta cagacagtca atgtggatga gaactaatcg 540
   ctgatcgtca gatcaaataa agttataaaa t                                571
<210> 141
   <211> 531
   <212> DNA
   <213> Homo sapiens
<400> 141
   tcgggagcca cacttggccc tcttcctctc caaagsgcca gaacctcctt ctctttggag 60
   aatggggagg cctcttggag acacagaggg tttcaccttg gatgacctct agagaaattg 120
   cccaagaagc ccaccttctg gtcccaacct gcagacccca cagcagtcag ttggtcaggc 180
   cctgctgtag aaggtcactt ggctccattg cctgcttcca accaatgggc aggagagaag 240
   gcctttattt ctcgcccacc cattcctcct gtaccagcac ctccgttttc agtcagtgtt 300
   gtccagcaac ggtaccgttt acacagtcac ctcagacaca ccatttcacc tcccttgcca 360
   agctgttagc cttagagtga ttgcagtgaa cactgtttac acaccgtgaa tccattccca 420
   tcagtccatt ccagttggca ccagcctgaa ccatttggta cctggtgtta actggagtcc 480
   tgtttacaag gtggagtcgg ggcttgctga cttctcttca tttgagggca c          531
<210> 142
   <211> 491
   <212> DNA
   <213> Homo sapiens
<220>
   <221> misc_feature
   <222> 410
   <223> n = A,T,C or G
<400> 142
   acctagacag aaggtgggtg agggaggact ggtaggaggc tgaggcaatt ccttggtagt 60
   ttgtcctgaa accctactgg agaagtcagc atgaggcacc tactgagaga agtgcccaga 120
   aactgctgac tgcatctgtt aagagttaac agtaaagagg tagaagtgtg tttctgaatc 180
   agagtggaag cgtctcaagg gtcccacagt ggaggtccct gagctacctc ccttccgtga 240
   gtgggaagag tgaagcccat gaagaactga gatgaagcaa ggatggggtt cctgggctcc 300
   aggcaagggc tgtgctctct gcagcaggga gccccacgag tcagaagaaa agaactaatc 360
   atttgttgca agaaaccttg cccggatact agcggaaaac tggaggcggn ggtgggggca 420
   caggaaagtg gaagtgattt gatggagagc agagaagcct atgcacagtg gccgagtcca 480
   cttgtaaagt g                                                      491
<210> 143
   <211> 515
   <212> DNA
   <213> Homo sapiens
<400> 143
   ttcaagcaat tgtaacaagt atatgtagat tagagtgagc aaaatcatat acaattttca 60
   tttccagttg ctattttcca aattgttctg taatgtcgtt aaaattactt aaaaattaac 120
   aaagccaaaa attatattta tgacaagaaa gccatcccta cattaatctt acttttccac 180
   tcaccggccc atctccttcc tctttttcct aactatgcca ttaaaactgt tctactgggc 240
   cgggcgtgtg gctcatgcct gtaatcccag cattttggga ggccaaggca ggcggatcat 300
   gaggtcaaga gattgagacc atcctggcca acatggtgaa accccgcctc gactaagaat 360
   acaaaaatta gctgggcatg gtggcgcatg cctgtagtct cagctactcg ggaggctgag 420
   gcagaagaat cgcttgaacc cgggaggcag aggatgcagt gagccccgat cgcgccactg 480
   cactctagcc tgggcgacag actgagactc tgctc                            515
<210> 144
   <211> 340
   <212> DNA
   <213> Homo sapiens
<400> 144
   tgtgccagtc tacaggccta tcagcagcga ctccttcagc aacagatggg gtcccctgtt 60
   cagcccaacc ccatgagccc ccagcagcat atgctcccaa atcaggccca gtccccacac 120
   ctacaaggcc agcagatccc taattctctc tccaatcaag tgcgctctcc ccagcctgtc 180
   ccttctccac ggccacagtc ccagcccccc cactccagtc cttccccaag gatgcagcct 240
   cagccttctc cacaccacgt ttccccacag acaagttccc cacatcctgg actggtagtt 300
   gcccaggcca accccatgga acaagggcat tttgccagcc                       340
<210> 145
   <211> 630
   <212> DNA
   <213> Homo sapiens
<400> 145
   tgtaaaaact tgtttttaat tttgtataaa ataaaggtgg tccatgccca cgggggctgt 60
   aggaaatcca agcagaccag ctggggtggg gggatgtagc ctacctcggg ggactgtctg 120
   tcctcaaaac gggctgagaa ggcccgtcag gggcccaggt cccacagaga ggcctgggat 180
   actcccccaa cccgaggggc agactggcca gtggggagcc cccatcgtgc cccagaggtg 240
   gccacaggct gaaggagggg cctgaggcac cgcagcctgc aacccccagg gctgcagtcc 300
   actaactttt tacagaataa aaggaacatg gggatgggga aaaaagcacc aggtcaggca 360
   gggcccgagg gccccagatc ccaggagggc caggactcag gatgccagca ccaccctagc 420
   agctcccaca gctcctggca caggaggccg ccacggattg gcacaggccg ctgctggcca 480
   tcacgccaca tttggagaac ttgtcccgac agaggtcagc tcggaggagc tcctcgtggg 540
   cacacactgt acgaacacag atctccttgt taatgacgta cacacggcgg aggctgcggg 600
   gacagggcac gggaggtctc agccccactt                                  630
<210> 146
   <211> 521
   <212> DNA
   <213> Homo sapiens
<400> 146
   atggctgctg gatttaggtg gtaatagggg ctgtgggcca taaatctgaa gccttgagaa 60
   ccttgggtct ggagagccat gaagagggaa ggaaaagagg gcaagtcctg aacctaacca 120
   atgacctgat ggattgctcg accaagacac agaagtgaag tctgtgtctg tgcacttccc 180
   acagactgga gtttttggtg ctgaatagag ccagttgcta aaaaattggg ggtttggtga 240
   agaaatctga ttgttgtgtg tattcaatgt gtgattttaa aaataaacag caacaacaat 300
   aaaaaccctg actggctgtt ttttccctgt attctttaca actatttttt gaccctctga 360
   aaattattat acttcaccta aatggaagac tgctgtgttt gtggaaattt tgtaattttt 420
   taatttattt tattctctct cctttttatt ttgcctgcag aatccgttga gagactaata 480
   aggcttaata tttaattgat ttgtttaata tgtatataaa t                         521
<210> 147
   <211> 562
   <212> DNA
   <213> Homo sapiens
<400> 197
<210> 148
   <211> 820
   <212> DNA
   <213> Homo sapiens
<400> 148
   gaaggagtcg ggatactcag cattgatgca ccccaatttc aaagcggcat tcttcggcag 60
   gtctctggga caatctctag ggtcactacc tggaaactcg ttagggtaca actgaatgct 120
   gaaaggaaag aacacctgca gaaccggaca gaaattcacc ccggcgatca gctgattgat 180
   ctcggtcgac cagaagtcat ggctaaagat gacgaggacg ttgtcaattc cctgggcttt 240
   tcgaagtgag tccagcagca gtctgaggta ttcgggccgg ttatgcacct ggaccaccag 300
   caccagctcc cggggggccc aggtgccagc cttatctaca ttcctcaggg tctgatcaaa 360
   gttcagctgg tacaccaggg accggtaccg cagcgtcagg ttgtccgctc gggctggggg 420
   accgccggga ccagggaagc cgccgacacg ttggagaccc tgcggatgcc cacagccaca 480
   gaggggtggt ccccaccgcg gccgccggca ccccgcgcgg gttcggcgtc cagcaacggt 540
   ggggcgaggg cctcgttctt cctttgtcgc ccattgctgc tccagaggac gaagccgcag 600
   gcggccacca cgagcgtcag gattagcacc ttccgtttgt agatgcggaa cctcatggtc 660
   tccagggccg ggagcgcagc tacagctcga gcgtcggcgc cgccgctagg agccgcggct 720
   cggcttcgtc tccgtcctct ccattcagca ccacgggtcc cggaaaaagc tcagccscgg 780
   tcccaaccgc accctagctt cgttacctgc gcctcgcttg                       820
<210> 149
   <211> 501
   <212> DNA
   <213> Homo sapiens
<400> 149
   cagattttta tttgcagtcg tcactggggc cgtttcttgc tgcttatttg tctgctagcc 60
   tgctcttcca gctgcatggc caggcgcaag gccttgatga catctcgcag ggctgagaaa 120
   tgcttggctt gctgggccag agcagattcc gctttgttca caaaggtctc caggtcatag 180
   tctggctgct cggtcatctc agagagctca agccagtctg gtccttgctg tatgatctcc 240
   ttgagctctt ccatagcctt ctcctccagc tccctgatct gagtcatggc ttcgttaaag 300
   ctggacatct gggaagacag ttcctcctct tccttggata aattgcctgg aatcagcgcc 360
   ccgttagagc aggcttccat ctcttctgtt tccatttgaa tcaactgctc tccactgggc 420
   ccactgtggg ggctcagctc cttgaccctg ctgcatatct taagggtgtt taaaggatat 480
   tcacaggagc ttatgcctgg t                                           501
<210> 150
   <211> 511
   <212> DNA
   <213> Homo sapiens
<220>
   <221> misc_feature
   <222> 457, 479
   <223> n = A,T,C or G
<400> 150
   ctcctcttgg tacatgaacc caagttgaaa gtggacttaa caaagtatct ggagaaccaa 60
   gcattctgct ttgactttgc atttgatgaa acagcttcga atgaagttgt ctacaggttc 120
   acagcaaggc cactggtaca gacaatcttt gaaggtggaa aagcaacttg ttttgcatat 180
   ggccagacag gaagtggcaa gacacatact atgggcggag acctctctgg gaaagcccag 240
   aatgcatcca aagggatcta tgccatggcc ttccgggacg tcttcttctg aagaatcaac 300
   cctgctaccg gaagttgggc ctggaagtct atgtgacatt cttcgagatc tacaatggga 360
   agctgtttga cctgctcaac aagaaggcca agcttgcgcg tgctggaaga cggcaagcaa 420
   caggtgcaag tggtgggggc ttgcaggaac atctggntaa ctctgcttga tgatggcant 480
   caagatgatc gacatgggca gcgcctgcag a                                511
<210> 151
   <211> 566
   <212> DNA
   <213> Homo sapiens
<400> 151
   tcccgaattc aagcgacaaa ttggawagtg aaatggaaga tgcctatcat gaacatcagg 60
   caaatctttt gcgccaagat ctgatgagac gacaggaaga attaagacgc atggaagaac 120
   ttcacaatca agaaatgcag aaacgtaaag aaatgcaatt gaggcaagag gaggaacgac 180
   gtagaagaga ggaagagatg atgattcgtc aacgtgagat ggaagaacaa atgaggcgcc 240
   aaagagagga aagttacagc cgaatgggct acatggatcc acgggaaaga gacatgcgaa 300
   tgggtggcgg aggagcaatg aacatgggag atccctatgg ttcaggaggc cagaaatttc 360
   cacctctagg aggtggtggt ggcataggtt atgaagctaa tcctggcgtt ccaccagcaa 420
   ccatgagtgg ttccatgatg ggaagtgaca tgcgtactga gcgctttggg cagggaggtg 480
   cggggcctgt gggtggacag ggtcctagag gaatggggcc tggaactcca gcaggatatg 540
   gtagagggag agaagagtac gaaggc                                      566

<210> 152
   <211> 518
   <212> DNA
   <213> Homo sapiens
<400> 152
   ttcgtgaaga ccctgactgg taagaccatc actctcgaag tggagcccga gtgacaccat 60
   tgagaatgtc aaggcaaaga tccaagacaa ggaaggcatc cctcctgacc agcakaggtt 120
   gatctttgct gggaaacagc tggaagatgg acgcaccctg tctgactaca acatccagaa 180
   agagtccacc ctgcacctgg tgctccgtct cagaggtggg atgcaaatct tcgtgaagac 240
   cctgactggt aagaccatca ccctcgaggt ggagcccagt gacaccatcg agaatgtcaa 300
   ggcaaagatc caagataagg aaggcatccc tcctgatcag cagaggttga tctttgctgg 360
   gaaacagctg gaagatggac gcaccctgtc tgactacaac atccagaaag agtccactct 420
   gcacttggtc ctgcgcttga gggggggtgt ctaagtttcc ccttttaagg tttcaacaaa 480
   tttcattgca ctttcctttc aataaagttg ttgcattc                        518
<210> 153
   <211> 542
   <212> DNA
   <213> Homo sapiens
<400> 153
   gcgcgggtgc gtgggccact gggtgaccga cttagcctgg ccagactctc agcacctgga 60
   agcgccccga gagtgacagc gtgaggctgg gagggaggac ttggcttgag cttgttaaac 120
   tctgctctga gcctccttgt cgcctgcatt tagatggctc ccgcaaagaa gggtggcgag 180
   aagaaaaagg gccgttctgc catcaacgaa gtggtaaccc gagaatacac catcaacatt 240
   cacaagcgca tccatggagt gggcttcaag aagcgtgcac ctcgggcact caaagagatt 300
   cggaaatttg ccatgaagga gatgggaact ccagatgtgc gcattgacac caggctcaac 360
   aaagctgtct gggccaaagg aataaggaat gtgccatacc gaatccgtgt gcggctgtcc 420
   agaaaacgta atgaggatga agattcacca aataagctat atactttggt tacctatgta 480
   cctgttacca ctttcaaaaa tctacagaca gtcaatgtgg atgagaacta atcgctgatc 540
   gt                                                                542
<210> 154
   <211> 411
   <212> DNA
   <213> Homo sapiens
<400> 154
   aattctttat ttaaatcaac aaactcatct tcctcaagcc ccagaccatg gtaggcagcc 60
   ctccctctcc atcccctcac cccacccctt agccacagtg aagggaatgg aaaatgagaa 120
   gccacgaggg cccctgccag ggaaggctgc cccagatgtg tggtgagcac agtcagtgca 180
   gctgtggctg gggcagcagc tgccacaggc tcctccctat aaattaagtt cctgcagcca 240
   cagctgtggg agaagcatac ttgtagaagc aaggccagtc cagcatcaga aggcagaggc 300
   agcatcagtg actcccagcc atggaatgaa cggaggacac agagctcaga gacagaacag 360
   gccaggggga agaaggagag acagaatagg ccagggcatg gcggtgaggg a          411
<210> 155
   <211> 421
   <212> DNA
   <213> Homo sapiens
<220>
   <221> misc_feature
   <222> 173
   <223> n = A,T,C or G
<400> 155
   tgatgaatct gggtgggctg gcagtagccc gagatgatgg gctcttctct ggggatccca 60
   actggttccc taagaaatcc aaggagaatc ctcggaactt ctcggataac cagctgcaag 120
   agggcaagaa cgtgatcggg ttacagatgg gcaccaaccg cggggcgtct cangcaggca 180
   tgactggcta cgggatgcca cgccagatcc tctgatccca ccccaggcct tgcccctgcc 240
   ctcccacgaa tggttaatat atatgtagat atatatttta gcagtgacat tcccagagag 300
   ccccagagct ctcaagctcc tttctgtcag ggtggggggt tcaagcctgt cctgtcacct 360
   ctgaagtgcc tgctggcatc ctctccccca tgcttactaa tacattccct tccccatagc 420
   c                                                                 421
<210> 156
   <211> 670
   <212> DNA
   <213> Homo sapiens
<400> 156
   agcggagctc cctcccctgg tggctacaac ccacacacgc caggctcagg catcgagcag 60
   aactccagcg actgggtaac cactgacatt caggtgaagg tgcgggacac ctacctggat 120
   acacaggtgg tgggacagac aggtgtcatc cgcagtgtca cggggggcat gtgctctgtg 180
   tacctgaagg acagtgagaa ggttgtcagc atttccagtg agcacctgga gcctatcacc 240
   cccaccaaga acaacaaggt gaaagtgatc ctgggcgagg atcgggaagc cacgggcgtc 300
   ctactgagca ttgatggtga ggatggcatt gtccgtatgg accttgatga gcagctcaag 360
   atcctcaacc tccgcttcct ggggaagctc ctggaagcct gaagcaggca gggccggtgg 420
   acttcgtcgg atgaagagtg atcctccttc cttccctggc ccttggctgt gacacaagat 480
   cctcctgcag ggctaggcgg attgttctgg atttcctttt gtttttcctt ttaggtttcc 540
   atcttttccc tccctggtgc tcattggaat ctgagtagag tctgggggag ggtccccacc 600
   ttcctgtacc tcctccccac agcttgcttt tgttgtaccg tctttcaata aaaagaagct 660
   gtttggtcta                                                        670
<210> 157
   <211> 421
   <212> DNA
   <213> Homo sapiens
<400> 157
   ggttcacagc actgctgctt gtgtgttgcc ggccaggaat tccaggctca caaggctatc 60
   ttagcagctc gttctccggt ttttagtgcc atgtttgaac atgaaatgga ggagagcaaa 120
   aagaatcgag ttgaaatcaa tgatgtggag cctgaagttt ttaaggaaat gatgtgcttc 180
   atttacacgg ggaaggctcc aaacctcgac aaaatggctg atgatttgct ggcagctgct 240
   gacaagtatg ccctggagcg cttaaaggtc atgtgtgagg atgccctctg cagtaacctg 300
   tccgtggaga acgctgcaga aattctcatc ctggccgacc tccacagtgc agatcagttg 360
   aaaactcagg cagtggattt catcaactat catgcttcgg atgtcttgga gacctcttgg 420
   g                                                                 421
<210> 158
   <211> 321
   <212> DNA
   <213> Homo sapiens
<400> 158
   tcgtagccat ttttctgctt ctttggagaa tgacgccaca ctgactgctc attgtcgttg 60
   gttccatgcc aattggtgaa atagaacctc atccggtagt ggagccggag ggacatcttg 120
   tcatcaacgg tgatggtgcg atttggagca taccagagct tggtgttctc gccatacagg 180
   gcaaagaggt tgtgacaaag aggagagata cggcatgcct gtgcagccct gatgcacagt 240
   tcctctgctg tgtactctcc actgcccagc cggaggggct ccctgtccga cagatagaag 300
   atcacttcca cccctggctt g                                           321
<210> 159
   <211> 596
   <212> DNA
   <213> Homo sapiens
<400> 159
   tggcacactg ctcttaagaa actatgawga tctgagattt ttttgtgtat gtttttgact 60
   cttttgagtg gtaatcatat gtgtctttat agatgtacat acctccttgc acaaatggag 120
   gggaattcat tttcatcact gggagtgtcc ttagtgtata aaaaccatgc tggtatatgg 180
   cttcaagttg taaaaatgaa agtgacttta aaagaaaata ggggatggtc caggatctcc 240
   actgataaga ctgtttttaa gtaacttaag gacctttggg tctacaagta tatgtgaaaa 300
   aaatgagact tactgggtga ggaaattcat tgtttaaaga tggtcgtgtg tgtgtgtgtg 360
   tgtgtgtgtg ttgtgttgtg ttttgttttt taagggaggg aatttattat ttaccgttgc 420
   ttgaaattac tgkgtaaata tatgtytgat aatgatttgc tytttgvcma ctaaaattag 480
   gvctgtataa gtwctaratg cmtccctggg kgttgatytt ccmagatatt gatgatamcc 540
   cttaaaattg taaccygcct ttttcccttt gctytcmatt aaagtctatt cmaaag     596
<210> 160
   <211> 515
   <212> DNA
   <213> Homo sapiens
<400> 160
   gggggtaggc tctttattag acggttattg ctgtactaca gggtcagagt gcagtgtaag 60
   cagtgtcaga ggcccgcgtt cagcccaaga atgtggattt tctctcccta ttgatcacag 120
   tgggtgggtt tcttcagaaa agccccagag gcagggacca gtgagctcca aggttagaag 180
   tggaactgga aggcttcagt cacatgctgc ttccacgctt ccaggctggg cagcaaggag 240
   gagatgccca tgacgtgcca ggtctcccca tctgacacca gtgaagtctg gtaggacagc 300
   agccgcacgc ctgcctctgc caggaggcca atcatggtag gcagcattgc agggtcagag 360
   gtctgagtcc ggaataggag caggggcagg tccctgcgga gaggcacttc tggcctgaag 420
   acagctccat tgagcccctg cagtacaggy gtagtgcctt ggaccaagcc cacagcctgg 480
   taaggggcgc ctgccagggc cacggccagg aggca                            515
<210> 161
   <211> 936
   <212> DNA
   <213> Homo sapiens
<400> 161
   taatttctta gtcgtttgga atccttaagc atgcaaaagc tttgaacaga agggttcaca 60
   aaggaaccag ggttgtctta tggcatccag ttaagccaga gctgggaatg cctctgggtc 120
   atccacatca ggagcagaag cacttgactt gtcggtcctg ctgccacggt ttgggcgccc 180
   accacgccca cgtccacctc gtcctcccct gccgccacgt cctgggcggc caaggtctcc 240
   aaaattgatc tccagctgag acgttatatc atttgctggc ttccggaaat gatggtccat 300
   aaccgaatct tcagcatgag cctcttcact ctttgattta tgaagaacaa atcccttctt 360
   ccactgccca tcagcacctt catttggttt tcggatatta aattctactt ttgcccggtc 420
   cttattttga atagccttcc actcatccaa agtcatctct tttggaccct cctcttttac 480
   ctcttcaact tcattctcct tattttcagt gtctgccact ggatgatgtt cttcaccttc 540
   aggtgtttcc tcagtcacat ttgattgatc caagtcagtt aattcgtctt tgacagttcc 600
   ccagttgtga gatccgctac ctccacgttt gtcctcgtgc ttcaggccag atctatcact 660
   tccactatgc ctatcaaatt cacgtttgcc acgagaatca aatccatctc ctcggcccat 720
   tccacgtcca cggccccctc gacctcttcc aagaccacca cgacctcgaa taggtcggtc 780
   aataatcggt ctatcaactg aaaattcgcc tccttcaccc ttttcttcaa gtggcttttc 840
   gaatcttcgt tcacgaggtg gtcgcctttc tggtcttcta tcaattattt tcccttcacc 900
   ctgaagttgt tgatcaggtc ttcttccaac tcgtgc 936
<210> 162
   <211> 950
   <212> DNA
   <213> Homo sapiens
<400> 162
   aagcggatgg acctgagtca gccgaatcct agccccttcc cttgggcctg ctgtggtgct 60
   cgacatcagt gacagacgga agcagcagac catcaaggct acgggaggcc cggggcgctt 120
   gcgaagatga agtttggctg cctctccttc cggcagcctt atgctggctt tgtcttaaat 180
   ggaatcaaga ctgtggagac gcgctggcgt cctctgctga gcagccagcg gaactgtacc 240
   atcgccgtcc acattgctca cagggactgg gaaggcgatg cctgtcggga gctgctggtg 300
   gagagactcg ggatgactcc tgctcagatt caggccttgc tcaggaaagg ggaaaagttt 360
   ggtcgaggag tgatagcggg actcgttgac attggggaaa ctttgcaatg ccccgaagac 420
   ttaactcccg atgaggttgt ggaactagaa aatcaagctg cactgaccaa cctgaagcag 480
   aagtacctga ctgtgatttc aaaccccagg tggttactgg agcccatacc taggaaagga 540
   ggcaaggatg tattccaggt agacatccca gagcacctga tccctttggg gcatgaagtg 600
   tgacaagtgt gggctcctga aaggaatgtt ccrgagaaac cagctaaatc atggcacctt 660
   caatttgcca tcgtgacgca gacctgtata aattaggtta aagatgaatt tccactgctt 720
   tggagagtcc cacccactaa gcactgtgca tgtaaacagg ttcctttgct cagatgaagg 780
   aagtaggggg tggggctttc cttgtgtgat gcctccttag gcacacaggc aatgtctcaa 840
   gtactttgac cttagggtag aaggcaaagc tgccagtaaa tgtctcagca ttgctgctaa 900
   ttttggtcct gctagtttct ggattgtaca aataaatgtg ttgtagatga 950
<210> 163
   <211> 475
   <212> DNA
   <213> Homo sapiens
<220>
   <221> misc_feature
   <222> 301, 317, 331, 458, 464, 470
   <223> n = A,T,C or G
<400> 163
   tcgagcggcc gcccgggcag gtgtcggagt ccagcacggg aggcgtggtc ttgtagttgt 60
   tctccggctg cccattgctc tcccactcca cggcgatgtc gctgggatag aagcctttga 120
   ccaggcaggt caggctgacc tggttcttgg tcatctcctc ccgggatggg ggcagggtgt 180
   acacctgtgg ttctcggggc tgccctttgg ctttggagat ggttttctcg atgggggctg 240
   ggagggcttt gttggagacc ttgcacttgt actccttgcc attcaaccag tcctggtgca 300
   ngacggtgag gacgctnacc acacggtacg ngctggtgta ctgctcctcc cgcggctttg 360
   tcttggcatt atgcacctcc acgccgtcca cgtaccaatt gaacttgacc tcagggtctt 420
   cgtggctcac gtccaccacc acgcatgtaa cctcaaanct cggncgcgan cacgc      475
<210> 164
   <211> 476
   <212> DNA
   <213> Homo sapiens
<400> 164
   agcgtggtcg cggccgaggt ctgaggttac atgcgtggtg gtggacgtga gccacgaaga 60
   ccctgaggtc aagttcaact ggtacgtgga cggcgtggag gtgcataatg ccaagacaaa 120
   gccgcgggag gagcagtaca acagcacgta ccgtgtggtc agcgtcctca ccgtcctgca 180
   ccaggactgg ctgaatggca aggagtacaa gtgcaaggtc tccaacaaag ccctcccagc 240
   ccccatcgag aaaaccatct ccaaagccaa agggcagccc cgagaaccac aggtgtacac 300
   cctgccccca tcccgggagg agatgaccaa gaaccaggtc agcctgacct gcctggtcaa 360
   aggcttctat cccagcgaca tcgcccgtgg agtgggagag caatgggcag ccggagaaca 420
   actacaagac cacgcctccc gtgctggact ccgacacctg ccgggcggcc gctcga 476
<210> 165
   <211> 256
   <212> DNA
   <213> Homo sapiens
<220>
   <221> misc_feature
   <222> 10, 37, 249
   <223> n = A,T,C or G
<400> 165
   agcgtggttn cggccgaggt cccaaccaag gctgcancct ggatgccatc aaagtcttct 60
   gcaacatgga gactggtgag acctgcgtgt accccactca gcccagtgtg gcccagaaga 120
   actggtacat cagcaagaac cccaaggaca agaggcatgt ctggttcggc gagagcatga 180
   ccgatggatt ccagttcgag tatggcggcc agggctccga ccctgccgat gtggacctgc 240
   ccgggcggnc gctcga                                                256

<210> 166
   <211> 332
   <212> DNA
   <213> Homo sapiens
<400> 166
   agcgtggtcg cggccgaggt caagaacccc gcccgcacct gccgtgacct caagatgtgc 60
   cactctgact ggaagagtgg agagtactgg attgacccca accaaggctg caacctggat 120
   gccatcaaag tcttctgcaa catggagact ggtgagacct gcgtgtaccc cactcagccc 180
   agtgtggccc agaagaactg gtacatcagc aagaacccca aggacaagag gcatgtctgg 240
   ttcggcgaga gcatgaccga tggattccag ttcgagtatg gcggccaggg ctccgaccct 300
   gccgatgtgg acctgcccgg gcggccgctc ga                               332
<210> 167
   <211> 332
   <212> DNA
   <213> Homo sapiens
<220>
   <221> misc_feature
   <222> 77, 109, 136, 184, 198
   <223> n = A,T,C or G
<400> 167
   tcgagcggtc gcccgggcag gtccacatcg gcagggtcgg agccctggcc gccatactcg 60
   aactggaatc catcggncat gctctcgccg aaccagacat gcctcttgnc cttggggttc 120
   ttgctgatgt accagntctt ctgggccaca ctgggctgag tggggtacac gcaggtctca 180
   ccantctcca tgttgcanaa gactttgatg gcatccaggt tgcagccttg gttggggtca 240
   atccagtact ctccactctt ccagacagag tggcacatct tgaggtcacg gcaggtgcgg 300
   gcggggttct tgacctcggt cgcgaccacg ct                               332
<210> 168
   <211> 276
   <212> DNA
   <213> Homo sapiens
<220>
   <221> misc_feature
   <222> 72, 84
   <223> n = A, T, C or G
<400> 168
   tcgagcggcc gcccgggcag gtcctcctca gagcggtagc tgttcttatt gccccggcag 60
   cctccataga tnaagttatt gcangagttc ctctccacgt caaagtacca gcgtgggaag 120
   gatgcacggc aaggcccagt gactgcgttg gcggtgcagt attcttcata gttgaacata 180
   tcgctggagt ggacttcaga atcctgcctt ctgggagcac ttgggacaga ggaatccgct 240
   gcattcctgc tggtggacct cggccgcgac cacgct                           276
<210> 169
   <211> 276
   <212> DNA
   <213> Homo sapiens
<400> 169
   agcgtggtcg cggccgaggt ccaccagcag gaatgcagcg gattcctctg tcccaagtgc 60
   tcccagaagg caggattctg aagaccactc cagcgatatg ttcaactatg aagaatactg 120
   caccgccaac gcagtcactg ggccttgccg tgcatccttc ccacgctggt actttgacgt 180
   ggagaggaac tcctgcaata acttcatcta tggaggctgc cggggcaata agaacagcta 240
   ccgctctgag gaggacctgc ccgggcggcc gctcga 276
<210> 170
   <211> 332
   <212> DNA
   <213> Homo sapiens
<220>
   <221> misc_feature
   <222> 294
   <223> n = A,T,C or G
<400> 170
   tcgagcggcc gcccgggcag gtccacatcg gcagggtcgg agccctggcc gccatactcg 60
   aactggaatc catcggtcat gctctcgccg aaccagacat gcctcttgtc cttggggttc 120
   ttgctgatgt accagttctt ctgggccaca ctgggctgag tggggtacac gcaggtctca 180
   ccagtctcca tgttgcagaa gactttgatg gcatccaggt tgcagccttg gttggggtca 240
   atccagtact ctccactctt ccagccagaa tggcacatct tgaggtcacg gcangtgcgg 300
   gcggggttct tgacctcggc cgcgaccacg ct 332
<210> 171
   <211> 333
   <212> DNA
   <213> Homo sapiens
<400> 171
   agcgtggtcg cggccgaggt caagaaaccc cgcccgcacc tgccgtgacc tcaagatgtg 60
   ccactctggc tggaagagtg gagagtactg gattgacccc aaccaaggct gcaacctgga 120
   tgccatcaaa gtcttctgca acatggagac tggtgagacc tgcgtgtacc ccactcagcc 180
   cagtgtggcc cagaagaact ggtacatcag caagaacccc aaggacaaga ggcatgtctg 240
   gctcggcgag agcatgaccg atggattcca gttcgagtat ggcggccagg gctccgaccc 300
   tgccgatgtg gacctgcccg ggcggccgct cga 333
<210> 172
   <211> 527
   <212> DNA
   <213> Homo sapiens
<220>
   <221> misc_feature
   <222> 46, 125, 140, 148, 220, 229, 291, 388, 456
   <223> n = A,T,C or G
<400> 172
   agcgtggtcg cggccgaggt cctgtcagag tggcactggt agaagntcca ggaaccctga 60
   actgtaaggg ttcttcatca gtgccaacag gatgacatga aatgatgtac tcagaagtgt 120
   cctgnaatgg ggcccatgan atggttgnct gagagagagc ttcttgtcct acattcggcg 180
   ggtatggtct tggcctatgc cttatggggg tggccgttgn gggcggtgng gtccgcctaa 240
   aaccatgttc ctcaaagatc atttgttgcc caacactggg ttgctgacca naagtgccag 300
   gaagctgaat accatttcca gtgtcatacc cagggtgggt gacgaaaggg gtcttttgaa 360
   ctgtggaagg aacatccaag atctctgntc catgaagatt ggggtgtgga agggttacca 420
   gttggggaag ctcgctgtct ttttccttcc aatcangggc tcgctcttct gaatattctt 480
   cagggcaatg acataaattg tatattcggt tcccggttcc aggccag 527
<210> 173
   <211> 635
   <212> DNA
   <213> Homo sapiens
<220>
   <221> misc_feature
   <222> 444, 453, 517, 540, 546, 551, 573, 593
   <223> n = A,T,C or G
<400> 173
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaagaata atcagaagag cgagcccctg 240
   attggaagga aaaagacaga cgagcttccc caactggtaa cccttccaca ccccaatctt 300
   catggaccag agatcttgga tgttccttcc acagttcaaa agaccccttt cgtcacccac 360
   cctgggtatg acactggaaa tggtattcag cttcctggca cttctggtca gcaacccagt 420
   gttgggcaac aaatgatctt tgangaacat ggntttaggc ggaccacacc ggccacaacg 480
   ggcaccccca taaggcatag gccaagaaca tacccgncga atgtaggaca agaagctctn 540
   tctcanacaa ncatctcatg ggccccattc cangacactt ctgagtacat canttcatgg 600
   catcctggtg gcactgataa aaacccttac agtta 635
<210> 174
   <211> 572
   <212> DNA
   <213> Homo sapiens
<220>
   <221> misc_feature
   <222> 457, 511, 520, 552, 568
   <223> n = A,T,C or G
<400> 174
   agcgtggtcg cgggcgaggt cctgtcagag tggcactggt agaagttcca ggaaccctga 60
   actgtaaggg ttcttcatca gtgccaacag gatgacatga aatgatgtac tcagaagtgt 120
   cctggaatgg ggcccatgag atggttgtct gagagagagc ttcttgtcct acattcggcg 180
   ggtatggtct tggcctatgc cttatggggg tggccgttgt gggcggtgtg gtccgcctaa 240
   aaccatgttc ctcaaagatc atttgttgcc caacactggg ttgctgacca gaagtgccag 300
   gaagctgaat accatttcca gtgtcatacc cagggtgggt gacgaaaggg gtcttttgaa 360
   ctgtggaagg aacatccaag atctctggtc catgaagatt ggggtgtgga agggttacca 420
   gttggggaag ctcgtctgtc tttttccttc caatcanggg ctcgctcttc tgattattct 480
   tcagggcaat gacataaatt gtatattcgg ntcccgggtn cagccaataa taataaccct 540
   ctgtgacacc anggcggggc cgaagganca ct 572
<210> 175
   <211> 372
   <212> DNA
   <213> Homo sapiens
<220>
   <221> misc_feature
   <222> 247
   <223> n = A, T, C or G
<400> 175
   agcgtggtcg cggccgaggt cctcaccaga ggtaccacct acaacatcat agtggaggca 60
   ctgaaagacc agcagaggca taaggttcgg gaagaggttg ttaccgtggg caactctgtc 120
   aacgaaggct tgaaccaacc tacggatgac tcgtgctttg acccctacac agtttcccat 180
   tatgccgttg gagatgagtg ggaacgaatg tctgaatcag gctttaaact gttgtgccag 240
   tgcttangct ttggaagtgg tcatttcaga tgtgattcat ctagatggtg ccatgacaat 300
   ggtgtgaact acaagattgg agagaagtgg gaccgtcagg gagaaaatgg acctgcccgg 360
   gcggccgctc ga                                                     372
<210> 176
   <211> 372
   <212> DNA
   <213> Homo sapiens
<220>
   <221> misc_feature
   <222> 251
   <223> n = A, T, C or G
<400> 176
   tcgagcggcc gcccgggcag gtccattttc tccctgacgg tcccacttct ctccaatctt 60
   gtagttcaca ccattgtcat ggcaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ntgacagagt tgcccacggt aacaacctct tcccgaacct tatgcctctg 300
   ctggtctttc agtgcctcca ctatgatgtt gtaggtggta cctctggtga ggacctcggc 360
   cgcgaccacg ct                                                     372
<210> 177
   <211> 269
   <212> DNA
   <213> Homo sapiens
<220>
   <221> misc_feature
   <222> 94, 225
   <223> n = A,T,C or G
<400> 177
   agcgtggccg cggccgaggt ccattggctg gaacggcatc aacttggaag ccagtgatcg 60
   tctcagcctt ggttctccag ctaatggtga tggnggtctc agtagcatct gtcacacgag 120
   cccttcttgg tgggctgaca ttctccagag tggtgacaac accctgagct ggtctgcttg 180
   tcaaagtgtc cttaagagca tagacactca cttcatattt ggcgnccacc ataagtcctg 240
   atacaaccac ggaatgacct gtcaggaac                                   269
<210> 178
   <211> 529
   <212> DNA
   <213> Homo sapiens
<400> 178
   tcgagcggcc gcccgggcag gtcctcagac cgggttctga gtacacagtc agtgtggttg 60
   ccttgcacga tgatatggag agccagcccc tgattggaac ccagtccaca gctattcctg 120
   caccaactga cctgaagttc actcaggtca cacccacaag cctgagcgcc cagtggacac 180
   cacccaatgt tcagctcact ggatatcgag tgcgggtgac ccccaaggag aagaccggac 240
   caatgaaaga aatcaacctt gctcctgaca gctcatccgt ggttgtatca ggacttatgg 300
   cggccaccaa atatgaagtg agtgtctatg ctcttaagga cactttgaca agcagaccag 360
   ctcagggtgt tgtcaccact ctggagaatg tcagcccacc aagaagggct cgtgtgacag 420
   atgctactga gaccaccatc accattagct ggagaaccaa gactgagacg atcactggct 480
   tccaagttga tgccgttcca gccaatggac ctcggccgcg accacgctt            529

<210> 179
   <211> 454
   <212> DNA
   <213> Homo sapiens
<220>
   <221> misc_feature
   <222> 64
   <223> n = A,T,C or G
<400> 179
   agcgtggtcg cggccgaggt ctggccgaac tgccagtgta cagggaagat gtacatgtta 60
   tagntcttct cgaagtcccg ggccagcagc tccacggggt ggtctcctgc ctccaggcgc 120
   ttctcattct catggatctt cttcacccgc agcttctgct tctcagtcag aaggttgttg 180
   tcctcatccc tctcatacag ggtgaccagg acgttcttga gccagtcccg catgcgcagg 240
   gggaattcgg tcagctcaga gtccaggcaa ggggggatgt atttgcaagg cccgatgtag 300
   tccaagtgga gcttgtggcc cttcttggtg ccctccaagg tgcactttgt ggcaaagaag 360
   tggcaggaag agtcgaaggt cttgttgtca ttgctgcaca ccttctcaaa ctcgccaatg 420
   ggggctgggc agacctgccc gggcggccgc tcga                             454
<210> 180
   <211> 454
   <212> DNA
   <213> Homo sapiens
<220>
   <221> misc_feature
   <222> 55, 299, 317, 332, 342, 348
   <223> n = A,T,C or G
<400> 180
   tcgagcggcc gcccgggcag gtctgcccag cccccattgg cgagtttgag aaggngtgca 60
   gcaatgacaa caagaccttc gactcttcct gccacttctt tgccacaaag tgcaccctgg 120
   agggcaccaa gaagggccac aagctccacc tggactacat cgggccttgc aaatacatcc 180
   ccccttgcct ggactctgag ctgaccgaat tccccctgcg catgcgggac tggctcaaga 240
   acgtcctggt caccctgtat gagagggatg aggacaacaa ccttctgact gagaagcana 300
   agctgcgggt gaagaanatc catgagaatg anaagcgcct gnaggcanga gaccaccccg 360
   tggagctgct ggcccgggac ttcgagaaga actataacat gtacatcttc cctgtacact 420
   ggcagttcgg ccagacctcg gccgcgacca cgct 454
<210> 181
   <211> 102
   <212> DNA
   <213> Homo sapiens
<220>
<221> misc_feature
   <222> 8, 47, 60, 67
   <223> n = A,T,C or G
<400> 181
   agcgtggntg cggacgacgc ccacaaagcc attgtatgta gttttanttc agctgcaaan 60
   aataccncca gcatccacct tactaaccag catatgcaga ca 102
<210> 182
   <211> 337
   <212> DNA
   <213> Homo sapiens
<220>
   <221> misc_feature
   <222> 169, 195, 253, 314
   <223> n = A, T, C or G
<400> 182
   tcgagcggtc gcccgggcag gtctgggcgg atagcaccgg gcatattttg gaatggatga 60
   ggtctggcac cctgagcagc ccagcgagga cttggtctta gttgagcaat ttggctagga 120
   ggatagtatg cagcacggtt ctgagtctgt gggatagctg ccatgaagna acctgaagga 180
   ggcgctggct ggtangggtt gattacaggg ctgggaacag ctcgtacact tgccattctc 240
   tgcatatact ggntagtgag gcgagcctgg cgctcttctt tgcgctgagc taaagctaca 300
   tacaatggct ttgnggacct cggccgcgac cacgctt 337
<210> 183
   <211> 374
   <212> DNA
   <213> Homo sapiens
<400> 183
   tcgagcggcc gcccgggcag gtccattttc tccctgacgg tcccacttct ctccaatctt 60
   gtagttcaca ccattgtcat gacaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ttgacagaag ttgcccacgg taacaacctc ttcccgaacc ttatgcctct 300
   gctggtcttt caagtgcctc cactatgatg ttgtaggtgg cacctctggt gaggacctcg 360
   gccgcgacca cgct                                                   374
<210> 184
   <211> 375
   <212> DNA
   <213> Homo sapiens
<220>
   <221> misc_feature
   <222> 30, 174, 248, 285, 306, 332, 345, 368
   <223> n = A,T,C or G
<400> 184
   agcgtggttt gcggccgagg tcctcaccan aggtgccacc tacaacatca tagtggaggc 60
   actgaaagac cagcagaggc ataaggttcg ggaagaggtt gttaccgtgg gcaactctgt 120
   caacgaaggc ttgaaccaac ctacggatga ctcgtgcttt gacccctaca cagnttccca 180
   ttatgccgtt ggagatgagt gggaacgaat gtctgaatca ggctttaaac tgttgtgcca 240
   gtgcttangc tttggaagtg gtcatttcag atgtgattca tctanatggt gtcatgacaa 300
   tggtgngaac tacaagattg gagagaagtg gnaccgtcag ggganaaaat ggacctgccc 360
   gggcggcncg ctcga                                                  375
<210> 185
   <211> 148
   <212> DNA
   <213> Homo sapiens
<220>
   <221> misc_feature
   <222> 28, 36, 86
   <223> n = A,T,C or G
<400> 185
   agcgtggtcg cggccgaggt ctggcttnct gctcangtga ttatcctgaa ccatccaggc 60
   caaataagcg ccggctatgc ccctgnattg gattgccaca cggctcacat tgcatgcaag 120
   tttgctgagc tgaaggaaaa gattgatc                                    148
<210> 186
   <211> 397
   <212> DNA
   <213> Homo sapiens
<220>
   <221> misc_feature
   <222> 78
   <223> n = A,T,C or G
<400> 186
   tcgagcggcc gcccgggcag gtccaattga aacaaacagt tctgagaccg ttcttccacc 60
   actgattaag agtggggngg cgggtattag ggataatatt catttagcct tctgagcttt 120
   ctgggcagac ttggtgacct tgccagctcc agcagccttc tggtccactg ctttgatgac 180
   acccaccgca actgtctgtc tcatatcacg aacagcaaag cgacccaaag gtggatagtc 240
   tgagaagctc tcaacacaca tgggcttgcc aggaaccata tcaacaatgg gcagcatcac 300
   cagacttcaa gaatttaagg gccatcttcc agctttttac cagaacggcg atcaatcttt 360
   tccttcagct cagcaaactt gcatgcaatg tgagccg                          397
<210> 187
   <211> 584
   <212> DNA
   <213> Homo sapiens
<220>
   <221> misc_feature
   <222> 145, 286, 363, 365, 425, 433, 452, 462, 471, 512, 514, 534, 536, 590, 565, 583
   <223> n = A,T,C or G
<400> 187
   tcgagcggcc gcccgggcag gtccagaggg ctgtgctgaa gtttgctgct gccactggag 60
   ccactccaat tgctggccgc ttcactcctg gaaccttcac taaccagatc caggcagcct 120
   tccgggagcc acggcttctt gtggntactg accccagggc tgaccaccag cctctcacgg 180
   aggcatctta tgttaaccta cctaccattg cgctgtgtaa cacagattct cctctgcgct 240
   atgtggacat tgccatccca tgcaacaaca agggagctca ctcagngggg tttgatgtgg 300
   tggatgctgg ctcgggaagt tctgcgcatg cgtggcacca tttcccgtga acacccatgg 360
   gangncatgc ctgatctgga cttctacaga gatcctgaag agattgaaaa agaagaacag 420
   gctgnttgct ganaaagcaa gtgaccaagg angaaatttc angggtgaaa nggactgctc 480
   ccgctcctga attcactgct actcaacctg angntgcaga ctggtcttga aggngnacan 540
   gggccctctg ggcctattta agcancttcg gtcgcgaaca cgnt                  584
<210> 188
   <211> 579
   <212> DNA
   <213> Homo sapiens
<220>
   <221> misc_feature
   <222> 7, 136, 486
   <223> n = A, T, C or G
<400> 188
   agcgtgngtc gcggccgagg tgctgaatag gcacagaggg cacctgtaca ccttcagacc 60
   agtctgcaac ctcaggctga gtagcagtga actcaggagc gggagcagtc cattcaccct 120
   gaaattcctc cttggncact gccttctcag cagcagcctg ctcttctttt tcaatctctt 180
   caggatctct gtagaagtac agatcaggca tgacctccca tgggtgttca cgggaaatgg 240
   tgccacgcat gcgcagaact tcccgagcca gcatccacca catcaaaccc actgagtgag 300
   ctcccttgtt gttgcatggg atgggcaatg tccacatagc gcagaggaga atctgtgtta 360
   cacagcgcaa tggtaggtag gttaacataa gatgcctccg cgagaagctg gtggtcagcc 420
   ctggggtcaa gtaaccacaa gaagccgtgg ctcccggaag gctgcctgga tctggttagt 480
   gaaggntcca ggagtgaagc ggccaacaat tggagtggct tcagtggcaa gcagcaaact 540
   tcagcacaag ccctctggac ctgcccggcg gccgctcga                        579
<210> 189
   <211> 374
   <212> DNA
   <213> Homo sapiens
<220>
   <221> misc_feature
   <222> 41, 280, 314, 330, 350, 353
   <223> n = A,T,C or G
<400> 189
   tcgagcggcc gcccgggcag gtccattttc tccctgacgg ncccacttct ctccaatctt 60
   gtagttcaca ccattgtcat ggcaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ttgacagagt tgcccacggt aacaacctcn tccccgaacc ttatgcctct 300
   gctgggcttt cagngcctcc actatgatgn tgtagggggg cacctctggn gangacctcg 360
   gccgcgacca cgct                                                   374
<210> 190
   <211> 373
   <212> DNA
   <213> Homo sapiens
<220>
   <221> misc_feature
   <222> 247, 304, 306, 332, 337
   <223> n = A, T, C or G
<400> 190
   agcgtggtcg cggccgaggt cctcaccaga ggtgccacct acaacatcat agtggaggca 60
   ctgaaagacc agcagaggca taaggctcgg gaagaggttg ttaccgtggg caactctgtc 120
   aacgaaggct tgaaccaacc tacggatgac tcgtgctttg acccctacac agtttcccat 180
   tatgccgttg gagatgagtg ggaacgaatg tctgaatcag gctttaaact gttgtgccag 240
   tgcttangct ttggaagtgg gtcatttcag atgtgattca tctagatggt gccatgacaa 300
   tggngngaac tacaagattg gagagaagtg gnaccgncag ggagaaaatg gacctgcccg 360
   ggcggccgct cga                                                    373

<210> 191
   <211> 354
   <212> DNA
   <213> Homo sapiens
<220>
   <221> misc_feature
   <222> 218, 299, 306, 326, 333, 337, 341
   <223> n = A,T,C or G
<400> 191
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacacgc aggtctcacc 180
   agtctccatg ttgcagaaga ctttgatggc atccaggntg caaccttggt tggggtcaat 240
   ccagtactct ccactcttcc agccagagtg gcacatcttg aggtcacggc aggtgcggnc 300
   gggggntttt gcggctgccc tctggncttc ggntgtnctc natctgctgg ctca       354
<210> 192
   <211> 587
   <212> DNA
   <213> Homo sapiens
<220>
   <221> misc_feature
   <222> 276
   <223> n = A,T,C or G
<400> 192
   tcgagcggcc gcccgggcag gtctcgcggt cgcactggtg atgctggtcc tgttggtccc 60
   cccggccctc ctggacctcc tggcccccct ggtcctccca gcgctggttt cgacttcagc 120
   ttcctgcccc agccacctca agagaaggct cacgatggtg gccgctacta ccgggctgat 180
   gatgccaatg tggttcgtga ccgtgacctc gaggtggaca ccaccctcaa gagcctgagc 240
   cagcagatcg agaacatccg gagcccagag ggcagncgca agaaccccgc ccgcacctgc 300
   cgtgacctca agatgtgcca ctctgactgg aagagtggag agtactggat tgaccccaac 360
   caagctgcaa cctggatgcc atcaaagtct tctgcaacat ggagactggt gagacctgcg 420
   tgtaccccac tcagcccagt gtggcccaaa agaactggta catcagcaag aaccccaagg 480
   acaagaagca tgtctggttc ggcgagaaca tgaccgatgg attccagttc gagtatggcg 540
   ggcagggctc cgaccctgcc gatggggacc ttggccgcga acacgct               587
<210> 193
   <211> 98
   <212> DNA
   <213> Homo sapiens
<220>
   <221> misc_feature
   <222> 8, 9, 33, 58, 71, 90
   <223> n = A,T,C or G
<400> 193
   agcgtggnng cggccgaggt ataaatatcc agnccatatc ctccctccac acgctganag 60
   atgaagctgt ncaaagatct cagggtggan aaaaccat                         98
<210> 194
   <211> 240
   <212> DNA
   <213> Homo sapiens
<400> 194
   tcgagcggcc gcccgggcag gtccttcaga cttggactgt gtcacactgc caggcttcca 60
   gggctccaac ttgcagacgg cctgttgtgg gacagtctct gtaatcgcga aagcaaccat 120
   ggaagacctg ggggaaaaca ccatggtttt atccaccctg agatctttga acaacttcat 180
   ctctcagcgt gcggagggag gctctggact ggatatttct acctcggccg cgaccacgct 240
<210> 195
   <211> 400
   <212> DNA
   <213> Homo sapiens
<220>
   <221> misc_feature
   <222> 22, 37, 39, 105, 268, 276, 302, 323, 331, 335, 347, 351, 371, 378
   <223> n = A,T,C or G
<400> 195
   cgagcgggcg accgggcagg tncagactcc aatccanana accatcaagc cagatgtcag 60
   aagctacacc atcacaggtt tacaaccagg cactgactac aaganctacc tgcacacctt 120
   gaatgacaat gctcggagct cccctgtggt catcgacgcc tccactgcca ttgatgcacc 180
   atccaacctg cgtttcctgg ccaccacacc caattccttg ctggtatcat ggcagccgcc 240
   acgtgccagg attaccggta catcatcnag tatganaagc ctgggcctcc tcccagagaa 300
   gnggtccctc ggccccgccc tgntgtccca naggntacta ttactgngcc ngcaaccggc 360
   aaccgatatc nattttgnca ttggccttca acaataatta                       400
<210> 196
   <211> 494
   <212> DNA
   <213> Homo sapiens
<220>
   <221> misc_feature
   <222> 19, 83, 168, 252, 271, 292, 43.0
   <223> n = A,T,C or G
<400> 196
   agcgtggttc gcggccgang tcctgtcaga gtggcactgg tagaagttcc aggaaccctg 60
   aactgtaagg gttcttcatc agngccaaca ggatgacatg aaatgatgta ctcagaagtg 120
   tcctggaatg gggcccatga gatggttgtc tgagagagag cttcttgncc tgtctttttc 180
   cttccaatca ggggctcgct cttctgatta ttcttcaggg caatgacata aattgtatat 240
   tcgggtcccg gntccaggcc agtaatagta ncctctgtga caccagggcg gngccgaggg 300
   accacttctc tgggaggaga cccaggcttc tcatacttga tgatgtaacc ggtaatcctg 360
   gcacgtggcg gctgccatga taccagcaag gaattggggt gtggtggcca ggaaacgcag 420
   gttggatggn gcatcaatgg cagtggaggc cgtcgatgac cacaggggga gctccgacat 480
   tgtcattcaa ggtg                                                   494
<210> 197
   <211> 118
   <212> DNA
   <213> Homo sapiens
<220>
   <221> misc_feature
   <222> 8, 71, 96
   <223> n = A,T,C or G
<400> 197
   agcgtggncg cggccgaggt gcagcgcggg ctgtgccacc ttctgctctc tgcccaacga 60
   taaggagggt ncctgccccc aggagaacat taactntccc cagctcggcc tctgccgg 118
<210> 198
   <211> 403
   <212> DNA
   <213> Homo sapiens
<220>
   <221> misc_feature
   <222> 41, 53, 98, 195, 350
   <223> n = A, T, C or G
<400> 198
   tcgagcggcc gcccgggcag gttttttttg ctgaaagtgg ntactttatt ggntgggaaa 60
   gggagaagct gtggtcagcc caagagggaa tacagagncc cgaaaaaggg gagggcaggt 120
   gggctggaac cagacgcagg gccaggcaga aactttctct cctcactgct cagcctggtg 180
   gtggctggag ctcanaaatt gggagtgaca caggacacct tcccacagcc attgcggcgg 240
   catttcatct ggccaggaca ctggctgtcc acctggcact ggtcccgaca gaagcccgag 300
   ctggggaaag ttaatgttca cctgggggca ggaaccctcc ttatcattgn gcagagagca 360
   gaaggtggca cagcccgcgc tgcacctcgg ccgcgaccac gct 403
<210> 199
   <211> 167
   <212> DNA
   <213> Homo sapiens
<220>
   <221> misc_feature
   <222> 92, 107
   <223> n = A,T,C or G
<400> 199
   tcgagcggcc gcccgggcag gtccaccata agtcctgata caaccacgga tgagctgtca 60
   ggagcaaggt tgatttcttt cattggtccg gncttctcct tgggggncac ccgcactcga 120
   tatccagtga gctgaacatt gggtggcgtc cactgggcgc tcaggct 167
<210> 200
   <211> 252
   <212> DNA
   <213> Homo sapiens
<220>
   <221> misc_feature
   <222> 210, 226, 227, 230, 236
   <223> n = A,T,C or G
<400> 200
   tcgagcggtt cgcccgggca ggtccaccac acccaattcc ttgctggtat catggcagcc 60
   gccacgtgcc aggattaccg gctacatcat caagtatgag aagcctgggt ctcctcccag 120
   agaagcggtc cctcggcccc gccctggtgt cacagaggct actattactg gcctggaacc 180
   gggaaccgaa tatacaattt atgtcattgn cctgaagaat aatcannaan agcgancccc 240
   tgattggaag ga                                                     252

<210> 201
   <211> 91
   <212> DNA
   <213> Homo sapiens
<400> 201
   agcgtggtcg cggccgaggt tgtacaagct tttttttttt tttttttttt tttttttttt 60
   tttttttttt tttttttttt tttttttttt t                                91
<210> 202
   <211> 368
   <212> DNA
   <213> Homo sapiens
<220>
   <221> misc_feature
   <222> 9, 354
   <223> n = A,T,C or G
<400> 202
   tcgagcggnc gcccgggcag gtctgccaac accaagattg gcccccgccg catccacaca 60
   gtccgtgtgc ggggaggtaa caagaaatac cgtgccctga ggttggacgt ggggaatttc 120
   tcctggggct cagagtgttg tactcgtaaa acaaggatca tcgatgttgt ctacaatgca 180
   tctaataacg agctggttcg taccaagacc ctggtgaaga attgcatcgt gctcatcgac 240
   agcacaccgt accgacagtg gtacgagtcc cactatgcgc tgcccctggg ccgcaagaag 300
   ggagccaagc tgactcctga ggaagaagag attttaaaca aaaaacgatc taanaaaaaa 360
   aaaacaat                                                          368
<210> 203
   <211> 340
   <212> DNA
   <213> Homo sapiens
<400> 203
   agcgtggtcg cggccgaggt gaaatggtat tcagcttcct ggcacttctg gtcagcaacc 60
   cagtgttggg caacaaatga tctttgagga acatggtttt aggcggacca caccgcccac 120
   aacggccacc cccataaggc ataggccaag accatacccg ccgaatgtag gacaagaagc 180
   tctctctcag acaaccatct catgggcccc attccaggac acttctgagt acatcatttc 240
   atgtcatcct gttggcactg atgaagaacc cttacagttc agggttcctg gaacttctac 300
   cagtgccact ctgacaggac ctgcccgggc ggccgctcga                       340
<210> 204
   <211> 341
   <212> DNA
   <213> Homo sapiens
<400> 204
   tcgagcggcc gcccgggcag gtcctgtcag agtggcactg gtagaagttc caggaaccct 60
   gaactgtaag ggttcttcat cagtgccaac aggatgacat gaaatgatgt actcagaagt 120
   gtcctggaat ggggcccatg agatggttgt ctgagagaga gcttcttgtc ctacattcgg 180
   cgggtatggt cttggcctat gccttatggg ggtggccgtt gtgggcggtg tggtccgcct 240
   aaaaccatgt tcctcaaaga tcatttgttg cccaacactg ggttgctgac cagaagtgcc 300
   aggaagctga ataccatttc acctcggccg cgaccacgct a                     341
<210> 205
   <211> 770
   <212> DNA
   <213> Homo sapiens
<220>
   <221> misc_feature
   <222> 529, 591, 623, 626, 629, 630, 656, 702, 709, 712, 717, 743, 746, 749, 759, 762, 766
   <223> n = A,T,C or G
<400> 205
   tcgagcggcc gcccgggcag gtctcccttc ttgcggccca ggggcagcgc atagtgggac 60
   tcgtaccact gtcggtacgg tgtgctgtcg atgagcacga tgcaattctt caccagggtc 120
   ttggtacgaa ccagctcgtt attagatgca ttgtagacaa catcgatgat ccttgtttta 180
   cgagtacaac actctgagcc ccaggagaaa ttccccacgt ccaacctcag ggcacggtat 240
   ttcttgttac ctccccgcac acggactgtg tggatgcggc gggggccaag ctgactcctg 300
   aggaagaaga gattttaaac aaaaaacgat ctaaaaaaat tcagaagaaa tatgatgaaa 360
   ggaaaaagaa tgccaaaatc agcagtctcc tggaggagca gttccagcag ggcaagcttc 420
   ttgcgtgcat cgcttcaagg ccgggacagt gtgaccgagc agatggctat gtgctagagg 480
   gcaaagaagt ggagttctat cttaagaaaa tcagggccca gaatggtgng tcttcaacta 540
   atccaaaggg gagtttcaga ccagtgcaat cagcaaaaac attgatactg ntggccaaat 600
   ttattggtgc agggcttgca cantangann ggctgggtct tggggcttgg attggnacaa 660
   gctttggcag ccttttcttt ggttttgcca aaaacctttt gntgaagang anacctnggg 720
   cggacccctt aaccgattcc acnccnggng gcgttctang gncccncttg            770
<210> 206
   <211> 810
   <212> DNA
   <213> Homo sapiens
<220>
   <221> misc_feature
   <222> 574, 621, 625, 636, 668, 673, 704, 728, 743, 767, 772, 786, 789, 807, 809, 810
   <223> n = A,T,C or G
<400> 206
   agcgtggtcg cggccgaggt ctgctgcttc agcgaagggt ttctggcata accaatgata 60
   aggctgccaa agactgttcc aataccagca ccagaaccag ccactcctac tgttgcagca 120
   cctgcaccaa taaatttggc agcagtatca atgtctctgc tgattgcact ggtctgaaac 180
   tccctttgga ttagctgaga cacaccattc tgggccctga ttttcctaag atagaactcc 240
   aactctttgc cctctagcac atagccatct gctcggtcac actgtcccgg ccttgaagcg 300
   atgcacgcaa gaagcttgcc ctgctggaac tgctcctcca ggagactgct gattttggca 360
   ttctttttcc tttcatcata tttcttctga atttttttag atcgtttttt gtttaaaatc 420
   tcttcttcct caggagtcag cttggccccc gccgcatcca cacagtccgt gtgcggggag 480
   gtaacaagaa ataccgtgcc ctgaggttgg acgtggggaa tttctcctgg ggctcagagt 540
   ggtgtactcg taaaacaagg atcatcgatg gtgnctacaa tgcatctaat aacgagctgg 600
   gtcggaccca aagaacctgg ngaanaaatg gatcgnctca tcgacaggac accgtacccg 660
   acaggggnac gantcccact atgcgcttgc ccctgggccg caanaaagga aaactgcccg 720
   ggcggccntc gaaagcccaa ttntggaaaa aatccatcac actgggnggc cngtcgagca 780
   tgcatntana ggggcccatt ccccctnann 810
<210> 207
   <211> 257
   <212> DNA
   <213> Homo sapiens
<400> 207
   tcgagcggcc gcccgggcag gtccccaacc aaggctgcaa cctggatgcc atcaaagtct 60
   tctgcaacat ggagactggt gagacctgcg tgtaccccac tcagcccagt gtggcccaga 120
   agaactggta catcagcaag aaccccaagg acaagaggca tgtctggttc ggcgagagca 180
   tgaccgatgg attccagttc gagtatggcg gccagggctc cgaccctgcc gatgtggacc 240
   tcggccgcga ccacgct 257
<210> 208
   <211> 257
   <212> DNA
   <213> Homo sapiens
<400> 208
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacacgc aggtctcacc 180
   agtctccatg ttgcagaaga ctttgatggc atccaggttg cagccttggt tggggacctg 240
   cccgggcggc cgctcga 257
<210> 209
   <211> 747
   <212> DNA
   <213> Homo sapiens
<220>
   <221> misc_feature
   <222> 453, 538, 540, 542, 546, 554, 556, 598, 659, 670, 679, 689, 693, 711, 723, 724, 731, 747
   <223> n = A,T,C or G
<400> 209
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaagaata atcagaagag cgagcccctg 240
   attggaagga aaaagacaga cgagcttccc caactggtaa cccttccaca ccccaatctt 300
   catggaccag agatcttgga tgttccttcc acagttcaaa agaccccttt cgtcacccac 360
   cctgggtatg acactggaaa tggtattcag cttcctggca cttctggtca gcaacccagt 420
   gttgggcaac aaatgatctt tgaggaacat ggntttaggc ggaccacacc gcccacaacg 480
   gccaccccca taaggcatag gccaagacca tacccgccga atgtaggaca agaagctntn 540
   tntcanacac catntnatgg gccccattcc aggacacttc tgagtacatc atttatgnca 600
   tctgtggcac ttgatgaaaa cccttacagt tcagggttct ggaactttta ccaggcctnt 660
   tacaggactn ggccggacnc cttaagccna ttncaccctg gggcgttcta nggtcccact 720
   cgnncactgg ngaaaatggc tactgtn                                     747
<210> 210
   <211> 872
   <212> DNA
   <213> Homo sapiens
<220>
   <221> misc_feature
   <222> 165, 174, 181, 256, 260, 269, 271, 277, 286, 289, 294, 298, 300, 301, 303, 308, 311, 321, 325, 328, 329, 333, 338, 342, 346, 349, 351, 357, 359, 364, 366, 379, 385, 395, 396, 397, 407, 408, 410, 414, 415, 429, 431, 434, 435, 440, 443
   <223> n = A,T,C or G
   <221> misc_feature
   <222> 444, 446, 447, 448, 449, 450, 451, 464, 470, 472, 475, 479, 483, 484, 485, 488, 494, 496, 497, 504, 508, 509, 511, 513, 517, 522, 524, 526, 532, 533, 542, 543, 553, 559, 566, 567, 571, 572, 578, 582, 588, 591, 594, 595, 596, 600, 606
   <223> n = A,T,C or G
<221> misc_feature
   <222> 612, 614, 617, 618, 629, 630, 631, 652, 654, 655, 661, 663, 664, 666, 671, 673, 678, 679, 681, 688, 690, 691, 698, 706, 707, 708, 714, 719, 721, 723, 726, 741, 751, 761, 762, 769, 770, 778, 779, 781, 782, 785, 791, 802, 807, 808, 812
   <223> n = A,T,C or G
<221> misc_feature
   <222> 815, 820, 827, 828, 838, 841, 844, 851, 857, 864, 866, 869, 872
   <223> n = A,T,C or G
<400> 210
   agcgtggtcg cggccgaggt ccactagagg tctgtgtgcc attgcccagg cagagtctct 60
   gcgttacaaa ctcctaggag ggcttgctgt gcggagggcc tgctatggtg tgctgcggtt 120
   catcatggag agtggggcca aaggctgcga ggttgtggtg tctgngaaac tccnaggaca 180
   ngagggctaa attccatgaa gtttgtggat ggcctgatga tccacaatcg gagaccctgt 240
   taactactac cgtctnaccn cctgctgtnc ncccccnttt ctgctnaana catngggntn 300
   ntncttgncc ntccttgggt ngaanatnna atngcctncc cnttcntanc nctactngnt 360
   ccananttgg cctttaaana atccnccttg ccttnnncac tgttcanntn tttnntcgta 420
   aaccctatna nttnnattan atnntnnnnn nctcaccccc ctcntcattn anccnatang 480
   ctnnnaantc cttnanncct cccncccnnt ncnctcntac tnantncttc tnncccatta 540
   cnnagctctt tcntttaana taatgnngcc nngctctnca tntctacnat ntgnnnaatn 600
   cccccncccc cnancgnntt tttgacctnn naacctcctt tcctcttccc tncnnaaatt 660
   ncnnanttcc ncnttccnnc ntttcggntn ntcccatnct ttccannnct tcantctanc 720
   ncnctncaac ttattttcct ntcatccctt nttctttaca nnccccctnn tctactcnnc 780
   nnttncatta natttgaaac tnccacnnct anttncctcn ctctacnntt ttattttncg 840
   ntcnctctac ntaatanttt aatnanttnt cn                               872

<210> 211
   <211> 517
   <212> DNA
   <213> Homo sapiens
<220>
   <221> misc_feature
   <222> 462, 464, 506
   <223> n = A,T,C or G
<400> 211
   tcgagcggcc gcccgggcag gtctgccaag gagaccctgt tatgctgtgg ggactggctg 60
   gggcatggca ggcggctctg gcttcccacc cttctgttct gagatggggg tggtgggcag 120
   tatctcatct ttgggttcca caatgctcac gtggtcaggc aggggcttct tagggccaat 180
   cttaccagtt gggtcccagg gcagcatgat cttcaccttg atgcccagca caccctgtct 240
   gagcaacacg tggcgcacaa gcagtgtcaa cgtagtaagt taacagggtc tccgctgtgg 300
   atcatcaggc catccacaaa cttcatggat ttagccctct gtcctcggag tttcccagac 360
   accacaacct cgcagccttt ggccccactc tccatgatga accgcagcac accatagcag 420
   gccctccgca caagcaagcc ctcctaagaa tttgtaacgc ananactctg ctggcaatgg 480
   cacacaaacc tctagtggac ctcggncgcg accacgc                          517
<210> 212
   <211> 695
   <212> DNA
   <213> Homo sapiens
<220>
   <221> misc_feature
   <222> 432, 476, 522, 547, 621, 624, 647, 679
   <223> n = A,T,C or G
<400> 212
   tcgagcggcc gcccgggcag gtctggtcca ggatagcctg cgagtcctcc tactgctact 60
   ccagacttga catcatatga atcatactgg ggagaatagt tctgaggacc agtagggcat 120
   gattcacaga ttccaggggg gccaggagaa ccaggggacc ctggttgtcc tggaatacca 180
   gggtcaccat ttctcccagg aataccagga gggcctggat ctcccttggg gccttgaggt 240
   ccttgaccat taggagggcg agtaggagca gttggaggct gtgggcaaac tgcacaacat 300
   tctccaaatg gaatttctgg gttggggcag tctaattctt gatccgtcac atattatgtc 360
   atcgcagaga acggatcctg agtcacagac acatatttgg catggttctg gcttccagac 420
   atctctatcc gncataggac tgaccaagat gggaacatcc tccttcaaca agcttnctgt 480
   tgtgccaaaa ataatagtgg gatgaagcag accgagaagt anccagctcc cctttttgca 540
   caaagcntca tcatgtctaa atatcagaca tgagacttct ttgggcaaaa aaggagaaaa 600
   agaaaaagca gttcaaagta nccnccatca agttggttcc ttgcccnttc agcacccggg 660
   ccccgttata aaacacctng ggccggaccc ccctt                            695
<210> 213
   <211> 804
   <212> DNA
   <213> Homo sapiens
<220>
   <221> misc_feature
   <222> 552, 555, 592, 624, 629, 633, 658, 695, 697, 698, 700, 702, 745, 753, 755, 762, 773, 786, 788, 793, 795
   <223> n = A,T,C or G
<400> 213
   agcgtggtcg cggccgaggt gttttatgac gggcccggtg ctgaagggca gggaacaact 60
   tgatggtgct actttgaact gcttttcttt tctccttttt gcacaaagag tctcatgtct 120
   gatatttaga catgatgagc tttgtgcaaa aggggagctg gctacttctc gctctgcttc 180
   atcccactat tattttggca caacaggaag ctgttgaagg aggatgttcc catcttggtc 240
   agtcctatgc ggatagagat gtctggaagc cagaaccatg ccaaatatgt gtctgtgact 300
   caggatccgt tctctgcgat gacataatat gtgacgatca agaattagac tgccccaacc 360
   cagaaattcc atttggagaa tgttgtgcag tttgcccaca gcctccaact gctcctactc 420
   gccctcctaa tggtcaagga cctcaaggcc ccaagggaga tccaggccct cctggtattc 480
   ctgggagaaa tggtgaccct ggtattccag gacaaccagg gtcccctggt tctcctggcc 540
   cccctggaat cnggngaatc atgccctact ggtcctcaaa ctattctccc anatgattca 600
   tatgatgtca agtctgggat agcnagtang ganggactcg caggctattc tggaccanac 660
   ctgccggggg ggcgttcgaa agcccgaatc tgcananntn cnttcacact ggcggccgtc 720
   gagctgcttt aaaagggcca ttccnccttt agngnggggg antacaatta ctnggcggcg 780
   ttttanancg cgngnctggg aaat                                        804
<210> 214
   <211> 594
   <212> DNA
   <213> Homo sapiens
<220>
   <221> misc_feature
   <222> 452, 509, 585
   <223> n = A,T,C or G
<400> 214
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacacgc aggtctcacc 180
   agtctccatg ttgcagaaga ctttgatggc atccaggttg cagccttggt tggggtcaat 240
   ccagtactct ccactcttcc agtcagagtg gcacatcttg aggtcacggc aggtgcgggc 300
   ggggttcttg cggctgccct ctgggctccg gatgttctcg atctgctggc tcaggctctt 360
   gagggtggtg tccacctcga ggtcacggtc acgaaccaca ttggcatcat cagcccggta 420
   gtagcggcca ccatcgtgag ccttctcttg angtggctgg ggcaggaact gaagtcgaaa 480
   ccagcgctgg gaggaccagg gggaccaana ggtccaggaa gggcccgggg gggaccaaca 540
   ggaccagcat caccaagtgc gacccgcgag aacctgcccg gccgnccgct cgaa       594
<210> 215
   <211> 590
   <212> DNA
   <213> Homo sapiens
<220>
   <221> misc_feature
   <222> 8, 9
   <223> n = A,T,C or G
<400> 215
   tcgagcgnnc gcccgggcag gtctcgcggt cgcactggtg atgctggtcc tgttggtccc 60
   cccggccctc ctggacctcc tggtccccct ggtcctccca gcgctggttt cgacttcagc 120
   ttcctgcccc agccacctca agagaaggct cacgatggtg gccgctacta ccgggctgat 180
   gatgccaatg tggttcgtga ccgtgacctc gaggtggaca ccaccctcaa gagcctgagc 240
   cagcagatcg agaacatccg gagcccagag ggcagccgca agaaccccgc ccgcacctgc 300
   cgtgacctca agatgtgcca ctctgactgg aagagtggag agtactggat tgaccccaac 360
   caaggctgca acctggatgc catcaaagtc ttctgcaaca tggagactgg tgagacctgc 420
   gtgtacccca ctcagcccag tgtggcccag aagaactggt acatcagcaa gaaccccaag 480
   gacaagaggc atgtctggtt cggcgagagc atgaccgatg gattccagtt cgagtatggc 540
   ggccagggct cccaccctgc cgatgtggac ctccggccgc gaccaccctt            590
<210> 216
   <211> 801
   <212> DNA
   <213> Homo sapiens
<220>
   <221> misc_feature
   <222> 2, 22; 25, 26, 328, 373, 385, 440, 473, 534, 571, 572, 573, 582, 587, 589, 593, 600, 605, 617, 633, 642, 653, 672, 681, 685, 696, 699, 709, 715, 717, 726, 731, 739, 742, 745, 758, 769, 772, 778, 780, 788, 789, 791, 793, 796
   <223> n = A,T,C or G
<400> 216
   tngagcggcc gcccgggcag gntgnnaacg ctggtcctgc tggtcctcct ggcaaggctg 60
   gtgaagatgg tcaccctgga aaacccggac gacctggtga gagaggagtt gttggaccac 120
   agggtgctcg tggtttccct ggaactcctg gacttcctgg cttcaaaggc attaggggac 180
   acaatggtct ggatggattg aagggacagc ccggtgctcc tggtgtgaag ggtgaacctg 240
   gtgcccctgg tgaaaatgga actccaggtc aaacaggagc ccgtgggctt cctggtgaga 300
   gaggaccgtg ttggtgcccc tggcccanac ctcggccgcg accacgctaa gcccgaattt 360
   ccagcacact ggnggccgtt actantggat ccgagctcgg taccaagctt ggcgtaatca 420
   tggtcatagc tgtttcctgn gtgaaattgt tatccgctca caatttcaca cancatacga 480
   agccggaaag cataaagtgt aaagccttgg ggtgctaatg agtgagctaa ctcncattaa 540
   attgcgttgc gctcactgcc cgcttttcca nnngggaaac cntggcntng ccngcttgcn 600
   ttaantgaaa tccgccnacc cccggggaaa agncggtttg cngtattggg gcnctttttc 660
   cctttcctcg gnttacttga nttantgggc tttggncgnt tcgggttgng gcgancnggt 720
   tcaacntcac nccaaaggng gnaanacggt tttcccanaa tccgggggnt ancccaangn 780
   aaaacatnng ncnaangggc t                                           801
<210> 217
   <211> 349
   <212> DNA
   <213> Homo sapiens
<220>
   <221> misc_feature
   <222> 10, 157, 170
   <223> n = A,T,C or G
<400> 217
   agcgtggttn gcggccgagg tctgggccag gggcaccaac acgtcctctc tcaccaggaa 60
   gcccacgggc tcctgtttga cctggagttc cattttcacc aggggcacca ggttcaccct 120
   tcacaccagg agcaccgggc tgtcccttca atccatncag accattgtgn cccctaatgc 180
   ctttgaagcc aggaagtcca ggagttccag ggaaaccacc gagcaccctg tggtccaaca 240
   actcctctct caccaggtcg tccgggtttt ccagggtgac catcttcacc agccttgcca 300
   ggaggaccag caggaccagc gttaccaacc tgcccgggcg gccgctcga             349
<210> 218
   <211> 372
   <212> DNA
   <213> Homo sapiens
<400> 218
   tcgagcggcc gcccgggcag gtccattttc tccctgacgg tcccacttct ctccaatctt 60
   gtagttcaca ccattgtcat ggcaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ttgacagagt tgcccacggt aacaacctct tcccgaacct tatgcctctg 300
   ctggtctttc agtgcctcca ctatgatgtt gtaggtggca cctctggtga ggacctcggc 360
   cgcgaccacg ct                                                     372
<210> 219
   <211> 374
   <212> DNA
   <213> Homo sapiens
<400> 219
   agcgtggtcg cggccgaggt cctcaccaga ggtgccacct acaacatcat agtggaggca 60
   ctgaaagacc agcagaggca taaggttcgg gaagaggttg ttaccgtggg caactctgtc 120
   aacgaaggct tgaaccaacc tacggatgac tcgtgctttg acccctacac agtttcccat 180
   tatgccgttg gagatgagtg ggaacgaatg tctgaatcag gctttaaact gttgtgccag 240
   tgcttaggct ttggaagtgg tcatttcaag atgtgattca tctagatggt gccatgacaa 300
   tggtgtgaac tacaagattg gagagaagtg ggaccgtcag ggagaaaatg gacctgcccg 360
   ggccggccgc tcga                                                   374
<210> 220
   <211> 828
   <212> DNA
   <213> Homo sapiens
<220>
   <221> misc_feature
   <222> 8, 9, 557, 571, 587, 588, 601, 642, 643, 647, 654, 664, 681, 688, 698, 719, 720, 725, 734, 738, 743, 744, 757, 765, 773, 778, 780, 782, 783, 793, 798, 805, 809, 822, 827
   <223> n = A,T,C or G
<400> 220
   tcgagcgnnc gcccgggcag gtccagtagt gccttcggga ctgggttcac ccccaggtct 60
   gcggcagttg tcacagcgcc agccccgctg gcctccaaag catgtgcagg agcaaatggc 120
   accgagatat tccttctgcc actgttctcc tacgtggtat gtcttcccat catcgtaaca 180
   cgttgcctca tgagggtcac acttgaattc tccttttccg ttcccaagac atgtgcagct 240
   catttggctg gctctatagt ttggggaaag tttgttgaaa ctgtgccact gacctttact 300
   tcctccttct ctactggagc tttcgtacct tccacttctg ctgttggtaa aatggtggat 360
   cttctatcaa tttcattgac agtacccact tctcccaaac atccagggaa atagtgattt 420
   cagagcgatt aggagaacca aattatgggg cagaaataag gggcttttcc acaggttttc 480
   ctttggagga agatttcagt ggtgacttta aaagaatact caacagtgtc ttcatcccca 540
   tagcaaaaga agaaacngta aatgatggaa ngcttctgga gatgccnnca tttaagggac 600
   ncccagaact tcaccatcta caggacctac ttcagtttac annaagncac atantctgac 660
   tcanaaagga cccaagtagc nccatggnca gcactttnag cctttcccct ggggaaaann 720
   ttacnttctt aaancctngg ccnngacccc cttaagncca aattntggaa aanttccntn 780
   cnnctggggg gcngttcnac atgcntttna agggcccaat tnccccnt              828
<210> 221
   <211> 476
   <212> DNA
   <213> Homo sapiens
<400> 221
   tcgagcggcc gcccgggcag gtgtcggagt ccagcacggg aggcgtggtc ttgtagttgt 60
   tctccggctg cccattgctc tcccactcca cggcgatgtc gctgggatag aagcctttga 120
   ccaggcaggt caggctgacc tggttcttgg tcatctcctc ccgggatggg ggcagggtgt 180
   acacctgtgg ttctcggggc tgccctttgg ctttggagat ggttttctcg atgggggctg 240
   ggagggcttt gttggagacc ttgcacttgt actccttgcc attcagccag tcctggtgca 300
   ggacggtgag gacgctgacc acacggtacg tgctgttgta ctgctcctcc cgcggctttg 360
   tcttggcatt atgcacctcc acgccgtcca cgtaccagtt gaacttgacc tcagggtctt 420
   cgtggctcac gtccaccacc acgcatgtaa cctcagacct cggccgcgac cacgct     476
<210> 222
   <211> 477
   <212> DNA
   <213> Homo sapiens
<400> 222
<210> 223
   <211> 361
   <212> DNA
   <213> Homo sapiens
<400> 223
   tcgagcggcc gcccgggcag gttgaatggc tcctcgctga ccaccccggt gctggtggtg 60
   ggtacagagc tccgatgggt gaaaccattg acatagagac tgtccctgtc cagggtgtag 120
   gggcccagct cagtgatgcc gtgggtcagc tggctcagct tccagtacag ccgctctctg 180
   tccagtccag ggcttttggg gtcaggacga tgggtgcaga cagcatccac tctggtggct 240
   gccccatcct tctcaggcct gagcaaggtc agtctgcaac cagagtacag agagctgaca 300
   ctggtgttct tgaacaaggg cataagcaga ccctgaagga cacctcggcc gcgaccacgc 360
   t                                                                 361

<210> 224
   <211> 361
   <212> DNA
   <213> Homo sapiens
<400> 224
   agcgtggtcg cggccgaggt gtccttcagg gtctgcttat gcccttgttc aagaacacca 60
   gtgtcagctc tctgtactct ggttgcagac tgaccttgct caggcctgag aaggatgggg 120
   cagccaccag agtggatgct gtctgcaccc atcgtcctga ccccaaaagc cctggactgg 180
   acagagagcg gctgtactgg aagctgagcc agctgaccca cggcatcact gagctgggcc 240
   cctacaccct ggacagggac agtctctatg tcaatggttt cacccatcgg agctctgtac 300
   ccaccaccag caccggggtg gtcagcgagg agccattcaa cctgcccggg cggccgctcg 360
   a                                                                 361
<210> 225
   <211> 766
   <212> DNA
   <213> Homo sapiens
<220>
   <221> misc_feature
   <222> 574, 610, 631, 643, 657, 660, 666, 688, 712, 735, 747 <223> n = A,T,C or G
<400> 225
   agcgtggtcg cggccgaggt cctgtcagag tggcactggt agaagttcca ggaaccctga 60
   actgtaaggg ttcttcatca gtgccaacag gatgacatga aatgatgtac tcagaagtgt 120
   cctggaatgg ggcccatgag atggttgtct gagagagagc ttcttgtcct acattcggcg 180
   ggtatggtct tggcctatgc cttatggggg tggccgttgt gggcggtgtg gtccgcctaa 240
   aaccatgttc ctcaaagatc atttgttgcc caacactggg ttgctgacca gaagtgccag 300
   gaagctgaat accatttcca gtgtcatacc cagggtgggt gacgaaaggg gtcttttgaa 360
   ctgtggaagg aacatccaag atctctggtc catgaagatt ggggtgtgga agggttacca 420
   gttggggaag ctcgtctgtc tttttccttc caatcagggg ctcgctcttc tgattattct 480
   tcagggcaat gacataaatt gtatattcgg tcccggttcc aggccagtaa tagtagcctc 540
   tgtgacacca gggcggggcc gagggaccct tctnttggaa gagaccagct tctcatactt 600
   gatgatgagn ccggtaatcc tggcacgtgg nggttgcatg atnccaccaa ggaaatnggn 660
   gggggnggac ctgcccggcg gccgttcnaa agcccaattc cacacacttg gnggccgtac 720
   tatggatccc actcngtcca acttggngga atatggcata actttt                766
<210> 226
   <211> 364
   <212> DNA
   <213> Homo sapiens
<400> 226
   tcgagcggcc gcccgggcag gtccttgacc ttttcagcaa gtgggaaggt gtaatccgtc 60
   tccacagaca aggccaggac tcgtttgtac ccgttgatga tagaatgggg tactgatgca 120
   acagttgggt agccaatctg cagacagaca ctggcaacat tgcggacacc ctccaggaag 180
   cgagaatgca gagtttcctc tgtgatatca agcacttcag ggttgtagat gctgccattg 240
   tcgaacacct gctggatgac cagcccaaag gagaaggggg agatgttgag catgttcagc 300
   agcgtggctt cgctggctcc cactttgtct ccagtcttga tcagacctcg gccgcgacca 360
   cgct                                                              364
<210> 227
   <211> 275
   <212> DNA
   <213> Homo sapiens
<400> 227
   agcgtggtcg cggccgaggt ctgtcctaca gtcctcagga ctctactccc tcagcagcgt 60
   ggtgaccgtg ccctccagca acttcggcac ccagacctac acctgcaacg tagatcacaa 120
   gcccagcaac accaaggtgg acaagagagt tgagcccaaa tcttgtgaca aaactcacac 180
   atgcccaccg tgcccagcac ctgaactcct ggggggaccg tcagtcttcc tcttcccccg 240
   catccccctt ccaaacctgc ccgggcggcc gctcg                            275
<210> 228
   <211> 275
   <212> DNA
   <213> Homo sapiens
<400> 228
   cgagcggccg cccgggcagg tttggaaggg ggatgcgggg gaagaggaag actgacggtc 60
   cccccaggag ttcaggtgct gggcacggtg ggcatgtgtg agttttgtca caagatttgg 120
   gctcaactct cttgtccacc ttggtgttgc tgggcttgtg atctacgttg caggtgtagg 180
   tctgggtgcc gaagttgctg gagggcacgg tcaccacgct gctgagggag tagagtcctg 240
   aggactgtag gacagacctc ggccgcgacc acgct                            275
<210> 229
   <211> 40
   <212> DNA
   <213> Homo sapiens
<220>
   <221> misc_feature
   <222> 1, 4, 5, 13, 15, 17, 29
   <223> n = A,T,C or G
<400> 229
   nggnnggtcc ggncngncag gaccactcnt cttcgaaata 40
<210> 230
   <211> 208
   <212> DNA
   <213> Homo sapiens
<400> 230
   agcgtggtcg cggccgaggt cctcacttgc ctcctgcaaa gcaccgatag ctgcgctctg 60
   gaagcgcaga tctgttttaa agtcctgagc aatttctcgc accagacgct ggaagggaag 120
   tttgcgaatc agaagttcag tggacttctg ataacgtcta atttcacgga gcgccacagt 180
   accaggacct gcccgggcgg ccgctcga                                   208
<210> 231
   <211> 208
   <212> DNA
   <213> Homo sapiens
<220>
   <221> misc_feature
   <222> 33
   <223> n = A,T,C or G
<400> 231
   tcgagcggcc gcccgggcag gtcctggtac tgnggcgctc cgtgaaatta gacgttatca 60
   gaagtccact gaacttctga ttcgcaaact tcccttccag cgtctggtgc gagaaattgc 120
   tcaggacttt aaaacagatc tgcgcttcca gagcgcagct atcggtgctt tgcaggaggc 180
   aagtgaggac ctcggccgcg accacgct                                   208
<210> 232
   <211> 332
   <212> DNA
   <213> Homo sapiens
<400> 232
   tcgagcggcc gcccgggcag gtccacatcg gcagggtcgg agccctggcc gccatactcg 60
   aactggaatc catcggtcat gctctcgccg aaccagacat gcctcttgtc cttggggttc 120
   ttgctgatgt accagttctt ctgggccaca ctgggctgag tggggtacac gcaggtctca 180
   ccagtctcca tgttgcagaa gactttgatg gcatccaggt tgcagccttg gttggggtca 240
   atccagtact ctccactctt ccagtcagag tggcacatct tgaggtcacg gcaggtgcgg 300
   gcggggttct tgacctcggc cgcgaccacg ct                              332
<210> 233
   <211> 415
   <212> DNA
   <213> Homo sapiens
<220>
   <221> misc_feature
   <222> 6, 15, 19, 21
   <223> n = A,T,C or G
<400> 233
   gtgggnttga acccntttna nctccgcttg gtaccgagct cggatccact agtaacggcc 60
   gccagtgtgc tggaattcgg cttagcgtgg tcgcggccga ggtcaagaac cccgcccgca 120
   cctgccgtga cctcaagatg tgccactctg actggaagag tggagagtac tggattgacc 180
   ccaaccaagg ctgcaacctg gatgccatca aagtcttctg caacatggag actggtgaga 240
   cctgcgtgta ccccactcag cccagtgtgg cccagaagaa ctggtacatc agcaagaacc 300
   ccaaggacaa gaggcatgtc tggttcggcg agagcatgac cgatggattc cagttcgagt 360
   atggcggcca gggctccgac cctgccgatg tggacctgcc cgggcggccg ctcga 415

<210> 234
   <211> 776
   <212> DNA
   <213> Homo sapiens
<220>
   <221> misc_feature
   <222> 505, 550, 574, 601, 604, 608, 612, 649, 656, 657, 680, 711, 750, 776
   <223> n = A,T,C or G
<400> 234
   agcgtggtcg cggccgaggt ctgggatgct cctgctgtca cagtgagata ttacaggatc 60
   acttacggag aaacaggagg aaatagccct gtccaggagt tcactgtgcc tgggagcaag 120
   tctacagcta ccatcagcgg ccttaaacct ggagttgatt ataccatcac tgtgtatgct 180
   gtcactggcc gtggagacag ccccgcaagc agcaagccaa tttccattaa ttaccgaaca 240
   gaaattgaca aaccatccca gatgcaagtg accgatgttc aggacaacag cattagtgtc 300
   aagtggctgc cttcaagttc ccctgttact ggttacagag taaccaccac tcccaaaaat 360
   ggaccaggac caacaaaaac taaaactgca ggtccagatc aaacagaaat gactattgaa 420
   ggcttgcagc ccacagtgga gtatgtggtt aagtgtctat gctcagaatc caagcggaga 480
   gaagtcagcc tctggttcag actgnaagta accaacattg atcgcctaaa ggactggcat 540
   tcactgatgn ggatgccgat tccatcaaaa ttgnttggga aaacccacag gggcaagttt 600
   ncangtcnag gnggacctac tcgagccctg aggatggaat ccttgactnt tccttnncct 660
   gatggggaaa aaaaaccttn aaaacttgaa ggacctgccc gggcggccgt ncaaaaccca 720
   attccacccc cttgggggcg ttctatgggn cccactcgga ccaaacttgg ggtaan 776
<210> 235
   <211> 805
   <212> DNA
   <213> Homo sapiens
<220>
   <221> misc_feature
   <222> 637, 684, 705, 724, 733, 756, 778, 793, 796, 804
   <223> n = A,T,C or G
<400> 235
   tcgagcggcc gcccgggcag gtccttgcag ctctgcagtg tcttcttcac catcaggtgc 60
   agggaatagc tcatggattc catcctcagg gctcgagtag gtcaccctgt acctggaaac 120
   ttgcccctgt gggctttccc aagcaatttt gatggaatcg gcatccacat cagtgaatgc 180
   cagtccttta gggcgatcaa tgttggttac tgcagtctga accagaggct gactctctcc 240
   gcttggattc tgagcataga cactaaccac atactccact gtgggctgca agccttcaat 300
   agtcatttct gtttgatctg gacctgcagt tttagttttt gttggtcctg gtccattttt 360
   gggagtggtg gttactctgt aaccagtaac aggggaactt gaaggcagcc acttgacact 420
   aatgctgttg tcctgaacat cggtcacttg catctgggat ggtttgtcaa tttctgttcg 480
   gtaattaatg gaaattggct tgctgcttgc ggggcttgtc tccacggcca gtgacagcat 540
   acacagtgat ggtataatca actccaggtt taagccgctg atggtagctg aaactttgct 600
   ccaggcacaa gtgaactcct gacagggcta tttcctnctg ttctccgtaa gtgatcctgt 660
   aatatctcac tgggacagca ggangcattc caaaacttcg ggcgngaccc cctaagccga 720
   attntgcaat atncatcaca ctggcgggcg ctcgancatt cattaaaagg cccaatcncc 780
   cctataggga gtntantaca attng                                      805
<210> 236
   <211> 262
   <212> DNA
   <213> Homo sapiens
<400> 236
   tcgagcggcc gcccgggcag gtcacttttg gtttttggtc atgttcggtt ggtcaaagat 60
   aaaaactaag tttgagagat gaatgcaaag gaaaaaaata ttttccaaag tccatgtgaa 120
   attgtctccc atttttttgg cttttgaggg ggttcagttt gggttgcttg tctgtttccg 180
   ggttgggggg aaagttggtt gggtgggagg gagccaggtt gggatggagg gagtttacag 240
   gaagcagaca gggccaacgt cg                                         262
<210> 237
   <211> 372
   <212> DNA
   <213> Homo sapiens
<400> 237
   agcgtggtcg cggccgaggt cctcaccaga ggtgccacct acaacatcat agtggaggca 60
   ctgaaagacc agcagaggca taaggttcgg gaagaggttg ttaccgtggg caactctgtc 120
   aacgaaggct tgaaccaacc tacggatgac tcgtgctttg acccctacac agtttcccat 180
   tatgccgttg gagatgagtg ggaacgaatg tctgaatcag gctttaaact gttgtgccag 240
   tgcttaggct ttggaagtgg tcatttcaga tgtgattcat ctagatggtg ccatgacaat 300
   ggtgtgaact acaagattgg agagaagtgg gaccgtcagg gagaaaatgg acctgcccgg 360
   gcggccgctc ga                                                    372
<210> 238
   <211> 372
   <212> DNA
   <213> Homo sapiens
<400> 238
   tcgagcggcc gcccgggcag gtccattttc tccctgacgg tcccacttct ctccaatctt 60
   gtagttcaca ccattgtcat ggcaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ttgacagagt tgcccacggt aacaacctct tcccgaacct tatgcctctg 300
   ctggtctttc agtgcctcca ctatgatgtt gtaggtggca cctctggtga ggacctcggc 360
   cgcgaccacg ct                                                     372
<210> 239
   <211> 720
   <212> DNA
   <213> Homo sapiens
<220>
   <221> misc_feature
   <222> 478, 557, 563, 566, 620, 660, 663, 672, 673, 684, 693, 695
   <223> n = A,T,C or G
<400> 239
   tcgagcggcc gcccgggcag gtccaccata agtcctgata caaccacgga tgagctgtca 60
   ggagcaaggt tgatttcttt cattggtccg gtcttctcct tgggggtcac ccgcactcga 120
   tatccagtga gctgaacatt gggtggtgtc cactgggcgc tcaggcttgt gggtgtgacc 180
   tgagtgaact tcaggtcagt tggtgcagga atagtggtta ctgcagtctg aaccagaggc 240
   tgactctctc cgcttggatt ctgagcatag acactaacca catactccac tgtgggctgc 300
   aagccttcaa tagtcatttc tgtttgatct ggacctgcag ttttagtttt tgttggtcct 360
   ggtccatttt tgggagtggt ggttactctg taaccagtaa caggggaact tgaaggcagc 420
   cacttgacac taatgctgtt gtcctgaaca tcggtcactt gcatctggga tggtttgnca 480
   atttctgttc ggtaattaat ggaaattggc ttgctgcttg cggggctgtc tccacggcca 540
   gtgacagcat acacagngat ggnatnatca actccaagtt taaggccctg atggtaactt 600
   taaacttgct cccagccagn gaacttccgg acagggtatt tcttctggtt ttccgaaagn 660
   gancctggaa tnntctcctt ggancagaag gancntccaa aacttgggcc ggaacccctt 720
<210> 240
   <211> 691
   <212> DNA
   <213> Homo sapiens
<220>
   <221> misc_feature
   <222> 564, 582, 640, 651, 666, 669, 690
   <223> n = A,T,C or G
<400> 240
   agcgtggtcg cggccgaggt cctgtcagag tggcactggt agaagttcca ggaaccctga 60
   actgtaaggg ttcttcatca gtgccaacag gatgacatga aatgatgtac tcagaagtgt 120
   cctggaatgg ggcccatgag atggttgtct gagagagagc ttcttgtcct acattcggcg 180
   ggtatggtct tggcctatgc cttatggggg tggccgttgt gggcggtgtg gtccgcctaa 240
   aaccatgttc ctcaaagatc atttgttgcc caacactggg ttgctgacca gaagtgccag 300
   gaagctgaat accatttcca gtgtcatacc cagggtgggt gacgaaaggg gtcttttgaa 360
   ctgtggaagg aacatccaag atctctggtc catgaagatt ggggtgtgga agggttacca 420
   gttggggaag ctcgtctgtc tttttccttc caatcagggg ctcgctcttc tgattattct 480
   tcagggcaat gacataaatt gtatattcgg ttcccggttc caggccagta atagtagcct 540
   cttgtgacac caggcggggc ccanggacca cttctctggg angagaccca gcttctcata 600
   cttgatgatg taacccggta atcctgcacg tggcggctgn catgatacca ncaaggaatt 660
   gggtgnggng gacctgcccg gcggccctcn a 691
<210> 241
   <211> 808
   <212> DNA
   <213> Homo sapiens
<220>
   <221> misc_feature
   <222> 680, 715, 721, 728, 735, 749, 757, 762, 772, 776, 779, 781, 792, 796, 800, 808
   <223> n = A,T,C or G
<400> 241
   agcgtggtcg cggccgaggt ctgggatgct cctgctgtca cagtgagata ttacaggatc 60
   acttacggag aaacaggagg aaatagccct gtccaggagt tcactgtgcc tgggagcaag 120
   tctacagcta ccatcagcgg ccttaaacct ggagttgatt ataccatcac tgtgtatgct 180
   gtcactggcc gtggagacag ccccgcaagc agcaagccaa tttccattaa ttaccgaaca 240
   gaaattgaca aaccatccca gatgcaagtg accgatgttc aggacaacag cattagtgtc 300
   aagtggctgc cttcaagttc ccctgttact ggttacagag taaccaccac tcccaaaaat 360
   ggaccaggac caacaaaaac taaaactgca ggtccagatc aaacagaaat gactattgaa 420
   ggcttgcagc ccacagtgga gtatgtggtt agtgtctatg ctcagaatcc aagcggagag 480
   agtcagcctc tggttcagac tgcagtaacc actattcctg caccaactga cctgaagttc 540
   actcaggtca cacccacaag cctgagccgc cagtggacac cacccaatgt tcactcactg 600
   gatatcgagt gcgggtgacc cccaaggaga agacccggac ccatgaaaga aatcaacctt 660
   gctcctgaca gctcatccgn gggtgtatca ggacttatgg gggactgccc cggcnggccg 720
   ntcgaaancg aattntgaaa tttccttcnc actgggnggc gnttcgagct tncttntana 780
   nggcccaatt cncctntagn gggtcgtn 808
<210> 242
   <211> 26
   <212> DNA
   <213> Homo sapiens
<220>
   <221> misc_feature
   <222> 22
   <223> n = A,T,C or G
<400> 242
   agcgtggtcg cggccgaggt cnagga 26
<210> 243
   <211> 697
   <212> DNA
   <213> Homo sapiens
<220>
   <221> misc_feature
   <222> 496, 541, 624, 662, 679, 688
   <223> n = A,T,C or G
<400> 243
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaagaata atcagaagag cgagcccctg 240
   attggaagga aaaagacaga cgagcttccc caactggtaa cccttccaca ccccaatctt 300
   catggaccag agatcttgga tgttccttcc acagttcaaa agaccccttt cgtcacccac 360
   cctgggtatg acactggaaa tggtattcag cttcctggca cttctggtca gcaacccagt 420
   gttgggcaac aaatgatctt tgaggaacat ggttttaggc ggaccacacc gcccacaacg 480
   ggcaccccca taaggnatag gccaagacca taccccgccg aatgtaggac aagaagctct 540
   ntctcaacaa ccatctcatg ggccccattc caggacactt ctgagtacat catttcatgt 600
   catcctggtg ggcacttgat gaanaaccct tacagttcag ggttcctgga acttctacca 660
   gngccacttc tgacagganc ttgggcgnga ccaccct                          697

<210> 244
   <211> 373
   <212> DNA
   <213> Homo sapiens
<400> 244
   agcgtggtcg cggccgaggt ccattttctc cctgacggtc ccacttctct ccaatcttgt 60
   agttcacacc attgtcatgg caccatctag atgaatcaca tctgaaatga ccacttccaa 120
   agcctaagca ctggcacaac agtttaaagc ctgattcaga cattcgttcc cactcatctc 180
   caacggcata atgggaaact gtgtaggggt caaagcacga gtcatccgta ggttggttca 240
   agccttcgtt gacagagttg cccacggtaa caacctcttc ccgaacctta tgcctctgct 300
   ggtctttcag tgcctccact atgatgttgt aggtggcacc tctggtgagg acctgcccgg 360
   gcggcccgct cga                                                    373
<210> 245
   <211> 307
   <212> DNA
   <213> Homo sapiens
<400> 245
   agcgtggtcg cggccgaggt gtgccccaga ccaggaattc ggcttcgacg ttggccctgt 60
   ctgcttcctg taaactccct ccatcccaac ctggctccct cccacccaac caactttccc 120
   cccaacccgg aaacagacaa gcaacccaaa ctgaaccccc tcaaaagcca aaaaaatggg 180
   agacaatttc acatggactt tggaaaatat ttttttcctt tgcattcatc tctcaaactt 240
   agtttttatc tttgaccaac cgaacatgac caaaaaccaa aagtgacctg cccgggcggc 300
   cgctcga                                                           307
<210> 246
   <211> 372
   <212> DNA
   <213> Homo sapiens
<400> 246
   tcgagcggcc gcccgggcag gtcctcacca gaggtgccac ctacaacatc atagtggagg 60
   cactgaaaga ccagcagagg cataaggttc gggaagaggt tgttaccgtg ggcaactctg 120
   tcaacgaagg cttgaaccaa cctacggatg actcgtgctt tgacccctac acagtttccc 180
   attatgccgt tggagatgag tgggaacgaa tgtctgaatc aggctttaaa ctgttgtgcc 240
   agtgcttagg ctttggaagt ggtcatttca gatgtgattc atctagatgg tgccatgaca 300
   atggtgtgaa ctacaagatt ggagagaagt gggaccgtca gggagaaaat ggacctcggc 360
   cgcgaccacg ct                                                     372
<210> 247
   <211>.348
   <212> DNA
   <213> Homo sapiens
<220>
   <221> misc_feature
   <222> 284, 297, 299, 322, 325, 338, 342, 345
   <223> n = A,T,C or G
<400> 247
<210> 248
   <211> 304
   <212> DNA
   <213> Homo sapiens
<220>
   <221> misc_feature
   <222> 125
   <223> n = A,T,C or G
<400> 248
   gaggactggc tcagctccca gtatagccgc tctctgtcca gtccaggacc agtgggatca 60
   aggcggaggg tgcagatggc gtccactcca gtggctgccc catgtttctc aagtctgagc 120
   aaagncagtc tgcagccaga gtacagaggg ccaacactgg tgctcttgaa cagggacctg 180
   agcaggccct gaaggaccct ctccgtggtg ttgaacttcc tggagccagg gtgctgcatg 240
   ttctcctcat accgcaggtt gttgatggtg aagttcagtg tgaatggctc ctcgctgacc 300
   accc                                                              304
<210> 249
   <211> 400
   <212> DNA
   <213> Homo sapiens
<220>
   <221> misc_feature
   <222> 308, 310, 312, 320, 331, 336, 383, 392, 396
   <223> n = A,T,C or G
<400> 249
   agcgtggtcg cggccgaggt ccaccacacc caattccttg ctggtatcat ggcagccgcc 60
   acgtgccagg attaccggct acatcatcaa gtatgagaag cctgggtctc ctcccagaga 120
   agtggtccct cggccccgcc ctggtgtcac agaggctact attactggcc tggaaccggg 180
   aaccgaatat acaatttatg tcattgccct gaagaataat cagaagagcg agcccctgat 240
   tggaaggaaa aagacagacg agcttcccca actggtaacc cttccacacc ccaatcttca 300
   tggaccanan ancttggatn gtcctttcac nggttnaaaa aacccttttc gcccccccac 360
   cttggggatt aaccttggga aanggggatt tnaccnttcc                       400
<210> 250
   <211> 400
   <212> DNA
   <213> Homo sapiens
<220>
   <221> misc_feature
   <222> 338, 357, 361, 369, 388, 394
   <223> n = A,T,C or G
<400> 250
   tcgagcggcc gcccgggcag gtcctgtcag agtggcactg gtagaagttc caggaaccct 60
   gaactgtaag ggttcttcat cagtgccaac aggatgacat gaaatgatgt actcagaagt 120
   gtcctggaat ggggcccatg agatggttgt ctgagagaga gcttcttgtc ctacattcgg 180
   cgggtatggt cttggcctat gccttatggg ggtggccgtt gtgggcggtg tggtccgcct 240
   aaaaccatgt tcctcaaaga tcatttgttg cccaacactg ggttgctgac cagaagtgcc 300
   aggaagctga ataccatttc cagtgtcata cccagggngg gtgaccaaag ggggtcnttt 360
   ngacctggng aaaggaacca tccaaaanct ctgncccatg                       400
<210> 251
   <211> 514
   <212> DNA
   <213> Homo sapiens
<220>
   <221> misc_feature
   <222> 8, 107, 312, 338, 351, 352, 357, 363, 366, 373, 380, 405, 421, 444, 508
   <223> n = A,T,C or G
<400> 251
   agcgtggncg cggccgaggt ctgaggatgt aaactcttcc caggggaagg ctgaagtgct 60
   gaccatggtg ctactgggtc cttctgagtc agatatgtga ctgatgngaa ctgaagtagg 120
   tactgtagat ggtgaagtct gggtgtccct aaatgctgca tctccagagc cttccatcat 180
   taccgtttct tcttttgcta tgggatgaga cactgttgag tattctctaa agtcaccact 240
   gaaatcttcc tccaaaggaa aacctgtgga aaagcccctt atttctgccc cataatttgg 300
   ttctcctaat cnctctgaaa tcactatttc cctggaangt ttgggaaaaa nngggcnacc 360
   tgncantgga aantggatan aaagatccca ccattttacc caacnagcag aaagtgggaa 420
   nggtaccgaa aagctccaag taanaaaaag gagggaagta aaggtcaagt gggcaccagt 480
   ttcaaacaaa actttcccca aactatanaa ccca                             514
<210> 252
   <211> 501
   <212> DNA
   <213> Homo sapiens
<220>
   <221> misc_feature
   <222> 20, 21, 25, 44, 343, 347, 356, 362, 387, 391, 398, 409, 428, 430, 453, 494
   <223> n = A,T,C or G
<400> 252
   aagcggccgc ccgggcaggn ncagnagtgc cttcgggact gggntcaccc ccaggtctgc 60
   ggcagttgtc acagcgccag ccccgctggc ctccaaagca tgtgcaggag caaatggcac 120
   cgagatattc cttctgccac tgttctccta cgtggtatgt cttcccatca tcgtaacacg 180
   ttgcctcatg agggtcacac ttgaattctc cttttccgtt cccaagacat gtgcagctca 240
   tttggctggc tctatagttt ggggaaagtt tgttgaaact gtgccactga cctttacttc 300
   ctccttctct actggagctt tccgtacctt ccacttctgc tgntggnaaa aagggnggaa 360
   cntcttatca atttcattgg acagtanccc nctttctncc caaaacatnc aagggaaaat 420
   attgattncn agagcggatt aaggaacaac ccnaattatg ggggccagaa ataaaggggg 480
   cttttccaca ggtnttttcc t                                          501
<210> 253
   <211> 226
   <212> DNA
   <213> Homo sapiens
<400> 253
   tcgagcggcc gcccgggcag gtctgcaggc tattgtaagt gttctgagca catatgagat 60
   aacctgggcc aagctatgat gttcgatacg ttaggtgtat taaatgcact tttgactgcc 120
   atctcagtgg atgacagcct tctcactgac agcagagatc ttcctcactg tgccagtggg 180
   caggagaaag agcatgctgc gactggacct cggccgcgac cacgct                226
<210> 254
   <211> 226
   <212> DNA
   <213> Homo sapiens
<400> 254
   agcgtggtcg cggccgaggt ccagtcgcag catgctcttt ctcctgccca ctggcacagt 60
   gaggaagatc tctgctgtca gtgagaaggc tgtcatccac tgagatggca gtcaaaagtg 120
   catttaatac acctaacgta tcgaacatca tagcttggcc caggttatct catatgtgct 180
   cagaacactt acaatagcct gcagacctgc ccgggcggcc gctcga                226
<210> 255
   <211> 427
   <212> DNA
   <213> Homo sapiens
<220>
   <221> misc_feature
   <222> 327, 403
   <223> n = A,T,C or G
<400> 255
   cgagcggccg cccgggcagg tccagactcc aatccagaga accaccaagc cagatgtcag 60
   aagctacacc atcacaggtt tacaaccagg cactgactac aagatctacc tgtacacctt 120
   gaatgacaat gctcggagct cccctgtggt catcgacgcc tccactgcca ttgatgcacc 180
   atccaacctg cgtttcctgg ccaccacacc caattccttg ctggtatcat ggcagccgcc 240
   acgtgccagg attaccggct acatcatcaa gtatgagaag cctgggtctc ctcccagaga 300
   agtggtccct cggccccgcc ctggtgncac agaagctact attactggcc tggaaccggg 360
   aaccgaatat acaatttatg tcattgccct gaagaataat canaagagcg agcccctgat 420
   tggaagg                                                           427
<210> 256
   <211> 535
   <212> DNA
   <213> Homo sapiens
<220>
   <221> misc_feature
   <222> 347, 456, 475
   <223> n = A,T,C or G
<400> 256
   agcgtggtcg cggccgaggt cctgtcagag tggcactggt agaagttcca ggaaccctga 60
   actgtaaggg ttcttcatca gtgccaacag gatgacatga aatgatgtac tcagaagtgt 120
   cctggaatgg ggcccatgag atggttgtct gagagagagc ttcttgtcct gtctttttcc 180
   ttccaatcag gggctcgctc ttctgattat tcttcagggc aatgacataa attgtatatt 240
   cggttcccgg ttccaggcca gtaatagtag cctctgtgac accagggcgg ggccgaggga 300
   ccacttctct gggaggagac ccaggcttct catacttgat gatgtanccg gtaatcctgg 360
   caccgtggcg gctgccatga taccagcaag gaattgggtg tggtggccaa gaaacgcagg 420
   ttggatggtg catcaatggc agtggaggcg tcgatnacca caggggagct ccgancattg 480
   tcattcaagg tggacaggta gaatcttgta atcaggtgcc tggtttgtaa acctg      535

<210> 257
   <211> 544
   <212> DNA
   <213> Homo sapiens
<220>
   <221> misc_feature
   <222> 495, 511
   <223> n = A,T,C or G
<400> 257
   tcgagcggcc gcccgggcag gtttcgtgac cgtgacctcg aggtggacac caccctcaag 60
   agcctgagcc agcagatcga gaacatccgg agcccagagg gcagccgcaa gaaccccgcc 120
   cgcacctgcc gtgacctcaa gatgtgccac tctgactgga agagtggaga gtactggatt 180
   gaccccaacc aaggctgcaa cctggatgcc atcaaagtct tctgcaacat ggagactggt 240
   gagacctgcg tgtaccccac tcagcccagt gtggcccaga agaactggta catcagcaag 300
   aaccccaagg acaagaagca tgtctggttc ggcgaaagca tgaccgatgg attccagttc 360
   gagtatggcg gccagggctc cgaccctgcc gatgtggacc tcggccgcga ccacgctaag 420
   cccgaattcc agcacactgg cggccgttac tagtgggatc cgagcttcgg taccaagctt 480
   ggcgtaatca tgggncatag ctgtttcctg ngtgaaaatg gtattccgct tcacaatttc 540
   ccac                                                              544
<210> 258
   <211> 418
   <212> DNA
   <213> Homo sapiens
<400> 258
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacacgc aggtctcacc 180
   agtctccatg ttgcagaaga ctttgatggc atccaggttg cagccttggt tggggtcaat 240
   ccagtactct ccactcttcc agtcagagtg gcacatcttg aggtcacggc aggtgcgggc 300
   ggggttcttg cggctgccct ctgggctccg gatgttctcg atctgctggc tcaagctctt 360
   gaagggtggt gtccacctcg aggtcacggt cacgaaacct gcccgggcgg ccgctcga   418
<210> 259
   <211> 377
   <212> DNA
   <213> Homo sapiens
<220>
   <221> misc_feature
   <222> 320, 326, 342, 352
   <223> n = A,T,C or G
<400> 259
   agcgtggtcg cggccgaggt caagaacccc gcccgcacct gccgtgacct caagatgtgc 60
   cactctgact ggaagagtgg agagtactgg attgacccca accaaggctg caacctggat 120
   gccatcaaag tcttctgcaa catggagact ggtgagacct gcgtgtaccc cactcagccc 180
   agtgtggccc agaagaactg gtacatcagc aagaacccca aggacaagag gcatgtctgg 240
   ttcggcgaga gcatgaccga tggattccag ttcgagtatg gcggccaggg ctccgaccct 300
   gccgatgtgg acctgcccgn gccggnccgc tcgaaaagcc cnaatttcca gncacacttg 360
   gccggccgtt actactg                                                377
<210> 260
   <211> 332
   <212> DNA
   <213> Homo sapiens
<400> 260
   tcgagcggcc gcccgggcag gtccacatcg gcagggtcgg agccctggcc gccatactcg 60
   aactggaatc catcggtcat gctctcgccg aaccagacat gcctcttgtc cttggggttc 120
   ttgctgatgt accagttctt ctgggccaca ctgggctgag tggggtacac gcaggtctca 180
   ccagtctcca tgttgcagaa gactttgatg gcatccaggt tgcagccttg gttggggtca 240
   atccagtact ctccactctt ccagtcagag tggcacatct tgaggtcacg gcaggtgcgg 300
   gcggggttct tgacctcggc cgcgaccacg ct                               332
<210> 261
   <211> 94
   <212> DNA
   <213> Homo sapiens
<400> 261
   cgagcggccg cccgggcagg tcccccccct tttttttttt tttttttttt tttttttttt 60
   tttttttttt tttttttttt tttttttttt tttt 94
<210> 262
   <211> 650
   <212> DNA
   <213> Homo sapiens
<220>
   <221> misc_feature
   <222> 412, 582, 612, 641, 646
   <223> n = A,T,C or G
<400> 262
   agcgtggtcg cggccgaggt ctggcattcc ttcgacttct ctccagccga gcttcccaga 60
   acatcacata tcactgcaaa aatagcattg catacatgga tcaggccagt ggaaatgtaa 120
   agaaggccct gaagctgatg gggtcaaatg aaggtgaatt caaggctgaa ggaaatagca 180
   aattcaccta cacagttctg gaggatggtt gcacgaaaca cactggggaa tggagcaaaa 240
   cagtctttga atatcgaaca cgcaaggctg tgagactacc tattgtagat attgcaccct 300
   atgacattgg tggtcctgat caagaatttg gtgtggacgt tggccctgtt tgctttttat 360
   aaaccaaact ctatctgaaa tcccaacaaa aaaaatttaa ctccatatgt gntcctcttg 420
   ttctaatctt ggcaaccagt gcaagtgacc gacaaaattc cagttattta tttccaaaat 480
   gtttggaaac agtataattt gacaaagaaa aaaggatact tctctttttt tggctggtcc 540
   accaaataca attcaaaagg ctttttggtt ttattttttt anccaattcc aatttcaaaa 600
   tgtctcaatg gngcttataa taaaataaac tttcaccctt nttttntgat 650
<210> 263
   <211> 573
   <212> DNA
   <213> Homo sapiens
<220>
   <221> misc_feature
   <222> 453, 458, 544
   <223> n = A,T,C or G
<400> 263
   agcgtggtcg cggccgaggt ctgggatgct cctgctgtca cagtgagata ttacaggatc 60
   acttacggag aaacaggagg aaatagccct gtccaggagt tcactgtgcc tgggagcaag 120
   tctacagcta ccatcagcgg ccttaaacct ggagttgatt ataccatcac tgtgtatgct 180
   gtcactggcc gtggagacag ccccgcaagc agcaagccaa tttccattaa ttaccgaaca 240
   gaaattgaca aaccatccca gatgcaagtg accgatgttc aggacaacag cattagtgtc 300
   aagtggctgc cttcaagttc ccctgttact ggttacagaa gtaaccacca ctcccaaaaa 360
   tggaccagga ccaacaaaaa ctaaaactgc aggtccagat caaacagaaa atggactatt 420
   gaaggcttgc agcccacagt ggaagtatgt ggntaggngt ctatgctcag aatcccaagc 480
   cggagaaagt cagccttctg gtttagactg cagtaaccaa cattgatcgc cctaaaggac 540
   tggncattca cttggatggt ggatgtccaa ttc 573
<210> 264
   <211> 550
   <212> DNA
   <213> Homo sapiens
<220>
   <221> misc_feature
   <222> 39, 174, 352, 526
   <223> n = A,T,C or G
<400> 264
   tcgagcggcc gcccgggcag gtccttgcag ctctgcagng tcttcttcac catcaggtgc 60
   agggaatagc tcatggattc catcctcagg gctcgagtag gtcaccctgt acctggaaac 120
   ttgcccctgt gggctttccc aagcaatttt gatggaatcg acatccacat cagngaatgc 180
   cagtccttta gggcgatcaa tgttggttac tgcagtctga accagaggct gactctctcc 240
   gcttggattc tgagcataga cactaaccac atactccact gtgggctgca agccttcaat 300
   agtcatttct gtttgatctg gacctgcagt tttaagtttt tggtggtcct gncccatttt 360
   tgggaagtgg ggggttactc tgtaaccagt aacaggggaa cttgaaggca gccacttgac 420
   actaatgctg ttgtcctgaa catcggtcac ttgcatctgg ggatggtttt gacaatttct 480
   ggttcggcaa attaatggaa attggcttgc tgcttggcgg ggctgnctcc acgggccagt 540
   gacagcatac                                                        550
<210> 265
   <211> 596
   <212> DNA
   <213> Homo sapiens
<220>
   <221> misc_feature
   <222> 347, 352, 353, 534, 555, 587
   <223> n = A, T, C or G
<400> 265
   tcgagcggcc gcccgggcag gtccttgcag ctctgcagtg tcttcttcac catcaggtgc 60
   agggaatagc tcatggattc catcctcagg gctcgagtag gtcaccctgt acctggaaac 120
   ttgcccctgt gggctttccc aagcaatttt gatggaatcg acatccacat cagtgaatgc 180
   cagtccttta gggcgatcaa tgttggttac tgcagtctga accagaggct gactctctcc 240
   gcttggattc tgagcataga cactaaccac atactccact gtgggctgca agccttcaat 300
   agtcatttct gtttgatctg gacctgcagt tttaagtttt tgttggncct gnnccatttt 360
   tggggaaggg gtggttactc ttgtaaccag taacagggga acttgaagca gccacttgac 420
   actaatgctg gtggcctgaa catcggtcac ttgcatctgg gatggtttgg tcaatttctg 480
   ttcggtaatt aatgggaaat tggcttactg gcttgcgggg gctgtctcca cggncagtga 540
   caagcataca caggngatgg gtataatcaa ctccaggttt aaggccnctg atggta     596
<210> 266
   <211> 506
   <212> DNA
   <213> Homo sapiens
<220>
   <221> misc_feature
   <222> 393, 473
   <223> n = A, T, C or G

<400> 266
   agcgtggtcg cggccgaggt ctgggatgct cctgctgtca cagtgagata ttacaggatc 60
   acttacggag aaacaggagg aaatagccct gtccaggagt tcactgtgcc tgggagcaag 120
   tctacagcta ccatcagcgg ccttaaacct ggagttgatt ataccatcac tgtgtatgct 180
   gtcactggcc gtggagacag ccccgcaagc agtaagccaa tttccattaa ttaccgaaca 240
   gaaattgaca aaccatccca gatgcaagtg accgatgttc aggacaacag cattagtgtc 300
   aagtggctgc cttcaagttc ccctgttact ggttacagag taaccaccac tcccaaaaat 360
   gggaccagga ccaacaaaaa actaaaactg canggtccag atcaaacaga aatgactatt 420
   gaaggcttgc agcccacagt ggagtatgtg ggttagtgtc tatgctcaga atnccaagcg 480
   gagagagtca gcctctggtt cagact                                     506
<210> 267
   <211> 548
   <212> DNA
   <213> Homo sapiens
<220>
   <221> misc_feature
   <222> 346, 358, 432, 510, 512
   <223> n = A,T,C or G
<400> 267
   tcgagcggcc gcccgggcag gtcagcgctc tcaggacgtc accaccatgg cctgggctct 60
   gctcctcctc accctcctca ctcagggcac agggtcctgg gcccagtctg ccctgactca 120
   gcctccctcc gcgtccgggt ctcctggaca gtcagtcacc atctcctgca ctggaaccag 180
   cagtgacgtt ggtgcttatg aatttgtctc ctggtaccaa caacacccag gcaaggcccc 240
   caaactcatg atttctgagg tcactaagcg gccctcaggg gtccctgatc gcttctctgg 300
   ctccaagtct ggcaacacgg cctccctgac cgtctctggg ctccangctg aggatgangc 360
   tgattattac tggaagctca tatgcaggca acaacaattg ggtgttcggc ggaagggacc 420
   aagctgaccg tnctaaggtc aagcccaagg cttgcccccc tcggtcactc tgttcccacc 480
   ctcctctgaa gaagctttca agccaacaan gncacactgg gtgtgtctca taagtggact 540
   ttctaccc                                                          548
<210> 268
   <211> 584
   <212> DNA
   <213> Homo sapiens
<220>
   <221> misc_feature
   <222> 98, 380, 421, 454, 495, 506, 512, 561, 565, 579
   <223> n = A,T,C or G
<400> 268
   agcgtggtcg cggccgaggt ctgtagcttc tgtgggactt ccactgctca ggcgtcaggc 60
   tcaggtagct gctggccgcg tacttgttgt tgctttgntt ggagggtgtg gtggtctcca 120
   ctcccgcctt gacggggctg ctatctgcct tccaggccac tgtcacggct cccgggtaga 180
   agtcacttat gagacacacc agtgtggcct tgttggcttg aagctcctca gaggagggtg 240
   ggaacagagt gaccgagggg gcagccttgg gctgacctag gacggtcagc ttggtccctc 300
   cgccgaacac ccaattgttg ttgcctgcat atgagctgca gtaataatca gcctcatcct 360
   cagcctggag cccagagacn gtcaagggag gcccgtgttt gccaagactt ggaagccaga 420
   naagcgatca gggacccctg agggccgctt tacngacctc aaaaaatcat gaatttgggg 480
   ggcctttgcc tgggngttgg ttggtnacca gnaaaacaaa atttcataaa gcaccaacgt 540
   cactgctggt ttccagtgca ngaanatggt gaactgaant gtcc 584
<210> 269
   <211> 368
   <212> DNA
   <213> Homo sapiens
<220>
   <221> misc_feature
   <222> 265, 329
   <223> n = A,T,C or G
<400> 269
   agcgtggtcg cggccgaggt ccagcatcag gagccccgcc ttgccggctc tggtcatcgc 60
   ctttcttttt gtggcctgaa acgatgtcat caattcgcag tagcagaact gccgtctcca 120
   ctgctgtctt ataagtctgc agcttcacag ccaatggctc ccatatgccc agttccttca 180
   tgtccaccaa agtacccgtc tcaccattta caccccaggt ctcacagttc tcctgggtgt 240
   gcttggcccg aagggaggta agtanacgga tggtgctggt cccacagttc tggatcaggg 300
   tacgaggaat gacctctagg gcctgggcna caagccctgt atggacctgc ccgggcgggc 360
   ccgctcga 368
<210> 270
   <211> 368
   <212> DNA
   <213> Homo sapiens
<220>
   <221> misc_feature
   <222> 54, 163, 219, 229, 316
   <223> n = A,T,C or G
<400> 270
   tcgagcggcc gcccgggcag gtccatacag ggctgttgcc caggccctag aggncattcc 60
   ttgtaccctg atccagaact gtgggaccag caccatccgt ctacttacct cccttcgggc 120
   caagcacacc caggagaact gtgagacctg gggtgtaaat ggngagacgg gtactttggt 180
   ggacatgaag gaactgggca tatgggagcc attggctgng aagctgcana cttataagac 240
   agcagtggag acggcagttc tgctactgcg aattgatgac atcgtttcag gccacaaaaa 300
   gaaaggcgat gaccanagcc ggcaaggcgg ggcttcctga tgctggacct cggccgccga 360
   ccacgctt 368
<210> 271
   <211> 424
   <212> DNA
   <213> Homo sapiens
<220>
   <221> misc_feature
   <222> 279, 329, 362, 384, 400
   <223> n = A,T,C or G
<400> 271
   agcgtggtcg cggccgaggt ccactagagg tctgtgtgcc attgcccagg cagagtctct 60
   gcgttacaaa ctcctaggag ggcttgctgt gcggagggcc tgctatggtg tgctgcggtt 120
   catcatggag agtggggcca aaggctgcga ggttgtggtg tctgggaaac tccgaggaca 180
   gagggctaaa tccatgaagt ttgtggatgg cctgatgatc cacagcggag accctgttaa 240
   ctactacgtt gacactgctg tgcgccacgt gttgctcana cagggtgtgc tgggcatcaa 300
   ggtgaagatc atgctgccct gggacccanc tggcaaaaat ggcccttaaa aaccccttgc 360
   cntgaccacg tgaaccattt gtgngaaccc caagatgaan atacttgccc accacccccc 420
   attc                                                              424
<210> 272
   <211> 541
   <212> DNA
   <213> Homo sapiens
<220>
   <221> misc_feature
   <222> 422, 492, 510, 513, 515, 525
   <223> n = A,T,C or G
<400> 272
   tcgagcggcc gcccgggcag gtctgccaag gagaccctgt tatgctgtgg ggactggctg 60
   gggcatggca ggcggctctg gcttcccacc cttctgttct gagatggggg tggtgggcag 120
   tatctcatct ttgggttcca caatgctcac gtggtcaggc aggggcttct tagggccaat 180
   cttaccagtt gggtcccagg gcagcatgat cttcaccttg atgcccagca caccctgtct 240
   gagcaacacg tggcgcacag cagtgtcaac gtagtagtta acagggtctc cgctgtggat 300
   catcaggcca tccacaaact tcatggattt agccctctgt cctcggagtt tcccaaaaca 360
   ccacaacctc gccagccttt gggccccact tcttcatgaa tgaaaccgca gcacaccatt 420
   ancaaggccc ttccgcacag gnaagccctt cctaaggagt tttgtaaacg caaaaaactc 480
   ttgcctgggg caaatgggca cacagacctn tantnggacc ttggnccgcg aaccaccgct 540
   t                                                                 541
<210> 273
   <211> 579
   <212> DNA
   <213> Homo sapiens
<220>
   <221> misc_feature
   <222> 223, 265, 277, 308, 329, 346, 360, 366, 429, 448, 517, 524, 531, 578
   <223> n = A,T,C or G
<400> 273
   agcgtggtcg cggccgaggt ctggccctcc tggcaaggct ggtgaagatg gtcaccctgg 60
   aaaacccgga cgacctggtg agagaggagt tgttggacca cagggtgctc gtggtttccc 120
   tggaactcct ggacttcctg gcttcaaagg cattagggga cacaatggtc tggatggatt 180
   gaagggacag cccggtgctc ctggtgtgaa gggtgaacct ggngcccctg gtgaaaatgg 240
   aactccaggt caaacaggag cccgngggct tcctggngag agaggacgtg ttggtgcccc 300
   tggcccanac ctgcccgggc ggccgctcna aaagccgaaa tccagnacac tggcggccgn 360
   tactantgga atccgaactt cggtaccaaa gcttggccgt aatcatggcc atagcttgtt 420
   ccctggggng gaaattggta ttccgctncc aattccacac aacataccga acccggaaag 480
   cattaaagtg taaaagccct gggggggcct aaatgangtg agcntaactc ncatttaatt 540
   ggcgttgcgc ttcactgccc cgcttttcca gtccgggna                        579
<210> 274
   <211> 330
   <212> DNA
   <213> Homo sapiens
<220>
   <221> misc_feature
   <222> 171
   <223> n = A,T,C or G
<400> 274
   tcgagcggcc gcccgggcag gtctgggcca ggggcaccaa cacgtcctct ctcaccagga 60
   agcccacggg ctcctgtttg acctggagtt ccattttcac caggggcacc aggttcaccc 120
   ttcacaccag gagcaccggg ctgtcccttc aatccatcca gaccattgtg ncccctaatg 180
   cctttgaagc caggaagtcc aggagttcca gggaaaccac gagcaccctg tggtccaaca 240
   actcctctct caccaggtcg tccgggtttt ccagggtgac catcttcacc agccttgcca 300
   ggagggccag acctcggccg cgaccacgct                                  330
<210> 275
   <211> 97
   <212> DNA
   <213> Homo sapiens
<220>
   <221> misc_feature
   <222> 2, 35, 72
   <223> n = A,T,C or G
<400> 275
   ancgtggtcg cggccgaggt cctcaccaga ggtgncacct acaacatcat agtggaggca 60
   ctgaaagacc ancagaggca taaggttcgg gaagagg                           97
<210> 276
   <211> 610
   <212> DNA
   <213> Homo sapiens
<220>
   <221> misc_feature
   <222> 358, 360, 363, 382, 424, 433, 464, 468, 477, 491, 499, 511, 558, 584, 588, 590
   <223> n = A, T, C or G
<400> 276
   tcgagcggcc gcccgggcag gtccattttc tccctgacgg tcccacttct ctccaatctt 60
   gtagttcaca ccattgtcat ggcaccatct agatgaatca catctgaaat gaccacttcc 120
   aaagcctaag cactggcaca acagtttaaa gcctgattca gacattcgtt cccactcatc 180
   tccaacggca taatgggaaa ctgtgtaggg gtcaaagcac gagtcatccg taggttggtt 240
   caagccttcg ttgacagagt tgtccacggt aacaacctct tcccgaacct tatgcctctg 300
   ctggtctttc agtgcctcca ctatgatgtt gtaggtggca cctctggtga ggacctcngn 360
   ccngaacaac gcttaagccc gnattctgca gaataatccc atcacacttg gcggccgctt 420
   cgancatgca tcntaaaagg ggccccaatt tcccccttat aagngaancc gtatttncca 480
   atttcactgg ncccgccgnt tttacaaacg ncggtgaact ggggaaaaac cctggcggtt 540
   acccaacttt aatcgccntt ggcagcacaa tccccccttt tcgnccancn tgggcgtaaa 600
   taaccgaaaa                                                        610
<210> 277
   <211> 38
   <212> DNA
   <213> Homo sapiens
<220>
   <221> misc_feature
   <222> 2, 5, 18, 21, 31
   <223> n = A,T,C or G
<400> 277
   ancgnggtcg cggccgangt nttttttctt nttttttt 38
<210> 278
   <211> 443
   <212> DNA
   <213> Homo sapiens
<220>
   <221> misc_feature
   <222> 156, 212, 233, 245, 327, 331, 336, 361, 364, 381, 391, 397, 419, 437
   <223> n = A,T,C or G
<400> 278
   agcgtggtcg cggccgaggt ctgaggttac atgcgtggtg gtggacgtga gccacgaaga 60
   ccctgaggtc aagttcaact ggtacgtgga cggcgtggag gtgcataatg ccaagacaaa 120
   gccgcgggag gagcagtaca acagcacgta ccgggnggtc agcgtcctca ccgtcctgca 180
   ccagaattgg ttgaatggca aggagtacaa gngcaaggtt tccaacaaag ccntcccagc 240
   ccccntcgaa aaaaccattt ccaaagccaa agggcagccc cgagaaccac aggtgtacac 300
   cctgccccca tcccgggagg aaaagancaa naaccnggtt cagccttaac ttgcttggtc 360
   naangctttt tatcccaacg nacttccccc ntggaantgg gaaaaaccaa tgggccaanc 420
   cgaaaaacaa ttacaanaac ccc                                         443
<210> 279
   <211> 348
   <212> DNA
   <213> Homo sapiens
<220>
   <221> misc_feature
   <222> 219, 256, 291, 297, 307, 314, 317
   <223> n = A,T,C or G
<400> 279
   tcgagcggcc gcccgggcag gtgtcggagt ccagcacggg aggcgtggtc ttgtagttgt 60
   tctccggctg cccattgctc tcccactcca cggcgatgtc gctgggatag aagcctttga 120
   ccaggcaggt caggctgacc tggttcttgg tcatctcctc ccgggatggg ggcagggtga 180
   acacctgggg ttctcggggc ttgccctttg gttttgaana tggttttctc gatgggggct 240
   ggaagggctt tgttgnaaac cttgcacttg actccttgcc attcacccag ncctggngca 300
   ggacggngag gacnctnacc acacggaacc gggctggtgg actgctcc             348
<210> 280
   <211> 149
   <212> DNA
   <213> Homo sapiens
<220>
   <221> misc_feature
   <222> 18, 34, 51, 118, 120, 140
   <223> n = A,T,C or G
<400> 280
   agcgtggtcg cggacgangt cctgtcagag tggnactggt agaagttcca ngaaccctga 60
   actgtaaggg ttcttcatca gtgccaacag gatgacatga aatgatgtac tcagaagngn 120
   cctggaatgg ggcccatgan atggttgcc                                  149

<210> 281
   <211> 404
   <212> DNA
   <213> Homo sapiens
<220>
   <221> misc_feature
   <222> 383, 386, 388, 393
   <223> n = A,T,C or G
<400> 281
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaagaata atcagaagag cgagcccctg 240
   attggaagga aaaagacaga cgagcttccc caactggtaa cccttccaca ccccaatctt 300
   catggaccag agatcttgga tgttccttcc acagttcaaa agaccccttt cggcaccccc 360
   cctgggtatg aacctgggaa aanggnantt aanctttcct ggca                 404
<210> 282
   <211> 507
   <212> DNA
   <213> Homo sapiens
<220>
   <221> misc_feature
   <222> 320, 341, 424, 450, 459, 487, 498
   <223> n = A, T, C or G
<400> 282
   agcgtggtcg cggccgaggt ctgggatgct cctgctgtca cagtgagata ttacaggatc 60
   acttacggag aaacaggagg aaatagccct gtccaggagt tcactgtgcc tgggagcaag 120
   tctacagcta ccatcagcgg ccttaaacct ggagttgatt ataccatcac tgtgtatgct 180
   gtcactggcc gtggagacag ccccgcaagc agcaagccaa tttccattaa ttaccgaaca 240
   gaaattgaca aaccatccca gatgcaagtg accgatgttc aggacaacag cattagtgtc 300
   aagtggctgc cttcaaggtn ccctggtact gggttacaga ntaaccacca ctcccaaaaa 360
   tggaccagga accacaaaaa cttaaactgc agggtccaga tcaaaacaga aatgactatt 420
   gaangcttgc agcccacagt gggagtatgn gggtagtgnc tatgcttcag aatccaagcg 480
   gaaaaangtc aagccttntg ggttcaa                                     507
<210> 283
   <211> 325
   <212> DNA
   <213> Homo sapiens
<220>
   <221> misc_feature
   <222> 216, 292, 303, 304
   <223> n = A,T,C or G
<400> 283
   tcgagcggcc gcccgggcag gtccttgcag ctctgcagtg tcttcttcac catcaggtgc 60
   agggaatagc tcatggattc catcctcagg gctcgagtag gtcaccctgt acctggaaac 120
   ttgcccctgt gggctttccc aagcaatttt gatggaatcg acatccacat cagtgaatgc 180
   cagtccttta gggcgatcaa tgttggttac tgcagnctga accagaggct gactctctcc 240
   gcttggattc tgagcataga cactaaccac atactccact gtgggctgca anccttcaat 300
   aanncatttc tgtttgatct ggacc                                       325
<210> 284
   <211> 331
   <212> DNA
   <213> Homo sapiens
<220>
   <221> misc_feature
   <222> 54, 59, 63, 121, 312, 327
   <223> n = A,T,C or G
<400> 284
   tcgagcggcc gcccgggcag gtctggtggg gtcctggcac acgcacatgg gggngttgnt 60
   ctnatccagc tgcccagccc ccattggcga gtttgagaag gtgtgcagca atgacaacaa 120
   naccttcgac tcttcctgcc acttctttgc cacaaagtgc accctggagg gcaccaagaa 180
   gggccacaag ctccacctgg actacatcgg gccttgcaaa tacatccccc cttgcctgga 240
   ctctgagctg accgaattcc cccttgcgca tgcgggactg gctcaagaac cgtcctggca 300
   cccttgtatg anagggatga agacacnacc c                                 331
<210> 285
   <211> 509
   <212> DNA
   <213> Homo sapiens
<220>
   <221> misc_feature
   <222> 316, 319, 327, 329, 339, 344, 357, 384, 398, 427, 443, 450, 478
   <223> n = A,T,C or G
<400> 285
   agcgtggtcg cggccgaggt ctgtcctaca gtcctcagga ctctactccc tcagcagcgt 60
   ggtgaccgtg ccctccagca acttcggcac ccagacctac acctgcaacg tagatcacaa 120
   gcccagcaac accaaggtgg acaagagagt tgagcccaaa tcttgtgaca aaactcacac 180
   atgcccaccg tgcccagcac ctgaactcct ggggggaccg tcagtcttcc tcttcccccg 240
   catccccctt ccaaacctgc ccgggcggcc gctcgaaagc cgaattccag cacactggcg 300
   gccggtacta gtgganccna acttggnanc caacctggng gaantaatgg gcataanctg 360
   tttctggggg gaaattggta tccngtttac aattcccnca caacatacga gccggaagca 420
   taaaagngta aaagcctggg ggnggcctan tgaagtgaag ctaaactcac attaattngc 480
   gttgccgctc actggcccgc ttttccagc                                    509
<210> 286
   <211> 336
   <212> DNA
   <213> Homo sapiens
<220>
   <221> misc_feature
   <222> 188, 251, 267
   <223> n = A,T,C or G
<400> 286
   tcgagcggcc gcccgggcag gtttggaagg gggatgcggg ggaagaggaa gactgacggt 60
   ccccccagga gttcaggtgc tgggcacggt gggcatgtgt gagttttgtc acaagatttg 120
   ggctcaactc tcttgtccac cttggtgttg ctgggcttgt gatctacgtt gcaggtgtag 180
   gtctgggngc cgaagttgct ggagggcacg gtcaccacgc tgctgaggga gtagagtcct 240
   gaggactgta ngacagacct cggccgngac cacgctaagc cgaattctgc agatatccat 300
   cacactggcg gccgctccga gcatgcattt tagagg                           336
<210> 287
   <211> 30
   <212> DNA
   <213> Homo sapiens
<220>
   <221> misc_feature
   <222> 8, 18
   <223> n = A, T, C or G
<400> 287
   agcgtggncg cggacganga caacaacccc 30
<210> 288
   <211> 316
   <212> DNA
   <213> Homo sapiens
<220>
   <221> misc_feature
   <222> 22, 130
   <223> n = A, T, C or G
<400> 288
   tcgagcggcc gcccgggcag gnccacatcg gcagggtcgg agccctggcc gccatactcg 60
   aactggaatc catcggtcat gctcttgccg aaccagacat gcctcttgtc cttggggttc 120
   ttgctgatgn accagttctt ctgggccaca ctgggctgag tggggtacac gcaggtctca 180
   ccagtctcca tgttgcagaa gactttgatg gcatccaggt tgcagccttg gttggggtca 240
   atccagtact ctccactctt ccagtcagag tggcacatct tgaggtcacg gcaggtgcgg 300
   gcggggttct tgacct                                                 316
<210> 289
   <211> 308
   <212> DNA
   <213> Homo sapiens
<220>
   <221> misc_feature
   <222> 36, 165, 191, 195, 218, 235
   <223> n = A,T,C or G
<400> 289
   agcgtggtcg cggccgaggt ccagcctgga gataanggtg aaggtggtgc ccccggactt 60
   ccaggtatag ctggacctcg tggtagccct ggtgagagag gtgaaactgg ccctccagga 120
   cctgctggtt tccctggtgc tcctggacag aatggtgaac ctggnggtaa aggagaaaga 180
   ggggctccgg ntganaaagg tgaaggaggc cctcctgnat tggcaggggc cccangactt 240
   agaggtggag ctggcccccc tggccccgaa ggaggaaagg gtgctgctgg tcctcctggg 300
   ccacctgg                                                          308
<210> 290
   <211> 324
   <212> DNA
   <213> Homo sapiens
<220>
   <221> misc_feature
   <222> 184
   <223> n = A,T,C or G
<400> 290
   tcgagcggcc gcccgggcag gtctgggcca ggaggaccaa taggaccagt aggacccctt 60
   gggccatctt tccctgggac accatcagca cctggaccgc ctggttcacc cttgtcaccc 120
   tttggaccag gacttccaag acctcctctt tctccaggca ttccttgcag accaggagta 180
   ccancagcac caggtggccc aggaggacca gcagcaccct ttcctccttc gggaccaggg 240
   ggaccagctc cacctctaag tcctggggcc cctgccaatc caggagggcc tccttcacct 300
   ttctcacccg gagcccctct ttct 324
<210> 291
   <211> 278
   <212> DNA
   <213> Homo sapiens
<220>
   <221> misc_feature
   <222> 249, 267
   <223> n = A, T, C or G
<400> 291
   tcgagcggcc gcccgggcag gtccaccggg atattcgggg gtctggcagg aatgggaggc 60
   atccagaacg agaaggagac catgcaaagc ctgaacgacc gcctggcctc ttacctggac 120
   agagtgagga gcctggagac cgacaaccgg aggctggaga gcaaaatccg ggagcacttg 180
   gagaagaagg gaccccaggt cagagactgg agccattact tcaagatcat cgaggacctg 240
   agggctcana tcttcgcaaa tactgcngac aatgcccg 278
<210> 292
   <211> 299
   <212> RNA
   <213> Homo sapiens
<220>
   <221> misc_feature
   <222> 6, 19, 25, 51, 53, 61, 63, 70, 109, 136, 157, 241, 276
   <223> n = A,T,C or G
<400> 292
   atgcgnggtc gcggccgang accanctctg gctcatactt gactctaaag ncntcaccag 60
   nanttacggn cattgccaat ctgcagaacg atgcgggcat tgtccgcant atttgcgaag 120
   atctgagccc tcaggncctc gatgatcttg aagtaanggc tccagtctct gacctggggt 180
   cccttcttct ccaagtgctc ccggattttg ctctccagcc tccggttctc ggtctccaag 240
   ncttctcact ctgtccagga aaagaggcca ggcggncgat cagggctttt gcatggact 299
<210> 293
   <211> 101
   <212> DNA
   <213> Homo sapiens
<400> 293
   agcgtggtcg cggccgaggt tgtacaagct tttttttttt tttttttttt tttttttttt 60
   tttttttttt tttttttttt tttttttttt tttttttttt t 101
<210> 294
   <211> 285
   <212> DNA
   <213> Homo sapiens
<220>
   <221> misc_feature
   <222> 64, 103, 110, 237, 282
   <223> n = A,T,C or G
<400> 294
   tcgagcggcc gcccgggcag gtctgccaac accaagattg gcccccgccg catccacaca 60
   gttngtgtgc ggggaggtaa caagaaatac cgtgccctga ggntggacgn ggggaatttc 120
   tcctggggct cagagtgttg tactcgtaaa acaaggatca tcgatgttgt ctacaatgca 180
   tctaataacg agctggttcg taccaagacc ctggtgaaga attgcatcgt gctcatngac 240
   agcacaccgt accgacagtg ggtaccgaag tcccactatg cncct                 285

<210> 295
   <211> 216
   <212> DNA
   <213> Homo sapiens
<400> 295
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaag                           216
<210> 296
   <211> 414
   <212> DNA
   <213> Homo sapiens
<220>
   <221> misc_feature
   <222> 7, 10, 33, 61, 62, 63, 88, 109, 122, 255, 298, 307, 340, 355, 386, 393
   <223> n = A,T,C or G
<400> 296
   agcgtgntcn cggccgagga tggggaagct cgnctgtctt tttccttcca atcaggggct 60
   nnntcttctg attattcttc agggcaanga cataaattgt atattcggnt cccggttcca 120
   gnccagtaat agtagcctct gtgacaccag ggcggggccg agggaccact tctctgggag 180
   gagacccagg cttctcatac ttgatgatga agccggtaat cctggcacgt gggcggctgc 240
   catgatacca ccaangaatt gggtgtggtg gacctgcccg ggcgggccgc tcgaaaancc 300
   gaattcntgc aagaatatcc atcacacttg ggcgggccgn tcgaaccatg catcntaaaa 360
   gggccccaat ttccccccta ttaggngaag ccncatttaa caaattccac ttgg       414
<210> 297
   <211> 376
   <212> DNA
   <213> Homo sapiens
<220>
   <221> misc_feature
   <222> 312, 326, 335, 361
   <223> n = A,T,C or G
<400> 297
   tcgagcggcc gcccgggcag gtctcgcggt cgcactggtg atgctggtcc tgttggtccc 60
   cccggccctc ctggacctcc tggtccccct ggtcctccca gcgctggttt cgacttcagc 120
   ttcctgcccc agccacctca agagaaggct cacgatggtg gccgctacta ccgggctgat 180
   gatgccaatg tggttcgtga ccgtgacctc gaggtggaca ccaccctcaa gagccttgag 240
   ccagcagaat cgaaaacatt cggaacccaa gaagggcaag cccgcaaaga aaccccgccc 300
   gcacctggcc gngaacctcc aagaangtgc ccacntcttg actgggaaaa aaagggaaaa 360
   ntacttggaa ttggac                                                 376
<210> 298
   <211> 357
   <212> DNA
   <213> Homo sapiens
<220>
   <221> misc_feature
   <222> 345, 346
   <223> n = A,T,C or G
<400> 298
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacacgc aggtctcacc 180
   agtctccatg ttgcagaaga ctttgatggc atccaggttg cagccttggt tggggtcaat 240
   ccagtactct ccactcttcc agtcagaagt ggcacatctt gaggtcacgg cagggtgcgg 300
   gcggggttct tgcgggctgc ccttctgggc tcccggaatg ttctnngaac ttgctgg    357
<210> 299
   <211> 307
   <212> DNA
   <213> Homo sapiens
<220>
   <221> misc_feature
   <222> 281, 285, 306
   <223> n = A,T,C or G
<400> 299
   agcgtggtcg cggccgaggt ccactagagg tctgtgtgcc attgcccagg cagagtctct 60
   gcgttacaaa ctcctaggag ggcttgctgt gcggagggcc tgctatggtg tgctgcggtt 120
   catcatggag agtggggcca aaggctgcga ggttgtggtg tctgggaaac tccgaggaca 180
   gagggctaaa tccatgaagt ttgtggatgg cctgatgatc cacagcggag accctgttaa 240
   ctactacgtt gacacttgct tgtgcgccac gtgttgctca nacangggtg ggctgggcat 300
   caaggng                                                           307
<210> 300
   <211> 351
   <212> DNA
   <213> Homo sapiens
<400> 300
   tcgagcggcc gcccgggcag gtctgccaag gagaccctgt tatgctgtgg ggactggctg 60
   gggcatggca ggcggctctg gcttcccacc cttctgttct gagatggggg tggtgggcag 120
   tatctcatct ttgggttcca caatgctcac gtggtcaggc aggggcttct tagggccaat 180
   cttaccagtt gggtcccagg gcagcatgat cttcaccttg atgcccagca caccctgtct 240
   gagcaacacg tggcgcacag caagtgtcaa cgtaagtaag ttaacagggt ctccgctgtg 300
   gatcatcagg ccatccacaa acttcatgga tttaaccctc tgtcctcgga g          351
<210> 301
   <211> 330
   <212> DNA
   <213> Homo sapiens
<400> 301
   tcgagcggcc gcccgggcag gtgtttcaga ggttccaagg tccactgtgg aggtcccagg 60
   agtgctggtg gtgggcacag aggtccgatg ggtgaaacca ttgacataga gactgttcct 120
   gtccagggtg taggggccca gctctttgat gccattggcc agttggctca gctcccagta 180
   cagccgctct ctgttgagtc cagggctttt ggggtcaaga tgatggatgc agatggcatc 240
   cactccagtg gctgctccat ccttctcgga cctgagagag gtcagtctgc agccagagta 300
   cagagggcca acactggtgt tctttgaata                                  330
<210> 302
   <211> 317
   <212> DNA
   <213> Homo sapiens
<220>
   <221> misc_feature
   <222> 129, 295
   <223> n = A,T,C or G
<400> 302
   agcgtggtcg cggccgaggt ctgtactggg agctaagcaa actgaccaat gacattgaag 60
   agctgggccc ctacaccctg gacaggaaca gtctctatgt caatggtttc acccatcaga 120
   gctctgtgnc caccaccagc actcctggga cctccacagt ggatttcaga acctcaggga 180
   ctccatcctc cctctccagc cccacaatta tggctgctgg ccctctcctg gtaccattca 240
   ccctcaactt caccatcacc aacctgcagt atggggagga catgggtcac cctgnctcca 300
   ggaagttcaa caccaca                                                317
<210> 303
   <211> 283
   <212> DNA
   <213> Homo sapiens
<220>
   <221> misc_feature
   <222> 139, 146, 195
   <223> n = A,T,C or G
<400> 303
   tcgagcggcc gcccggacag gtctgggcgg atagcaccgg gcatattttg gaatggatga 60
   ggtctggcac cctgagcagt ccagcgagga cttggtctta gttgagcaat ttggctagga 120
   ggatagtatg cagcacggnt ctgagnctgt gggatagctg ccatgaagta acctgaagga 180
   ggtgctggct ggtangggtt gattacaggg ttgggaacag ctcgtacact tgccattctc 240
   tgcatatact ggttagtgag gtgagcctgg ccctcttctt ttg                   283
<210> 304
   <211> 72
   <212> DNA
   <213> Homo sapiens
<220>
   <221> misc_feature
   <222> 59
   <223> n = A,T,C or G
<400> 304
   agcgtggtcg cggccgaggt gagccacagg tgaccggggc tgaagctggg gctgctggnc 60
   ctgctggtcc tg                                                     72
<210> 305
   <211> 245
   <212> DNA
   <213> Homo sapiens
<220>
   <221> misc_feature
   <222> 5, 11, 22, 98, 102
   <223> n = A,T,C or G
<400> 305
   cagcngctcc nacggggcct gngggaccaa caacaccgtt ttcaccctta ggccctttgg 60
   ctcctctttc tcctttagca ccaggttgac cagcagcncc ancaggacca gcaaatccat 120
   tggggccagc aggaccgacc tcaccacgtt caccagggct tccccgagga ccagcaggac 180
   cagcaggacc agcagcccca gcttcgcccc ggtcacctgt ggctcacctc ggccgcgacc 240
   acgct                                                             245
<210> 306
   <211> 246
   <212> DNA
   <213> Homo sapiens
<220>
   <221> misc_feature
   <222> 144, 159
   <223> n = A,T,C or G
<400> 306
   tcgagcggtc gcccgggcag gtccaccggg atagccgggg gtctggcagg aatgggaggc 60
   atccagaacg agaaggagac catgcaaagc ctgaacgacc gcctggcctc ttacctggac 120
   agagtgagga gcctggagac cganaaccgg aggctggana gcaaaatccg ggagcacttg 180
   gagaagaagg gaccccaggt caagagactg gagccattac ttcaagatca tcgagggacc 240
   tggagg                                                            246
<210> 307
   <211> 333
   <212> DNA
   <213> Homo sapiens
<220>
   <221> misc_feature
   <222> 5
   <223> n = A,T,C or G
<400> 307
   agcgnggtcg cggccgaggt ccagctctgt ctcatacttg actctaaagt catcagcagc 60
   aagacgggca ttgtcaatct gcagaacgat gcgggcattg tccgcagtat ttgcgaagat 120
   ctgagccctc aggtcctcga tgatcttgaa gtaatggctc cagtctctga cctggggtcc 180
   cttcttctcc aagtgctccc ggattttgct ctccagcctc cggttctcgg tctccaggct 240
   cctcactctg tccaggtaag aaggcccagg cggtcgttca ggctttgcat ggtctccttc 300
   tcgttctgga tgcctcccat tcctgccaga ccc                              333
<210> 308
   <211> 310
   <212> DNA
   <213> Homo sapiens
<400> 308
   tcgagcggcc gcccgggcag gtcaggaagc acattggtct tagagccact gcctcctgga 60
   ttccacctgt gctgcggaca tctccaggga gtgcagaagg gaagcaggtc aaactgctca 120
   gatcagtcag actggctgtt ctcagttctc acctgagcaa ggtcagtctg cagccagagt 180
   acagagggcc aacactggtg ttcttgaaca agggcttgag cagaccctgc agaaccctct 240
   tccgtggtgt tgaacttcct ggaaaccagg gtgttgcatg tttttcctca taatgcaagg 300
   ttggtgatgg                                                        310

<210> 309
   <211> 429
   <212> DNA
   <213> Homo sapiens
<400> 309
   agcgtggtcg cggccgaggt ccacatcggc agggtcggag ccctggccgc catactcgaa 60
   ctggaatcca tcggtcatgc tctcgccgaa ccagacatgc ctcttgtcct tggggttctt 120
   gctgatgtac cagttcttct gggccacact gggctgagtg gggtacaccg caggtctcac 180
   cagtctccat gttgcagaag actttgatgg catccaggtt gcagccttgg ttggggtcaa 240
   tccagtactc tccactcttc cagtcagaag tgggcacatc ttgaggtcac cggcaggtgc 300
   cgggccgggg gttcttgcgg cttgccctct gggctccgga tgttctcgat ctgcttggct 360
   caggctcttg agggtgggtg tccacctcga ggtcacggtc accgaaacct gcccgggcgg 420
   cccgctcga                                                         429
<210> 310
   <211> 430
   <212> DNA
   <213> Homo sapiens
<220>
   <221> misc_feature
   <222> 342
   <223> n = A,T,C or G
<400> 310
   tcgagcggtc gcccgggcag gtttcgtgac cgtgacctcg aggtggacac caccctcaag 60
   agcctgagcc agcagatcga gaacatccgg agcccagagg gcagccgcaa gaaccccgcc 120
   cgcacctgcc gtgacctcaa gatgtgccac tctgactgga agagtggaga gtactggatt 180
   gaccccaacc aaggctgcaa cctggatgcc atcaaagtct tctgcaacat ggagactggt 240
   gagacctgcg tgtaccccac tcagcccagt gtgggcccag aagaaactgg tacatcagca 300
   aggaacccca aggacaagag gcattgtctt ggttcggcga gnagcatgac ccgatggatt 360
   ccagtttcga gtattggcgg ccagggcttc ccgacccttg ccgatgtgga cctcggccgc 420
   gaccaccgct                                                        430
<210> 391
   <211> 2627
   <212> DNA
   <213> Homo sapiens
<400> 391
   ccacgcgtcc gcccacgcgt ccggaaggca gcggcagctc cactcagcca gtacccagat 60
   acgctgggaa ccttccccag ccatggcttc cctggggcag atcctcttct ggagcataat 120
   tagcatcatc attattctgg ctggagcaat tgcactcatc attggctttg gtatttcagg 180
   gagacactcc atcacagtca ctactgtcgc ctcagctggg aacattgggg aggatggaat 240
   cctgagctgc acttttgaac ctgacatcaa actttctgat atcgtgatac aatggctgaa 300
   ggaaggtgtt ttaggcttgg tccatgagtt caaagaaggc aaagatgagc tgtcggagca 360
   ggatgaaatg ttcagaggcc ggacagcagt gtttgctgat caagtgatag ttggcaatgc 420
   ctctttgcgg ctgaaaaacg tgcaactcac agatgctggc acctacaaat gttatatcat 480
   cacttctaaa ggcaagggga atgctaacct tgagtataaa actggagcct tcagcatgcc 540
   ggaagtgaat gtggactata atgccagctc agagaccttg cggtgtgagg ctccccgatg 600
   gttcccccag cccacagtgg tctgggcatc ccaagttgac cagggagcca acttctcgga 660
   agtctccaat accagctttg agctgaactc tgagaatgtg accatgaagg ttgtgtctgt 720
   gctctacaat gttacgatca acaacacata ctcctgtatg attgaaaatg acattgccaa 780
   agcaacaggg gatatcaaag tgacagaatc ggagatcaaa aggcggagtc acctacagct 840
   gctaaactca aaggcttctc tgtgtgtctc ttctttcttt gccatcagct gggcacttct 900
   gcctctcagc ccttacctga tgctaaaata atgtgccttg gccacaaaaa agcatgcaaa 960
   gtcattgtta caacagggat ctacagaact atttcaccac cagatatgac ctagttttat 1020
   atttctggga ggaaatgaat tcatatctag aagtctggag tgagcaaaca agagcaagaa 1080
   acaaaaagaa gccaaaagca gaaggctcca atatgaacaa gataaatcta tcttcaaaga 1140
   catattagaa gttgggaaaa taattcatgt gaactagaca agtgtgttaa gagtgataag 1200
   taaaatgcac gtggagacaa gtgcatcccc agatctcagg gacctccccc tgcctgtcac 1260
   ctggggagtg agaggacagg atagtgcatg ttctttgtct ctgaattttt agttatatgt 1320
   gctgtaatgt tgctctgagg aagcccctgg aaagtctatc ccaacatatc cacatcttat 1380
   attccacaaa ttaagctgta gtatgtaccc taagacgctg ctaattgact gccacttcgc 1440
   aactcagggg cggctgcatt ttagtaatgg gtcaaatgat tcacttttta tgatgcttcc 1500
   aaaggtgcct tggcttctct tcccaactga caaatgccaa agttgagaaa aatgatcata 1560
   attttagcat aaacagagca gtcggcgaca ccgattttat aaataaactg agcaccttct 1620
   ttttaaacaa acaaatgcgg gtttatttct cagatgatgt tcatccgtga atggtccagg 1680
   gaaggacctt tcaccttgac tatatggcat tatgtcatca caagctctga ggcttctcct 1740
   ttccatcctg cgtggacagc taagacctca gttttcaata gcatctagag cagtgggact 1800
   cagctggggt gatttcgccc cccatctccg ggggaatgtc tgaagacaat tttggttacc 1860
   tcaatgaggg agtggaggag gatacagtgc tactaccaac tagtggataa aggccaggga 1920
   tgctgctcaa cctcctacca tgtacaggac gtctccccat tacaactacc caatccgaag 1980
   tgtcaactgt gtcaggacta agaaaccctg gttttgagta gaaaagggcc tggaaagagg 2040
   ggagccaaca aatctgtctg cttcctcaca ttagtcattg gcaaataagc attctgtctc 2100
   tttggctgct gcctcagcac agagagccag aactctatcg ggcaccagga taacatctct 2160
   cagtgaacag agttgacaag gcctatggga aatgcctgat gggattatct tcagcttgtt 2220
   gagcttctaa gtttctttcc cttcattcta ccctgcaagc caagttctgt aagagaaatg 2280
   cctgagttct agctcaggtt ttcttactct gaatttagat ctccagaccc ttcctggcca 2340
   caattcaaat taaggcaaca aacatatacc ttccatgaag cacacacaga cttttgaaag 2400
   caaggacaat gactgcttga attgaggcct tgaggaatga agctttgaag gaaaagaata 2460
   ctttgtttcc agcccccttc ccacactctt catgtgttaa ccactgcctt cctggacctt 2520
   ggagccacgg tgactgtatt acatgttgtt atagaaaact gattttagag ttctgatcgt 2580
   tcaagagaat gattaaatat acatttccta caccaaaaaa aaaaaaa              2627
<210> 392
   <211> 309
   <212> PRT
   <213> Homo sapiens
<400> 392
   His Ala Ser Ala His Ala Ser Gly Arg Gln Arg Gln Leu His Ser Ala
    1               5                  10                  15
   Ser Thr Gln Ile Arg Trp Glu Pro Ser Pro Ala Met Ala Ser Leu Gly
               20                  25                  30
   Gln Ile Leu Phe Trp Ser Ile Ile Ser Ile Ile Ile Ile Leu Ala Gly
           35                  40                  45
   Ala Ile Ala Leu Ile Ile Gly Phe Gly Ile Ser Gly Arg His Ser Ile
       50                  55                  60
   Thr Val Thr Thr Val Ala Ser Ala Gly Asn Ile Gly Glu Asp Gly Ile
   65                  70                  75                  80
   Leu Ser Cys Thr Phe Glu Pro Asp Ile Lys Leu Ser Asp Ile Val Ile
                   85                  90                  95
   Gln Trp Leu Lys Glu Gly Val Leu Gly Leu Val His Glu Phe Lys Glu
               100                 105                 110
   Gly Lys Asp Glu Leu Ser Glu Gln Asp Glu Met Phe Arg Gly Arg Thr
           115                 120                 125
   Ala Val Phe Ala Asp Gln Val Ile Val Gly Asn Ala Ser Leu Arg Leu
       130                 135                 140
   Lys Asn Val Gln Leu Thr Asp Ala Gly Thr Tyr Lys Cys Tyr Ile Ile
   145                 150                 155                 160
   Thr Ser Lys Gly Lys Gly Asn Ala Asn Leu Glu Tyr Lys Thr Gly Ala
                   165                 170                 175
   Phe Ser Met Pro Glu Val Asn Val Asp Tyr Asn Ala Ser Ser Glu Thr
               180                 185                 190
   Leu Arg Cys Glu Ala Pro Arg Trp Phe Pro Gln Pro Thr Val Val Trp
           195                 200                 205
   Ala Ser Gln Val Asp Gln Gly Ala Asn Phe Ser Glu Val Ser Asn Thr
       210                 215                 220
   Ser Phe Glu Leu Asn Ser Glu Asn Val Thr Met Lys Val Val Ser Val
   225                 230                 235                 240
   Leu Tyr Asn Val Thr Ile Asn Asn Thr Tyr Ser Cys Met Ile Glu Asn
                   245                 250                 255
   Asp Ile Ala Lys Ala Thr Gly Asp Ile Lys Val Thr Glu Ser Glu Ile
               260                 265                 270
   Lys Arg Arg Ser His Leu Gln Leu Leu Asn Ser Lys Ala Ser Leu Cys
           275                 280                 285
   Val Ser Ser Phe Phe Ala Ile Ser Trp Ala Leu Leu Pro Leu Ser Pro
       290                 295                 300
   Tyr Leu Met Leu Lys
   305
<210> 393
   <211> 282
   <212> PRT
   <213> Homo sapiens
<400> 393
   Met Ala Ser Leu Gly Gln Ile Leu Phe Trp Ser Ile Ile Ser Ile Ile
    1               5                  10                  15
   Ile Ile Leu Ala Gly Ala Ile Ala Leu Ile Ile Gly Phe Gly Ile Ser
               20                  25                  30
   Gly Arg His Ser Ile Thr Val Thr Thr Val Ala Ser Ala Gly Asn Ile
           35                  40                  45
   Gly Glu Asp Gly Ile Leu Ser Cys Thr Phe Glu Pro Asp Ile Lys Leu
       50                  55                  60
   Ser Asp Ile Val Ile Gln Trp Leu Lys Glu Gly Val Leu Gly Leu Val
   65                  70                  75                  80
   His Glu Phe Lys Glu Gly Lys Asp Glu Leu Ser Glu Gln Asp Glu Met
                   85                  90                  95
   Phe Arg Gly Arg Thr Ala Val Phe Ala Asp Gln Val Ile Val Gly Asn
               100                 105                 110
   Ala Ser Leu Arg Leu Lys Asn Val Gln Leu Thr Asp Ala Gly Thr Tyr
           115                 120                 125
   Lys Cys Tyr Ile Ile Thr Ser Lys Gly Lys Gly Asn Ala Asn Leu Glu
       130                 135                 140
   Tyr Lys Thr Gly Ala Phe Ser Met Pro Glu Val Asn Val Asp Tyr Asn
   145                 150                 155                 160
   Ala Ser Ser Glu Thr Leu Arg Cys Glu Ala Pro Arg Trp Phe Pro Gln
                   165                 170                 175
   Pro Thr Val Val Trp Ala Ser Gln Val Asp Gln Gly Ala Asn Phe Ser
               180                 185                 190
   Glu Val Ser Asn Thr Ser Phe Glu Leu Asn Ser Glu Asn Val Thr Met
           195                 200                 205
   Lys Val Val Ser Val Leu Tyr Asn Val Thr Ile Asn Asn Thr Tyr Ser
       210                 215                 220
   Cys Met Ile Glu Asn Asp Ile Ala Lys Ala Thr Gly Asp Ile Lys Val
   225                 230                 235                 240
   Thr Glu Ser Glu Ile Lys Arg Arg Ser His Leu Gln Leu Leu Asn Ser
                   245                 250                 255
   Lys Ala Ser Leu Cys Val Ser Ser Phe Phe Ala Ile Ser Trp Ala Leu
               260                 265                 270
   Leu Pro Leu Ser Pro Tyr Leu Met Leu Lys
           275                 280
<210> 394
   <211> 20
   <212> PRT ,
   <213> Homo sapiens
<400> 394
   Met Ala Ser Leu Gly Gln Ile Leu Phe Trp Ser Ile Ile Ser Ile Ile
    1               5                  10                  15
   Ile Ile Leu Ala
               20
<210> 395
   <211> 20
   <212> PRT
   <213> Homo sapiens
<400> 395
   Ile Ile Ile Leu Ala Gly Ala Ile Ala Leu Ile Ile Gly Phe Gly Ile
    1               5                  10                  15
   Ser Gly Arg His
               20
<210> 396
   <211> 20
   <212> PRT
   <213> Homo sapiens
<400> 396
   Ile Ser Gly Arg His Ser Ile Thr Val Thr Thr Val Ala Ser Ala Gly
    1               5                  10                  15
   Asn Ile Gly Glu
               20
<210> 397
   <211> 20
   <212> PRT
   <213> Homo sapiens
<400> 397
   Gly Asn Ile Gly Glu Asp Gly Ile Leu Ser Cys Thr Phe Glu Pro Asp
    1               5                  10                  15
   Ile Lys Leu Ser
               20
<210> 398
   <211> 20
   <212> PRT
   <213> Homo sapiens
<400> 398
   Asp Ile Lys Leu Ser Asp Ile Val Ile Gln Trp Leu Lys Glu Gly Val
    1               5                  10                  15
   Leu Gly Leu Val
               20
<210> 399
   <211> 20
   <212> PRT
   <213> Homo sapiens
<400> 399
   Val Leu Gly Leu Val His Glu Phe Lys Glu Gly Lys Asp Glu Leu Ser
   1 5 10 15
   Glu Gln Asp Glu
   20
<210> 400
   <211> 20
   <212> PRT
   <213> Homo sapiens
<400> 400
   Ser Glu Gln Asp Glu Met Phe Arg Gly Arg Thr Ala Val Phe Ala Asp
   1 5 10 15
   Gln Val Ile Val
   20
<210> 401
   <211> 20
   <212> PRT
   <213> Homo sapiens
<400> 401
   Asp Gln Val Ile Val Gly Asn Ala Ser Leu Arg Leu Lys Asn Val Gln
   1 5 10 15
   Leu Thr Asp Ala
   20
<210> 402 .
   <211> 21
   <212> PRT
   <213> Homo sapiens
<400> 402
   Val Gln Leu Thr Asp Ala Gly Thr Tyr Lys Cys Tyr Ile Ile Thr Ser
   1 5 10 15
   Lys Gly Lys Gly Asn
   20
<210> 403
   <211> 20
   <212> PRT
   <213> Homo sapiens
<400> 403
   Lys Gly Lys Gly Asn Ala Asn Leu Glu Tyr Lys Thr Gly Ala Phe Ser
   1 5 10 15
   Met Pro Glu Val
   20
<210> 404
   <211> 20
   <212> PRT
   <213> Homo sapiens
<400> 404
   Ser Met Pro Glu Val Asn Val Asp Tyr Asn Ala Ser Ser Glu Thr Leu
    1               5                  10                  15
   Arg Cys Glu Ala
               20
<210> 405
   <211> 20
   <212> PRT
   <213> Homo sapiens
<400> 405
   Leu Arg Cys Glu Ala Pro Arg Trp Phe Pro Gln Pro Thr Val Val Trp
    1               5                  10                  15
   Ala Ser Gln Val
               20
<210> 406
   <211> 20
   <212> PRT
   <213> Homo sapiens
<400> 406
   Trp Ala Ser Gln Val Asp Gln Gly Ala Asn Phe Ser Glu Val Ser Asn
    1               5                  10                  15
   Thr Ser Phe Glu
               20

<210> 407
   <211> 20
   <212> PRT
   <213> Homo sapiens
<400> 407
   Asn Thr Ser Phe Glu Leu Asn Ser Glu Asn Val Thr Met Lys Val Val
    1               5                  10                  15
   Ser Val Leu Tyr
               20
<210> 408
   <211> 20
   <212> PRT
   <213> Homo sapiens
<400> 408
   Val Ser Val Leu Tyr Asn Val Thr Ile Asn Asn Thr Tyr Ser Cys Met
   1 5 10 15
   Ile Glu Asn Asp
   20
<210> 409
   <211> 20
   <212> PRT
   <213> Homo sapiens
<400> 409
   Met Ile Glu Asn Asp Ile Ala Lys Ala Thr Gly Asp Ile Lys Val Thr
   1 5 10 15
   Glu Ser Glu Ile
   20
<210> 410
   <211> 20
   <212> PRT
   <213> Homo sapiens
<400> 410
   Thr Glu Ser Glu Ile Lys Arg Arg Ser His Leu Gln Leu Leu Asn Ser
   1 5 10 15
   Lys Ala Ser Leu
   20
<210> 411
   <211> 20
   <212> PRT
   <213> Homo sapiens
<400> 411
   Ser Lys Ala Ser Leu Cys Val Ser Ser Phe Phe Ala Ile Ser Trp Ala
   1 5 10 15
   Leu Leu Pro Leu
<210> 412
   <211> 20
   <212> PRT
   <213> Homo sapiens
<900> 412
<210> 413
   <211> 35
   <212> PRT
   <213> Homo sapiens
<400> 413
   Ile Ser Gly Arg His Ser Ile Thr Val Thr Thr Val Ala Ser Ala Gly
    1               5                  10                  15
   Asn Ile Gly Glu Asp Gly Ile Leu Ser Cys Thr Phe Glu Pro Asp Ile
               20                  25                  30
   Lys Leu Ser
           35
<210> 414
   <211> 35
   <212> PRT
   <213> Homo sapiens
<400> 414
   Val Leu Gly Leu Val His Glu Phe Lys Glu Gly Lys Asp Glu Leu Ser
    1               5                  10                  15
   Glu Gln Asp Glu Met Phe Arg Gly Arg Thr Ala Val Phe Ala Asp Gln
               20                  25                  30
   Val Ile Val
           35
<210> 415
   <211> 65
   <212> PRT
   <213> Homo sapiens
<400> 415
   Lys Gly Lys Gly Asn Ala Asn Leu Glu Tyr Lys Thr Gly Ala Phe Ser
    1               5                  10                  15
   Met Pro Glu Val Asn Val Asp Tyr Asn Ala Ser Ser Glu Thr Leu Arg
               20                  25                  30
   Cys Glu Ala Pro Arg Trp Phe Pro Gln Pro Thr Val Val Trp Ala Ser
           35                  40                  45
   Gln Val Asp Gln Gly Ala Asn Phe Ser Glu Val Ser Asn Thr Ser Phe
       50                  55                  60
   Glu
           65
<210> 416
   <211> 10
   <212> PRT
   <213> Homo sapiens
<400> 416
   Lys Leu Ser Asp Ile Val Ile Gln Trp Leu
   1 5 10
<210> 417
   <211> 10
   <212> PRT
   <213> Homo sapiens
<400> 417
   Ser Leu Gly Gln Ile Leu Phe Trp Ser Ile
   1 5 10
<210> 418
   <211> 10
   <212> PRT
   <213> Homo sapiens
<400> 418
   Leu Leu Asn Ser Lys Ala Ser Leu Cys Val
   1 5 10
<210> 419
   <211> 10
   <212> PRT
   <213> Homo sapiens
<400> 419
   Ser Leu Cys Val Ser Ser Phe Phe Ala Ile
   1 5 10
<210> 420
   <211> 10
   <212> PRT
   <213> Homo sapiens
<400> 420
   Val Leu Tyr Asn Val Thr Ile Asn Asn Thr
   1 5 10
<210> 421
   <211> 10
   <212> PRT
   <213> Homo sapiens
<400> 421
   Ile Leu Phe Trp Ser Ile Ile Ser Ile Ile
   1 5 10

<210> 422
   <211> 10
   <212> PRT
   <213> Homo sapiens
<400> 422
   Leu Leu Pro Leu Ser Pro Tyr Leu Met Leu
   1               5                   10
<210> 423
   <211> 10
   <212> PRT
   <213> Homo sapiens
<400> 423
   Cys Met Ile Glu Asn Asp Ile Ala Lys Ala
   1               5                   10
<210> 424
   <211> 10
   <212> PRT
   <213> Homo sapiens
<400> 424
   Lys Thr Gly Ala Phe Ser Met Pro Glu Val
   1               5                   10
<210> 425
   <211> 10
   <212> PRT
   <213> Homo sapiens
<400> 425
   Trp Ala Leu Leu Pro Leu Ser Pro Tyr Leu
   1               5                   10
<210> 426
   <211> 10
   <212> PRT
   <213> Homo sapiens
<400> 426
   Ile Ile Leu Ala Gly Ala Ile Ala Leu Ile
   1               5                   10
<210> 427
   <211> 10
   <212> PRT
   <213> Homo sapiens
<400> 427
   Gln Leu Thr Asp Ala Gly Thr Tyr Lys Cys
   1 5 10
<210> 428
   <211> 10
   <212> PRT
   <213> Homo sapiens
<400> 428
   Ala Leu Leu Pro Leu Ser Pro Tyr Leu Met
   1 5 10
<210> 429
   <211> 10
   <212> PRT
   <213> Homo sapiens
<400> 429
   Gln Leu Leu Asn Ser Lys Ala Ser Leu Cys
   1 5 10
<210> 430
   <211> 10
   <212> PRT
   <213> Homo sapiens
<400> 430
   Ile Leu Ser Cys Thr Phe Glu Pro Asp Ile
   1 5 10
<210> 431
   <211> 10
   <212> PRT
   <213> Homo sapiens
<400> 431
   Trp Leu Lys Glu Gly Val Leu Gly Leu Val
   1 5 10
<210> 432
   <211> 10
   <212> PRT
   <213> Homo sapiens
<400> 432
   Leu Gln Leu Leu Asn Ser Lys Ala Ser Leu
   1 5 10
<210> 433
   <211> 10
   <212> PRT
   <213> Homo sapiens
<400> 433
   Gln Ile Leu Phe Trp Ser Ile Ile Ser Ile
   1 5 10
<210> 434
   <211> 10
   <212> PRT
   <213> Homo sapiens
<400> 434
   Gly Ile Ser Gly Arg His Ser Ile Thr Val
   1 5 10
<210> 435
   <211> 10
   <212> PRT
   <213> Homo sapiens
<400> 435
   Phe Glu Pro Asp Ile Lys Leu Ser Asp Ile
   1 5 10
<210> 436
   <211> 9
   <212> PRT
   <213> Homo sapiens
<400> 436
   Ala Leu Leu Pro Leu Ser Pro Tyr Leu
   1 5
<210> 437
   <211> 9
   <212> PRT
   <213> Homo sapiens
<400> 437
   Ser Leu Cys Val Ser Ser Phe Phe Ala
   1 5
<210> 438
   <211> 9
   <212> PRT
   <213> Homo sapiens
<400> 438
   Ile Leu Phe Trp Ser Ile Ile Ser Ile
   1 5
<210> 439
   <211> 9
   <212> PRT
   <213> Homo sapiens
<400> 439
   Gln Leu Leu Asn Ser Lys Ala Ser Leu
   1 5
<210> 440
   <211> 9
   <212> PRT
   <213> Homo sapiens
<400> 440
   Lys Val Val Ser Val Leu Tyr Asn Val
   1 5
<210> 441
   <211> 9
   <212> PRT
   <213> Homo sapiens
<400> 441
   Ile Leu Ala Gly Ala Ile Ala Leu Ile
   1 5
<210> 442
   <211> 9
   <212> PRT
   <213> Homo sapiens
<400> 442
   Trp Leu Lys Glu Gly Val Leu Gly Leu
   1 5
<210> 443
   <211> 9
   <212> PRT
   <213> Homo sapiens
<400> 443
   Ile Ile Leu Ala Gly Ala Ile Ala Leu
   1 5
<210> 444
   <211> 9
   <212> PRT
   <213> Homo sapiens
<400> 444
   Asn Val Thr Met Lys Val Val Ser Val
   1 5

<210> 445
   <211> 9
   <212> PRT
   <213> Homo sapiens
<400> 445
   Glu Met Phe Arg Gly Arg Thr Ala Val
   1 5
<210> 446
   <211> 9
   <212> PRT
   <213> Homo sapiens
<400> 446
   Ala Val Phe Ala Asp Gln Val Ile Val
   1 5
<210> 447
   <211> 9
   <212> PRT
   <213> Homo sapiens
<400> 447
   Leu Leu Pro Leu Ser Pro Tyr Leu Met
   1 5
<210> 448
   <211> 9
   <212> PRT
   <213> Homo sapiens
<400> 448
   Leu Leu Asn Ser Lys Ala Ser Leu Cys
   1 5
<210> 449
   <211> 9
   <212> PRT
   <213> Homo sapiens
<400> 449
   Val Ile Gln Trp Leu Lys Glu Gly Val
   1 5
<210> 450
   <211> 9
   <212> PRT
   <213> Homo sapiens
<400> 450
   Ala Ile Ser Trp Ala Leu Leu Pro Leu
   1 5
<210> 451
   <211> 9
   <212> PRT
   <213> Homo sapiens
<400> 451
   Ser Leu Gly Gln Ile Leu Phe Trp Ser
   1 5
<210> 452
   <211> 9
   <212> PRT
   <213> Homo sapiens
<400> 452
   Ile Ala Leu Ile Ile Gly Phe Gly Ile
   1 5
<210> 453
   <211> 9
   <212> PRT
   <213> Homo sapiens
<400> 453
   Cys Thr Phe Glu Pro Asp Ile Lys Leu
   1 5
<210> 454
   <211> 9
   <212> PRT
   <213> Homo sapiens
<400> 454
   Ile Val Gly Asn Ala Ser Leu Arg Leu
   1 5
<210> 455
   <211> 9
   <212> PRT
   <213> Homo sapiens
<400> 455
   Gly Gln Ile Leu Phe Trp Ser Ile Ile
   1 5

## Description

### Technical Field

The present invention relates generally to ovarian cancer therapy. The invention is more specifically related to polypeptides comprising a portion of an ovarian carcinoma protein, and to polynucleotides encoding such polypeptides, as well as antibodies that specifically recognize such polypeptides. Such polypeptides, polynucleotides and antibodies may be used in vaccines and pharmaceutical compositions for treatment of ovarian cancer.

### Background of the Invention

Ovarian cancer is a significant health problem for women in the United States and throughout the world. Although advances have been made in detection and therapy of this cancer, no vaccine or other universally successful method for prevention or treatment is currently available. Management of the disease currently relies on a combination of early diagnosis and aggressive treatment, which may include one or more of a variety of treatments such as surgery, radiotherapy, chemotherapy and hormone therapy. The course of treatment for a particular cancer is often selected based on a variety of prognostic parameters, including an analysis of specific tumor markers. However, the use of established markers often leads to a result that is difficult to interpret, and high mortality continues to be observed in many cancer patients.

Immunotherapies have the potential to substantially improve cancer treatment and survival. Such therapies may involve the generation or enhancement of an immune response to an ovarian carcinoma antigen. However, to date, relatively few ovarian carcinoma antigens are known and the generation of an immune response against such antigens has not been shown to be therapeutically beneficial. Patent applications WO 99/63083, WO 00/12758 and WO 00/36107 disclose the full length sequence of ovarian carcinoma antigen 08E.

Accordingly, there is a need in the art for improved methods for identifying ovarian tumor antigens and for using such antigens in the therapy of ovarian cancer. The present invention fulfills these needs and further provides other related advantages.

In accordance with the present invention there is provided an isolated antibody epitope of the ovarian carcinoma antigen O8E,said epitope consisting of the amino acid sequence set forth in SEQ ID NO: 398, or a variant thereof which has at least 90% identity along its whole length to the amino acid sequence set forth in SEQ ID No : 398.

In the second aspect there is provided an isolated antibody or antigen binding fragment thereof that specifically binds the antibody epitope of the present invention.

The present invention also provides the use of the epitope of the invention and antibody of the invention in therapy, including the use in to prevent the development of ovarian cancer and for the treatment of ovarian cancer.

The present invention also provides a polynucleotide which encodes the epitope of the present invention, together with its use in therapy and its use to prevent the development of ovarian cancer and the treatment of ovarian cancer.

There is also provided a pharmaceutical composition and vaccine including the epitope of the present invention, the antibody of the present invention or the polynucleotide of the present invention.

Briefly stated, this invention provides compositions and their use for the therapy of cancer, such as ovarian cancer. In one aspect, the present invention provides polypeptides comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished, as defined in the claims.

The present invention further provides polypeptide compositions consisting of an amino acid sequence selected from the group consisting of sequences recited in SEQ ID No: 398, or sequences at least 90% identical there to.

Within other aspects, the present invention provides pharmaceutical compositions and vaccines.

In other aspects, fusion proteins that comprise at least one polypeptide as described above, as well as polynucleotides encoding such fusion proteins are also disclosed.

Within related aspects, pharmaceutical compositions comprising a fusion protein or polynucleotide encoding a fusion protein in combination with a physiologically acceptable carrier are disclosed.

Vaccines are disclosed, within other aspects, comprising a fusion protein or polynucleotide encoding a fusion protein in combination with a non-specific immune response enhancer.

Within further aspects, the present disclosure provides a pharmaceutical composition or vaccine as recited above, for inhibiting the development of cancer in a patient.

The present invention discloses an antibody epitope recognized by the O8E polyclonal anti-sera which epitope is presented herein as SEQ ID NO: 398.

Further disclosed by the present disclosure 10-mer and 9-mer peptides predicted to bind HLA-0201 which peptides are disclosed herein as SEQ ID NO:416-435 and SEQ ID NO:436-455, respectively.

These and other aspects of the present invention will become apparent upon reference to the following detailed description and attached drawings.

According to another aspect, O8E polypeptides are described comprising at least an antibody epitope sequence set forth in SEQ ID NO: 398.

### BRIEF DESCRIPTION OF THE SEQUENCE IDENTIFIERS AND DRAWINGS

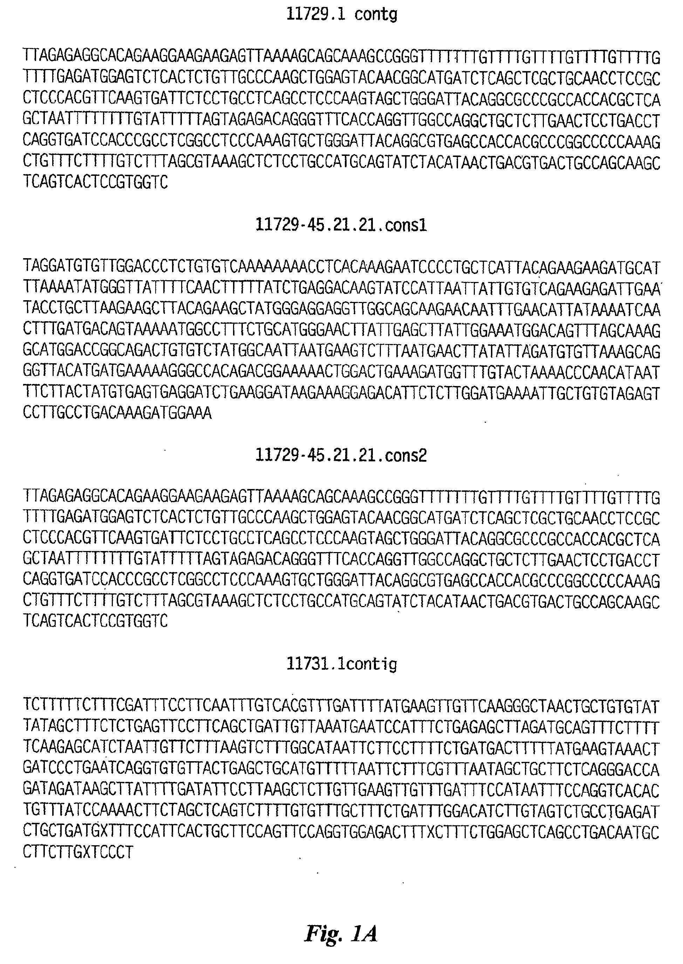

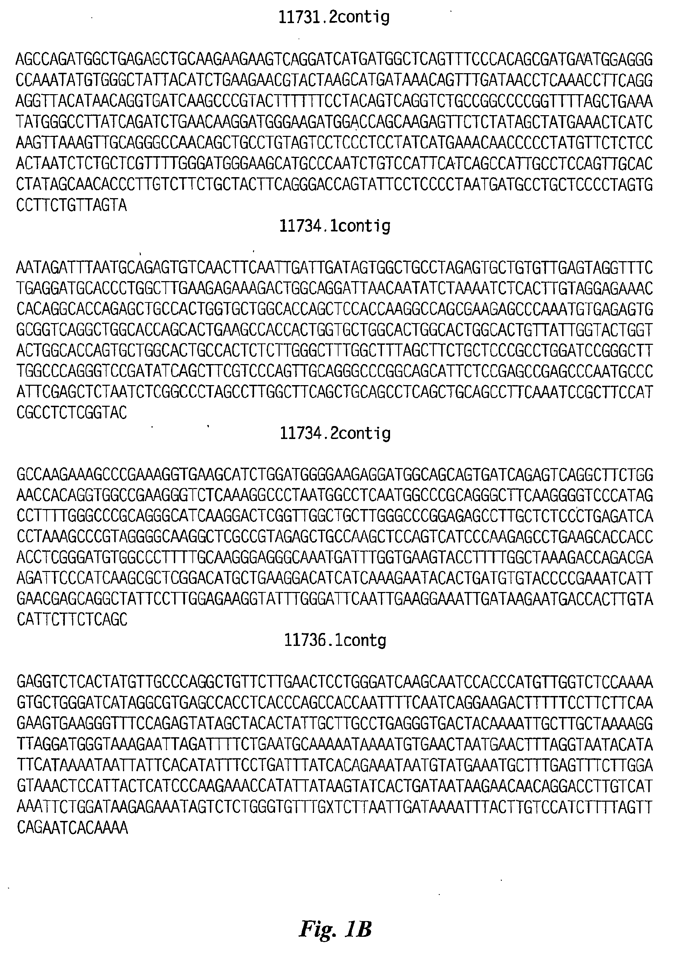

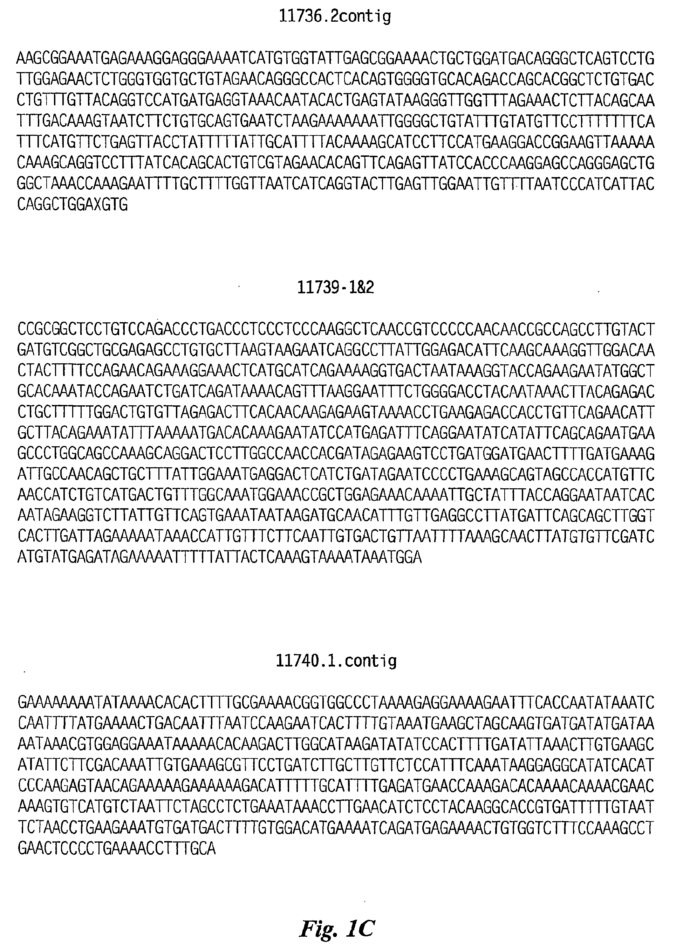

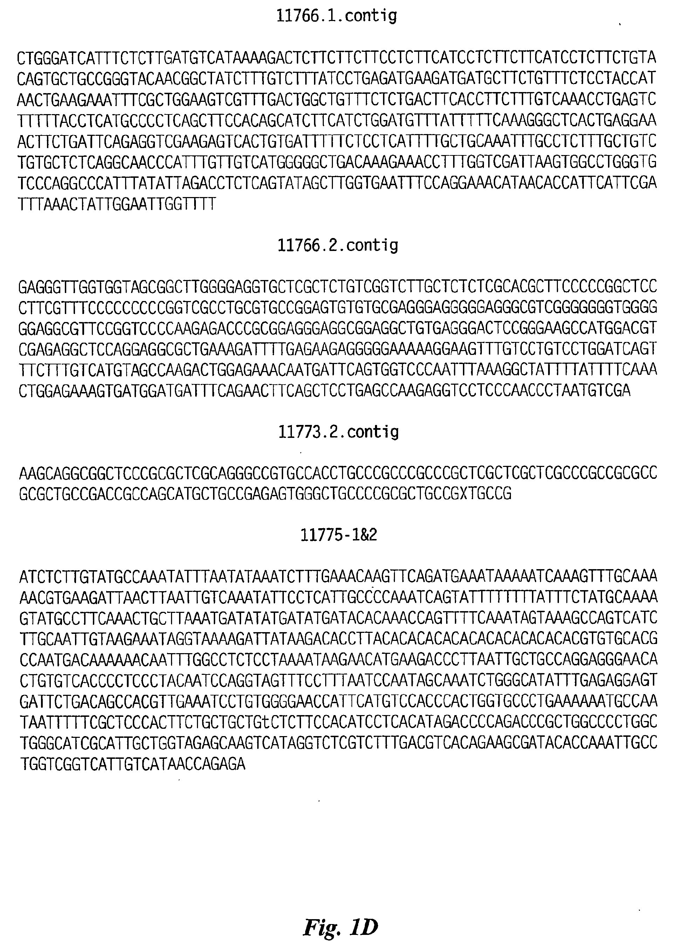

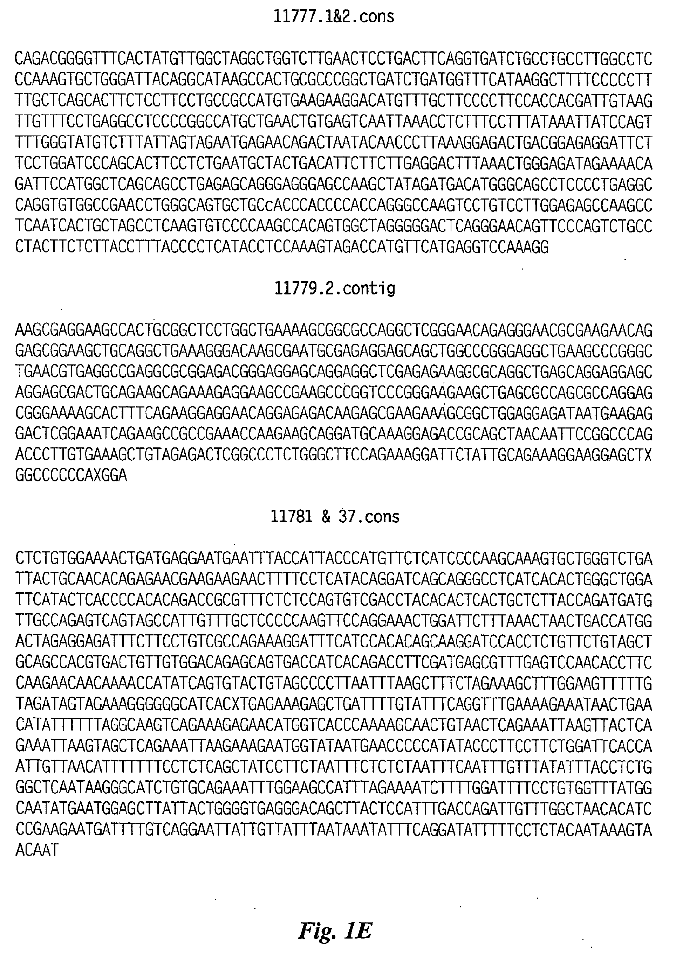

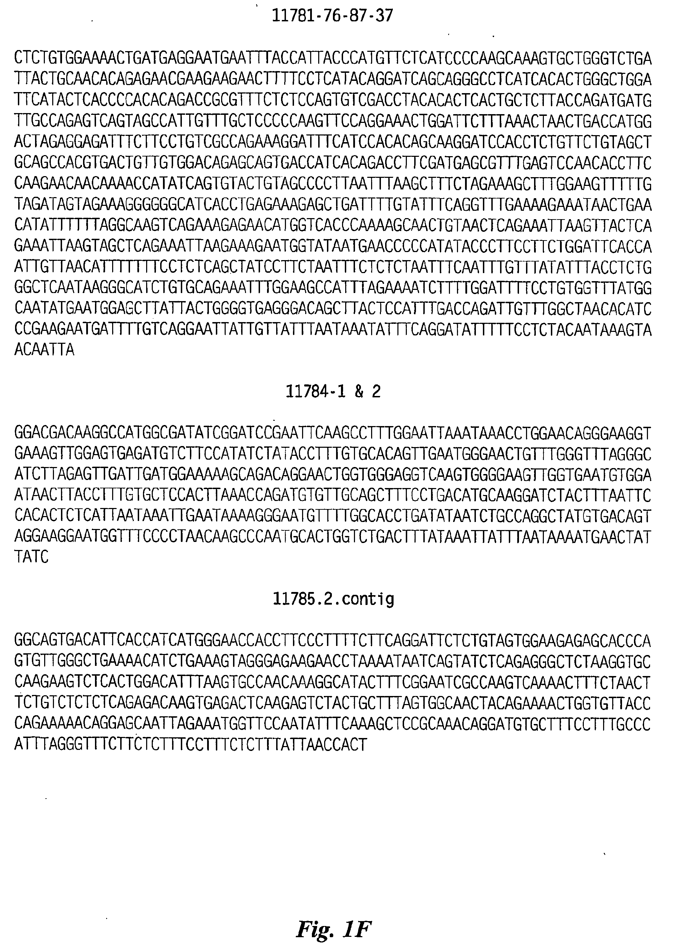

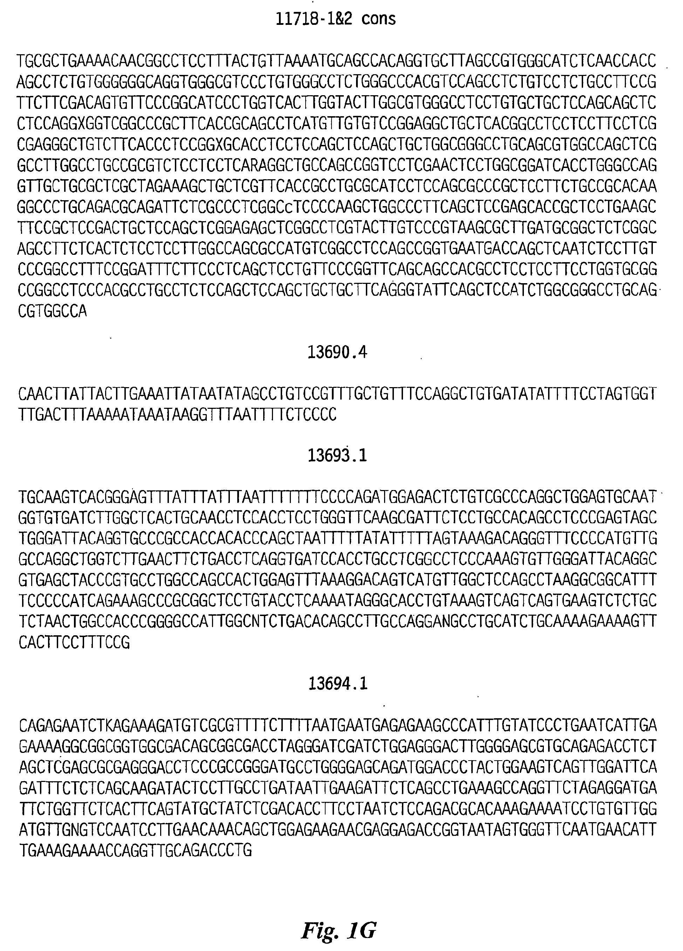

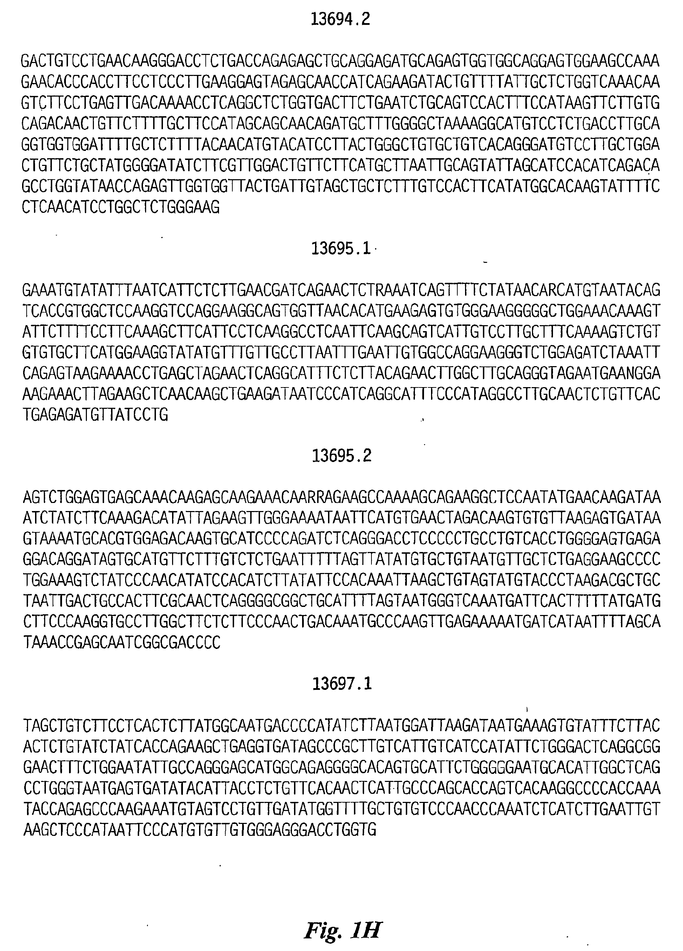

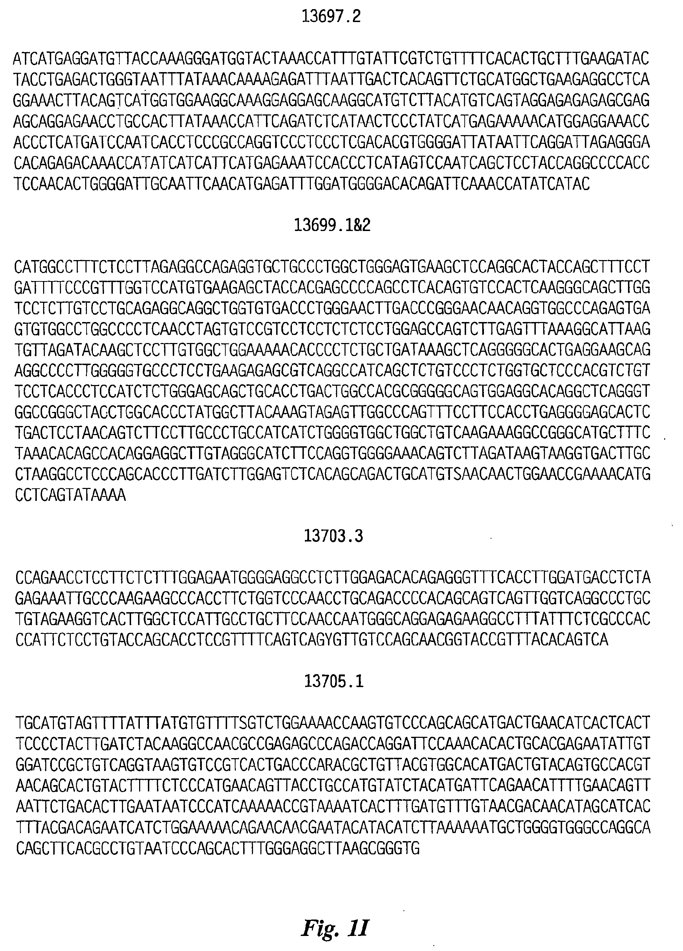

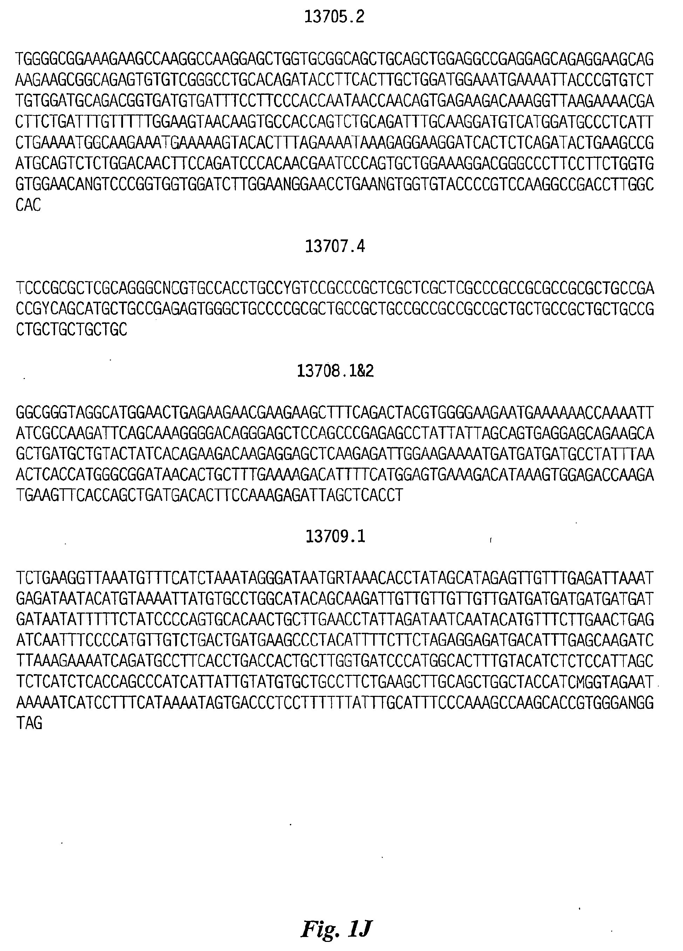

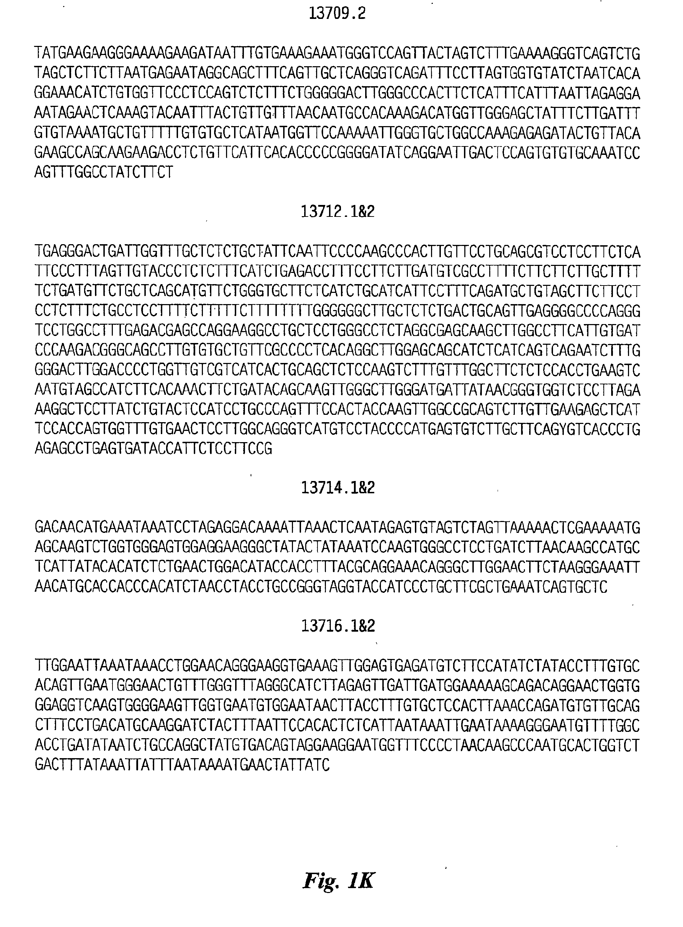

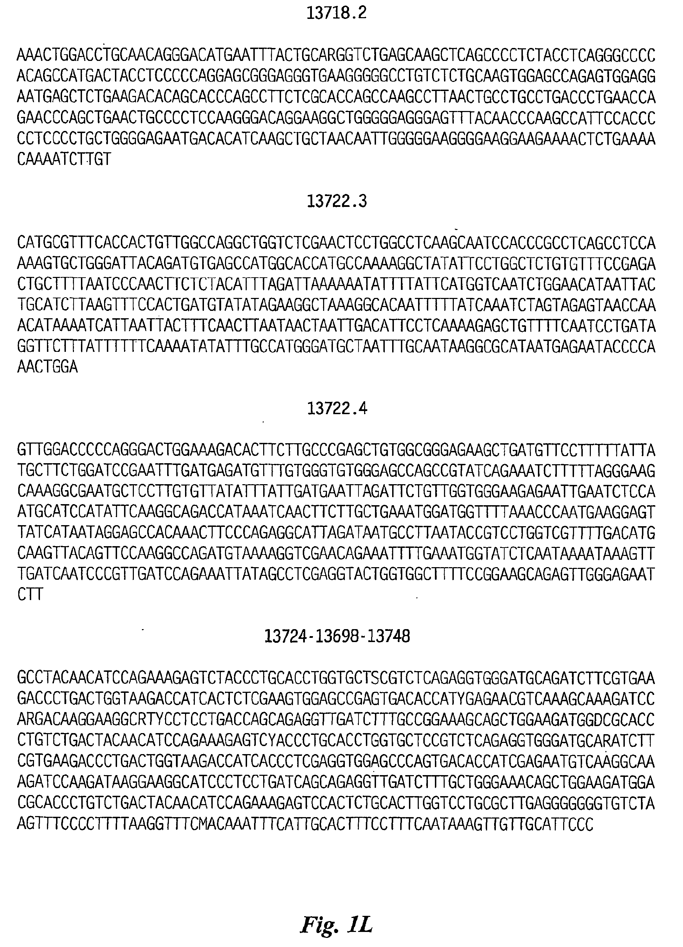

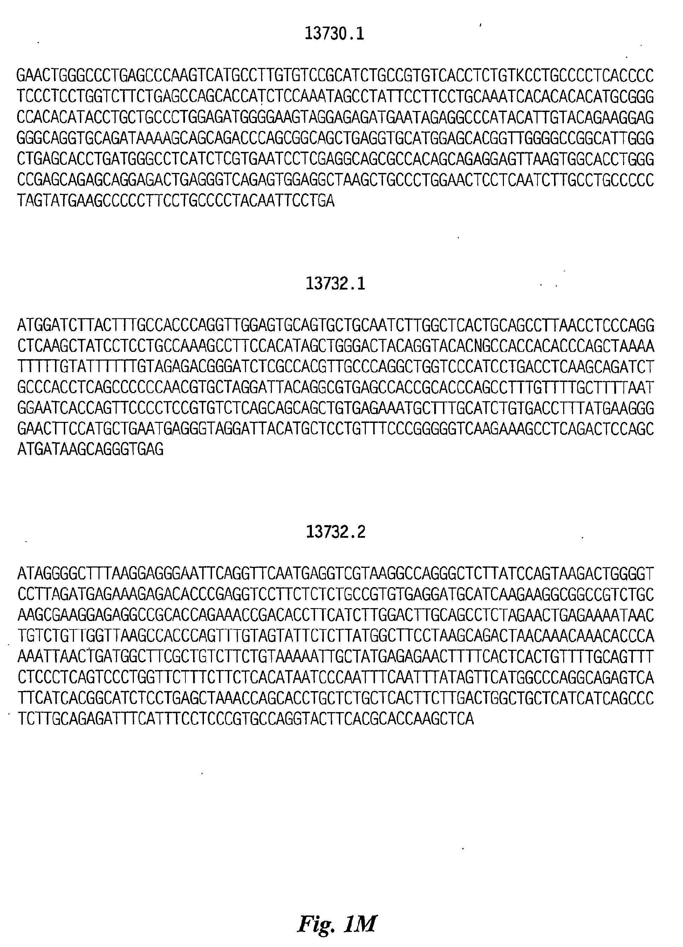

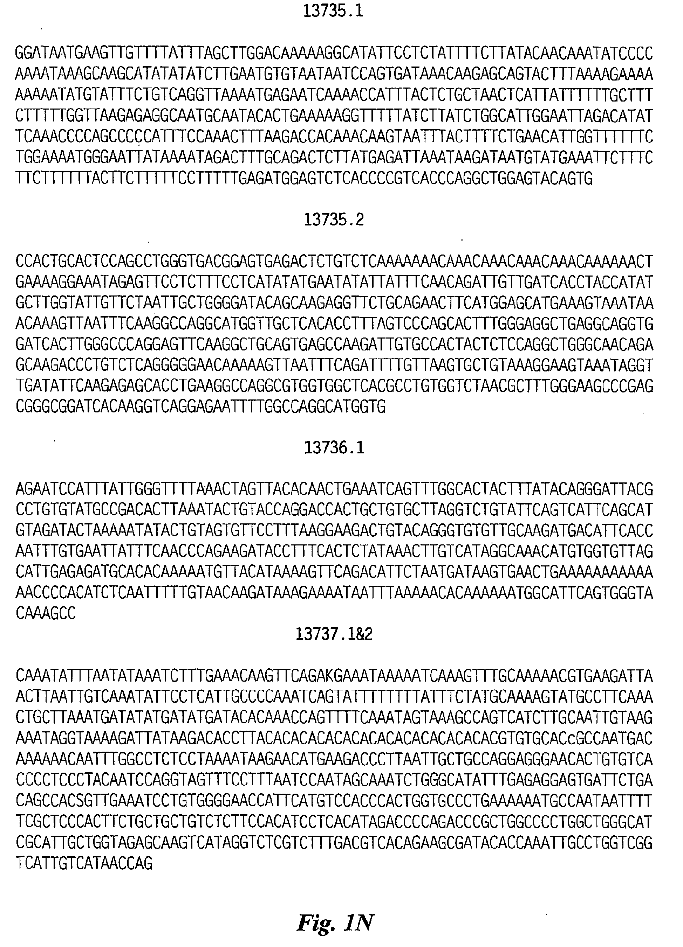

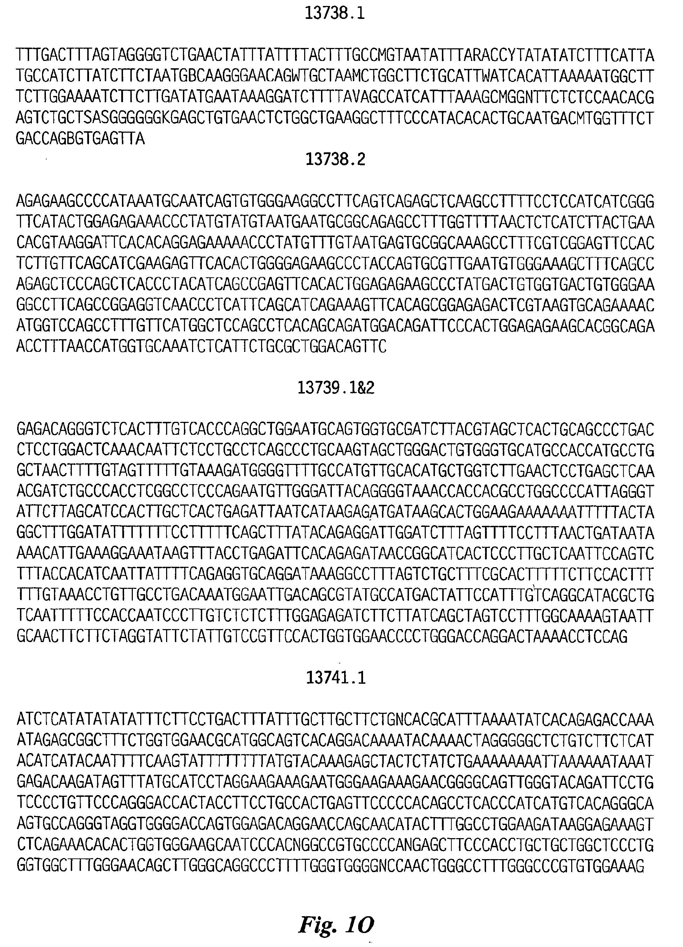

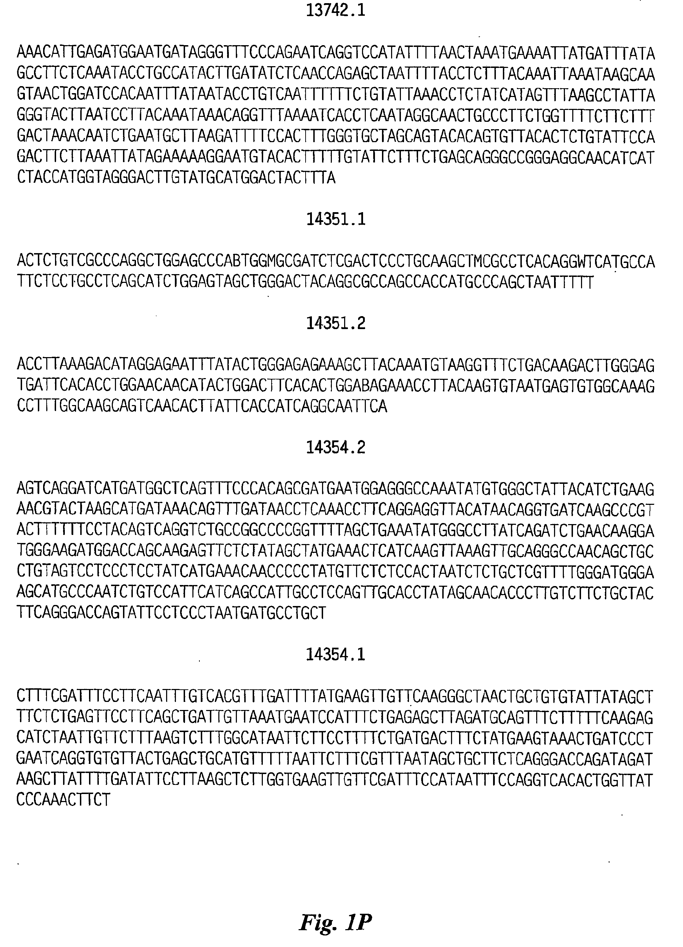

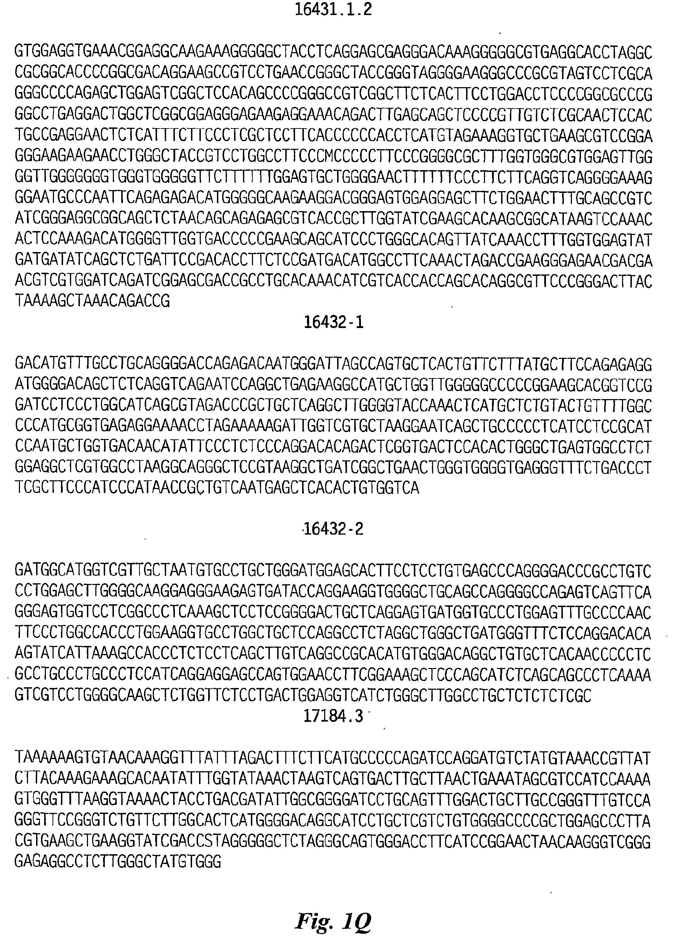

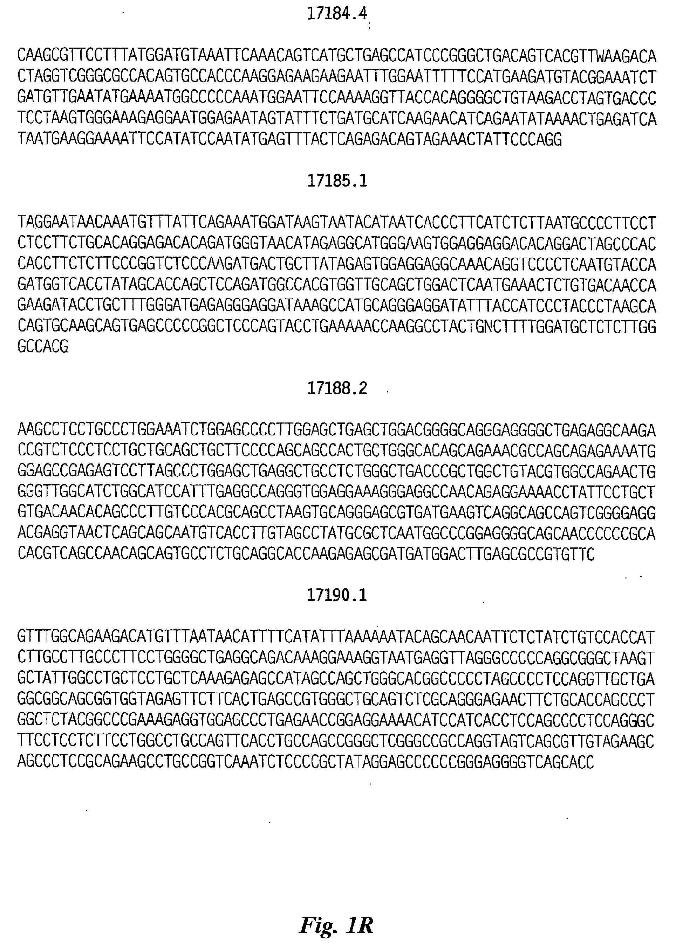

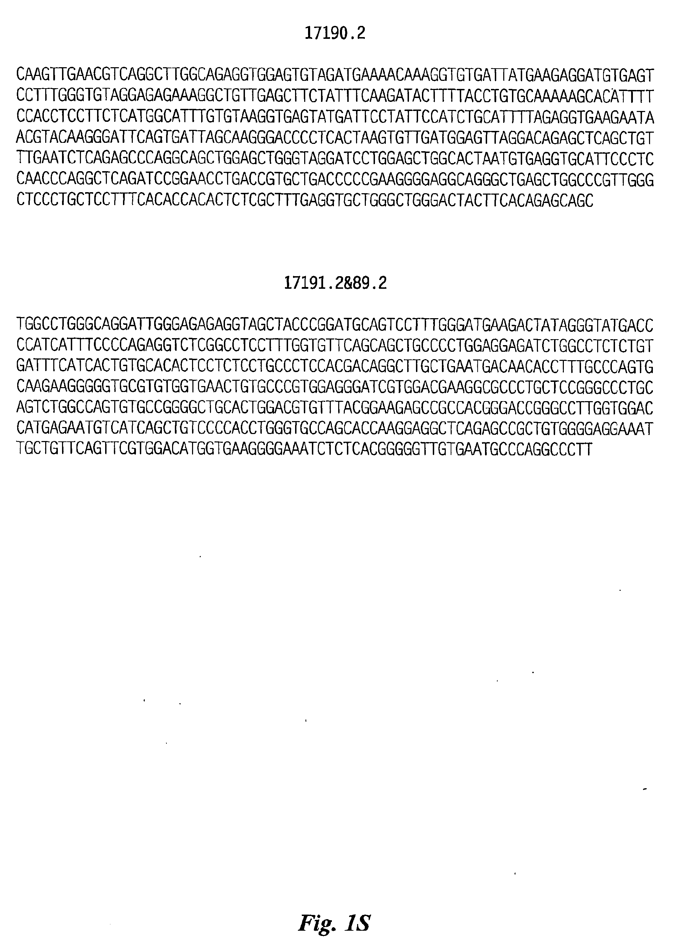

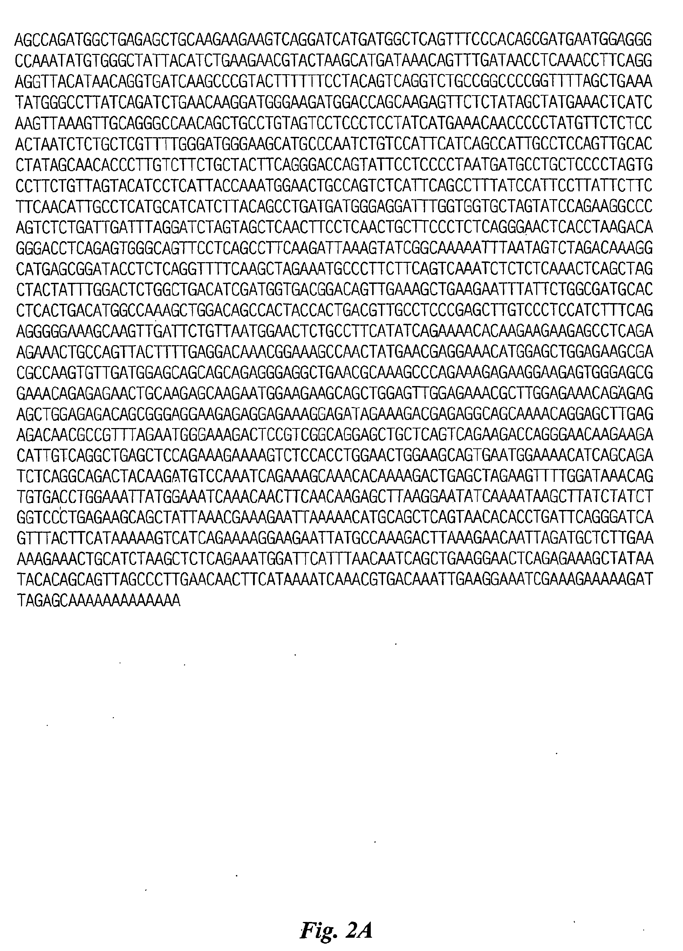

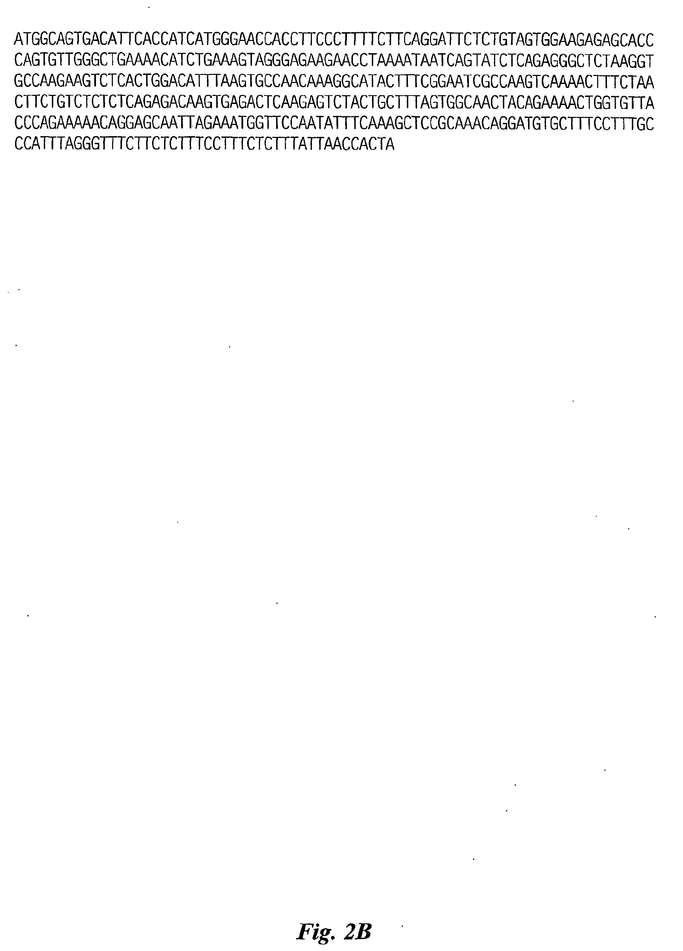

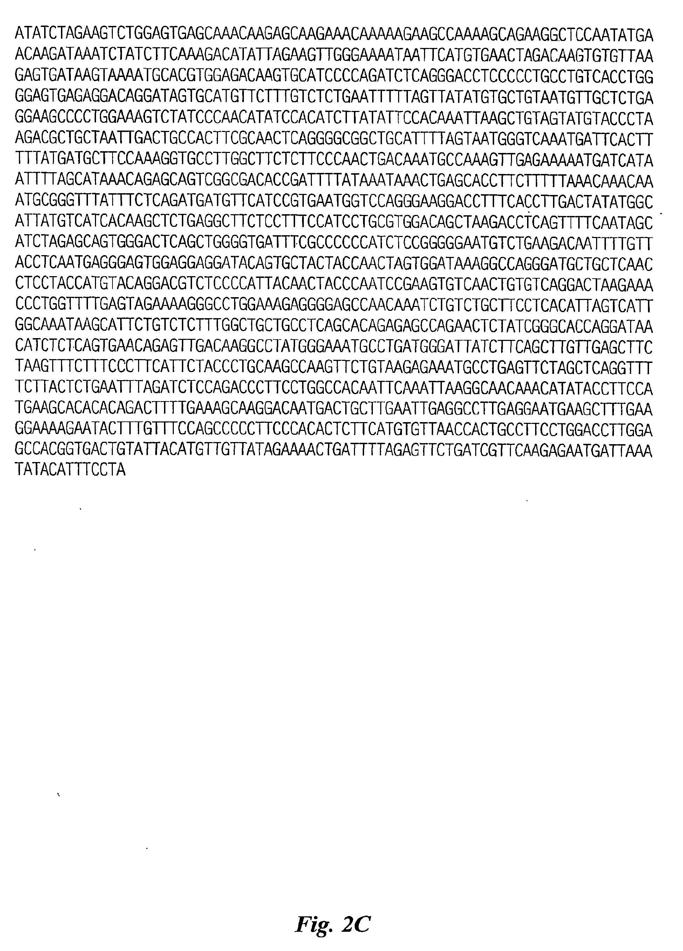

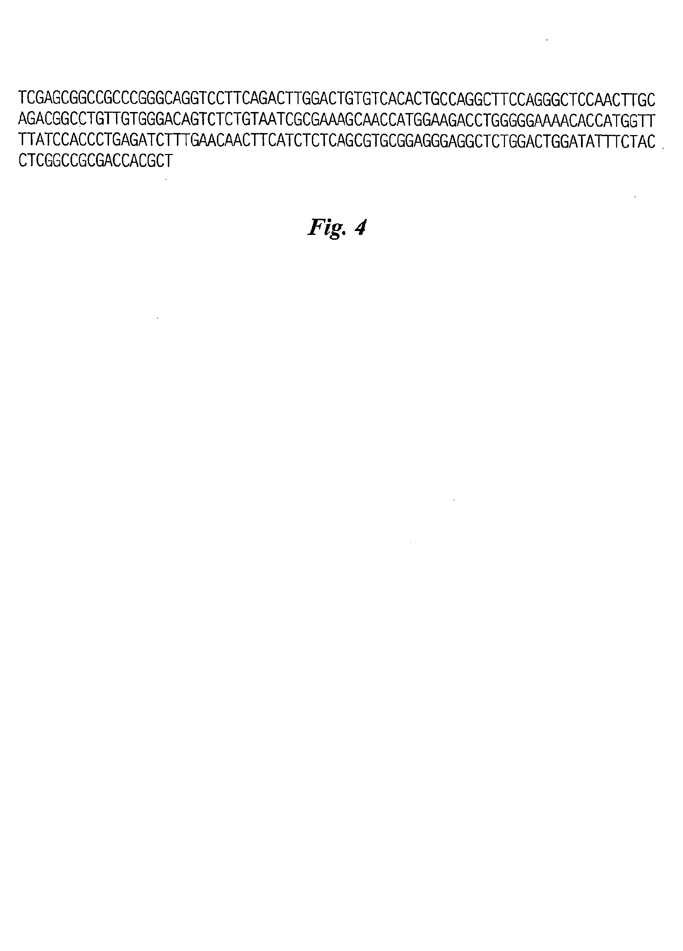

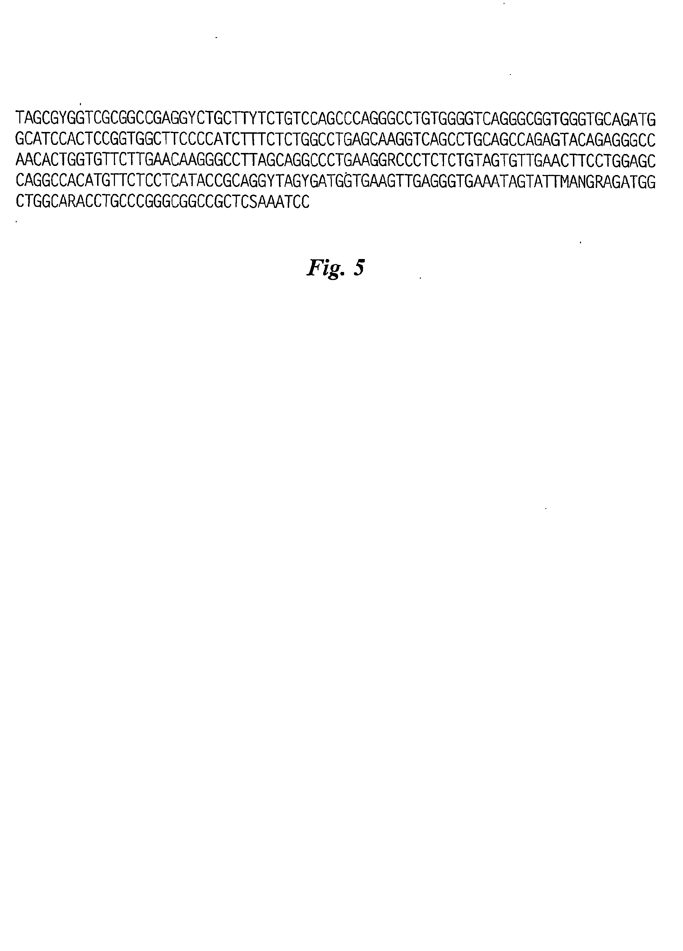

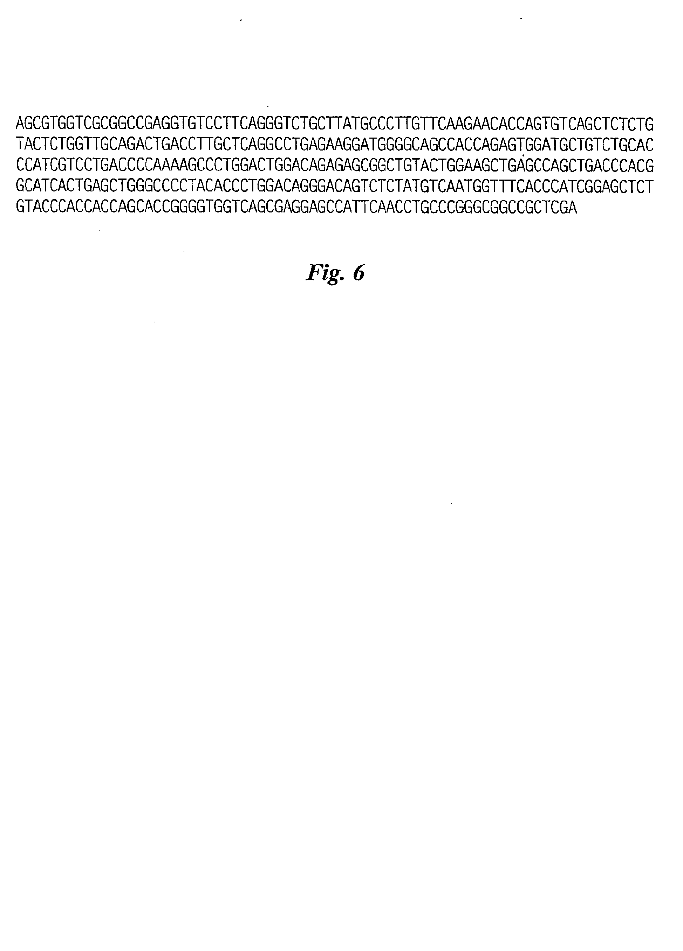

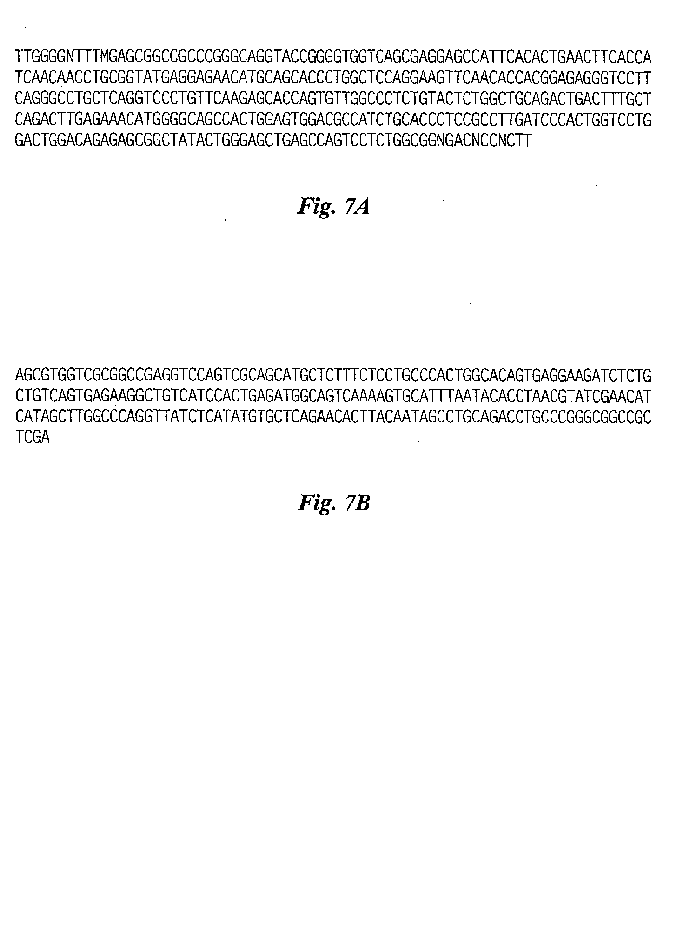

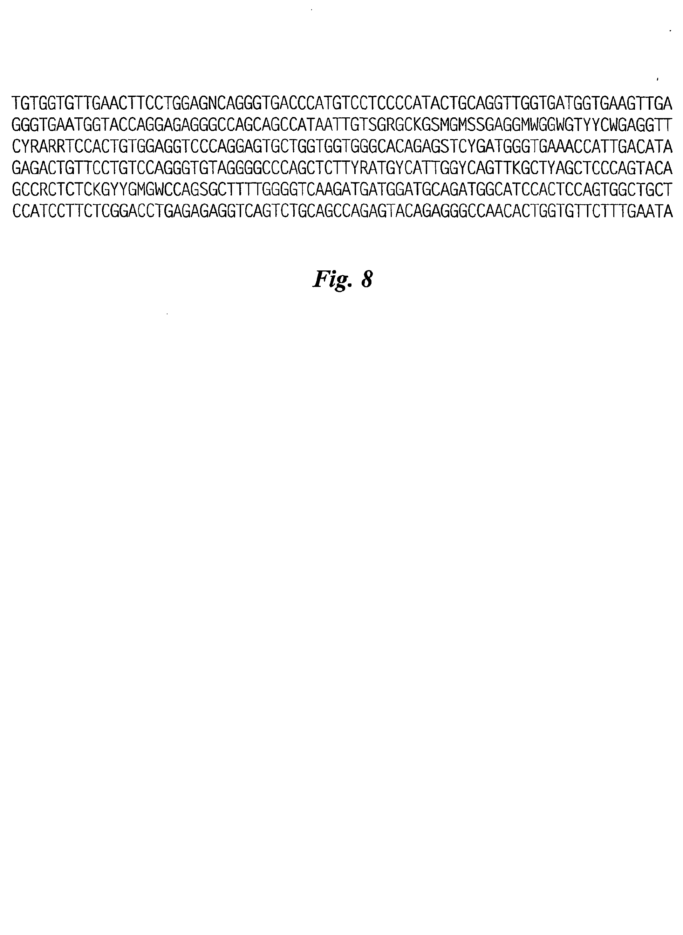

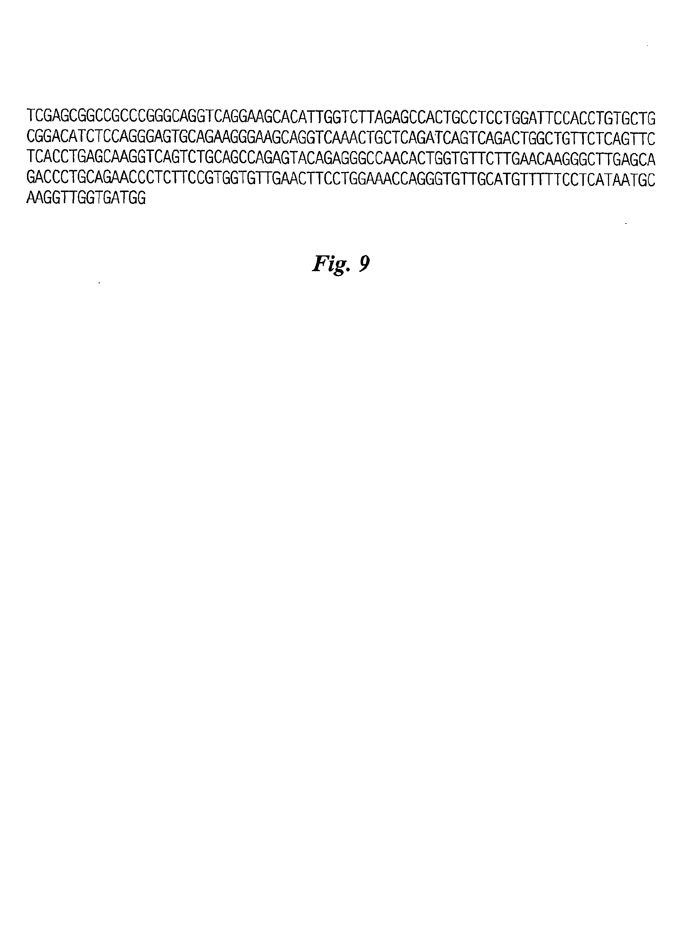

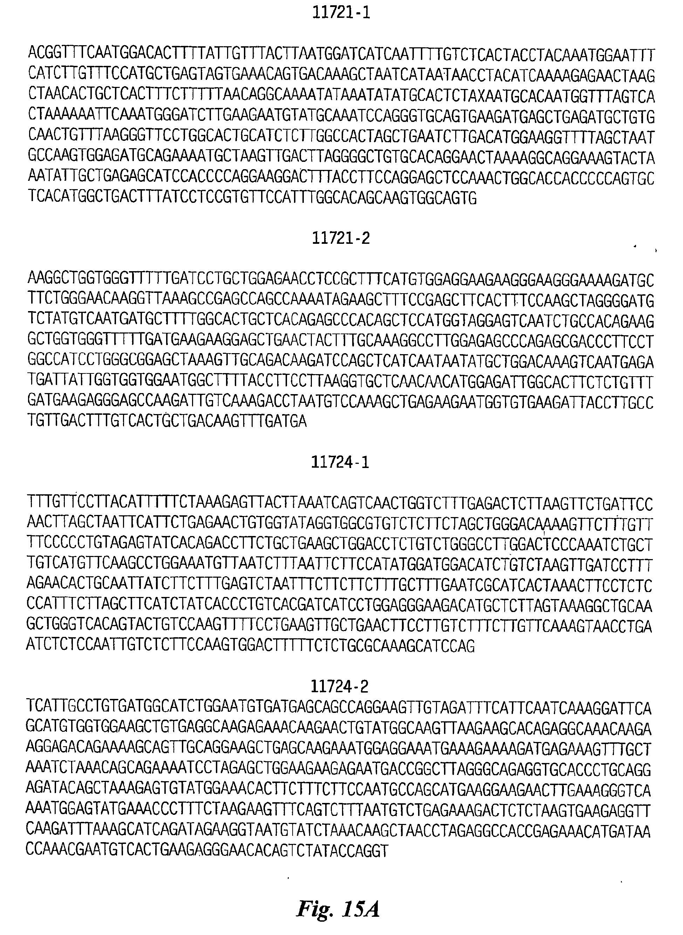

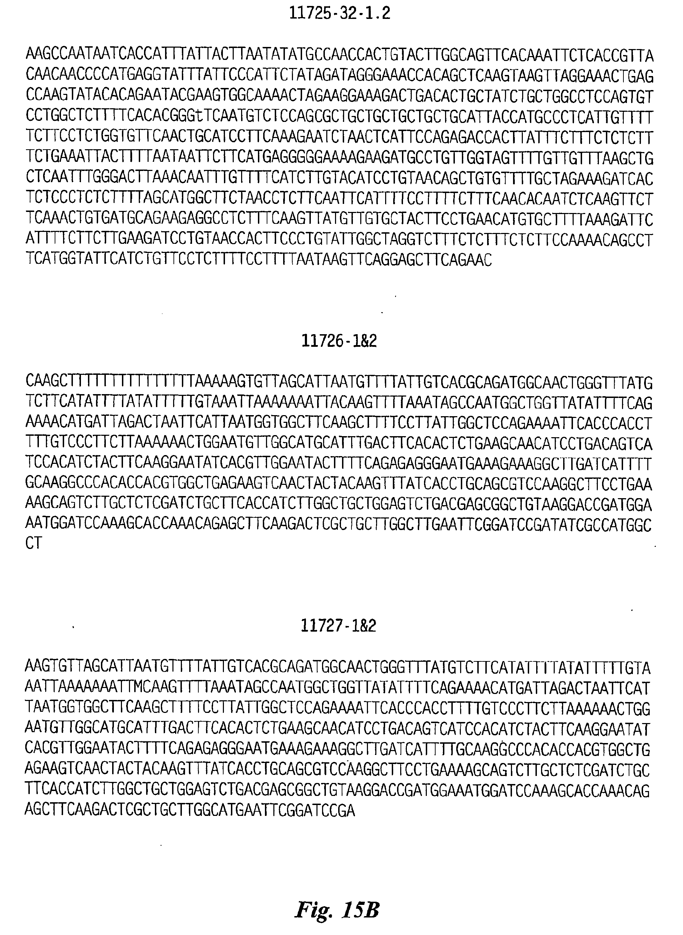

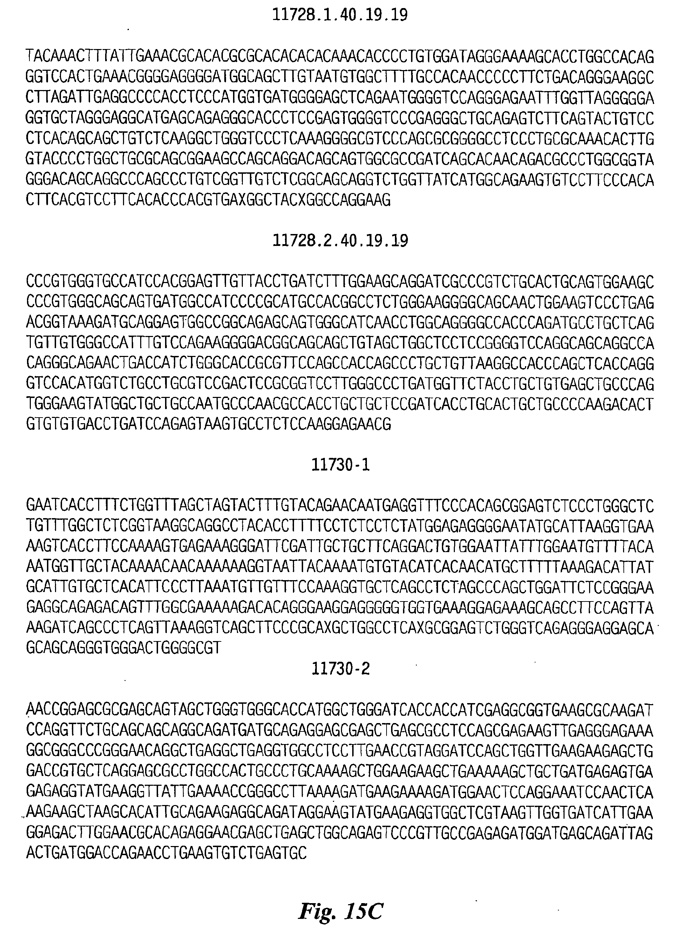

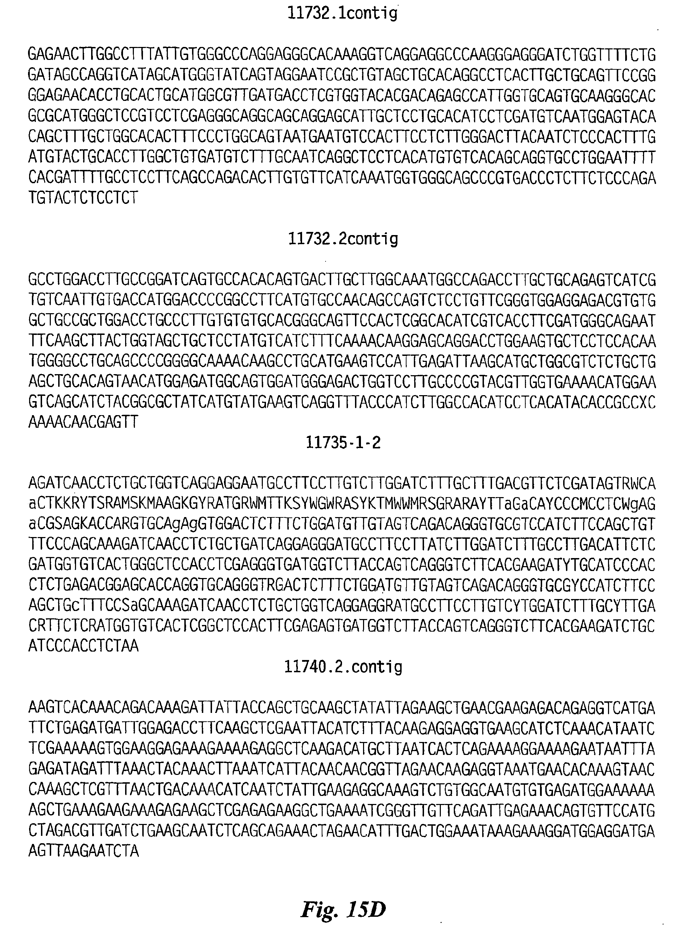

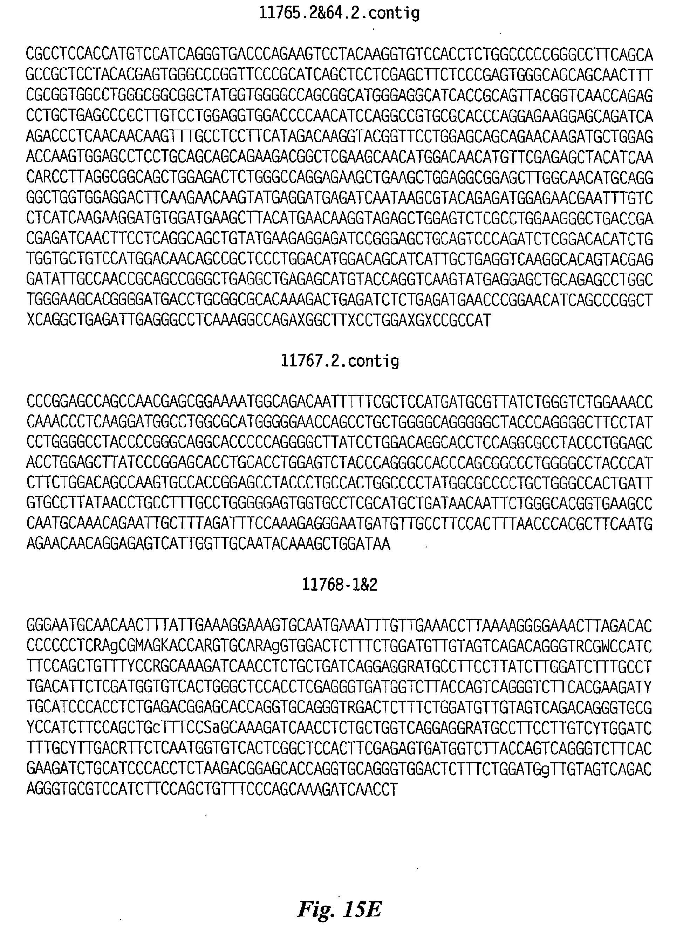

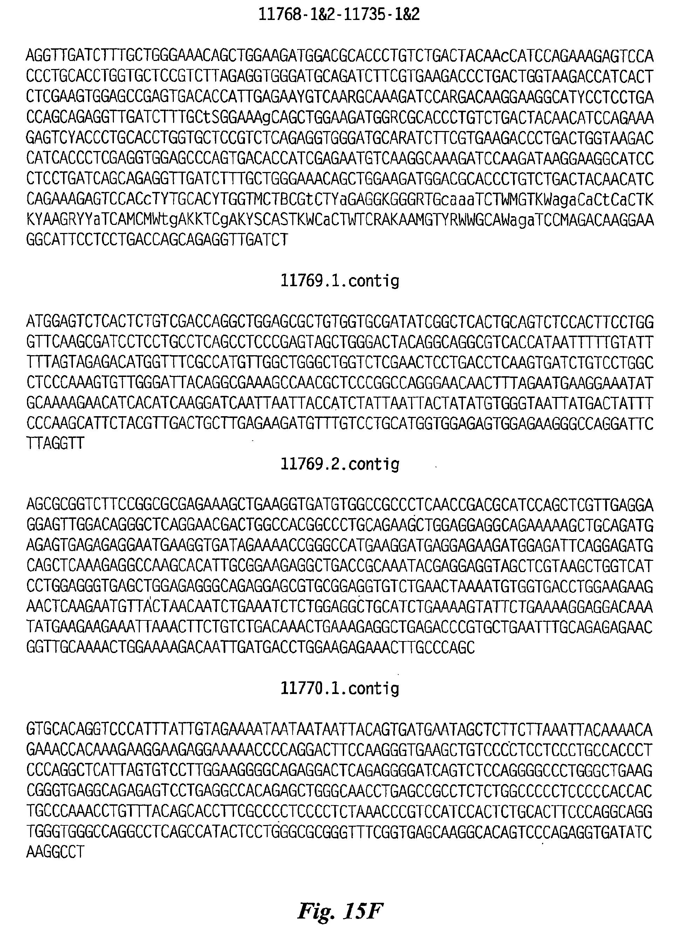

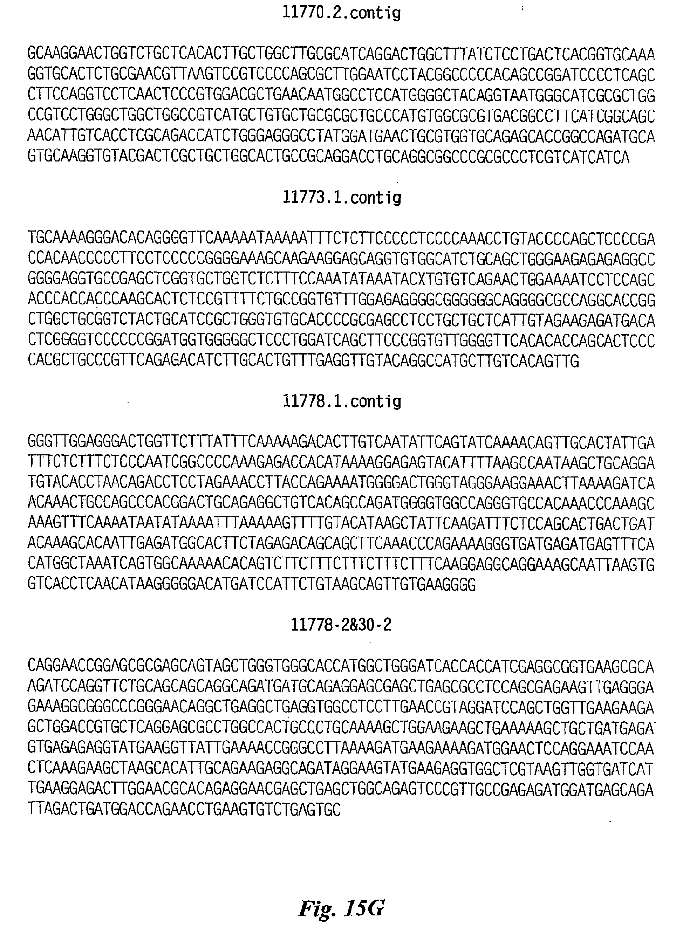

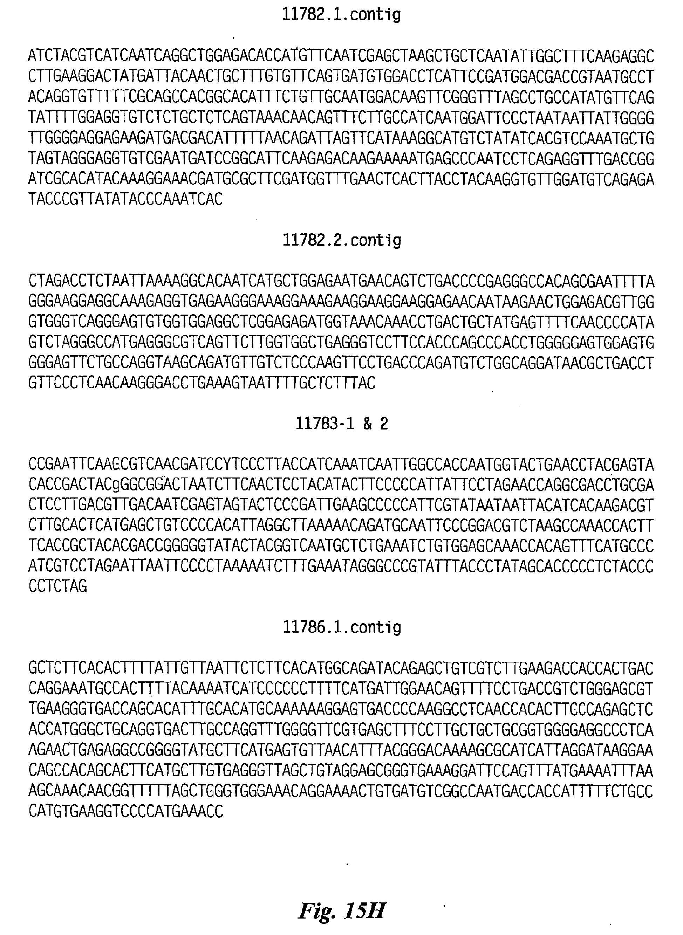

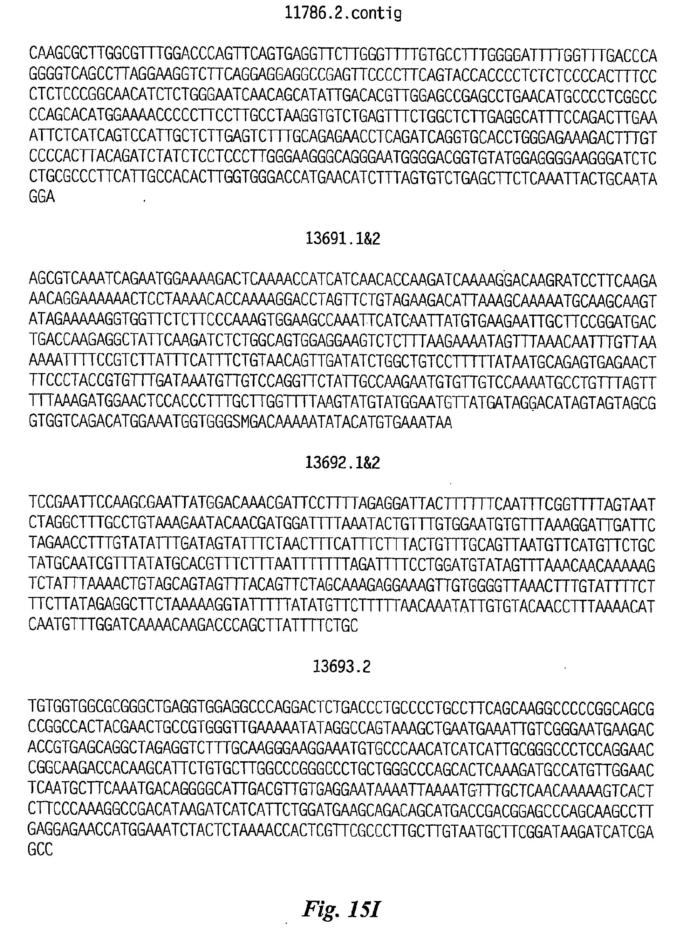

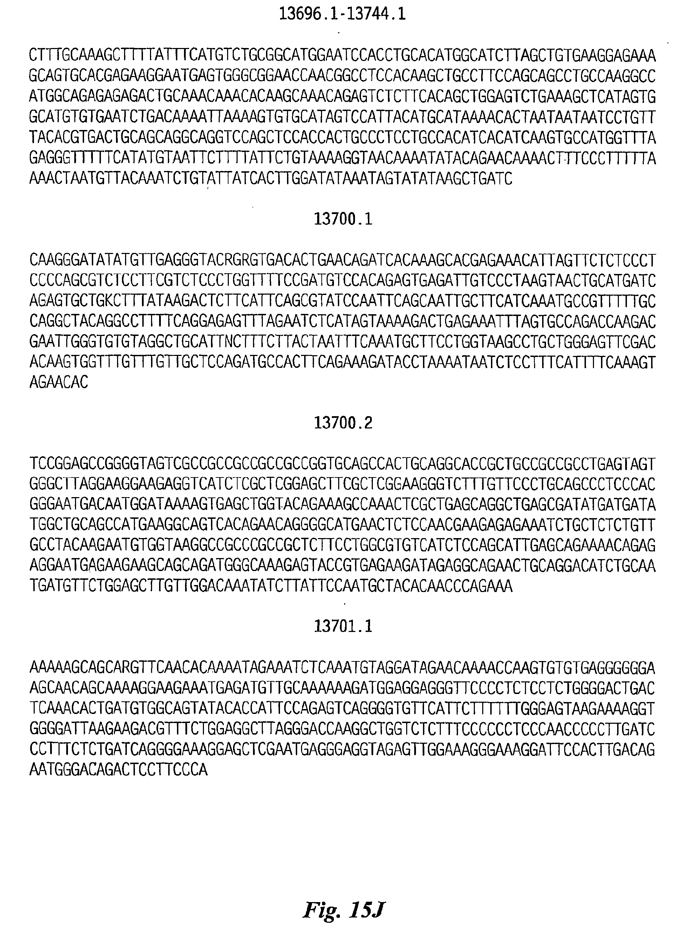

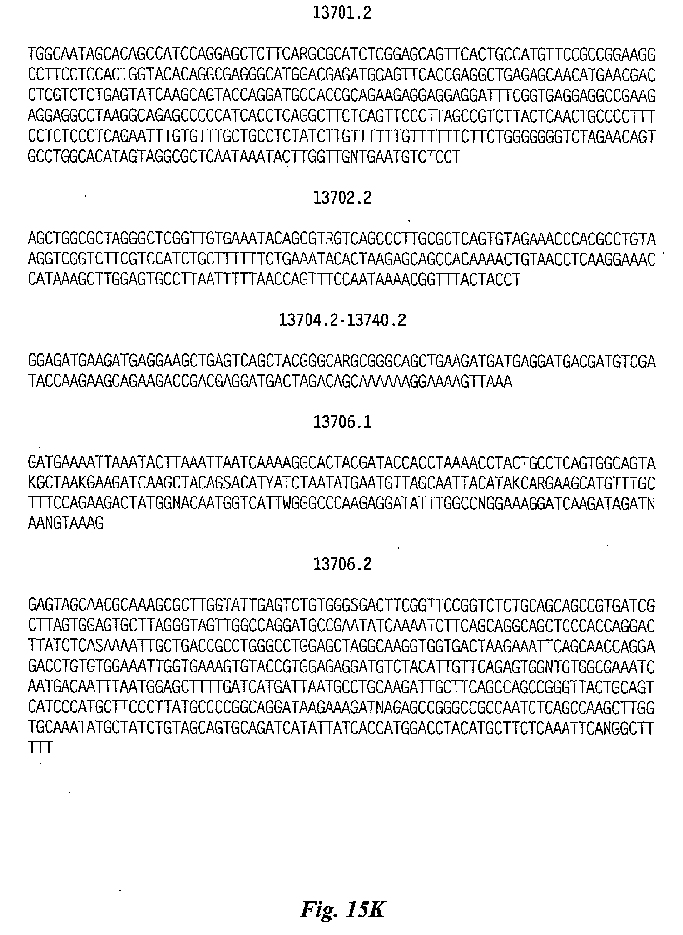

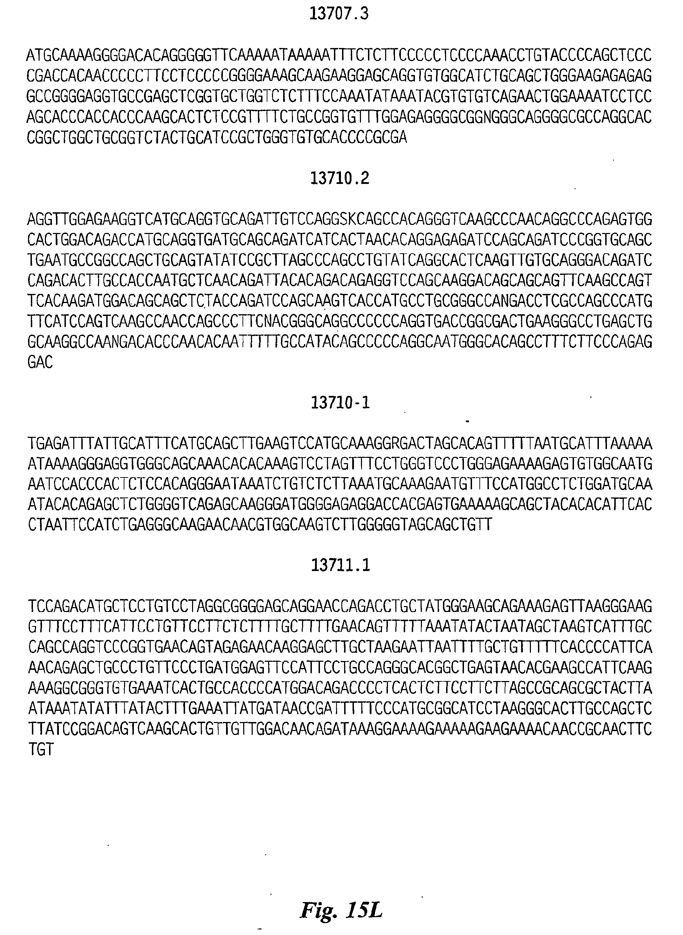

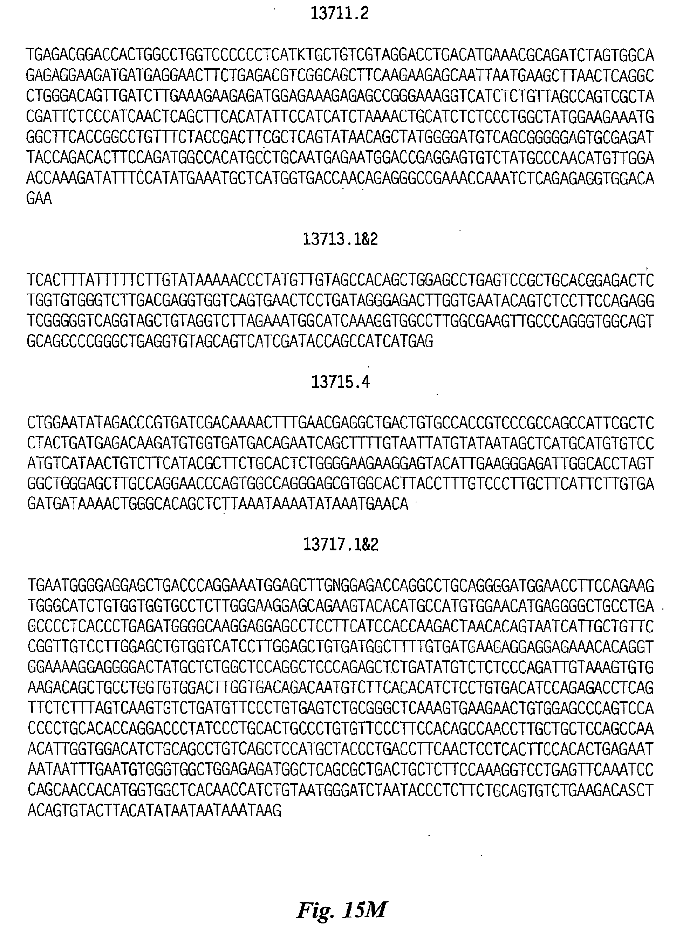

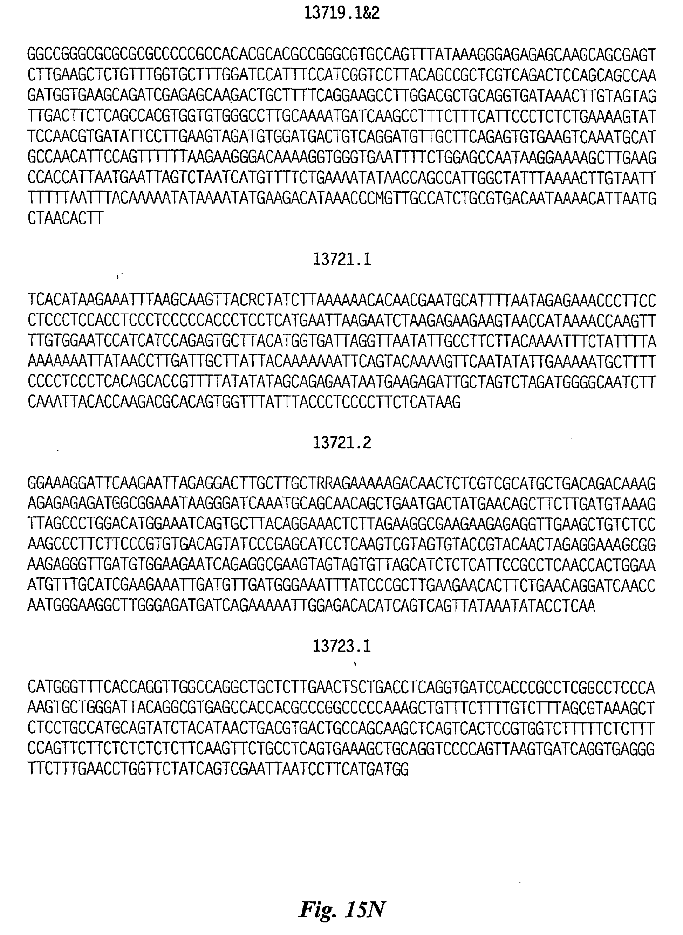

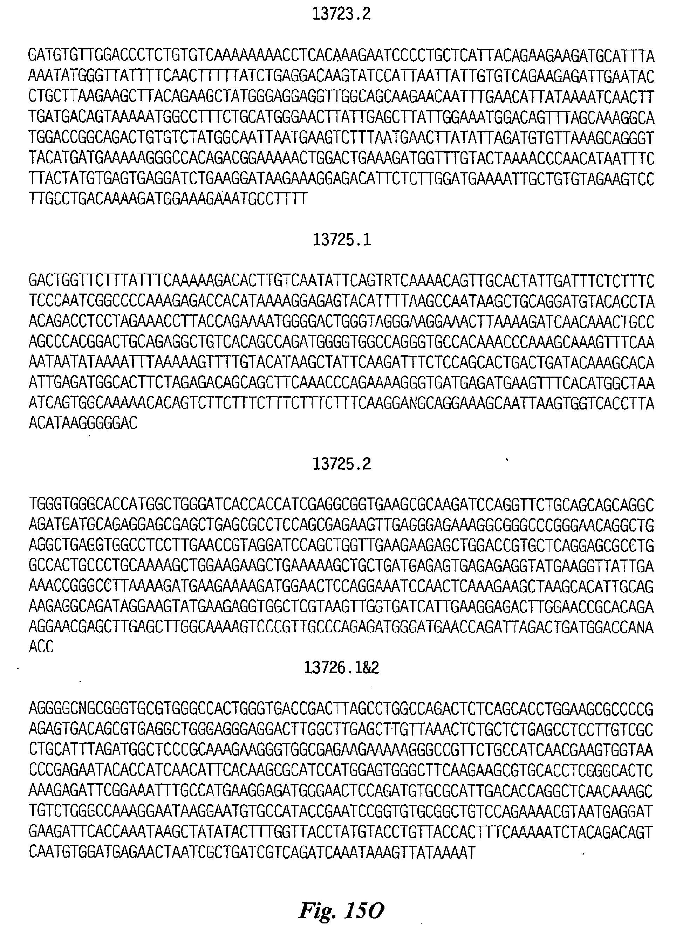

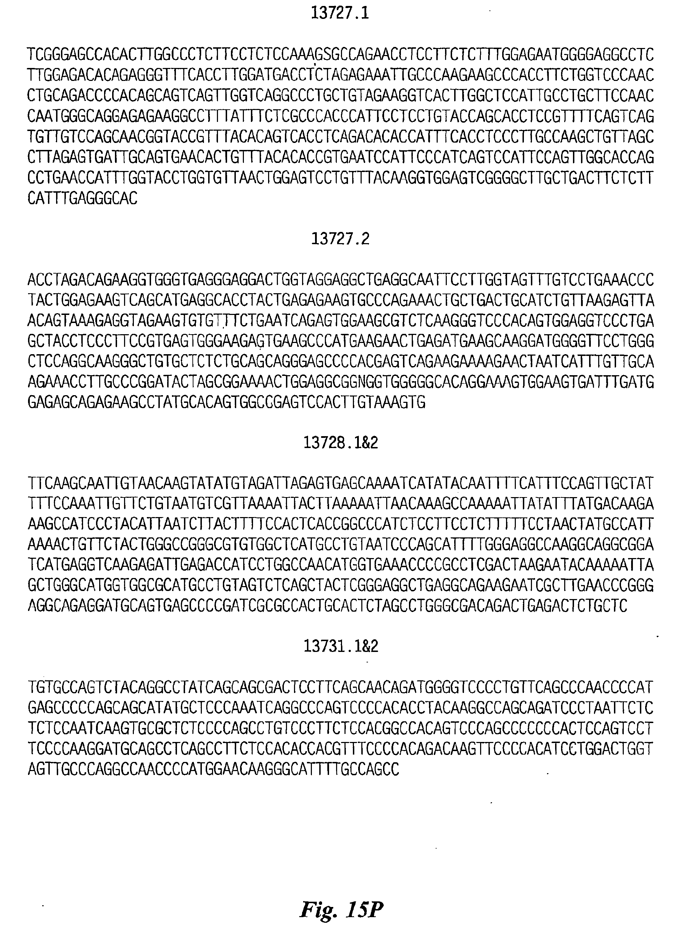

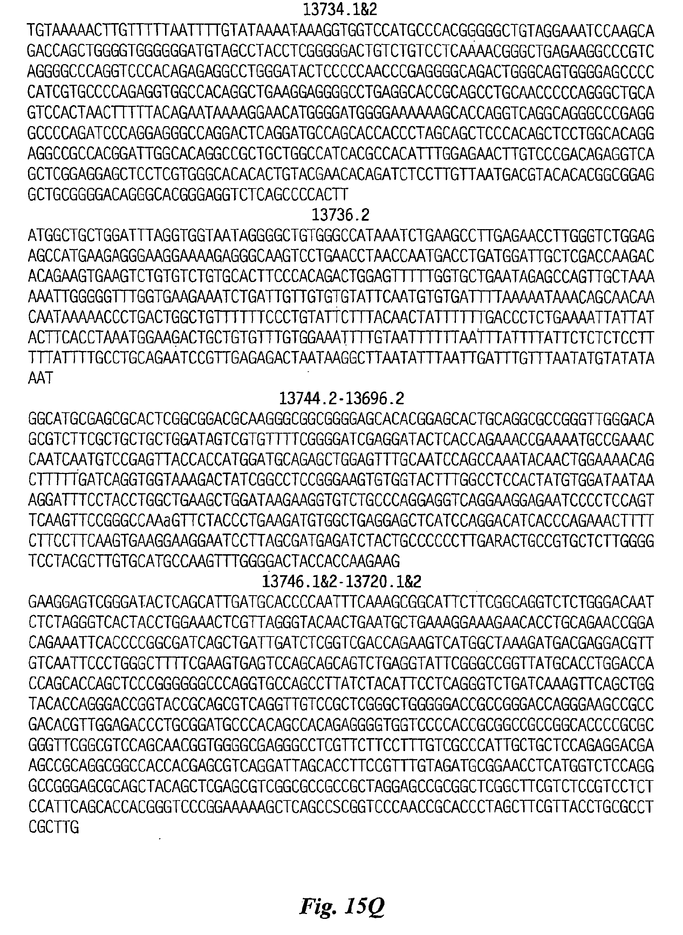

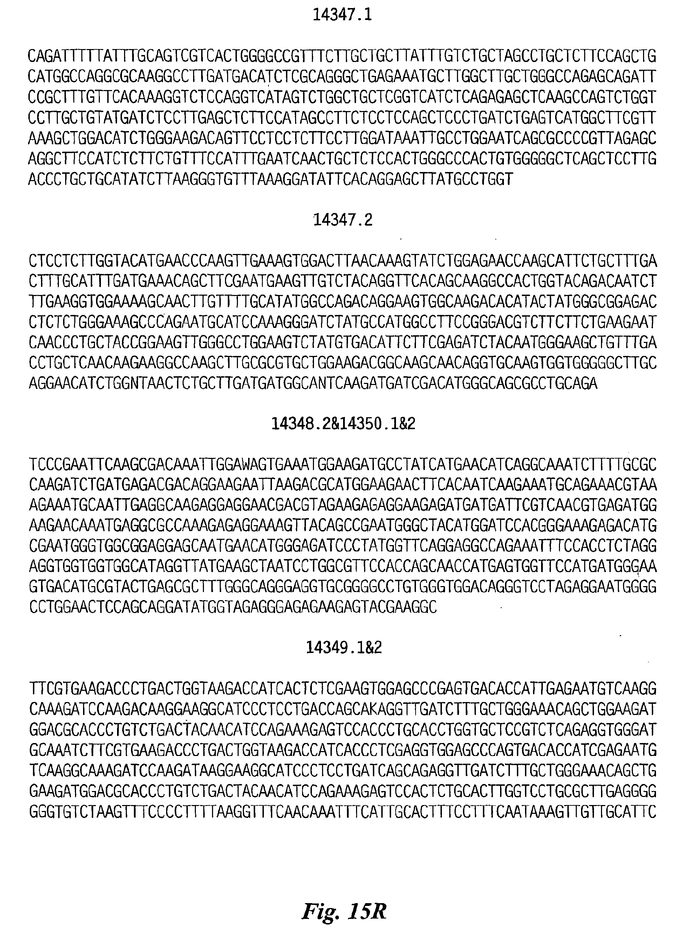

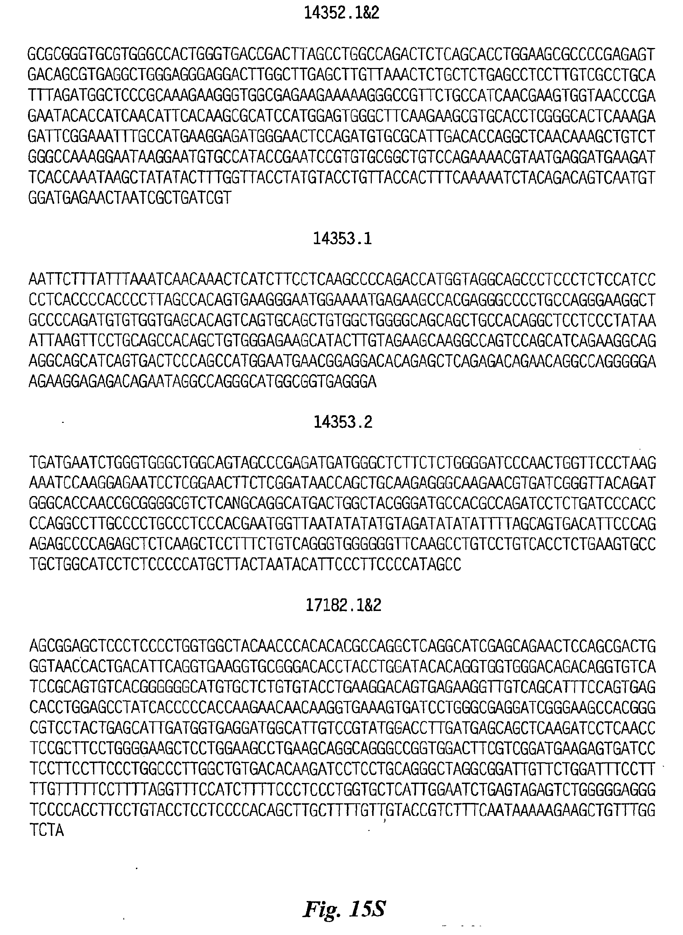

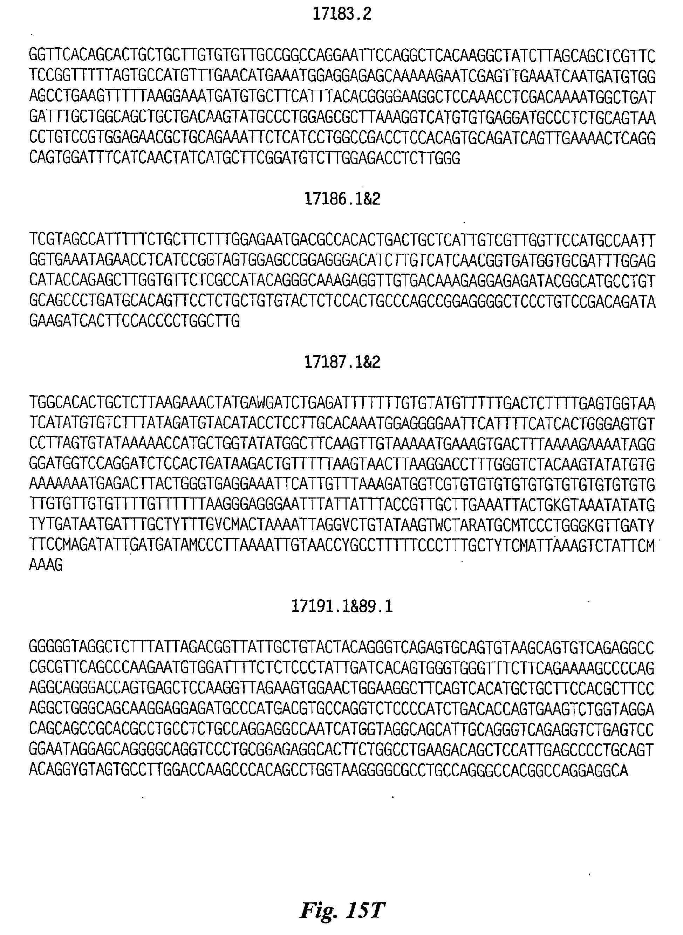

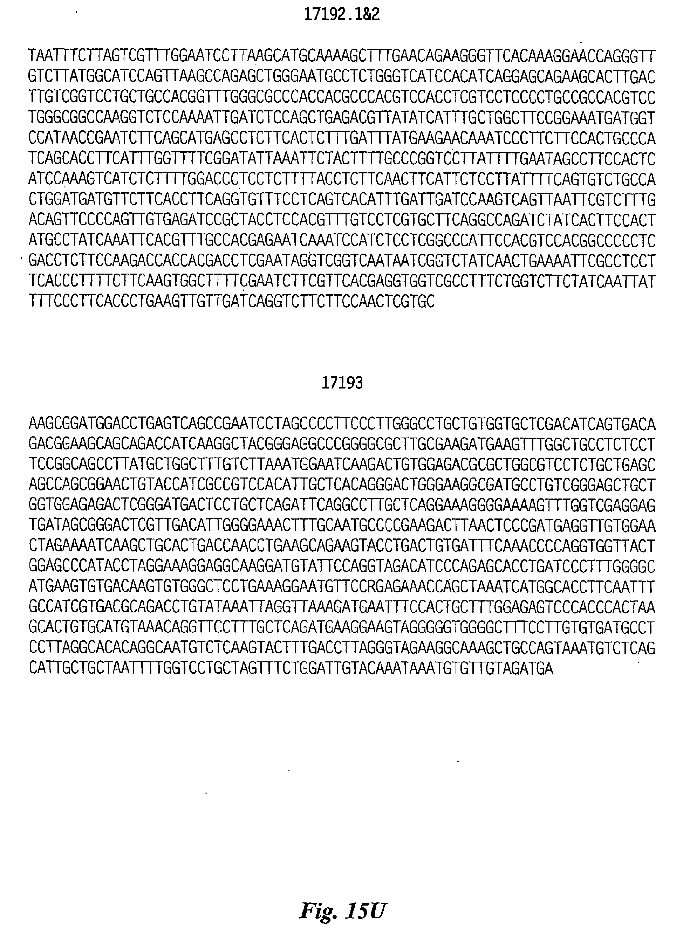

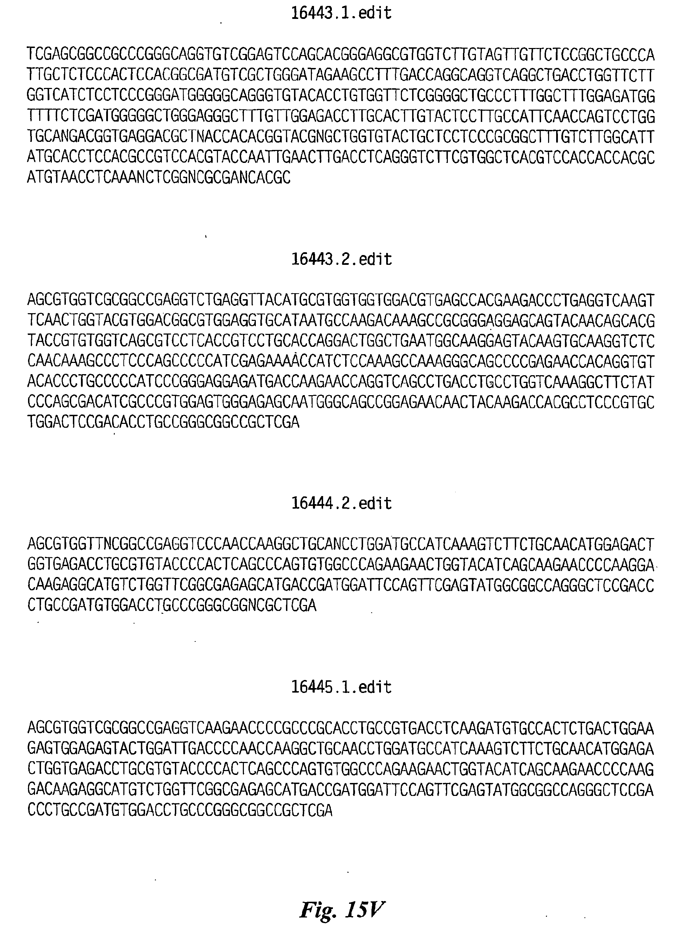

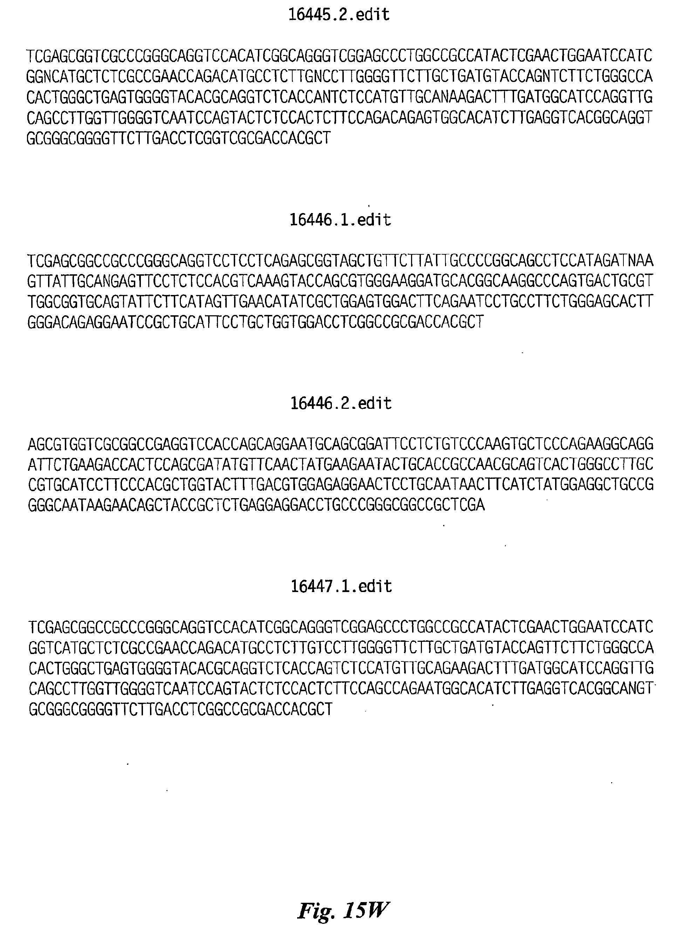

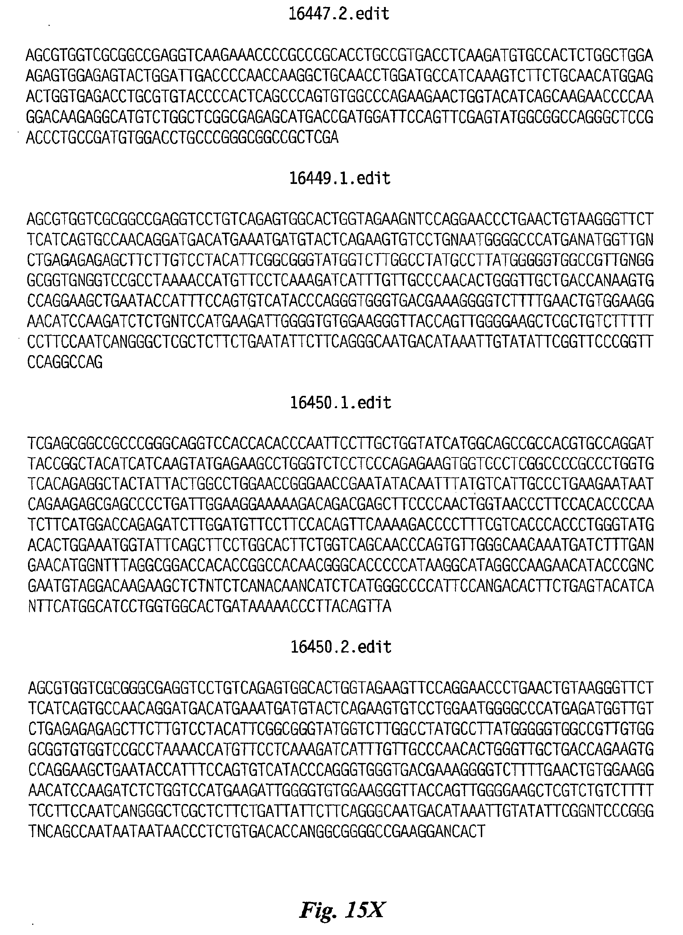

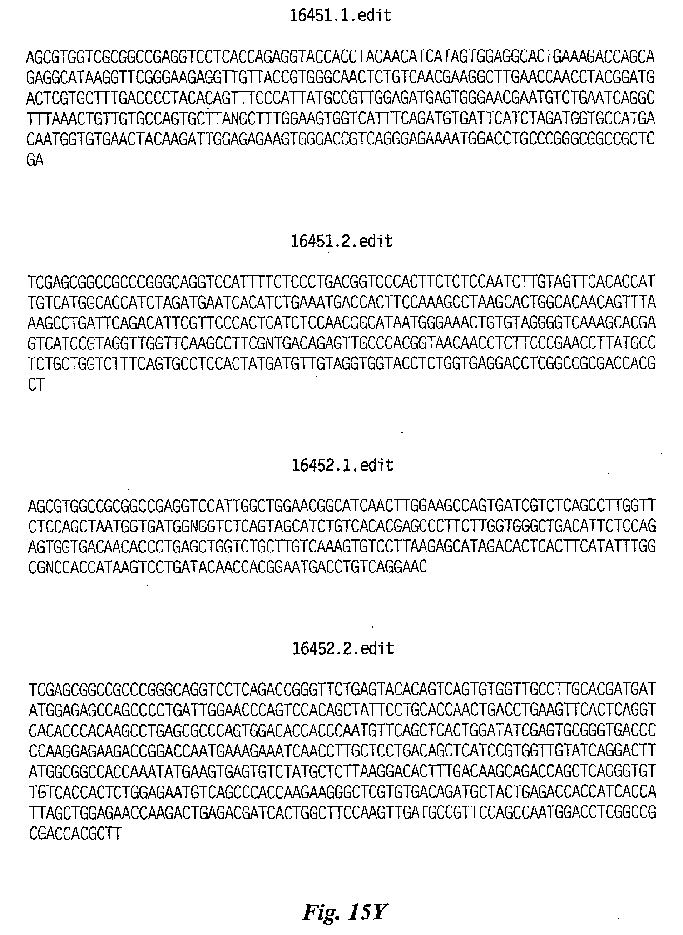

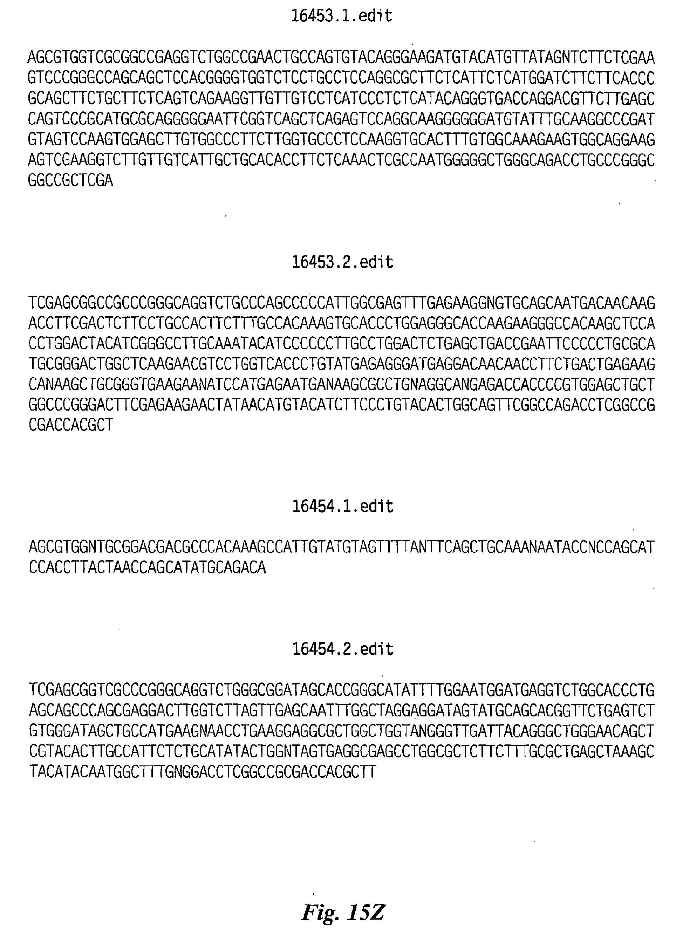

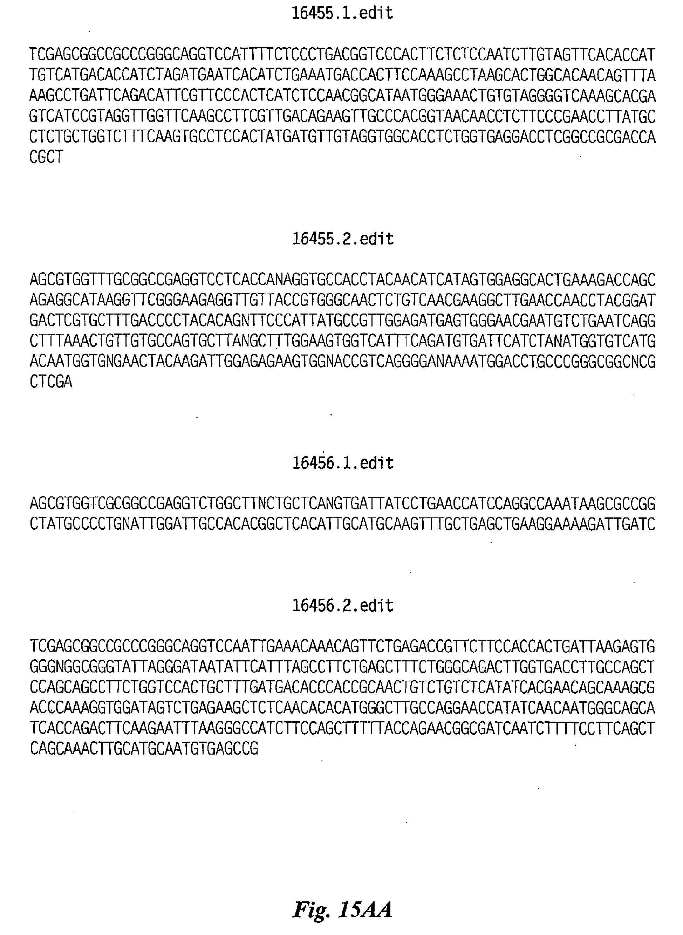

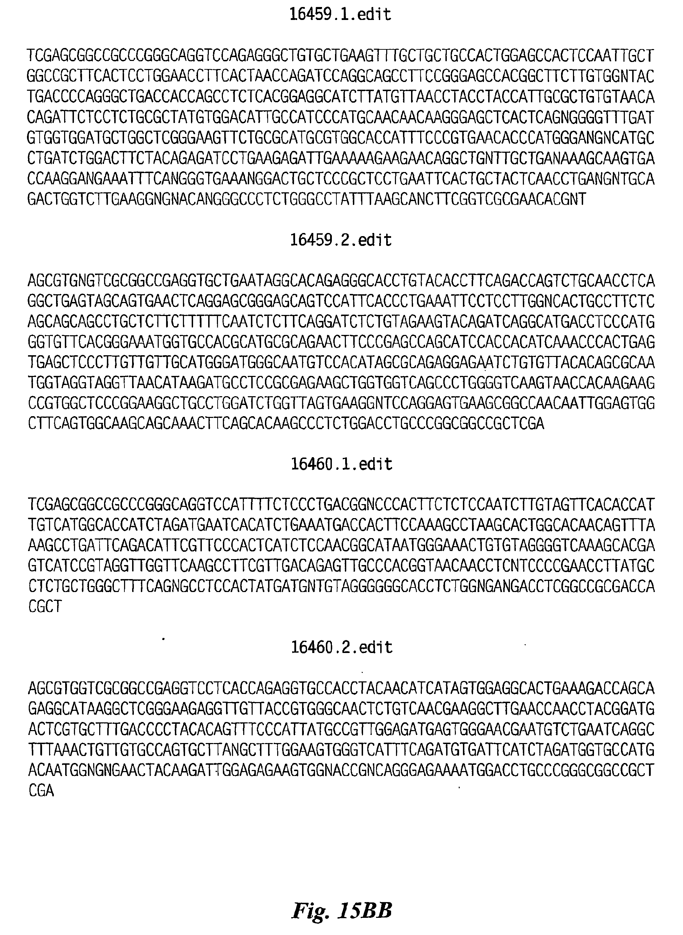

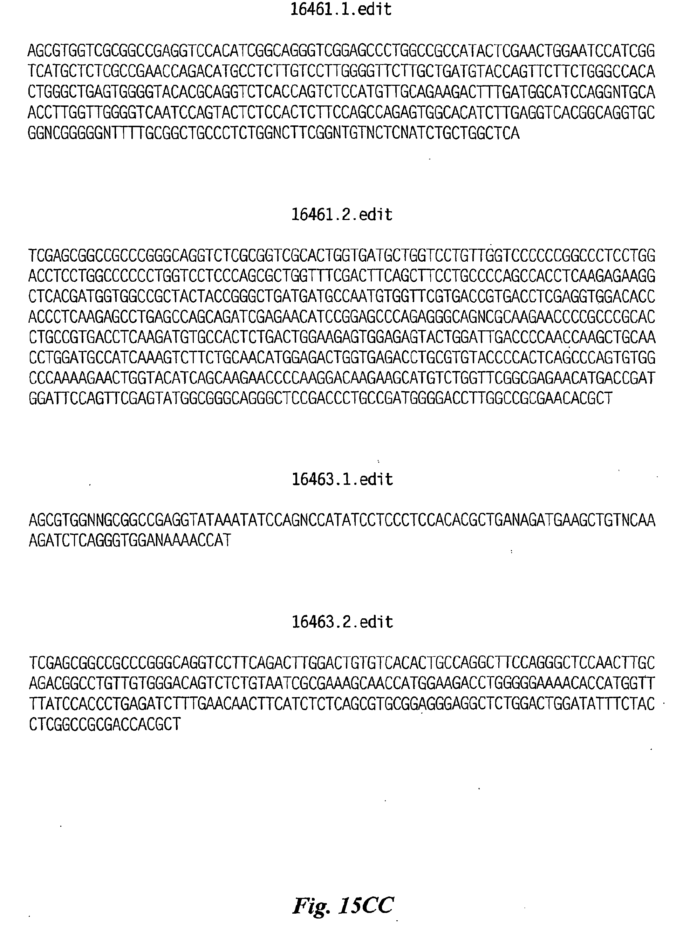

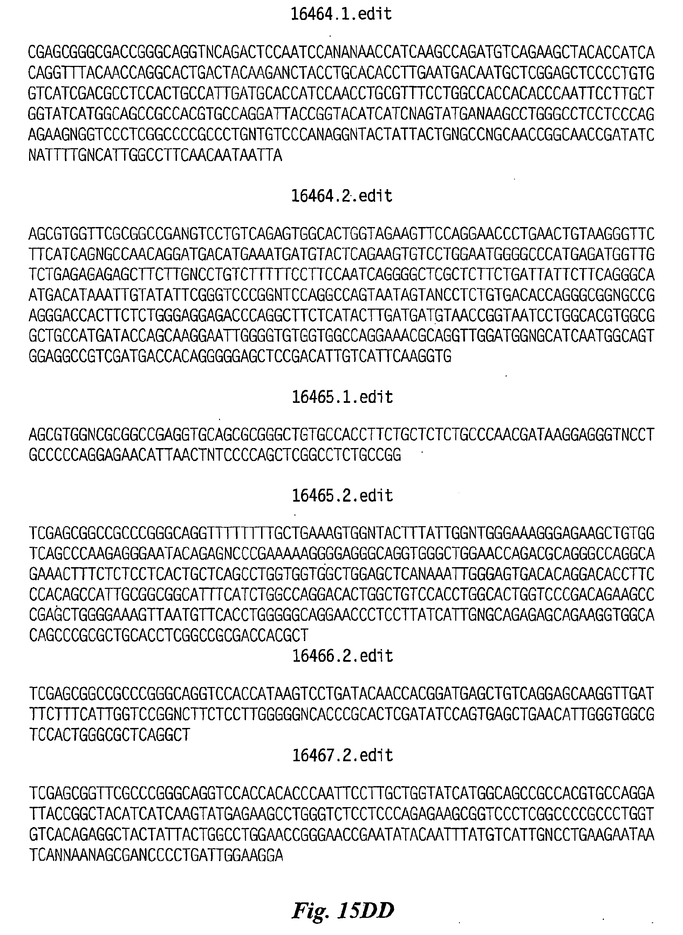

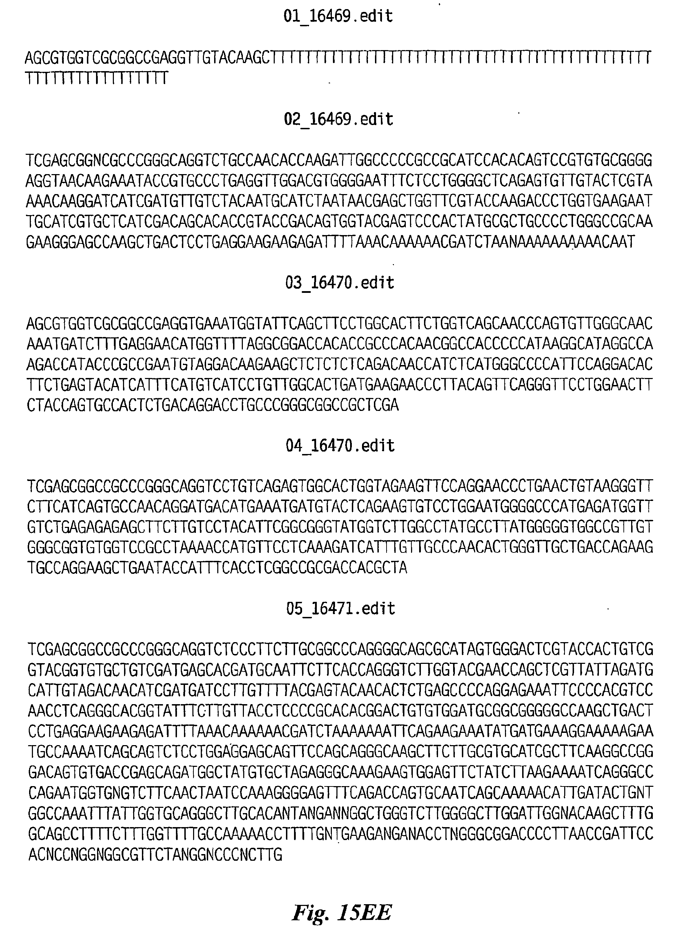

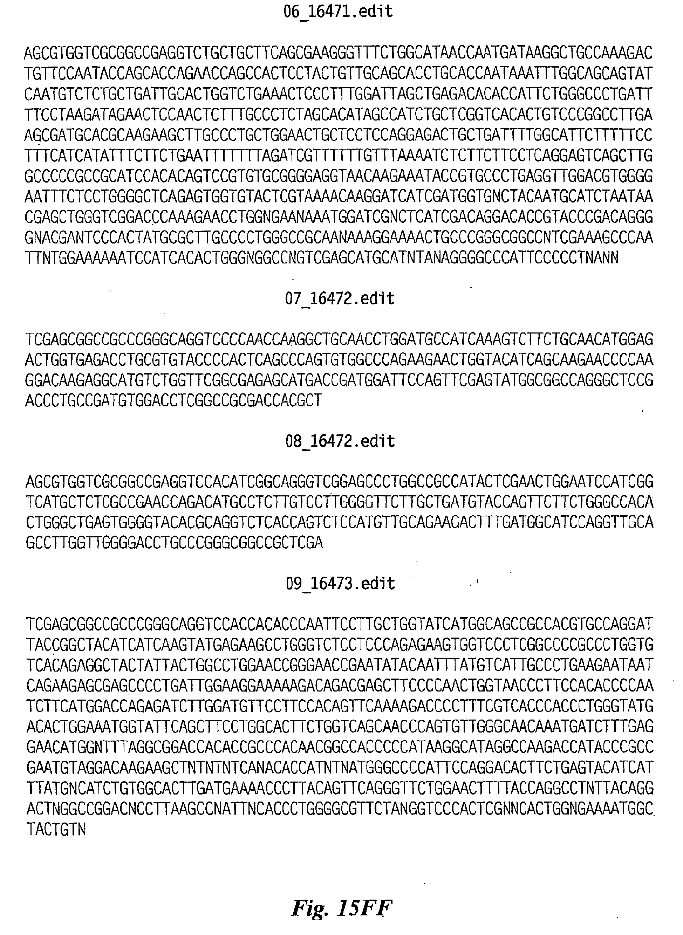

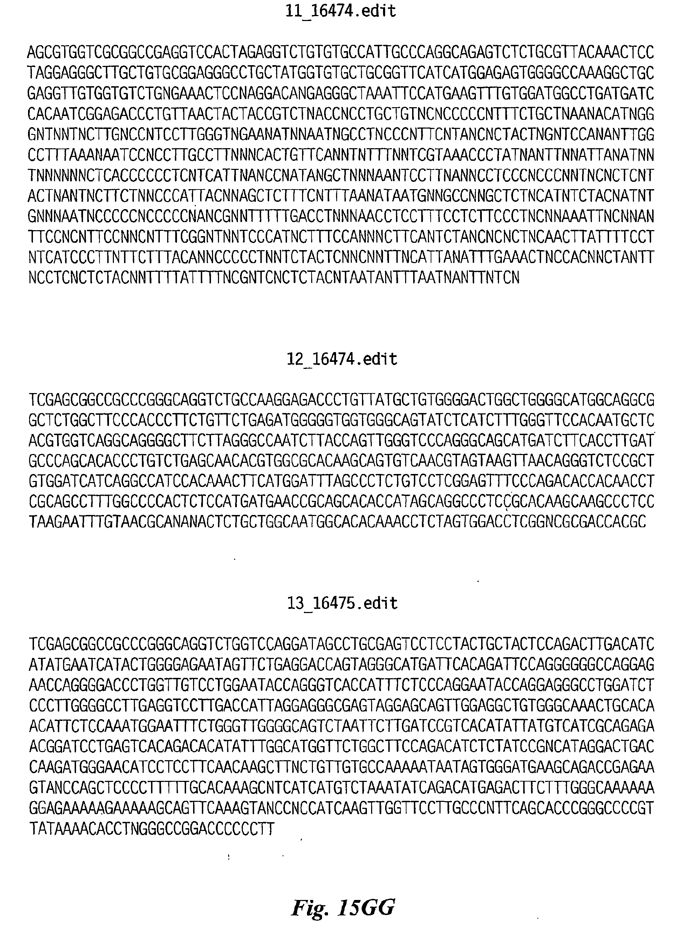

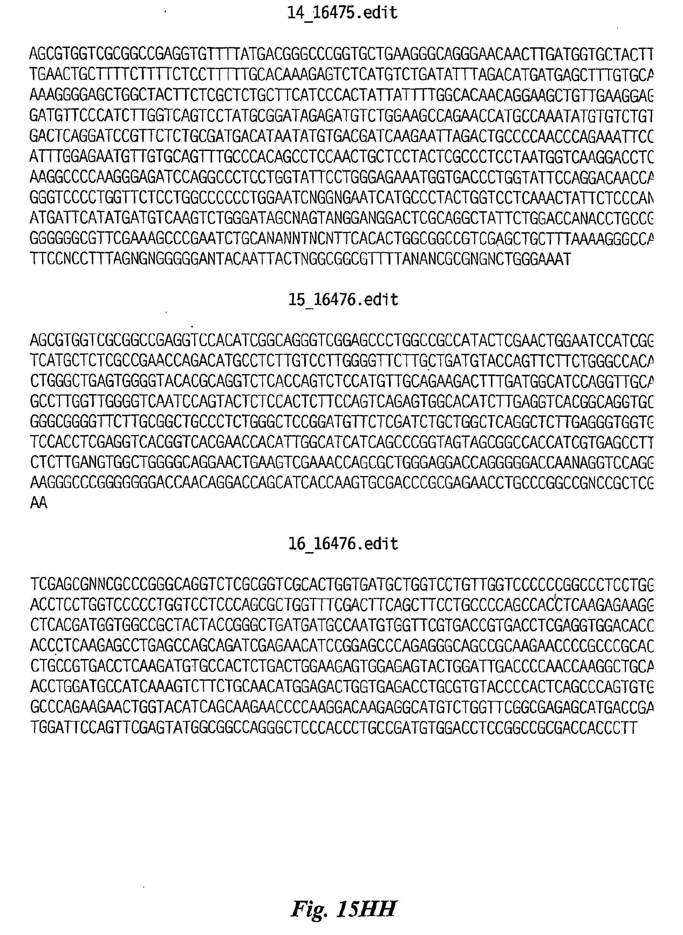

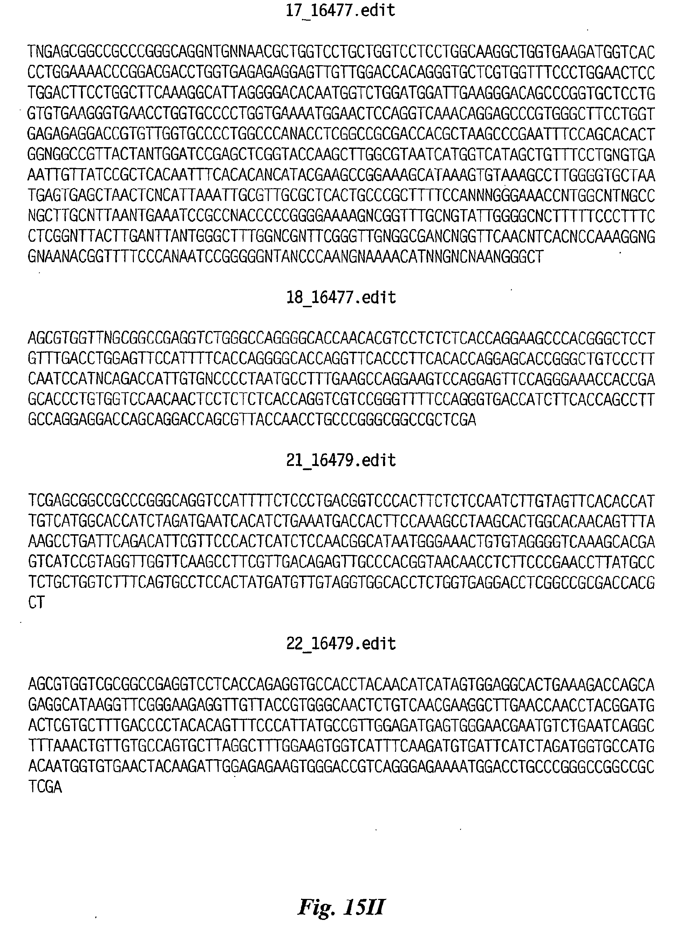

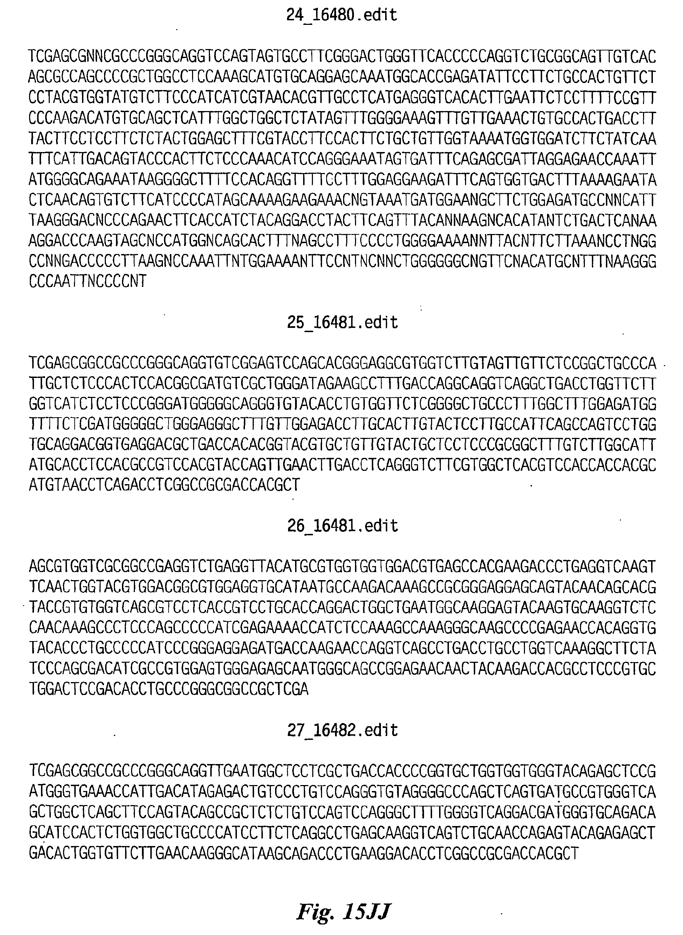

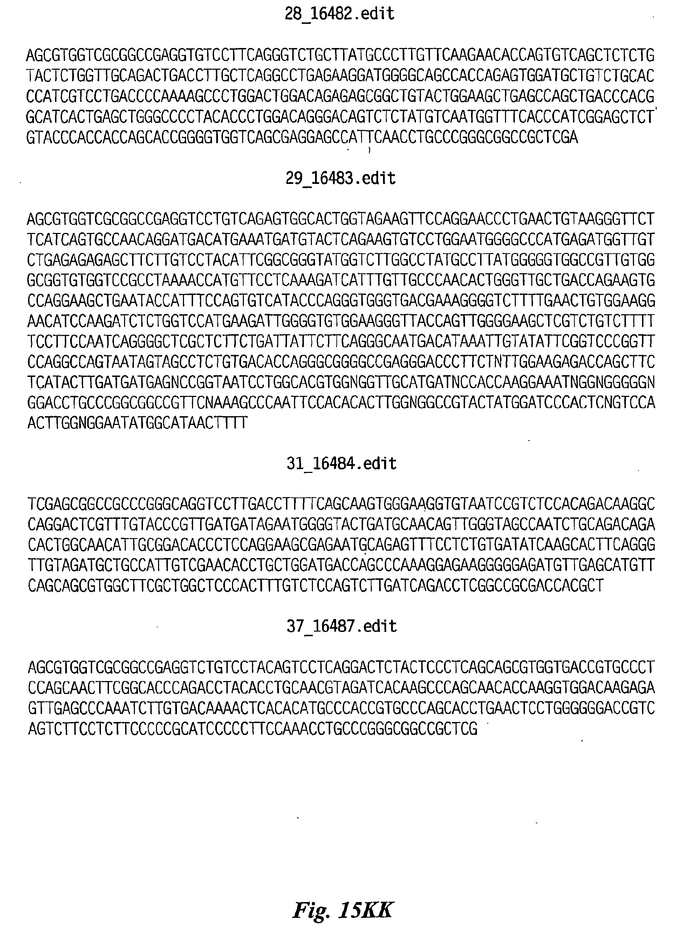

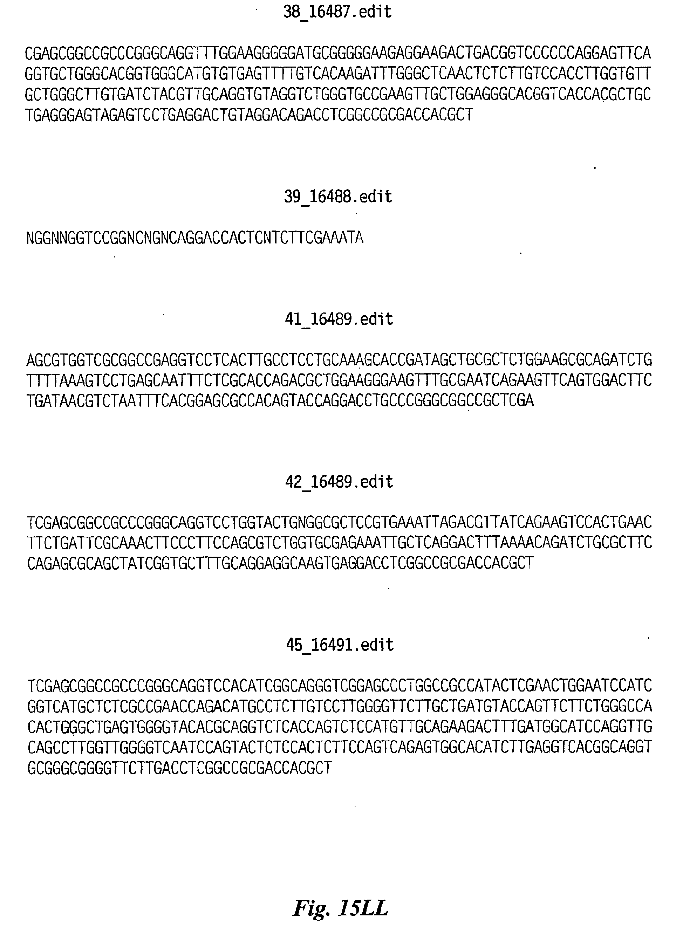

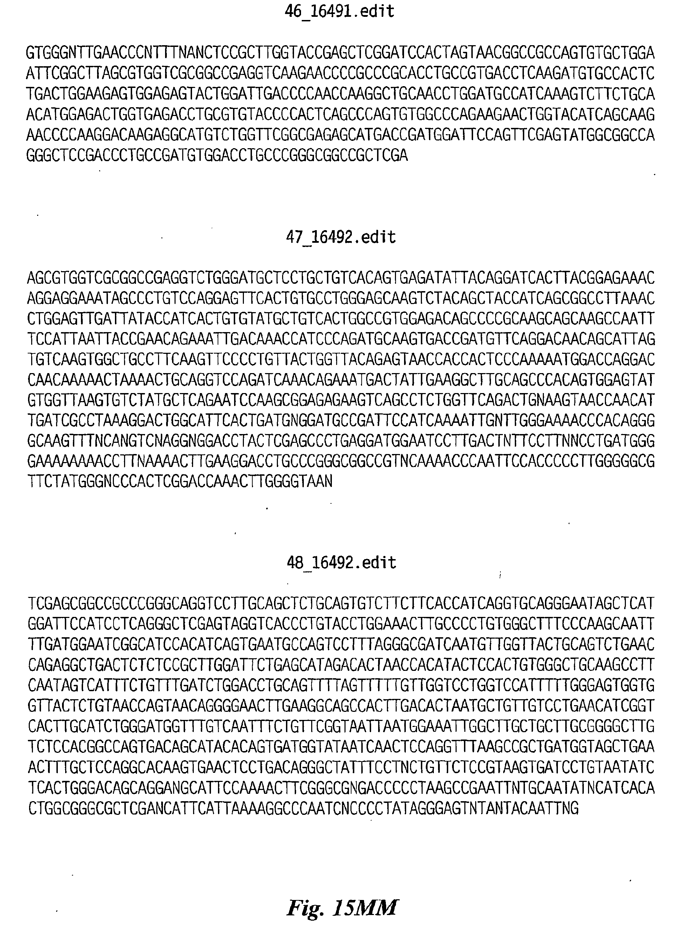

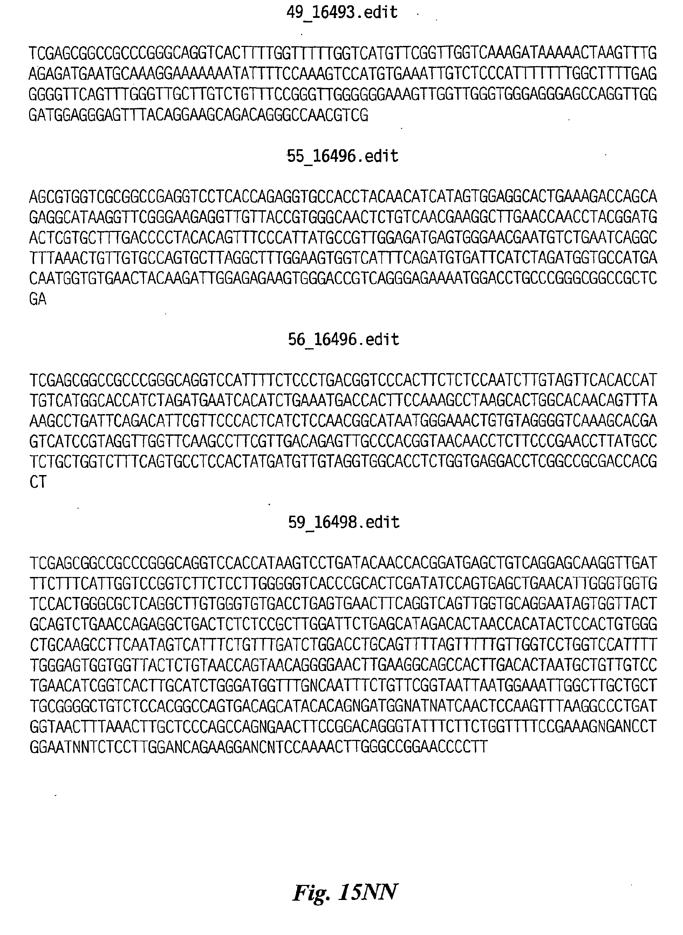

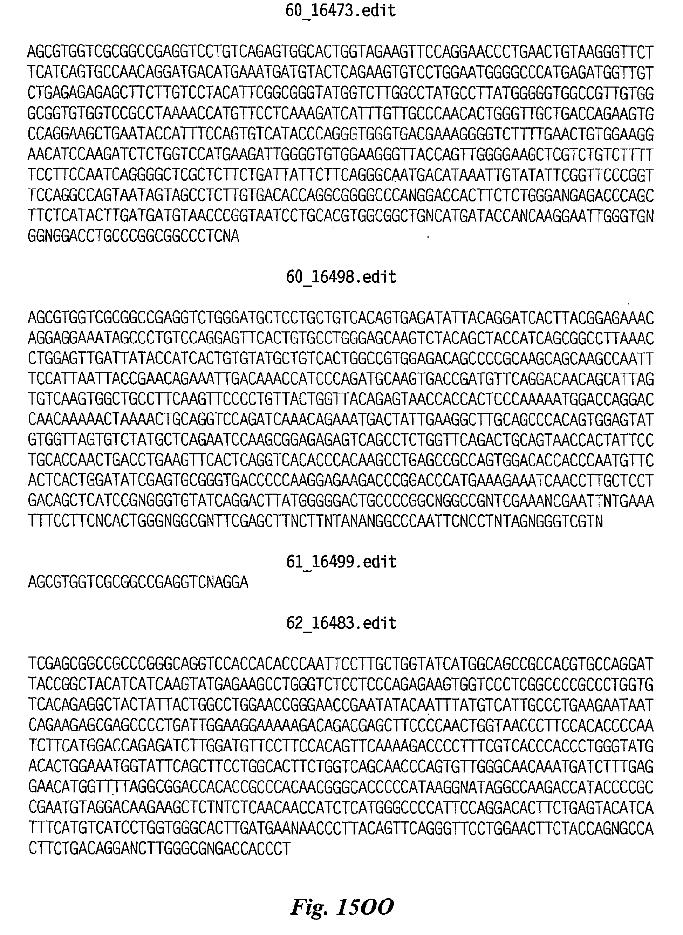

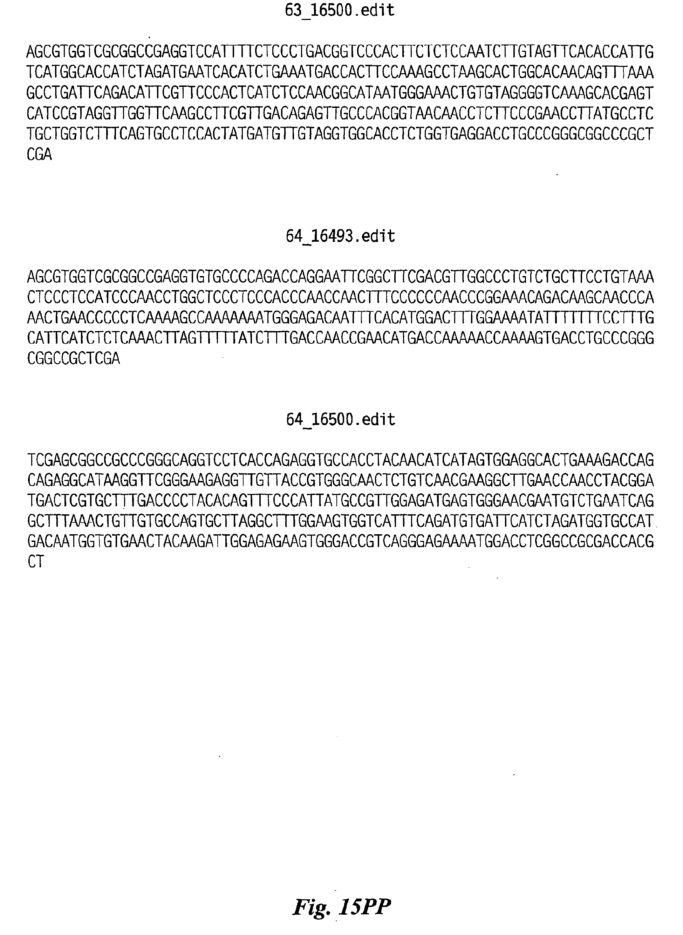

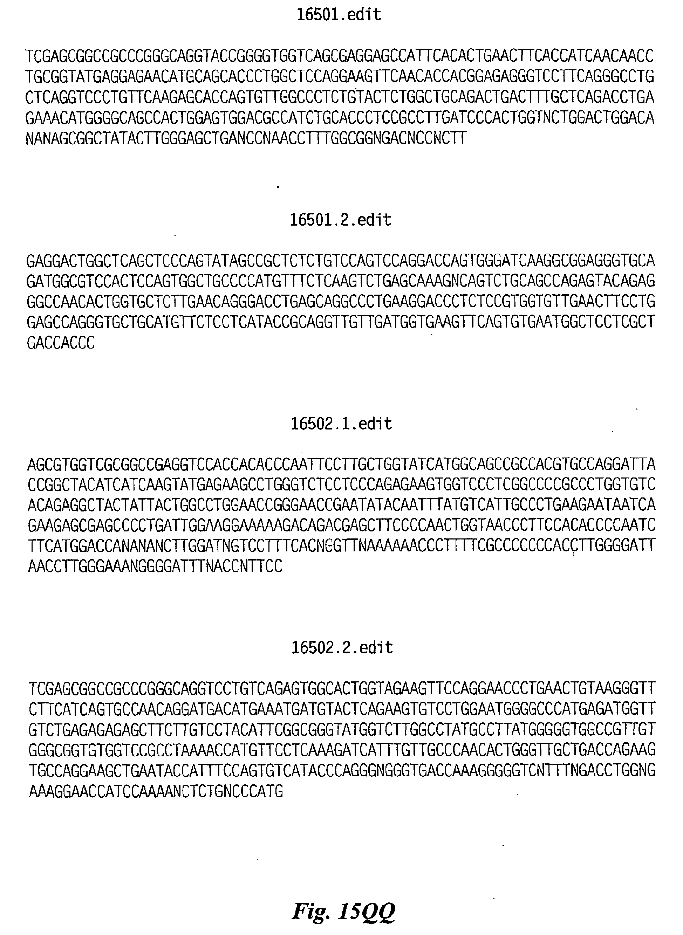

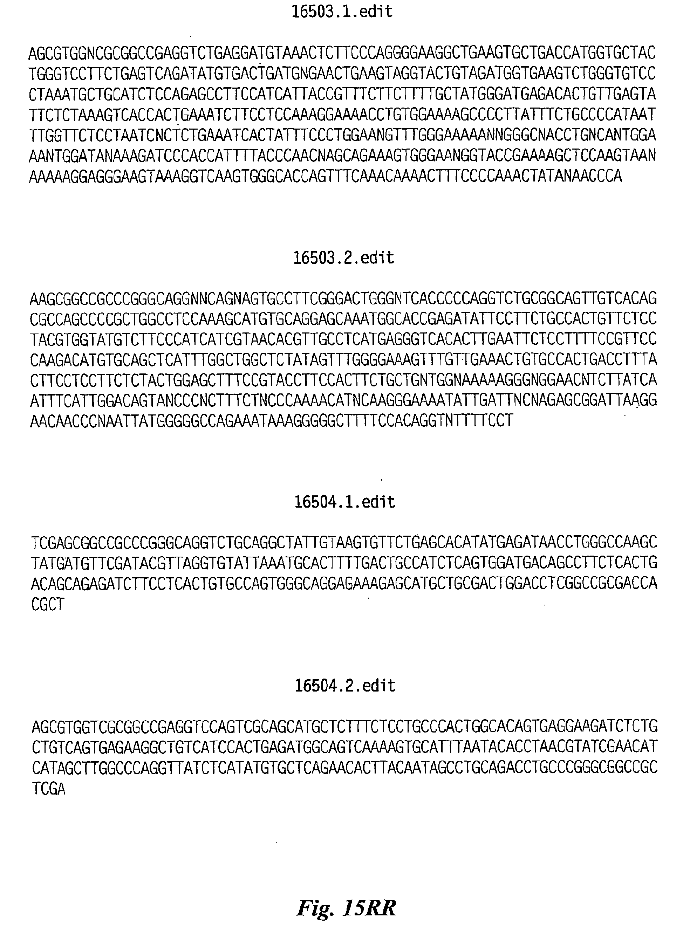

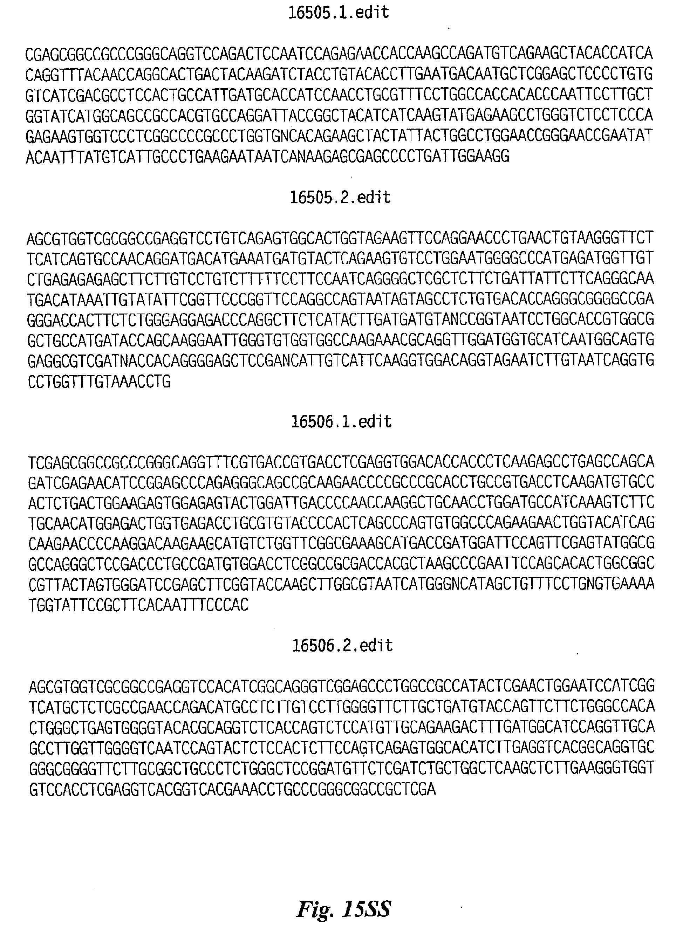

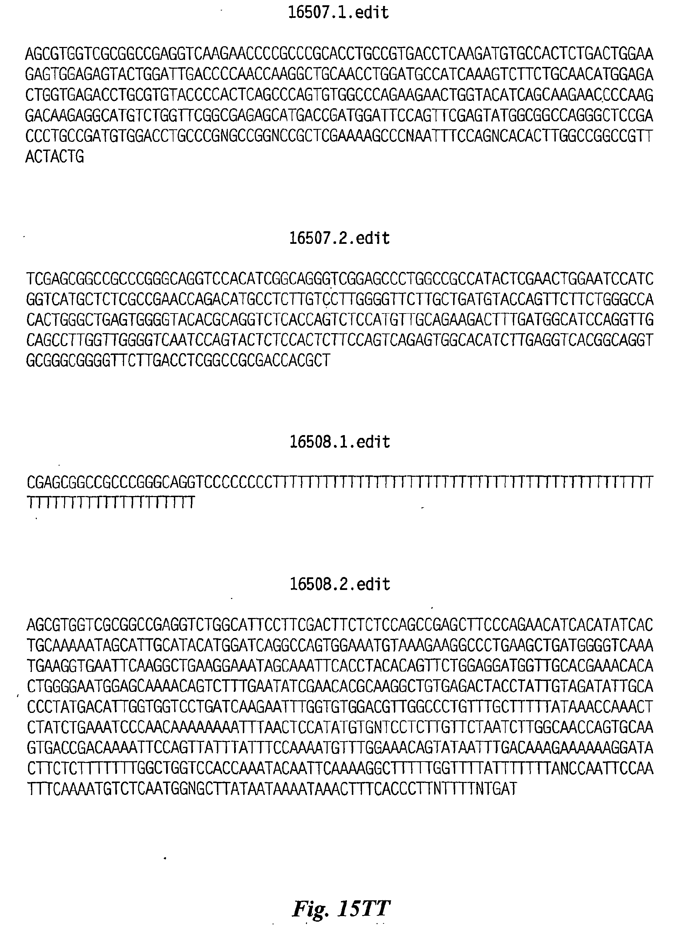

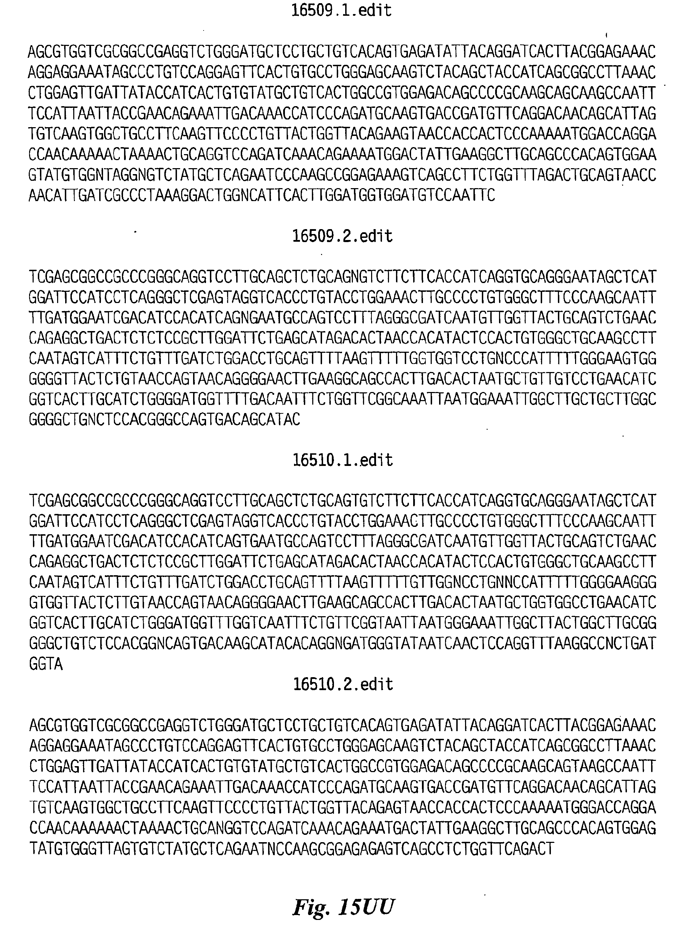

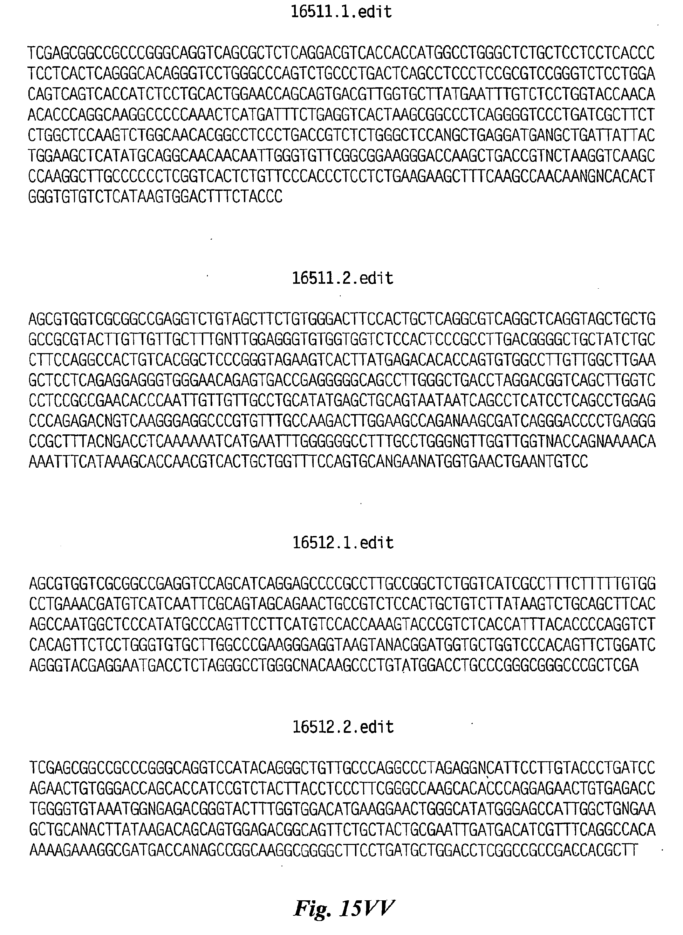

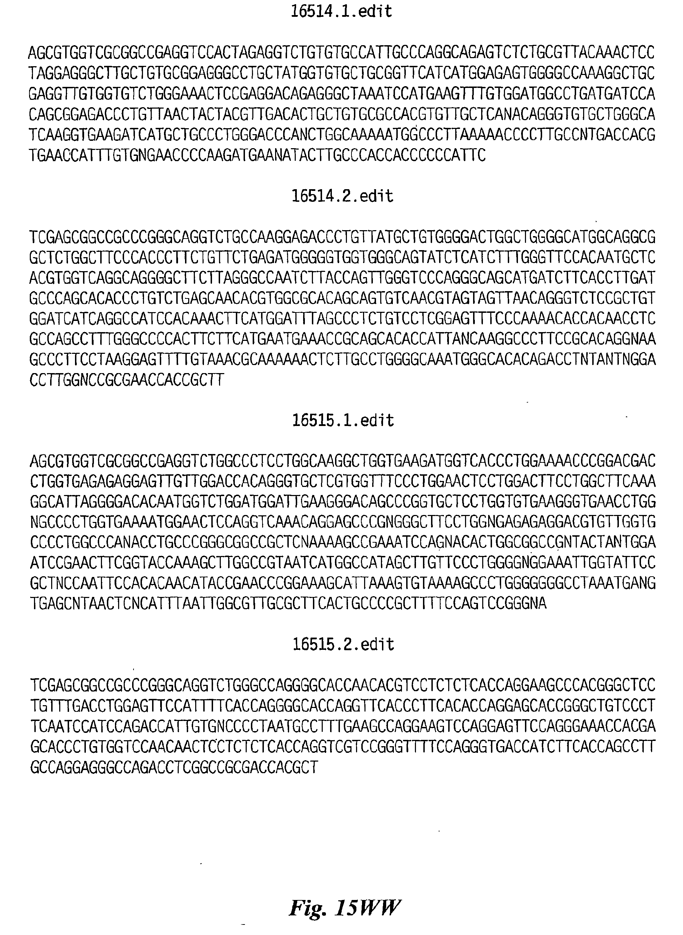

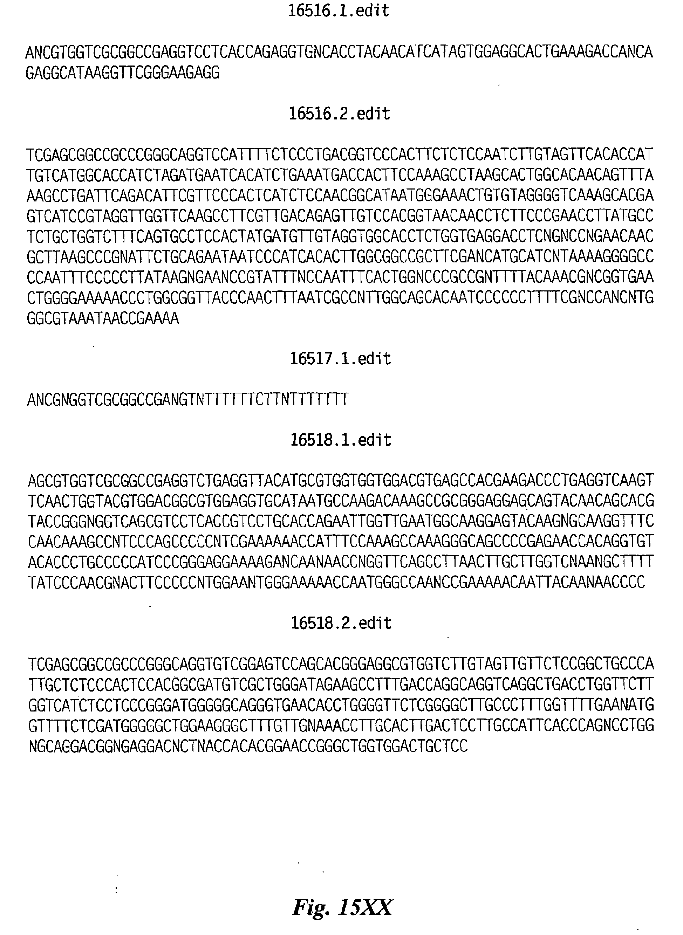

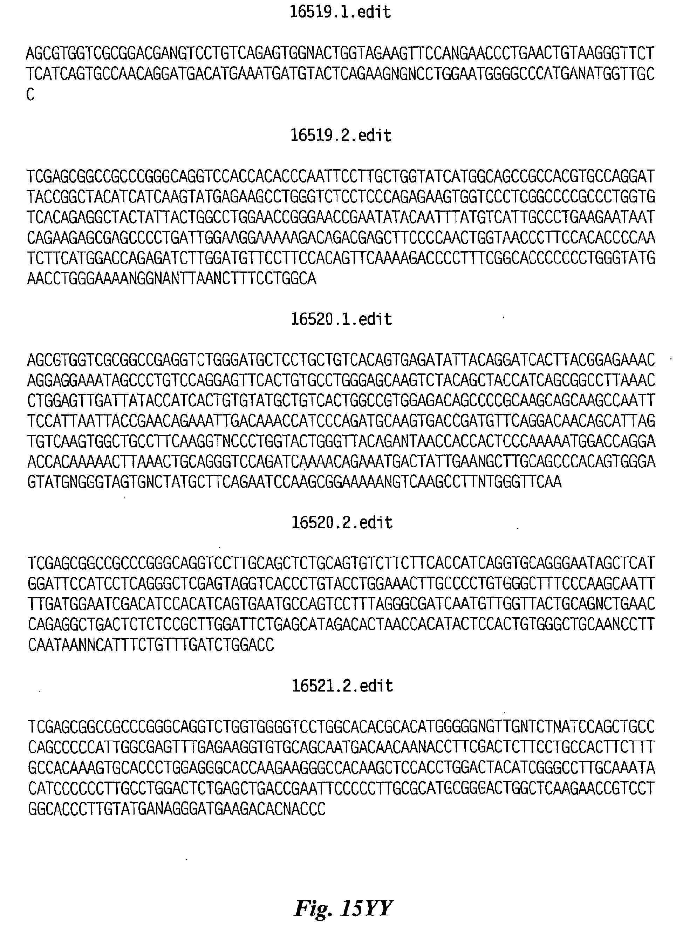

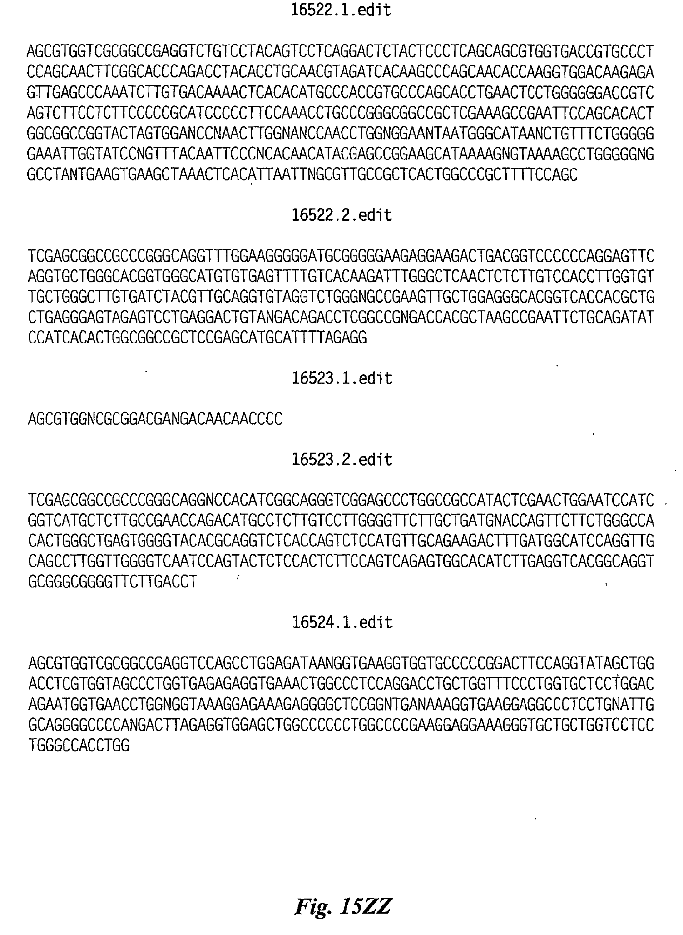

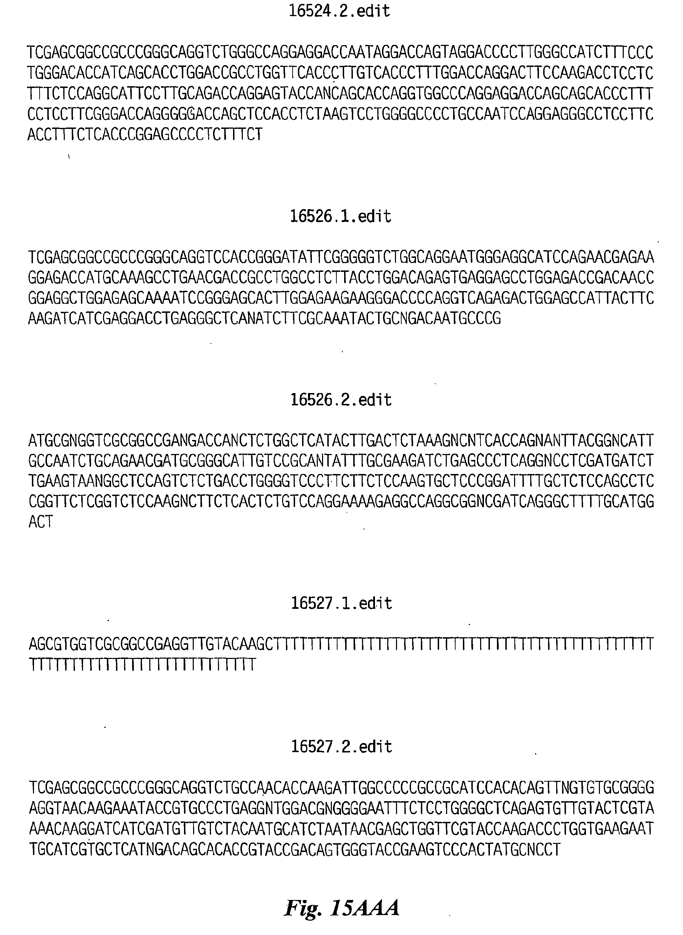

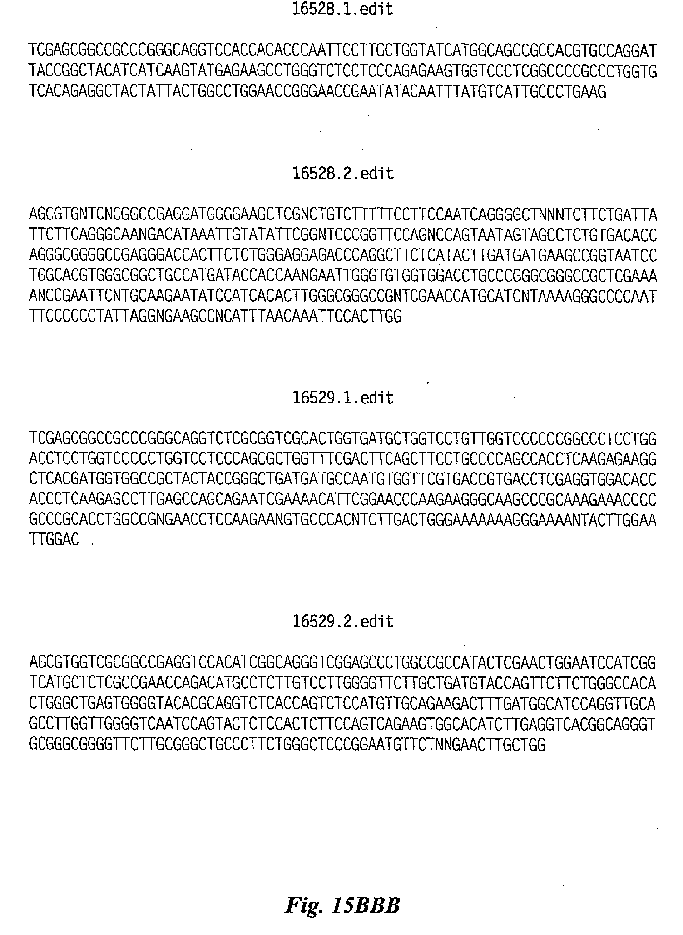

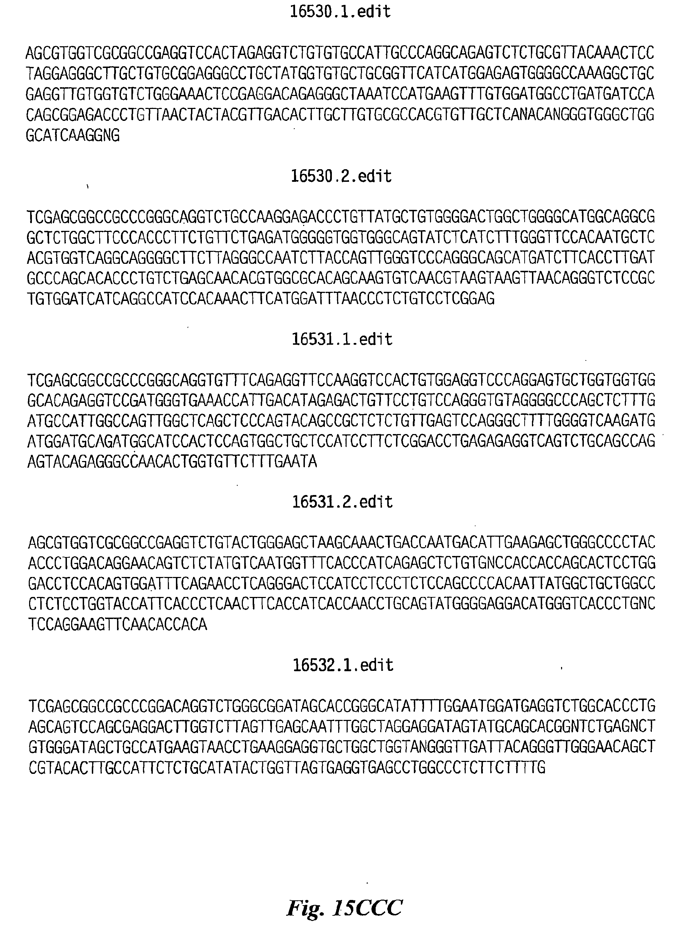

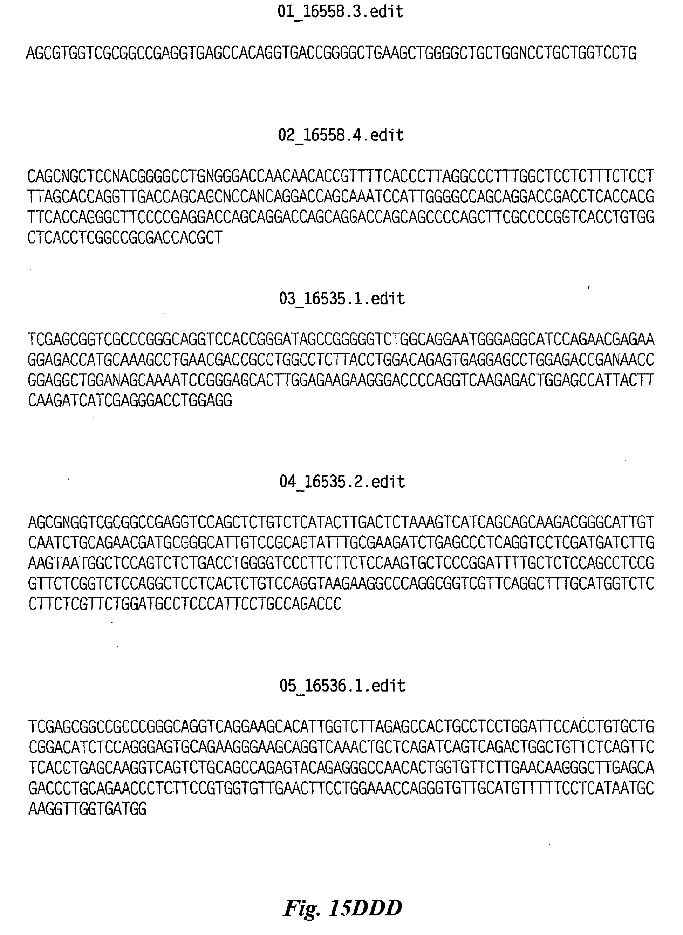

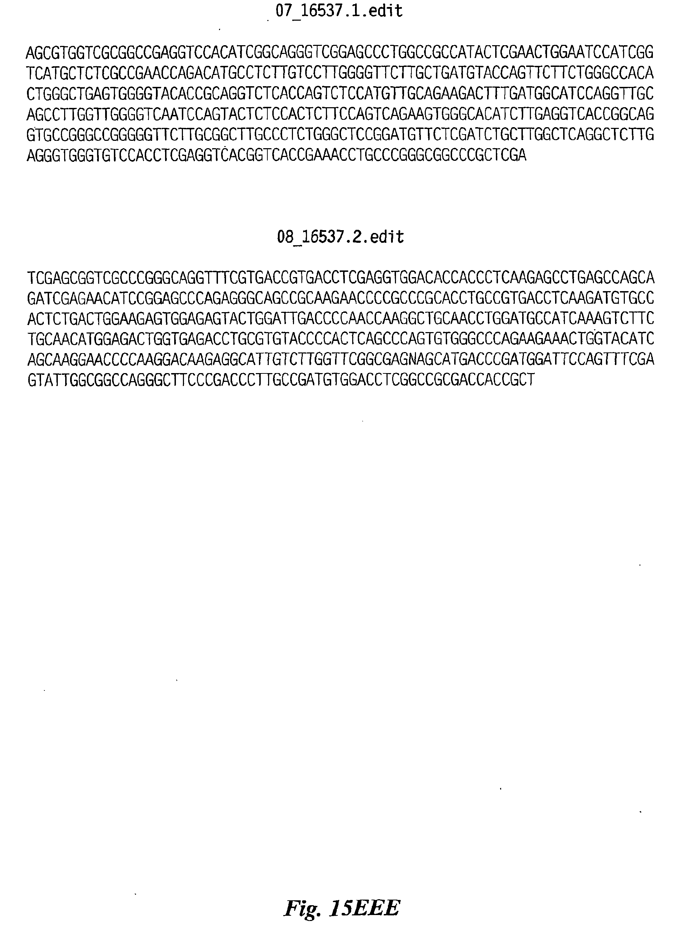

SEQ ID NO:1-71 are ovarian carcinoma antigen polynucleotides shown in Figures 1A-1S.
SEQ ID NO:72-74 are ovarian carcinoma antigen polynucleotides shown in Figures 2A-2C.
SEQ ID NO:75 is the ovarian carcinoma polynucleotide 3g (Figure 4).
SEQ ID NO:76 is the ovarian carcinoma polynucleotide 3f (Figure 5).
SEQ ID NO:77 is the ovarian carcinoma polynucleotide 6b (Figure 6).
SEQ ID NO:78 is the ovarian carcinoma polynucleotide 8e (Figure 7A).
SEQ ID NO:79 is the ovarian carcinoma polynucleotide 8h (Figure 7B).
SEQ ID NO:80 is the ovarian carcinoma polynucleotide 12e (Figure 8).
SEQ ID NO:81 is the ovarian carcinoma polynucleotide 12h (Figure 9).
SEQ ID NO:82-310 are ovarian carcinoma antigen polynucleotides shown in Figures 15A-15EEE.
SEQ ID NO:391 is a full length sequence of ovarian carcinoma polynucleotide O8E.
SEQ ID NO:392-393 are protein sequences encoded by O8E.
SEQ ID NO:394-415 are peptide sequences corresponding to the OE8 antibody epitopes.
SEQ ID NO:416-435 are potential HLA-A2 10-mer binding peptides predicted using the full length open-reading frame from OE8.
SEQ ID NO:436-455 are potential HLA-A2 9-mer binding peptides predicted using the full length open-reading frame from OE8.

Figures 1A-1S (SEQ ID NO:1-71) depict partial sequences of polynucleotides encoding representative secreted ovarian carcinoma antigens.
Figures 2A-2C depict full insert sequences for three of the clones of Figure 1. Figure 2A shows the sequence designated O7E (11731; SEQ ID NO:72), Figure 2B shows the sequence designated O9E (11785; SEQ ID NO:73) and Figure 2C shows the sequence designated O8E (13695; SEQ ID NO: 74).
Figure 3 presents results of microarray expression analysis of the ovarian carcinoma sequence designated O8E.
Figure 4 presents a partial sequence of a polynucleotide (designated 3g; SEQ ID NO:75) encoding an ovarian carcinoma sequence that is a splice fusion between the human T-cell leukemia virus type I oncoprotein TAX and osteonectin.
Figure 5 presents the ovarian carcinoma polynucleotide designated 3f (SEQ ID NO:76).
Figure 6 presents the ovarian carcinoma polynucleotide designated 6b (SEQ ID NO:77).
Figures 7A and 7B present the ovarian carcinoma polynucleotides designated 8e (SEQ ID NO:78) and 8h (SEQ ID NO:79).
Figure 8 presents the ovarian carcinoma polynucleotide designated 12c (SEQ ID NO:80).
Figure 9 presents the ovarian carcinoma polynucleotide designated 12h (SEQ ID NO:81).
Figure 10 depicts results of microarray expression analysis of the ovarian carcinoma sequence designated 3f.
Figure 11 depicts results of microarray expression analysis of the ovarian carcinoma sequence designated 6b.
Figure 12 depicts results of microarray expression analysis of the ovarian carcinoma sequence designated 8e.
Figure 13 depicts results of microarray expression analysis of the ovarian carcinoma sequence designated 12c.
Figure 14 depicts results of microarray expression analysis of the ovarian carcinoma sequence designated 12h.
Figures depict partial sequences of additional polynucleotides encoding representative secreted ovarian carcinoma antigens (SEQ ID NO:82-310).
Figure 16 is a diagram illustrating the location of various partial O8E sequences within the full length sequence.
Figure 17 is a graph illustrating the results of epitope mapping studies on O8E protein.
Figure 18 is graph of a fluorescence activated cell sorting (FACS) analysis of O8E cell surface expression.
Figure 19 is graph of a FACS analysis of O8E cell surface expression.
Figure 20 shows FACS analysis results for O8E transfected HEK293 cells demonstrating cell surface expression of O8E
Figure 21 shows FACS analysis results for SKBR3 breast tumor cells demonstrating cell surface expression of O8E.
Figure 22 shows 08E expression in HEK 293 cells. The cells were probed with anti-08E rabbit polyclonal antisera #2333L.
Figure 23 shows the ELISA analysis of anti-08E rabbit sera.
Figure 24 shows the ELISA analysis of affinity purified rabbit anti-08E polyclonal antibody.
Figure 25 is a graph determining antibody internalization of anti-O8E mAb showing that mAbs against amino acids 61-80 induces ligand internalization.

### DETAILED DESCRIPTION OF THE INVENTION

As noted above, the present invention is generally directed to compositions as defined by the claims and their use for the therapy of cancer, such as ovarian cancer. The compositions described herein may include immunogenic polypeptides, binding agents such as antibodies that bind to a polypeptide, antigen presenting cells (APCs) and/or immune system cells (*e.g*., T cells).

Polypeptides disclosed herein generally comprise at least an immunogenic portion of an ovarian carcinoma protein or a variant thereof. Certain ovarian carcinoma proteins have been identified using an immunoassay technique, and are referred to herein as ovarian carcinoma antigens. An "ovarian carcinoma antigen" is a protein that is expressed by ovarian tumor cells (preferably human cells) at a level that is at least two fold higher than the level in normal ovarian cells. Certain ovarian carcinoma antigens react detectably (within an immunoassay, such as an ELISA or Western blot) with antisera generated against serum from an immunodeficient animal implanted with a human ovarian tumor. Such ovarian carcinoma antigens are shed or secreted from an ovarian tumor into the sera of the immunodeficient animal. Accordingly, certain ovarian carcinoma antigens provided herein are secreted antigens. Certain nucleic acid sequences of the subject invention generally comprise a DNA or RNA sequence that encodes all or a portion of such a polypeptide, or that is complementary to such a sequence.

Ovarian carcinoma sequences are disclosed that are identified using techniques to evaluate altered expression within an ovarian tumor. Such sequences may be polynucleotide or protein sequences. Ovarian carcinoma sequences are generally expressed in an ovarian tumor at a level that is at least two fold, and preferably at least five fold, greater than the level of expression in normal ovarian tissue, as determined using a representative assay provided herein. Certain partial ovarian carcinoma polynucleotide sequences are presented herein. Proteins encoded by genes comprising such polynucleotide sequences (or complements thereof) are also considered ovarian carcinoma proteins.

Antibodies are generally immune system proteins, or antigen-binding fragments thereof, that are capable of binding to at least a portion of an ovarian carcinoma polypeptide as described herein. T cells that may be employed within the compositions provided herein are generally T cells (*e.g*., CD4⁺ and/or CD8⁺) that are specific for such a polypeptide. Certain methods described herein further employ antigen-presenting cells (such as dendritic cells or macrophages) that express an ovarian carcinoma polypeptide as provided herein.

### Ovarian Carcinoma Polynucleotides

Any polynucleotide that encodes an ovarian carcinoma protein or a portion or other variant thereof as described herein is part of the present disclosure. Preferred polynucleotides comprise at least 15 consecutive nucleotides, preferably at least 30 consecutive nucleotides, and more preferably at least 45 consecutive nucleotides, that encode a portion of an ovarian carcinoma protein. More preferably, a polynucleotide encodes an immunogenic portion of an ovarian carcinoma protein, such as an ovarian carcinoma antigen. Polynucleotides complementary to any such sequences are also disclosed. Polynucleotides may be single-stranded (coding or antisense) or double-stranded, and may be DNA (genomic, cDNA or synthetic) or RNA molecules. Additional coding or non-coding sequences may, but need not, be present within a polynucleotide as described, and a polynucleotide may, but need not, be linked to other molecules and/or support materials.

Polynucleotides may comprise a native sequence (*i.e.*, an endogenous sequence that encodes an ovarian carcinoma protein or a portion thereof) or may comprise a variant of such a sequence. Polynucleotide variants may contain one or more substitutions, additions, deletions and/or insertions such that the immunogenicity of the encoded polypeptide is not diminished, relative to a native ovarian carcinoma protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native ovarian carcinoma protein or a portion thereof.

The percent identity for two polynucleotide or polypeptide sequences may be readily determined by comparing sequences using computer algorithms well known to those of ordinary skill in the art, such as Megalign, using default parameters. Comparisons between two sequences are typically performed by comparing the sequences over a comparison window to identify and compare local regions of sequence similarity. A "comparison window" as used herein, refers to a segment of at least about 20 contiguous positions, usually 30 to about 75, or 40 to about 50, in which a sequence may be compared to a reference sequence of the same number of contiguous positions after the two sequences are optimally aligned. Optimal alignment of sequences for comparison may be conducted, for example, using the Megalign program in the Lasergene suite of bioinformatics software (DNASTAR, Inc., Madison, WI), using default parameters. Preferably, the percentage of sequence identity is determined by comparing two optimally aligned sequences over a window of comparison of at least 20 positions, wherein the portion of the polynucleotide or polypeptide sequence in the window may comprise additions or deletions *(i.e.,* gaps) of 20 % or less, usually 5 to 15 %, or 10 to 12%, relative to the reference sequence (which does not contain additions or deletions). The percent identity may be calculated by determining the number of positions at which the identical nucleic acid bases or amino acid residue occurs in both sequences to yield the number of matched positions, dividing the number of matched positions by the total number of positions in the reference sequence *(i.e.,* the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native ovarian carcinoma protein (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are also disclosed. Further, alleles of the genes comprising the polynucleotide sequences provided herein are described. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, an ovarian carcinoma polynucleotide may be identified, as described in more detail below, by screening a late passage ovarian tumor expression library with antisera generated against sera of immunocompetent mice after injection of such mice with sera from SCID mice implanted with late passage ovarian tumors. Ovarian carcinoma polynucleotides may also be identified using any of a variety of techniques designed to evaluate differential gene expression. Alternatively, polynucleotides may be amplified from cDNA prepared from ovarian tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (*e.g*., an ovarian carcinoma cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g*., by nick-translation or end-labeling with ³²P) using well known techniques. A bacterial or bacteriophage library is then screened by hybridizing filters containing denatured bacterial colonies (or lawns containing phage plaques) with the labeled probe *(see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR *(see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g*., NCBI BLAST searches), and such ESTs may be used to generate a contiguous full length sequence.

Certain nucleic acid sequences of cDNA molecules encoding portions of ovarian carcinoma antigens are provided in Figures 1A-1S (SEQ ID NO:1 to 71) and Figures 15A to 15EEE (SEQ ID NO: 82 to 310). The sequences provided in Figures 1A-1S appear to be novel. For sequences in Figures 15A-15EEE, database searches revealed matches having substantial identity. These polynucleotides were isolated by serological screening of an ovarian tumor cDNA expression library, using a technique designed to identify secreted tumor antigens. Briefly, a late passage ovarian tumor expression library was prepared from a SCID-derived human ovarian tumor (OV9334) in the vector λ-screen (Novagen). The sera used for screening were obtained by injecting immunocompetent mice with sera from SCID mice implanted with one late passage ovarian tumors. This technique permits the identification of cDNA molecules that encode immunogenic portions of secreted tumor antigens. The polynucleotides recited herein, as well as full length polynucleotides comprising such sequences, other portions of such full length polynucleotides, and sequences complementary to all or a portion of such full length molecules, are specifically described. It will be apparent to those of ordinary skill in the art that this technique can also be applied to the identification of antigens that are secreted from other types of tumors.

Other nucleic acid sequences of cDNA molecules encoding portions of ovarian carcinoma proteins are provided in Figures 4-9 (SEQ ID NO:75-81). These sequences were identified by screening a microarray of cDNAs for tumor-associated expression (*i.e.*, expression that is at least five fold greater in an ovarian tumor than in normal ovarian tissue, as determined using a representative assay provided herein). Such screens were performed using a Synteni microarray (Palo Alto, CA) according to the manufacturer's instructions (and essentially as described by Schena et al., Proc. Natl: Acad. Sci. USA 93:10614-10619, 1996 and Heller et al., Proc. Natl. Acad Sci. USA 94:2150-2155, 1997).

Any of a variety of well known techniques may be used to evaluate tumor-associated expression of a cDNA. For example, hybridization techniques using labeled polynucleotide probes may be employed. Alternatively, or in addition, amplification techniques such as real-time PCR may be used *(see* Gibson et al., Genome Research 6:995-1001, 1996; Heid et al., Genome Research 6:986-994, 1996). Real-time PCR is a technique that evaluates the level of PCR product accumulation during amplification. This technique permits quantitative evaluation of mRNA levels in multiple samples. Briefly, mRNA is extracted from tumor and normal tissue and cDNA is prepared using standard techniques. Real-time PCR may be performed, for example, using a Perkin Elmer/Applied Biosystems (Foster City, CA) 7700 Prism instrument. Matching primers and fluorescent probes may be designed for genes of interest using, for example, the primer express program provided by Perkin Elmer/Applied Biosystems (Foster City, CA). Optimal concentrations of primers and probes may be initially determined by those of ordinary skill in the art, and control *(e.g.,* β-actin) primers and probes may be obtained commercially from, for example, Perkin Elmer/Applied Biosystems (Foster City, CA). To quantitate the amount of specific RNA in a sample, a standard curve is generated alongside using a plasmid containing the gene of interest. Standard curves may be generated using the Ct values determined in the real-time PCR, which are related to the initial cDNA concentration used in the assay. Standard dilutions ranging from 10-10⁶ copies of the gene of interest are generally sufficient. In addition, a standard curve is generated for the control sequence. This permits standardization of initial RNA content of a tissue sample to the amount of control for comparison purposes.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis (*see* Adelman et al., DNA 2:183, 1983). Alternatively, RNA molecules may be generated by *in vitro* or *in vivo* transcription of DNA sequences encoding an ovarian carcinoma antigen, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo.*

A portion of a sequence complementary to a coding sequence *(i.e.,* an antisense polynucleotide) may also be used as a probe or to modulate gene expression. cDNA constructs that can be transcribed into antisense RNA may also be introduced into cells or tissues to facilitate the production of antisense RNA. An antisense polynucleotide may be used, as described herein, to inhibit expression of an ovarian carcinoma protein. Antisense technology can be used to control gene expression through triple-helix formation, which compromises the ability of the double helix to open sufficiently for the binding of polymerases, transcription factors or regulatory molecules (*see* Gee et al., In Huber and Carr, Molecular and Immunologic Approaches, Futura Publishing Co. (Mt. Kisco, NY; 1994). Alternatively, an antisense molecule may be designed to hybridize with a control region of a gene (*e.g*., promoter, enhancer or transcription initiation site), and block transcription of the gene; or to block translation by inhibiting binding of a transcript to ribosomes.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (*e.g*., avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e.,* an artificial membrane vesicle). The preparation and use of such systems is well known in the art.

### Ovarian Carcinoma Polypeptides

Polypeptides comprising at least an immunogenic portion of an ovarian carcinoma protein or a variant thereof are described. As noted above, certain ovarian carcinoma proteins are ovarian carcinoma antigens that are expressed by ovarian tumor cells and react detectably within an immunoassay (such as an ELISA) with antisera generated against serum from an immunodeficient animal implanted with an ovarian tumor. Other ovarian carcinoma proteins are encoded by ovarian carcinoma polynucleotides recited herein. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of an antigen that is recognized (*i.e.*, specifically bound) by a B-cell and/or T-cell surface antigen receptor. Such immunogenic portions generally comprise at least 5 amino acid residues, more preferably at least 10, and still more preferably at least 20 amino acid residues of an ovarian carcinoma protein or a variant thereof. Preferred immunogenic portions are encoded by cDNA molecules isolated as described herein. Further immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with ovarian carcinoma protein-specific antibodies, antisera and/or T-cell lines or clones. As used herein, antisera and antibodies are "ovarian carcinoma protein-specific" if they specifically bind to an ovarian carcinoma protein (*i.e.*, they react with the ovarian carcinoma protein in an ELISA or other immunoassay, and do not react detectably with unrelated proteins). Such antisera, antibodies and T cells may be prepared as described herein, and using well known techniques. An immunogenic portion of a native ovarian carcinoma protein is a portion that reacts with such antisera, antibodies and/or T-cells at a level that is not substantially less than the reactivity of the full length polypeptide (*e.g.,* in an ELISA and/or T-cell reactivity assay). Such immunogenic portions may react within such assays at a level that is similar to or greater than the reactivity of the full length protein. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native ovarian carcinoma protein. A polypeptide "variant," as used herein, is a polypeptide that differs from a native ovarian carcinoma protein in one or more substitutions, deletions, additions and/or insertions, such that the immunogenicity of the polypeptide is not substantially diminished. In other words, the ability of a variant to react with ovarian carcinoma protein-specific antisera may be enhanced or unchanged, relative to the native ovarian carcinoma protein, or may be diminished by less than 50%, and preferably less than 20%, relative to the native ovarian carcinoma protein. Such variants may generally be identified by modifying one of the above polypeptide sequences and evaluating the reactivity of the modified polypeptide with ovarian carcinoma protein-specific antibodies or antisera as described herein. Preferred variants include those in which one or more portions, such as an N-terminal leader sequence or transmembrane domain, have been removed. Other preferred variants include variants in which a small portion (*e.g.,* 1-30 amino acids, preferably 5-15 amino acids) has been removed from the N- and/or C-terminal of the mature protein.

Polypeptide variants preferably exhibit at least about 70%, more preferably at least about 90% and most preferably at least about 95% identity to the native polypeptide. Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide *(e.g.,* poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli*, yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Applied BioSystems, Inc. (Foster City, CA), and may be operated according to the manufacturer's instructions.

A polypeptide may be a fusion protein that comprises multiple polypeptides as described herein, or that comprises one polypeptide as described herein and a known tumor antigen, such as an ovarian carcinoma protein or a variant of such a protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also described that comprise a polypeptide as described together with an unrelated immunogenic protein. Preferably the immunogenic protein is capable of eliciting a recall response. Examples of such proteins include tetanus, tuberculosis and hepatitis proteins *(see,* for example, Stoute et al. New Engl. J. Med., 336:86-91, 1997).

An immunological fusion partner may be derived from protein D, a surface protein of the gram-negative bacterium Haemophilus influenza B (WO 91/18926). Preferably, a protein D derivative comprises approximately the first third of the protein (*e.g*., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E*. *coli* (thus functioning as an expression enhancer). The lipid tail ensures optimal presentation of the antigen to antigen present cells. Other fusion partners include the non-structural protein from influenzae virus, NS1 (hemaglutinin). Typically, the N-terminal 81 amino acids are used, although different fragments that include T-helper epitopes may be used.

The immunological fusion partner may be the protein known as LYTA, or a portion thereof (preferably a C-terminal portion). LYTA is derived from *Streptococcus pneumoniae*, which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E*. *coli* C-LYTA expressing plasmids useful for expression of fusion proteins. Purification of hybrid proteins containing the C-LYTA fragment at the amino terminus has been described (*see* Biotechnology 10:795-798, 1992). A repeat portion of LYTA may be incorporated into a fusion protein. A repeat portion is found in the C-terminal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### Binding Agents

The present disclosure further describes antibodies and antigen-binding fragments thereof, that specifically bind to an ovarian carcinoma protein. As used herein, an antibody, or antigen-binding fragment thereof, is said to "specifically bind" to an ovarian carcinoma protein if it reacts at a detectable level (within, for example, an ELISA) with an ovarian carcinoma protein, and does not react detectably with unrelated proteins under similar conditions. As used herein, "binding" refers to a noncovalent association between two separate molecules such that a "complex" is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant maybe determined using methods well known in the art.

Binding agents may be further capable of differentiating between patients with and without a cancer, such as ovarian cancer, using the representative assays provided herein. In other words, antibodies or other binding agents that bind to a ovarian carcinoma antigen will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (*e.g*., blood, sera, leukophoresis, urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.*, Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g*., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹ At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e.g*., covalently bonded) to a suitable monoclonal antibody either directly or indirectly (*e.g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g*., U.S. Patent No. 4,671,958, to Rodwell et al.

Where a therapeutic agent is more potent when free from the antibody portion of the immunoconjugates as described, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g.,* U.S. Patent No. 4,489,710, to Spitler), by irradiation of a photolabile bond *(e.g.,* U.S. Patent No. 4,625,014, to Senter et al.), by hydrolysis of derivatized amino acid side chains *(e.g.,* U.S. Patent No. 4,638,045, to Kohn et al.), by serum complement-mediated hydrolysis (*e.g*., U.S. Patent No. 4,671,958, to Rodwell et al.), and acid-catalyzed hydrolysis (*e.g*., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g.,* U.S. Patent No. 4,507,234, to Kato et al.), peptides and polysaccharides such as aminodextran *(e.g.,* U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Also described herein are anti-idiotypic antibodies that mimic an immunogenic portion of an ovarian carcinoma protein. Such antibodies may be raised against an antibody, or antigen-binding fragment thereof, that specifically binds to an immunogenic portion of an ovarian carcinoma protein, using well known techniques. Anti-idiotypic antibodies that mimic an immunogenic portion of an ovarian carcinoma protein are those antibodies that bind to an antibody, or antigen-binding fragment thereof, that specifically binds to an immunogenic portion of an ovarian carcinoma protein, as described herein.

### T Cells

Immunotherapeutic compositions may also, or alternatively, comprise T cells specific for an ovarian carcinoma protein. Such cells may generally be prepared in *vitro* or *ex vivo*, using standard procedures. For example, T cells may be present within (or isolated from) bone marrow, peripheral blood or a fraction of bone marrow or peripheral blood of a mammal, such as a patient, using a commercially available cell separation system, such as the CEPRATE^{™} system, available from CellPro Inc., Bothell WA (see also U.S. Patent No. 5,240,856; U.S. Patent No. 5,215,926; WO 89/06280; WO 91/16116 and WO 92/07243). Alternatively, T cells may be derived from related or unrelated humans, non-human animals, cell lines or cultures.

T cells may be stimulated with an ovarian carcinoma polypeptides, polynucleotide encoding an ovarian carcinoma polypeptide and/or an antigen presenting cell (APC) that expresses such a polypeptide. Such stimulation is performed under conditions and for a time sufficient to permit the generation of T cells that are specific for the polypeptide. Preferably, an ovarian carcinoma polypeptide or polynucleotide is present within a delivery vehicle, such as a microsphere, to facilitate the generation of specific T cells.

T cells are considered to be specific for an ovarian carcinoma polypeptide if the T cells kill target cells coated with an ovarian carcinoma polypeptide or expressing a gene encoding such a polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e.g*., by pulse-labeling cultures of T cells with tritiated thymidine and measuring the amount of tritiated thymidine incorporated into DNA). Contact with an ovarian carcinoma polypeptide (200 ng/ml - 100 µg/ml, preferably 100 ng/ml - 25 µl/ml) for 3 - 7 days should result in at least a two fold increase in proliferation of the T cells and/or contact as described above for 2-3 hours should result in activation of the T cells, as measured using standard cytokine assays in which a two fold increase in the level of cytokine release *(e.g.,* TNF or IFN-γ) is indicative of T cell activation (*see* Coligan et al., Current Protocols in Immunology, vol. 1, Wiley Interscience (Greene 1998). T cells that have been activated in response to an ovarian carcinoma polypeptide, polynucleotide or ovarian carcinoma polypeptide-expressing APC may be CD4⁺ and/or CD8⁺. Ovarian carcinoma polypeptide-specific T cells may be expanded using standard techniques. Within preferred embodiments, the T cells are derived from a patient or a related or unrelated donor and are administered to the patient following stimulation and expansion.

For therapeutic purposes, CD4⁺ or CD8⁺ T cells that proliferate in response to an ovarian carcinoma polypeptide, polynucleotide or APC can be expanded in number either *in vitro* or *in vivo.* Proliferation of such T cells *in vitro* may be accomplished in a variety of ways. For example, the T cells can be re-exposed to an ovarian carcinoma polypeptide, with or without the addition of T cell growth factors, such as interleukin-2, and/or stimulator cells that synthesize an ovarian carcinoma polypeptide. Alternatively, one or more T cells that proliferate in the presence of an ovarian carcinoma polypeptide can be expanded in number by cloning. Methods for cloning cells are well known in the art, and include limiting dilution. Following expansion, the cells may be administered back to the patient as described, for example, by Chang et al., Crit. Rev. Oncol. Hematol. 22:213, 1996.

### Pharmaceutical Compositions and Vaccines

Within certain aspects, polypeptides, polynucleotides, binding agents and/or immune system cells as described herein may be incorporated into pharmaceutical compositions or vaccines. Pharmaceutical compositions comprise one or more such compounds or cells and a physiologically acceptable carrier. Vaccines may comprise one or more such compounds or cells and a non-specific immune response enhancer. A non-specific immune response enhancer may be any substance that enhances an immune response to an exogenous antigen. Examples of non-specific immune response enhancers include adjuvants, biodegradable microspheres (*e.g*., polylactic galactide) and liposomes (into which the compound is incorporated; *see e.g.*, Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines as described may also contain other compounds, which may be biologically active or inactive. For example, one or more immunogenic portions of other tumor antigens may be present, either incorporated into a fusion polypeptide or as a separate compound within the composition or vaccine.

A pharmaceutical composition or vaccine may contain DNA encoding one or more of the polypeptides as described above, such that the polypeptide is generated *in situ*. As noted above, the DNA may be present within any of a variety of delivery systems known to those of ordinary skill in the art, including nucleic acid expression systems, bacteria and viral expression systems. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface. In a preferred embodiment, the DNA may be introduced using a viral expression system (*e.g.*, vaccinia or other pox virus, retrovirus, or adenovirus), which may involve the use of a non-pathogenic (defective), replication competent virus. Suitable systems are disclosed, for example, in Fisher-Hoch et al., PNAS 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.*, polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g*., neutral buffered saline or phosphate buffered saline), carbohydrates (*e.g*., glucose, mannose, sucrose or dextrans), mannitol, proteins, polypeptides or amino acids such as glycine, antioxidants, chelating agents such as EDTA or glutathione, adjuvants (*e.g*., aluminum hydroxide) and/or preservatives. Alternatively, compositions of the present invention may be formulated as a lyophilizate. Compounds may also be encapsulated within liposomes using well known technology.

Any of a variety of non-specific immune response enhancers may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI), Merck Adjuvant 65 (Merck and Company, Inc., Rahway, NJ), alum, biodegradable microspheres, monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (*e.g*., IFN-γ, IL-2 and IL-12) tend to favor the induction of cell mediated immune responses to an administered antigen. In contrast, high levels of Th2-type cytokines (*e.g.,* IL-4, IL-5, IL-6, IL-10 and TNF-β) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT; *see* US Patent Nos. 4,436,727; 4,877,611; 4,866,034 and 4,912,094). Also preferred is AS-2 (SmithKline Beecham). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation *(i.e.,* a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i.e.*, matched HLA haplotype). APCs may generally be isolated from any of a variety of biological fluids and organs, including tumor and peritumoral tissues, and may be autologous, allogeneic, syngeneic or xenogeneic cells.

Dendritic cells or progenitors thereof may be used as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity *(see* Timmerman and Levy, *Ann. Rev. Med*. *50*:507-529,1999). In general, dendritic cells may be identified based on their typical shape (stellate *in situ*, with marked cytoplasmic processes (dendrites) visible *in vitro*) and based on the lack of differentiation markers of B cells (CD19 and CD20), T cells (CD3), monocytes (CD14) and natural killer cells (CD56), as determined using standard assays. Dendritic cells may, of course, be engineered to express specific cell-surface receptors or ligands that are not commonly found on dendritic cells *in vivo* or *ex vivo*, and such modified dendritic cells are contemplated by the present disclosure. As an alternative to dendritic cells, secreted vesicles antigen-loaded dendritic cells (called exosomes) may be used within a vaccine *(see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, I-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor, mannose receptor and DEC-205 marker. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g*., CD40, CD80 and CD86).

APCs may generally be transfected with a polynucleotide encoding a ovarian carcinoma antigen (or portion or other variant thereof) such that the antigen, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the polypeptide, DNA (naked or within a plasmid vector) or RNA; or with antigen-expressing recombinant bacterium or viruses (*e.g.*, vaccinia, fowlpox, adenovirus or lentivirus vectors). Prior to loading, the polypeptide may be covalently conjugated to an immunological partner that provides T cell help (*e.g.,* a carrier molecule). Alternatively, a dendritic cell may be pulsed with a non-conjugated immunological partner, separately or in the presence of the polypeptide.

### Cancer Therapy

In further aspects of the present invention the compositions described herein may be used for immunotherapy of cancer, such as ovarian cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. Within certain preferred embodiments, a patient is afflicted with ovarian cancer. Such cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immuno response-modifying agents (such as tumor vaccines, bacterial adjuvants and/or cytokines).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (*see,* for example, Cheever et al., Immunological Reviews 157:177, 1997).

Alternatively, a vector expressing a polypeptide recited herein may be introduced into stem cells taken from a patient and clonally propagated *in vitro* for autologous transplant back into the same patient.

Routes and frequency of administration, as well as dosage, will vary from individual to individual, and may be readily established using standard techniques. In general, the pharmaceutical compositions and vaccines may be administered by injection (*e.g.*, intracutaneous, intramuscular, intravenous or subcutaneous), intranasally (*e.g.*, by aspiration), orally or in the bed of a resected tumor. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e.*, untreated) level.. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g.*, more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 100 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g.*, more frequent remissions, complete or partial, or longer disease-free survival) in treated patients as compared to non-treated patients. Increases in preexisting immune responses to an ovarian carcinoma antigen generally correlate with an improved clinical outcome. Such immune responses may generally be evaluated using standard proliferation, cytotoxicity or cytokine assays, which may be performed using samples obtained from a patient before and after treatment.

### Screens for Identifying Secreted Ovarian Carcinoma Antigens

Methods for identifying secreted tumor antigens are described. Within such methods, tumors are implanted into immunodeficient animals such as SCID mice and maintained for a time sufficient to permit secretion of tumor antigens into serum. In general, tumors may be implanted subcutaneously or within the gonadal fat pad of an immunodeficient animal and maintained for 1-9 months, preferably 1-4 months. Implantation may generally be performed as described in WO 97/18300. The serum containing secreted antigens is then used to prepare antisera in immunocompetent mice, using standard techniques and as described herein. Briefly, 50-100 µL of sera (pooled from three sets of immunodeficient mice, each set bearing a different SCID-derived human ovarian tumor) may be mixed 1:1 (vol:vol) with an appropriate adjuvant, such as RIBI-MPL or MPL + TDM (Sigma Chemical Co., St. Louis, MO) and injected intraperitoneally into syngeneic immunocompetent animals at monthly intervals for a total of 5 months. Antisera from animals immunized in such a manner may be obtained by drawing blood after the third, fourth and fifth immunizations. The resulting antiserum is generally pre-cleared of *E. coli* and phage antigens and used (generally following dilution, such as 1:200) in a serological expression screen.

The library is typically an expression library containing cDNAs from one or more tumors of the type that was implanted into SCID mice. This expression library may be prepared in any suitable vector, such as λ-screen (Novagen). cDNAs that encode a polypeptide that reacts with the antiserum may be identified using standard techniques, and sequenced. Such cDNA molecules may be further characterized to evaluate expression in tumor and normal tissue, and to evaluate antigen secretion in patients.

The methods provided herein have advantages over other methods for tumor antigen discovery. In particular, all antigens identified by such methods should be secreted or released through necrosis of the tumor cells. Such antigens may be present on the surface of tumor cells for an amount of time sufficient to permit targeting and killing by the immune system, following vaccination.

### Methods for Detecting Cancer

In general, a cancer may be detected in a patient based on the presence of one or more ovarian carcinoma proteins and/or polynucleotides encoding such proteins in a biological sample (such as blood, sera, urine and/or tumor biopsies) obtained from the patient. In other words, such proteins may be used as markers to indicate the presence or absence of a cancer such as ovarian cancer. In addition, such proteins may be useful for the detection of other cancers. The binding agents provided herein generally permit detection of the level of protein that binds to the agent in the biological sample. Polynucleotide primers and probes may be used to detect the level of mRNA encoding a tumor protein, which is also indicative of the presence or absence of a cancer. In general, an ovarian carcinoma-associated sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

The assay may involve the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length ovarian carcinoma proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present disclosure, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g*., Pierce Immunotechnology Catalog and Handbook, 1991, at A12-A13).

The assay may be a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.*, incubation time) is a period of time that is sufficient to detect the presence of polypeptide within a sample obtained from an individual with ovarian cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20^{™}. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as ovarian cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i.e.*, sensitivity) and false positive rates (100%-specificity) that correspond to each possible cut-off value for the diagnostic test result. The cut-off value on the plot that is the closest to the upper left-hand corner (*i.e.*, the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

The assay may be performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use ovarian carcinoma polypeptides to detect antibodies that bind to such polypeptides in a biological sample. The detection of such ovarian carcinoma protein specific antibodies may correlate with the presence of a cancer.

A cancer may also, or alternatively, be detected based on the presence of T cells that specifically react with an ovarian carcinoma protein in a biological sample. Within certain methods, a biological sample comprising CD4⁺ and/or CD8⁺ T cells isolated from a patient is incubated with an ovarian carcinoma protein, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with an ovarian carcinoma protein (*e.g.*, 5 - 25 µg/ml). It may be desirable to incubate another aliquot of a T cell sample in the absence of ovarian carcinoma protein to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding an ovarian carcinoma protein in a biological sample. For example, at least two oligonucleotide primers may be employed in a polymerase chain reaction (PCR) based assay to amplify a portion of an ovarian carcinoma protein cDNA derived from a biological sample, wherein at least one of the oligonucleotide primers is specific for (*i.e.*, hybridizes to) a polynucleotide encoding the ovarian carcinoma protein. The amplified cDNA is then separated and detected using techniques well known in the art, such as gel electrophoresis. Similarly, oligonucleotide probes that specifically hybridize to a polynucleotide encoding an ovarian carcinoma protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding an ovarian carcinoma protein that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. The oligonucleotide primers comprise at least 10 contiguous nucleotides, more preferably at least 15 contiguous nucleotides, of a DNA molecule having a sequence described herein. Techniques for both PCR based assays and hybridization assays are well known in the art (*see,* for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample such as a biopsy tissue and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

Ovarian carcinoma proteins and polynucleotides encoding such proteins may be used as markers for monitoring the progression of cancer. Assays as described above for the diagnosis of a cancer may be performed over time, and the change in the level of reactive polypeptide(s) evaluated. For example, the assays may be performed every 24-72 hours for a period of 6 months to 1 year, and thereafter performed as needed. In general, a cancer is progressing in those patients in whom the level of polypeptide detected by the binding agent increases over time. In contrast, the cancer is not progressing when the level of reactive polypeptide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple ovarian carcinoma protein markers may be assayed within a given sample. It will be apparent that binding agents specific for different proteins described herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### Diagnostic Kits

The present invention further describes kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to an ovarian carcinoma protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding an ovarian carcinoma protein in a biological sample. Such kits generally comprise at least one oligonucleotide probe or primer, as described above, that hybridizes to a polynucleotide encoding an ovarian carcinoma protein. Such an oligonucleotide may be used, for example, within a PCR or hybridization assay. Additional components that may be present within such kits include a second oligonucleotide and/or a diagnostic reagent or container to facilitate the detection of a polynucleotide encoding an ovarian carcinoma protein.

The following Examples are offered by way of illustration and not by way of limitation.

### EXAMPLES

### EXAMPLE 1 (for illustration only)

### IDENTIFICATION OF REPRESENTATIVE OVARIAN CARCINOMA PROTEIN cDNAs

This Example illustrates the identification of cDNA molecules encoding ovarian carcinoma proteins.

Anti-SCID mouse sera (generated against sera from SCID mice carrying late passage ovarian carcinoma) was pre-cleared of E. coli and phage antigens and used at a 1:200 dilution in a serological expression screen. The library screened was made from a SCID-derived human ovarian tumor (OV9334) using a directional RH oligo(dT) priming cDNA library construction kit and the λScreen vector (Novagen). A bacteriophage lambda screen was employed. Approximately 400,000 pfu of the amplified OV9334 library were screened.

196 positive clones were isolated. Certain sequences that appear to be novel are provided in Figures 1A-1S and SEQ ID NO:1 to 71. Three complete insert sequences are shown in Figures 2A-2C (SEQ ID NO:72 to 74). Other clones having known sequences are presented in Figures 15A-15EEE (SEQ ID NO:82 to 310). Database searches identified the following sequences that were substantially identical to the sequences presented in Figures 15A-15EEE.

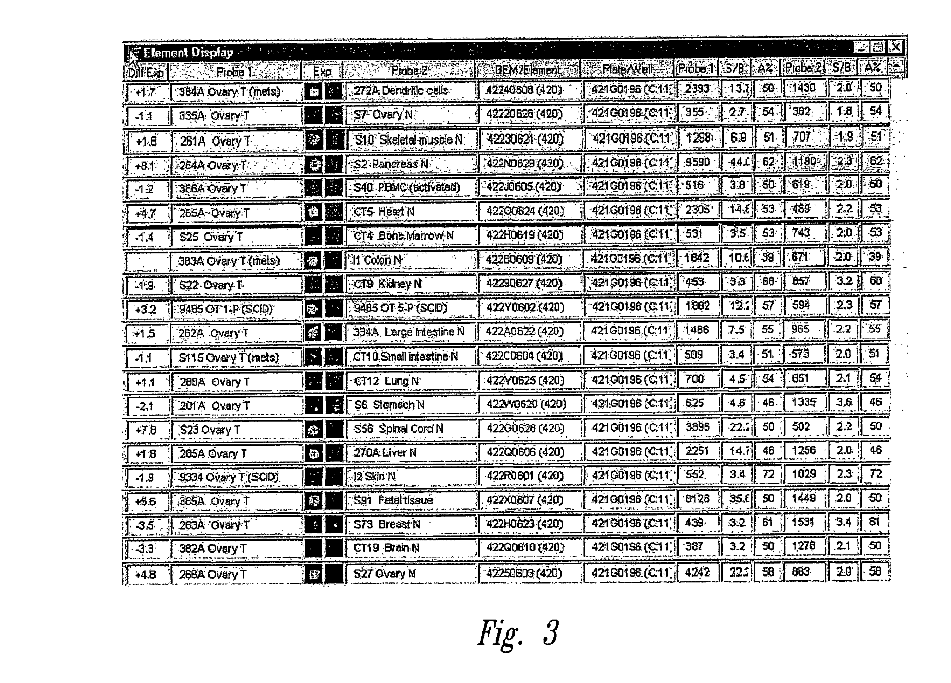

These clones were further characterized using microarray technology to determine mRNA expression levels in a variety of tumor and normal tissues. Such analyses were performed using a Synteni (Palo Alto, CA) microarray, according to the manufacturer's instructions. PCR amplification products were arrayed on slides, with each product occupying a unique location in the array. mRNA was extracted from the tissue sample to be tested, reverse transcribed and fluorescent-labeled cDNA probes were generated. The microarrays were probed with the labeled cDNA probes and the slides were scanned to measure fluorescence intensity. Data was analyzed using Synteni's provided GEMtools software. The results for one clone (13695, also referred to as O8E are shown in Figure 3.

### EXAMPLE 2 (For illustration only)

### IDENTIFICATION OF OVARIAN CARCINOMA cDNAs USING MICROARRAY TECHNOLOGY

This Example illustrates the identification of ovarian carcinoma polynucleotides by PCR subtraction and microarray analysis. Microarrays of cDNAs were analyzed for ovarian tumor-specific expression using a Synteni (Palo Alto, CA) microarray, according to the manufacturer's instructions (and essentially as described by Schena et al., Proc. Natl. Acad. Sci. USA 93:10614-10619; 1996 and Heller et al., Proc. Natl. Acad Sci. USA 94:2150-2155, 1997).

A PCR subtraction was performed using a tester comprising cDNA of four ovarian tumors (three of which were metastatic tumors) and a driver of cDNA form five normal tissues (adrenal gland, lung, pancreas, spleen and brain). cDNA fragments recovered from this subtraction were subjected to DNA microarray analysis where the fragments were PCR amplified, adhered to chips and hybridized with fluorescently labeled probes derived from mRNAs of human ovarian tumors and a variety of normal human tissues. In this analysis, the slides were scanned and the fluorescence intensity was measured, and the data were analyzed using Synteni's GEMtools software. In general, sequences showing at least a 5-fold increase in expression in tumor cells (relative to normal cells) were considered ovarian tumor antigens. The fluorescent results were analyzed and clones that displayed increased expression in ovarian tumors were further characterized by DNA sequencing and database searches to determine the novelty of the sequences.

Using such assays, an ovarian tumor antigen was identified that is a splice fusion between the human T-cell leukemia virus type I oncoprotein TAX (*see* Jin et al., Cell 93:81-91, 1998) and an extracellular matrix protein called osteonectin. A splice junction sequence exists at the fusion point. The sequence of this clone is presented in Figure 4 and SEQ ID NO:75. Osteonectin, unspliced and unaltered, was also identified from such assays independently.

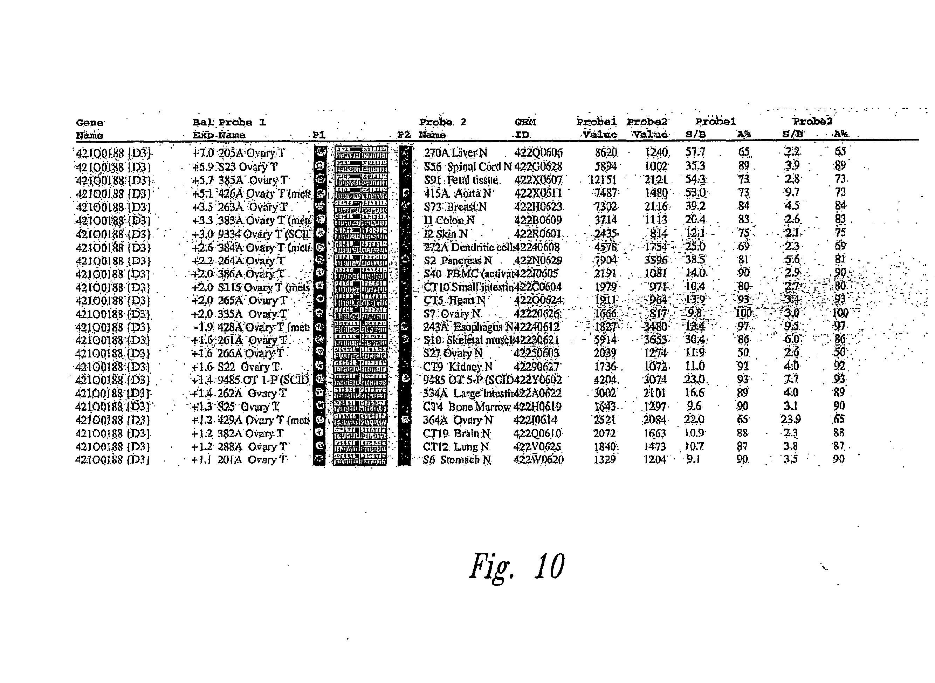

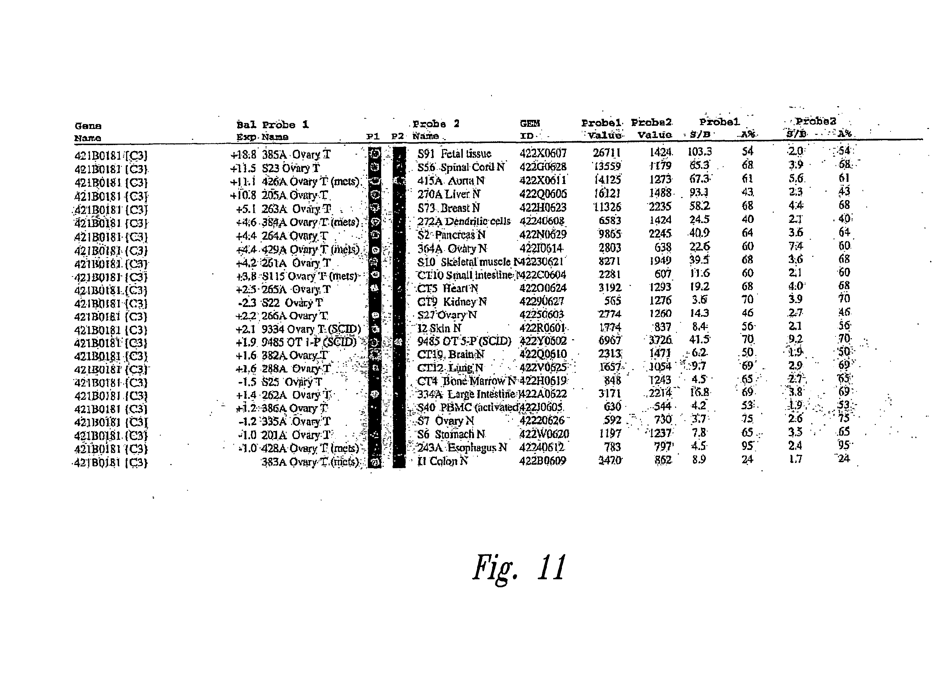

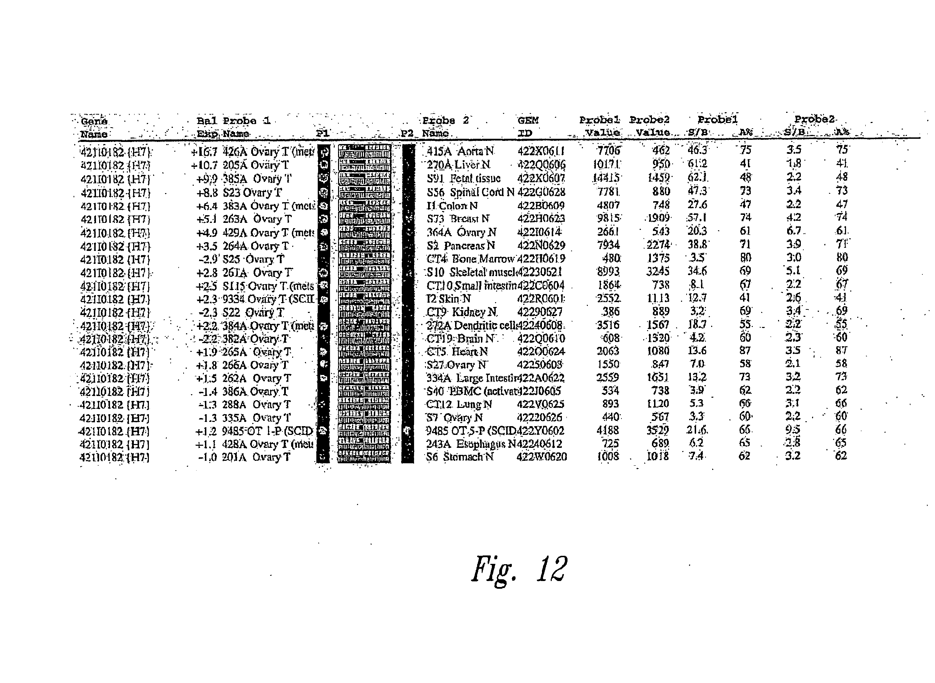

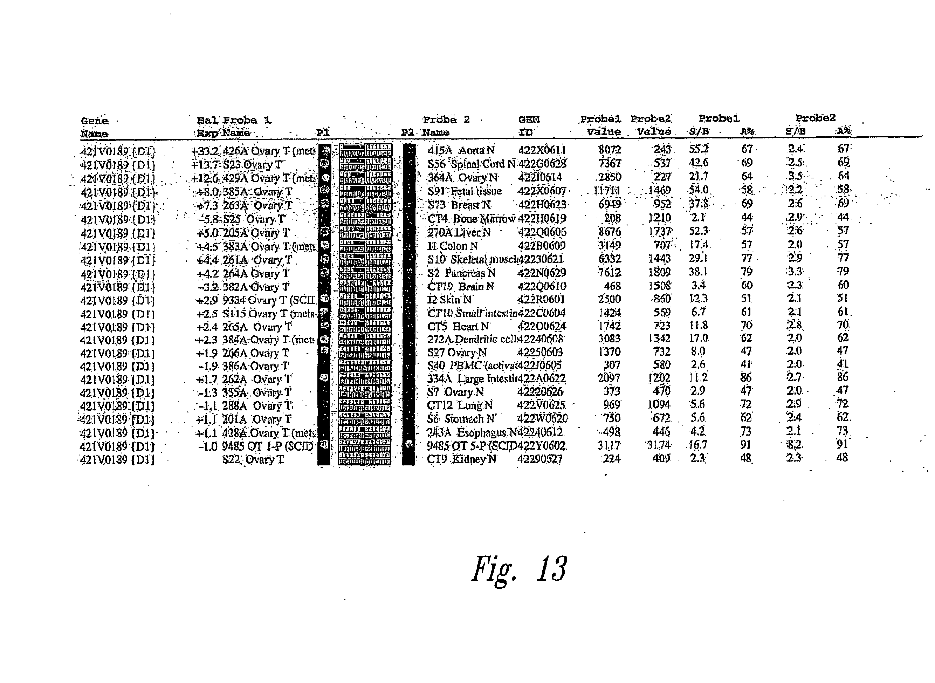

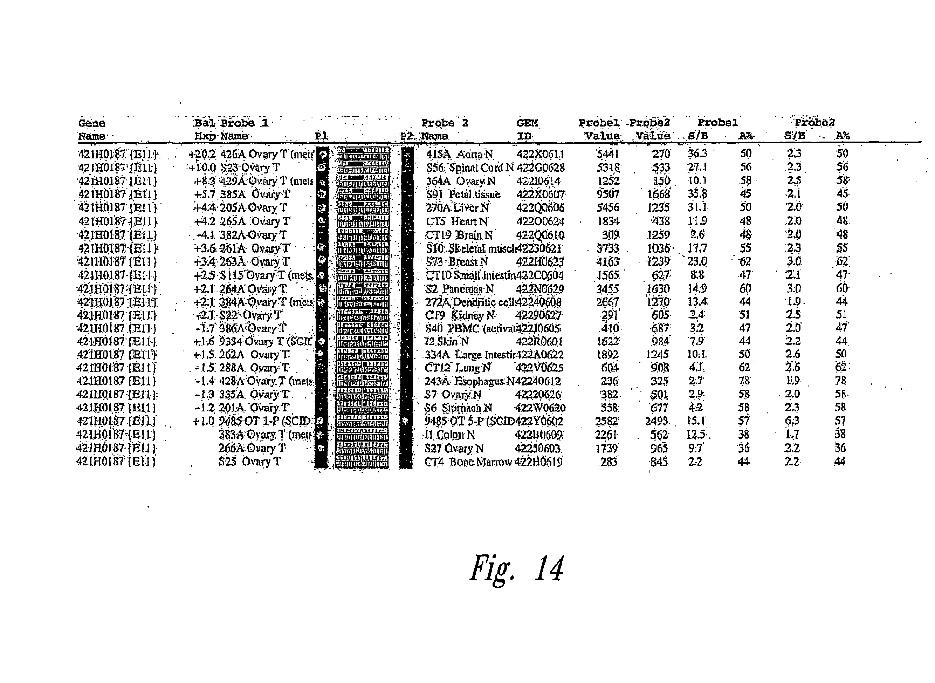

Further clones identified by this method are referred to herein as 3f, 6b, 8e, 8h, 12c and 12h. Sequences of these clones are shown in Figures 5 to 9 and SEQ ID NO:76 to 81. Microarray analyses were performed as described above, and are presented in Figures 10 to 14. A full length sequence encompassing clones 3f, 6b, 8e and 12h was obtained by screening an ovarian tumor (SCID-derived) cDNA library analysis indicates a Type 1a transmembrane protein localized to the plasma membrane.

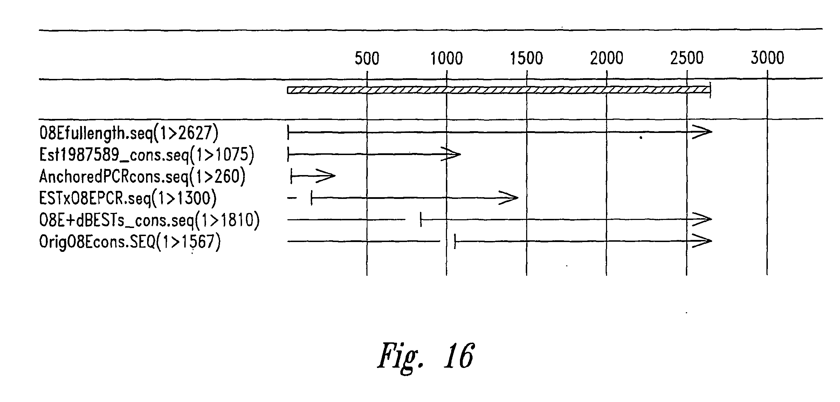

Additional sequence analysis resulted in a full length clone for O8E (2627 bp, which agrees with the message size observed by Northern analysis; SEQ ID NO:391). This nucleotide sequence was obtained as follows: the original O8E sequence (OrigO8Econs) was found to overlap by 33 nucleotides with a sequence from an EST clone (IMAGE#1987589). This clone provided 1042 additional nucleotides upstream of the original O8E sequence. The link between the EST and O8E was confirmed by sequencing multiple PCR fragments generated from an ovary primary tumor library using primers to the unique EST and the O8E sequence (ESTxO8EPCR). Full length status was further indicated when anchored PCR from the ovary tumor library gave several clones (AnchoredPCR cons) that all terminated upstream of the putative start methionine, but failed to yield any additional sequence information. Figure 16 presents a diagram that illustrates the location of each partial sequence within the full length O8E sequence.

Two protein sequences may be translated from the full length O8E. For "a" (SEQ ID NO:393) begins with a putative start methionine. A second form "b" (SEQ ID NO:392) includes 27 additional upstream residues to the 5' end of the nucleotide sequence.

### EXAMPLE 3

This example discloses the identification and characterization of antibody epitopes recognized by the O8E polyclonal anti-sera.

Rabbit anti-sera was raised against E. coli derived O8E recombinant protein and tested for antibody epitope recognition against 20 or 21 mer peptides that correspond to the O8E amino acid sequence. Peptides spanning amino acid regions 31 to 65, 76 to 110, 136 to 200 and 226 to 245 of the full length O8E protein were recognized by an acid eluted peak and/or a salt eluted peak from affinity purified anti-O8E sera. Thus, the corresponding amino acid sequences of the above peptides constitute the antibody epitopes recognized by affinity purified anti-O8E antibodies.

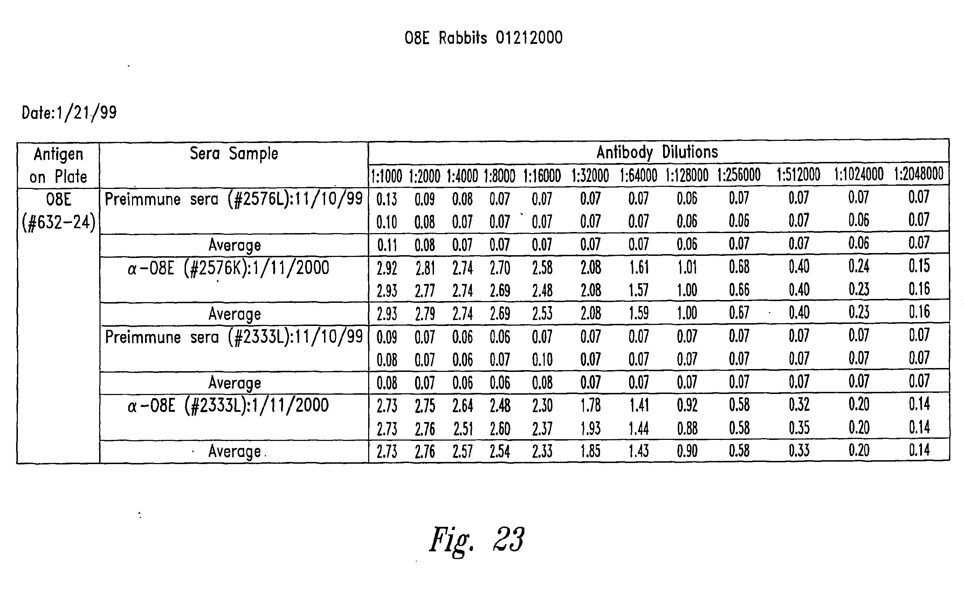

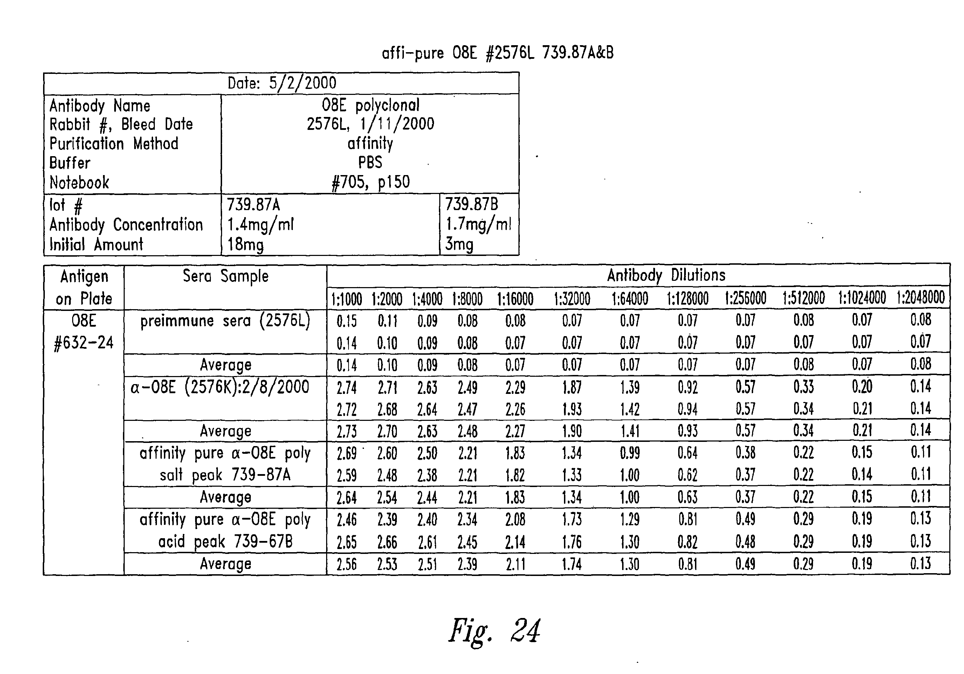

ELISA analysis of anti-08E rabbit sera is shown in Figure 23, and ELISA analysis of affinity purified rabbit anti-08E polyclonal antibody is shown in Figure 24.

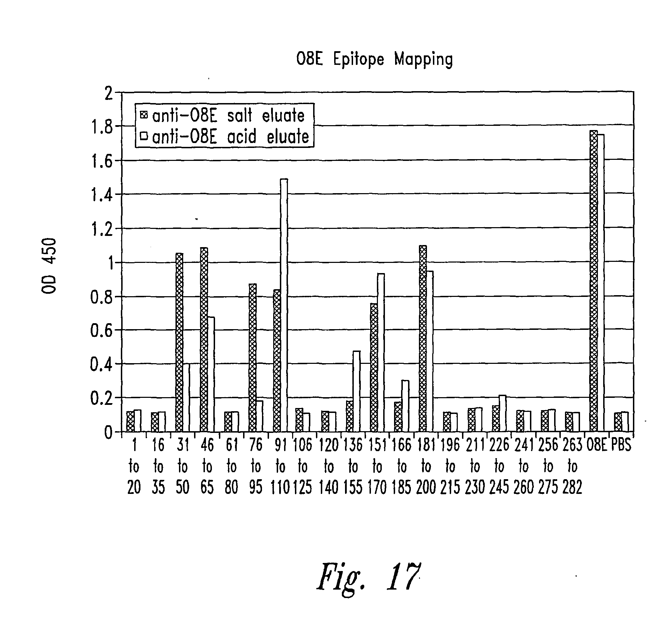

For epitope mapping, 20 or 21 mer peptides corresponding to the O8E protein were synthesized. For antibody affinity purification, rabbit anti-O8E sera was run over an O8E-sepharose column, then antibody was eluted with a salt buffer containing 0.5 M NaCl and 20 mM PO₄, followed by an acid elution step using 0.2 M Glycine, pH 2.3. Purified antibody was neutralized by the addition of 1M Tris, pH 8 and buffer exchanged into phosphate buffered saline (PBS). For enzyme linked immunosorbant assay (ELISA) analysis, O8E peptides and O8E recombinant protein were coated onto 96 well flat bottom plates at 2 µg/ml for 2 hours at room temperature (RT). Plates were then washed 5 times with PBS + 0.1 % Tween 20 and blocked with PBS + 1 % bovine serum albumin (BSA) for 1 hour. Affinity purified anti-O8E antibody, either an acid or salt eluted fraction, was then added to the wells at 1 µg/ml and incubated at RT for 1 hr. Plates were again washed, followed by the addition of donkey anti-rabbit-Ig-horseradish peroxidase (HRP) antibody for 1 hour at RT. Plates were washed, then developed by the addition of the chromagenic substrate 3, 3', 5, 5'-tetramethylbenzidine (TMB) (described by Bos et al., J. of Immunoassay 2:187-204 (1981); available from Sigma (St. Louis, MO)). The reaction was incubated 15 minutes at RT and then stopped by the addition of 1 N H₂SO₄. Plates were read at an optical density of 450 (OD450) in an automated plate reader. The sequences of peptides corresponding to the OE8 antibody epitopes are disclosed herein as SEQ ID NO: 394-415. Antibody epitopes recognized by the O8E polyclonal anti-sera are disclosed herein in Figure 17.

### EXAMPLE 4 (for illustration only)

This example discloses IHC analysis of O8E expression in ovarian cancer tissue samples.

For immunohistochemistry studies, paraffin-embedded formalin fixed ovarian cancer tissue was sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody (anti-O8E rabbit affinity purified polyclonal antibody) was added to each section for 25 min followed by a 25 min incubation with an anti-rabbit biotinylated antibody. Endogenous peroxidase activity was blocked by three 1.5 min incubations with hydrogen peroxidase. The avidin biotin complex/horse radish peroxidase system was used along with DAB chromogen to visualize antigen expression. Slides were counterstained with hematoxylin. One (papillary serous carcinoma) of six ovarian cancer tissue sections displayed O8E immunoreactivity. Upon optimization of the staining conditions, 4/5 ovarian cancer samples stained positive using the O8E polyclonal antibody. O8E expression was localized to the plasma membrane.

Six ovarian cancer tissues were analyzed with the anti-O8E rabbit polyclonal antibody. One (papillary serous carcinoma) of six ovarian cancer tissue samples stained positive for O8E expression. O8E expression was localized to the surface membrane.

### EXAMPLE 5 (for illustration only)

This example discloses O8E peptides that are predicted to bind HLA-A2 and to be immunogenic for CD8 T cell responses in humans.

Potential HLA-A2 binding peptides of O8E were predicted by using the full-length open-reading frame (ORF) from O8E and running it through "Episeek," a program used to predict MHC binding peptides. The program used is based on the algorithm published by Parker, K.C. et al., J. Immunol. 152(1):163-175 (1994) (incorporated by reference herein in its entirety). 10-mer and 9-mer peptides predicted to bind HLA-0201 are disclosed herein as SEQ ID NO: 416-435 and SEQ ID NO: 436-455, respectively.

### EXAMPLE 6 (for illustration only)

This example discloses O8E cell surface expression measured by fluoresence activated cell sorting.

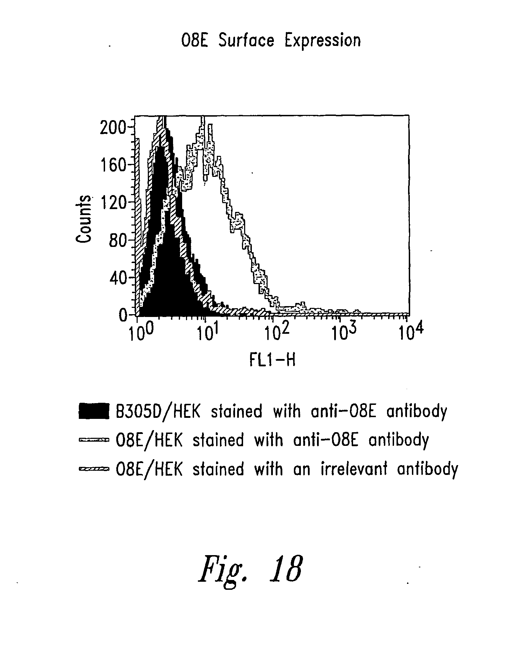

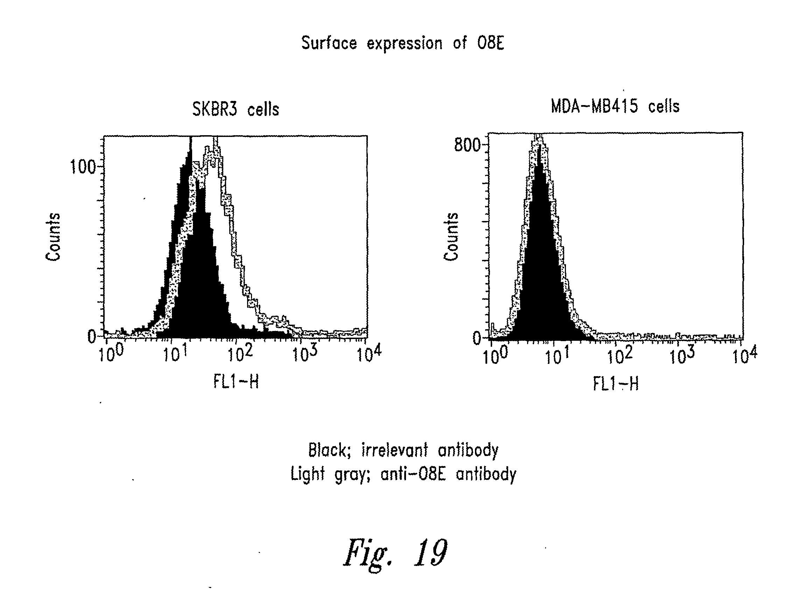

For FACS analysis, cells were washed with ice cold staining buffer (PBS/1% BSA/azide). Next, the cells were incubated for 30 minutes on ice with 10 micrograms/ml of affinity purified rabbit anti-B305D polyclonal antibody. The cells were washed 3 times with staining buffer and then incubated with a 1:100 dilution of a goat anti-rabbit Ig (H+L)-FITC reagent (Southern Biotechnology) for 30 minutes on ice. Following 3 washes, the cells were resuspended in staining buffer containing prodium iodide, a vital stain that allows for identification of permeable cells, and analyzed by FACS. O8E surface expression was confirmed on SKBR3 breast cancer cells and HEK293 cells that stably overexpress the cDNA for O8E. Neither MB415 cells nor HEK293 cells stably transfected with a control irrelevant plasmid DNA showed surface expression of O8E (Figures 18 and 19).

### EXAMPLE 7 (for illustration only)

This example further evaluates the expression and surface localization of O8E.

For expression and purification of antigen used for immunization, O8E expressed in an E. coli recombinant expression system was grown overnight in LB Broth with the appropriate antibiotics at 37°C in a shaking incubator. The next morning, 10 ml of the overnight culture was added to 500 ml of 2x YT plus appropriate antibiotics in a 2L-baffled Erlenmeyer flask. When the Optical Density (at 560 nanometers) of the culture reached 0.4-0.6 the cells were induced with IPTG (1 mM). 4 hours after induction with IPTG the cells were harvested by centrifugation. The cells were then washed with phosphate buffered saline and centrifuged again. The supernatant was discarded and the cells were either frozen for future use or immediately processed. Twenty milliliters of lysis buffer was added to the cell pellets and vortexed. To break open the E. coli cells, this mixture was then run through the French Press at a pressure of 16,000 psi. The cells were then centrifuged again and the supernatant and pellet were checked by SDS-PAGE for the partitioning of the recombinant protein. For protein that localized to the cell pellet, the pellet was resuspended in 10 mM Tris pH 8.0 , 1% CHAPS and the inclusion body pellet was washed and centrifuged again. This procedure was repeated twice more. The washed inclusion body pellet was solubilized with either 8 M urea or 6 M guanidine HCl containing 10 mM Tris pH 8.0 plus 10 mM imidazole. The solubilized protein was added to 5 ml of nickel-chelate resin (Qiagen) and incubated for 45 min to 1 hour at room temperature with continuous agitation. After incubation, the resin and protein mixture were poured through a disposable column and the flow through was collected. The column was then washed with 10-20 column volumes of the solubilization buffer. The antigen was then eluted from the column using 8M urea, 10 mM tris pH 8.0 and 300 mM imidazole and collected in 3 ml fractions. A SDS-PAGE gel was run to determine which fractions to pool for further purification. As a final purification step, a strong anion exchange resin such as Hi-Prep Q (Biorad) was equilibrated with the appropriate buffer and the pooled fractions from above were loaded onto the column. Each antigen was eluted off of the column with an increasing salt gradient. Fractions were collected as the column was run and another SDS-PAGE gel was run to determine which fractions from the column to pool. The pooled fractions were dialyzed against 10 mM Tris pH 8.0. This material was then evaluated for acceptable purity as determined by SDS-PAGE or HPLC, concentration as determined by Lowry assay or Amino Acid Analysis, identity as determined by amino terminal protein sequence, and endotoxin level as determined by the Limulus (LAL) assay. The proteins were then vialed after filtration through a 0.22 micron filter and the antigens were frozen until needed for immunization.

For generation of polyclonal anti-sera, 400 micrograms of each prostate antigen was combined with 100 micrograms of muramyldipeptide (MDP). Equal volume of Incomplete Freund's Adjuvant (IFA) was added and then mixed. Every four weeks animals were boosted with 100 micrograms of antigen mixed with an equal volume of IFA. Seven days following each boost the animal was bled. Sera was generated by incubating the blood at 4°C for 12-24 hours followed by centrifugation.

For characterization of polyclonal antisera, 96 well plates were coated with antigen by incubating with 50 microliters (typically 1 microgram) at 4°C for 20 hrs. 250 microliters of BSA blocking buffer was added to the wells and incubated at RT for 2 hrs. Plates were washed 6 times with PBS/0.01% tween. Anti-O8E rabbit sera or affinity purified anti-O8e antibody was diluted in PBS. Fifty microliters of diluted antibody was added to each well and incubated at RT for 30 min. Plates were washed as described above before 50 microliters of goat anti-rabbit horse radish peroxidase (HRP) at a 1:10000 dilution was added and incubated at RT for 30 min. Plates were washed as described above and 100 microliters of TMB microwell Peroxidase Substrate was added to each well. Following a 15 minute incubation in the dark at room temperature the colorimetric reaction was stopped with 100 microliters of 1N H2SO4 and read immediately at 450 nm. All polyclonal antibodies showed immunoreactivity to the O8E antigen.

For recombinant expression in mammalian HEK293 cells, full length O8E cDNA was subcloned into the mammalian expression vectors pcDNA3.1+ and pCEP4 (Invitrogen) which were modified to contain His and FLAG epitope tags, respectively. These constructs were transfected into HEK293 cells (ATCC) using Fugene 6 reagent (Roche). Briefly, HEK293 cells were plated at a density of 100,000 cells/ml in DMEM (Gibco) containing 10% FBS (Hyclone) and grown overnight. The following day, 2 ul of Fugene6 was added to 100 ul of DMEM containing no FBS and incubated for 15 minutes at room temperature. The Fugene6/DMEM mixture was then added to 1ug of O8E/pCEP4 or O8E/pcDNA3.1 plasmid DNA and incubated for 15 minutes at room temperature. The Fugene/DNA mix was then added to the HEK293 cells and incubated for 48-72 hrs at 37oC with 7% CO2. Cells were rinsed with PBS then collected and pelleted by centrifugation. For Western blot analysis, whole cell lysates were generated by incubating the cells in Triton-X100 containing lysis buffer for 30 minutes on ice. Lysates were then cleared by centrifugation at 10,000rpm for 5 minutes at 4 C. Samples were diluted with SDS-PAGE loading buffer containing betamercaptoethanol, then boiled for 10 minutes prior to loading the SDS-PAGE gel. Protein was transferred to nitrocellulose and probed using anti-O8E rabbit polyclonal sera #2333L at a dilution of 1:750. The blot was revealed with a goat anti-rabbit Ig coupled to HRP followed by incubation in ECL substrate.

For FACS analysis, cells were washed further with ice cold staining buffer (PBS+1%BSA+Azide). Next, the cells were incubated for 30 minutes on ice with 10ug/ml of Protein A purified anti-O8E polyclonal sera. The cells were washed 3 times with staining buffer and then incubated with a 1:100 dilution of a goat anti-rabbit Ig(H+L)-FITC reagent (Southern Biotechnology) for 30 minutes on ice. Following 3 washes, the cells were resuspended in staining buffer containing Propidium Iodide (PI), a vital stain that allows for the identification of permeable cells, and analyzed by FACS.

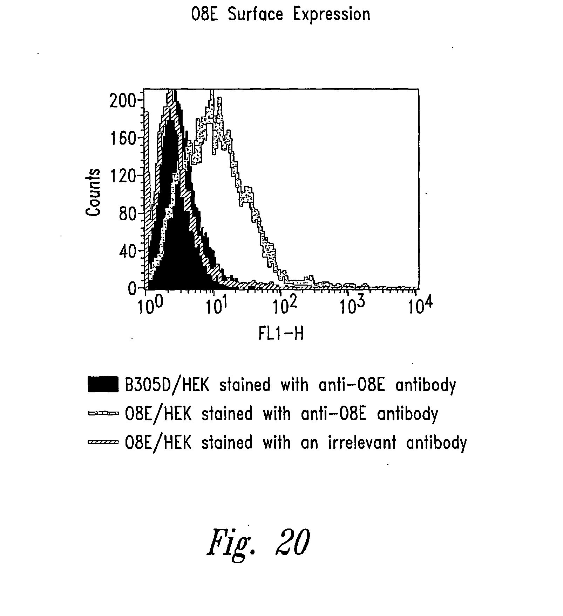

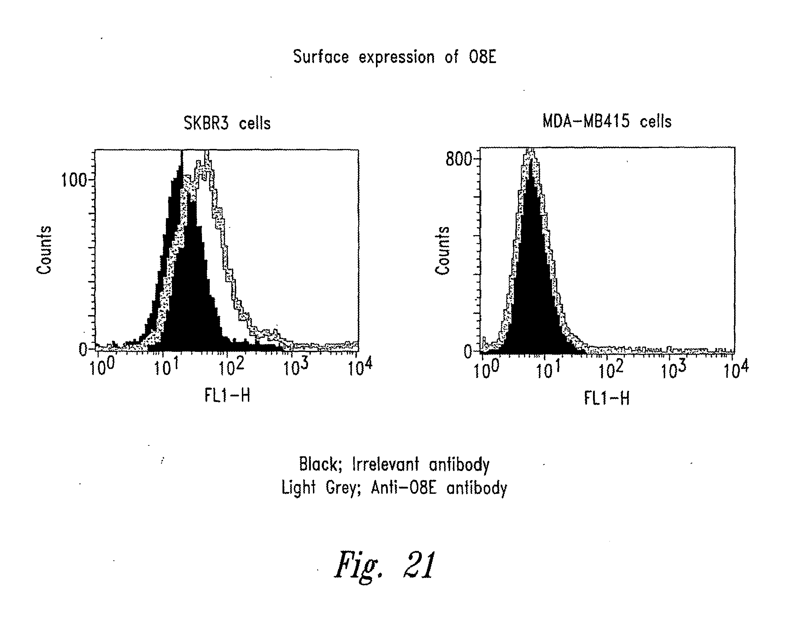

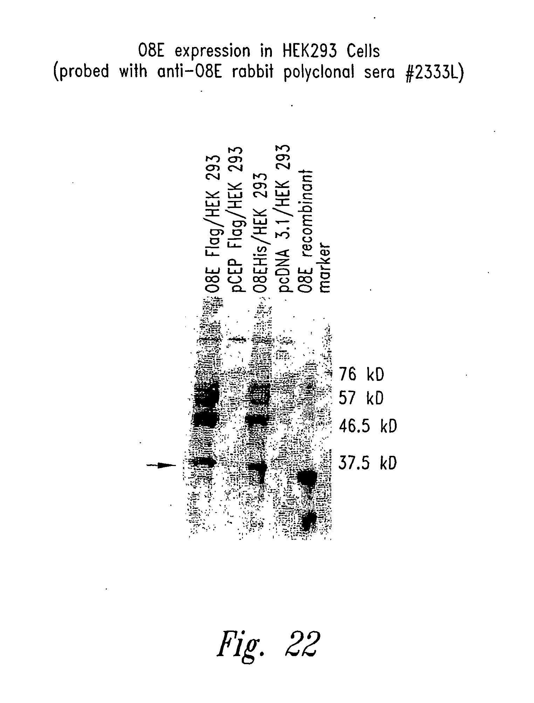

From these experiments, the results of which are illustrated in Figures 20-21, O8E expression was detected on the surface of transfected HEK293 cells and SKBR3 cells by FACS analysis using rabbit anti-O8E sera. Expression was also detected in transfected HEK293 cell lysates by Western blot analysis (Figure 22).

### EXAMPLE 8

### GENERATION AND CHARACTERIZATION OF ANTI-O8E MABS.

Mouse monoclonal antibodies were raised against E. coli derived O8E proteins as follows. A/J mice were immunized intraperitoneally (IP) with Complete Freund's Adjuvant (CFA) containing 50 µg recombinant O8E, followed by a subsequent IP boost with Incomplete Freund's Adjuvant (IFA) containing 10µg recombinant O8E protein. Three days prior to removal of the spleens, the mice were immunized intravenously with approximately 50µg of soluble O8E recombinant protein. The spleen of a mouse with a positive titer to O8E was removed, and a single-cell suspension made and used for fusion to SP2/0 myeloma cells to generate B cell hybridomas. The supernatants from the hybrid clones were tested by ELISA for specificity to recombinant O8E, and epitope mapped using peptides that spanned the entire O8E. sequence. The mAbs were also tested by flow cytometry for their ability to detect O8E on the surface of cells stably transfected with O8E and on the surface of a breast tumor cell line.

For ELISA analysis, 96 well plates were coated with either recombinant O8E protein or overlapping 20-mer peptides spanning the entire O8E molecule at a concentration of either 1-2µg/ml or 10µg/ml, respectively. After coating, the plates were washed 5 times with washing buffer (PBS + 0.1 % Tween-20) and blocked with PBS containing 0.5% BSA, 0.4% Tween-20. Hybrid supernatants or purified mAbs were then added and the plates incubated for 60 minutes at room temperature. The plates were washed 5 times with washing buffer and the secondary antibody, donkey-anti mouse Ig linked to horseradish peroxidase (HRP)(Jackson ImmunoResearch), was added for 60 minutes. The plates were again washed 5 times in washing buffer, followed by the addition of the peroxidase substrate. Of the hybridoma clones generated, 15 secreted mAbs that recognized the entire O8E protein. Epitope mapping revealed that of these 15 clones, 14 secreted mAbs that recognized the O8E amino acid residues 61-80 and one clone secreted a mAb that recognized amino acid residues 151-170.

For flow cytometric analysis, HEK293 cells which had been stably transfected with O8E and SKBR3 cells which express O8E mRNA, were harvested and washed in flow staining buffer (PBS+1%BSA+Azide). The cells were incubated with the supernatant from the mAb hybrids for 30 minutes on ice followed by 3 washes with staining buffer. The cells were incubated with goat-anti mouse Ig-FITC for 30 minutes on ice, followed by three washes with staining buffer before being resuspended in wash buffer containing propidium iodide. Flow cytometric analysis revealed that 15/15 mAbs were able to detect O8E protein expressed on the surface of O8E-transfected HEK293 cells. 6/6 mAbs tested on SKBR3 cells were able to recognize surface expressed O8E.

### EXAMPLE 12

### FUNCTIONAL CHARACTERIZATION OF ANTI-O8E MONOCLONAL ANTIBODIES

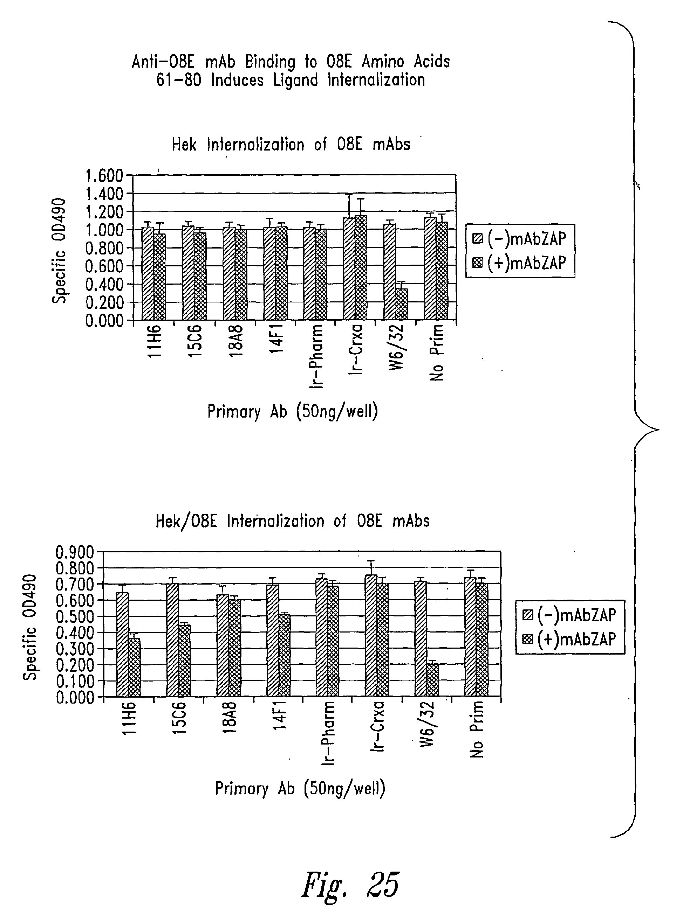

Mouse monoclonal antibodies (mAb) raised against E. coli derived O8E, as described in Example 8, were tested for their ability to promote O8E antigen internalization. Internalization of the antibody was determined using an in vitro cytotoxicity assay. Briefly, HEK293 and O8E/HEK transfected cells were plated into 96 well plates containing DME plus 10% heat-inactivated FBS in the presence of 50ng/well of purified anti-O8E or control antibodies. The isotype of the anti-O8E mAbs are as follows: 11A6-IgG1/kappa, 15C6-IgG2b/kappa, 18A8-IgG2b/kappa, and 14F1-IgG2a/kappa. W6/32 is a pan anti-human MHC class I mouse monoclonal antibody that serves as a positive control, and two irrelevant mAbs, Ir-Pharm and Ir-Crxa were included as negative controls. Following incubation with the O8E specific antibodies or the relevant controls antibodies, the mAb-zap, a goat anti-mouse Ig-saporin conjugated secondary antibody (Advanced Targeting Systems) was added at a concentration of 100ng/ml to half of the wells, and the plates were incubated for 48 to 72 hours at 37°C in a 7% CO₂ incubator. This assay takes advantage of the toxic nature of saporin, a ribozyme inactivating protein, which when internalized has a cytotoxic effect. Following incubation with the mAb-zap, internalization was quantitated by the addition of MTS reagent, followed by reading the OD490 of the plate on a microplate ELISA reader. Figure 25 depicts the results from these assays. The top panel represents HEK cells that have not been transfected with O8E and therefore O8E antibody should not bind and be internalized. Levels of proliferation were the same in all samples whether they were incubated with or without the mAb-zap, with the exception of the positive control Ab, W6/32. The lower panel represents cells that have been transfected with O8E and therefore should bind O8E specific antibodies. Antibodies from the hybridomas 11H6, 14F1, and 15C6, which recognize the amino acids 61-80 of O8E were able to promote internalization of the O8E surface protein as measured by decreased levels of proliferation due to the toxic nature of the mAb-zap (See Figure 25). The antibody generated by the hybridoma 18A8, which recognizes amino acids 151-170 of O8E, was unable to promote internalization as determined by normal levels of proliferation either in the absence or presence of the mAb-zap.

### CHARACTERIZATION OF THE OVARIAN TUMOR ANTIGEN, O772P

### EXAMPLE 14

### IMMUNOHISTOCHEMISTRY (IHC) ANALYSIS OF O8E EXPRESSION IN OVARIAN CANCER AND NORMAL TISSUES

In order to determine which tissues express the ovarian cancer antigen O8E, IHC analysis was performed on a diverse range of tissue sections using both polyclonal and monoclonal antibodies specific for O8E. The generation of O8E specific polyclonal antibodies is described in detail in Example 8. The monoclonal antibodies used for staining were 11A6 and 14F1, both of which are specific for amino acids 61-80 of O8E and 18A8, which recognizes amino acids 151-170 of O8E (see Example 12 for details on generation).

To perform staining, tissue samples were fixed in formalin solution for 12-24 hours and embedded in paraffin before being sliced into 8 micron sections. Steam heat induced epitope retrieval (SHEIR) in 0.1M sodium citrate buffer (pH 6.0) was used for optimal staining conditions. Sections were incubated with 10% serum/PBS for 5 minutes. Primary antibody was then added to each section for 25 minutes followed by 25 minutes of incubation with either anti-rabbit or anti-mouse biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize the antigen expression. Slides were counterstained with hematoxylin to visualize the cell nuclei.

Results using rabbit affinity purified polyclonal antibody to O8E (a.a. 29-283; for details on the generation of this Ab, see Example 3) are presented in Table 3. Results using the three monoclonal antibodies are presented in Table 4.

**Table 3**

| Immunohistochemistry analysis of O8E using polyclonal antibodies | |
|---|---|
| **Tissue** | **O8E Expression** |
| Ovarian Cancer | Positive |
| Breast Cancer | Positive |
| Normal Ovary | Positive |
| Normal Breast | Positive |
| Blood Vessel | Positive |
| Kidney | Negative |
| Lung | Negative |
| Colon | Negative |
| Liver | Negative |
| Heart | Negative |

**Table 4**

| Immunohistochemistry analysis of O8E using monoclonal antibodies | | | | | | |
|---|---|---|---|---|---|---|
| Normal Tissue | 11A6 | | 18A8 | | 14F1 | |
| | Endothelial | Epithelial | Endothelial | Epithelial | Endothelial | Epithelial |
| Skin | 2 | 2 | 0 | 0 | 1 | 1 |
| Skin | 1 | 1 | 0 | 0 | 1 | 1 |
| Breast | 0 | 1 | n/a | n/a | 1 | 1 |
| Colon | 0 | 0 | 0 | 0 | 0 | 0 |
| Jejunum | 0 | 0 | 0 | 0 | 0 | 0 |
| Colon | 0 | 0 | 0 | 0 | 0 | 0 |
| Colon | 0 | 0 | 0 | 0 | 0 | 0 |
| Ovary | 0 | 0 | 0 | 0 | 1 | 0 |
| Colon | 0 | 0 | 0 | 0 | 0 | 1 |
| Liver | 0 | 0 | 0 | 0 | 1 | 2 |
| Skin | 0 | 0 | 0 | 0 | 1 | 0 |
| Duodenum and Pancreas | 0 | 0 | 0 | 0 | 0 | 0 |
| Appendix | 0 | 0 | 0 | 0 | 0 | 0 |
| Ileum | 0 | 0 | 0 | 0 | 0 | 0 |

| | | | | | | |
|---|---|---|---|---|---|---|
| 0=no staining, 1=light staining, 2=moderate staining, n/a=not available | | | | | | |

## Claims

1. An isolated antibody epitope of the ovarian carcinoma antigen O8E, said epitope consisting of the amino acid sequence set forth in SEQ ID NO: 398 or a variant thereof which has at least 90% identity along its whole length to the amino acid sequence set forth in SEQ ID NO: 398.

2. An isolated antibody or antigen binding fragment thereof that specifically binds the antibody epitope of Claim 1.

3. The isolated antibody or antigen binding fragment of Claim 2, wherein the antibody is a monoclonal antibody.

4. The isolated antibody or antigen binding fragment of Claim 2, wherein the antibody is effective for promoting internalization of the O8E protein.

5. The epitope of claim 1 for use in therapy.

6. The antibody of claims 2, 3 or 4 for use in therapy.

7. The use of the epitope of claim 1 for the manufacture of a medicament to be used to prevent the development of ovarian cancer or for the treatment of ovarian cancer.

8. The use of the antibody of any one of claims 2, 3 or 4 for the manufacture of a medicament to prevent the development of ovarian cancer or for the treatment of ovarian cancer.

9. A polynucleotide, which encodes the epitope of claim 1.

10. The polynucleotide of claim 9 for use in therapy.

11. The use of the polynucleotide of claim 9 for the manufacture of medicament to prevent the development of ovarian cancer or for the treatment of ovarian cancer.

12. A pharmaceutical composition comprising,
a) a epitope of claim 1, or
b) an antibody of claims 2, 3 or 4; or
c) a polynucleotide of claim 9; and
a physiologically acceptable carrier.

13. A vaccine comprising,
a) a epitope of claim 1, or
b) an antibody of claims 2, 3 or 4; or
c) a polynucleotide of claim 9; and
a non-specific immune response enhancer.

## Patentansprüche

1. Ein isoliertes Antikörper-Epitop des Eierstockkarzinom-Antigens O8E wobei das Epitop aus der in SEQ ID NO: dargelegten Aminosäuresequenz besteht, oder eine Variante davon, die eine Identität von mindestens 90 % entlang ihrer gesamten Länge mit der in SEQ ID NO: 398 dargelegten Aminosäuresequenz aufweist.

2. Ein isolierter Antikörper oder ein Antigen-bindendes Fragment davon, das spezifisch das Antikörper-Epitop nach Anspruch 1 bindet.

3. Der isolierte Antikörper oder das Antigen-bindende Fragment nach Anspruch 2, wobei der Antikörper ein monoklonaler Antikörper ist.

4. Der isolierte Antikörper oder das Antigen-bindende . Fragment nach Anspruch 2, wobei der Antikörper zur Beschleunigung der Internalisierung des 08E-Proteins wirksam ist.

5. Das Epitop nach Anspruch 1 zur Verwendung bei der Therapie.

6. Der Antikörper nach Anspruch 2, 3 oder 4 zur Verwendung bei der Therapie.

7. Die Verwendung des Epitops nach Anspruch 1 zur Herstellung eines Medikaments, das zur Verhinderung der Entwicklung von Eierstockkrebs oder zur Behandlung von Eierstockkrebs zu verwenden ist.

8. Die Verwendung des Antikörpers nach einem der Ansprüche 2, 3 oder 4 zur Herstellung eines Medikaments zur Verhinderung der Entwicklung von Eierstockkrebs oder zur Behandlung von Eierstockkrebs.

9. Ein Polynukleotid, das das Epitop nach Anspruch 1 kodiert.

10. Das Polynukleotid nach Anspruch 9 zur Verwendung bei der Therapie.

11. Die Verwendung des Polynukleotids Anspruch 9 zur Herstellung eines Medikaments zur Verhinderung der Entwicklung von Eierstockkrebs oder zur Behandlung von Eierstockkrebs.

12. Pharmazeutische Zusammensetzung, die folgendes umfasst:
a) ein Epitop nach Anspruch 1, oder
b) einen Antikörper nach den Ansprüchen 2, 3 oder 4; oder
c) ein Polynukleotid nach Anspruch 9; und
einen physiologisch verträglichen Träger.

13. Ein Impfstoff, der folgendes umfasst:
a) ein Epitop nach Anspruch 1, oder
b) einen Antikörper nach den Ansprüchen 2, 3 oder 4; oder
c) ein Polynukleotid nach Anspruch 9; und
einen nicht spezifischen Immunantwört-Verstärker.

## Revendications

1. Epitope pour anticorps isolé de l'antigène du carcinome ovarien O8E ledit épitope constitué de la séquence d'acides aminés représentée par SEQ ID NO : 398 ou d'un variant de celle-ci qui présente au moins 90 % d'identité sur toute sa longueur avec la séquence d'acides aminés représentée par SEQ ID NO : 398.

2. Anticorps isolé ou fragment de celui-ci se liant à l'antigène qui se lie spécifiquement à l'épitope pour anticorps selon la revendication 1.

3. Anticorps isolé ou fragment se liant à l'antigène selon la revendication 2, où l'anticorps est un anticorps monoclonal.

4. Anticorps isolé ou fragment se liant à l'antigène selon la revendication 2, où l'anticorps est efficace pour favoriser l'intemalisation de la protéine O8E

5. Epitope selon la revendication 1 pour une utilisation en thérapie.

6. Anticorps selon les revendications 2, 3 ou 4 pour une utilisation en thérapie.

7. Utilisation de l'épitope selon la revendication 1 pour la fabrication d'un médicament à utiliser pour empêcher le développement d'un cancer ovarien ou pour le traitement d'un cancer ovarien.

8. Utilisation de l'anticorps selon l'une quelconque des revendications 2, 3 ou 4 pour la fabrication d'un médicament pour empêcher le développement d'un cancer ovarien ou pour le traitement d'un cancer ovarien.

9. Polynucléotide, qui code pour l'épitope selon la revendication 1.

10. Polynucléotide selon la revendication 9 pour une utilisation en thérapie.

11. Utilisation du polynucléotide selon la revendication 9 pour la fabrication d'un médicament pour empêcher le développement d'un cancer ovarien ou pour le traitement d'un cancer ovarien.

12. Composition pharmaceutique comprenant,
a) un épitope selon la revendication 1, ou
b) un anticorps selon les revendications 2, 3 ou 4 ; ou
c) un polynucléotide selon la revendication 9 ; et
un support physiologiquement acceptable.

13. Vaccin comprenant,
a) un épitope selon la revendication 1, ou
b) un anticorps selon les revendications 2, 3 ou 4 ; ou
c) un polynucléotide selon la revendication 9 ; et
un amplificateur de la réponse immunitaire non spécifique.
